# EUROPEAN PATENT SPECIFICATION

(11) **EP 4 247 809 B1**
(45) Date of publication and mention of the grant of the patent: **21.10.2026**
(21) Application number: 21815453.2
(22) Date of filing: 17.11.2021
(51) Int. Cl.: C07D 405/12, C07D 495/04, C07D 487/04, C07D 513/04, C07D 471/04, C07D 491/04, A61K 31/538, A61P 33/00, A61P 33/10

(54) **N-(2,3-DIHYDRO-1,4-BENZOXAZIN-4-YL)-3-ISOPROPYL-7-(2,3,5-TRIFLUOROPHENYL)BENZO-THIOPHENE-2-CARBOXAMIDE DERIVATIVES AND SIMILAR COMPOUNDS FOR THE TREATMENT OF HEARTWORM INFECTIONS**
N-(2,3-DIHYDRO-1,4-BENZOXAZIN-4-YL)-3-ISOPROPYL-7-(2,3,5-TRIFLUOROPHENYL)BENZO-THIOPHEN-2-CARBOXAMID-DERIVATE UND ÄHNLICHE VERBINDUNGEN ZUR BEHANDLUNG VON HERZWURMINFEKTIONEN
DÉRIVÉS DE N-(2,3-DIHYDRO-1,4-BENZOXAZIN-4-YL)-3-ISOPROPYL-7-(2,3,5-TRIFLUOROPHÉNYL)BENZO-THIOPHÉNE-2-CARBOXAMIDE ET COMPOSÉS SIMILAIRES POUR LE TRAITEMENT D'INFECTIONS DE DIROFILARIA IMMITIS

(30) Priority: 18.11.2020 US 202063115478 P; 04.12.2020 US 202063121501 P; 09.12.2020 US 202063123268 P
(43) Date of publication of application: 27.09.2023
(73) Proprietor: Elanco Tiergesundheit AG, 4058 Basel (CH)
(72) Inventor: GRIEBENOW, Nils, 41541 Dormagen (DE); TAHTAOUI, Chouaib, 68170 Rixheim (FR); DUCRAY, Pierre, 68128 Village-Neuf (FR); RAGEOT, Denise, 68300 Saint-Louis (FR)
(74) Representative: Cohausz & Florack
(86) International application number: PCT/EP2021/081991
(87) International publication number: WO 2022/106469

(56) References cited:
- WO-A1-2020/191091
- WO-A1-2020/247747
- WO-A1-2021/242581

## Description

### FIELD

The present invention relates to medicinal chemistry, pharmacology, and veterinary and human medicine. More particularly, the present invention relates to compounds of formula (I) and their use in the control of endoparasites, for example heartworms, in warm-blooded animals.

### BACKGROUND

Heartworm (*Dirofilaria immitis*) is a parasitic roundworm that is spread from host to host through the bites of mosquitoes. The lifecycle starts when a female mosquito takes a blood meal from an infected host. The mosquito ingests immature heartworms which then molt to the infective larvae stage and travel to the mosquitoes' mouth parts. The mosquito then feeds on a susceptible host, such as a dog or cat, depositing the infective larvae. The larvae then molt to the next larval stage in the new host and then migrate through the
body, eventually ending up in the blood vessels. As the larvae migrate through the tissues they molt into juvenile adults. The juvenile adults eventually move into the blood vessels of the lungs where they mature into sexually active adults. The adult heartworms then breed and release immature heartworms completing the cycle. Heartworm infection may result in serious disease for the host.

Adult heartworm infections may be treated with arsenic-based compounds; the treatment is time consuming, cumbersome, and often only partly successful. Accordingly, treatment is focused on the control of heartworm infection. Heartworm control is currently performed exclusively by year round periodical administration of drugs. Typical treatments include macrocyclic lactones such as ivermectin, moxidectin, and milbemycin oxime. Unfortunately, developing resistance of *Dirofilaria immitis* to macrocyclic lactones has been observed. Accordingly, there is a need for new compounds which effectively control heartworm infections either by way of prophylaxis or by directly killing heartworms. Certain treatments of endoparasites are described in WO/2017/178416, WO/2018/087036, WO/2018/197401, WO/2019/025341, WO/2019/002132, WO/2020/014068, WO/2020/131629, WO/2020/131631, WO/2020/191091, WO 2020/247747, and WO 2021/242581.

### SUMMARY

The present invention provides compounds of formula (I) which effectively treat and/or control endoparasites (e.g., heartworm) in warm-blooded animals.

In one embodiment, the present invention provides compounds of formula (I) 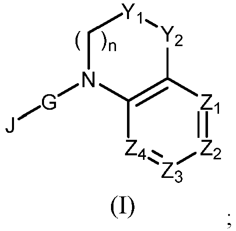 wherein
n is 0 or 1;
J is selected from the group consisting of 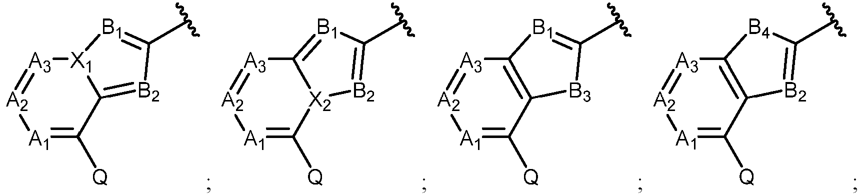 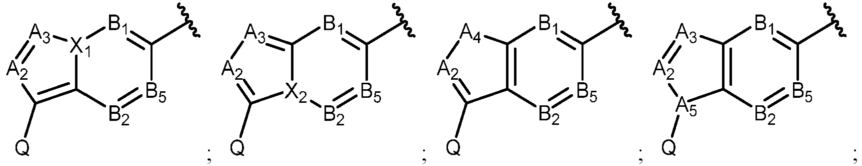 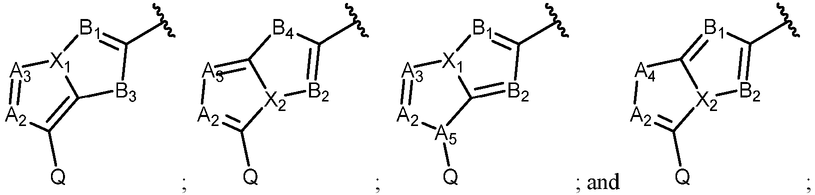
A₁ is selected from the group consisting of N and CR_{A1};
A₂ is selected from the group consisting of N and CR_{A2};
A₃ is selected from the group consisting of N and CR_{A3};
A₄ is selected from the group consisting of O, S, and NR_{A4};
A₅ is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 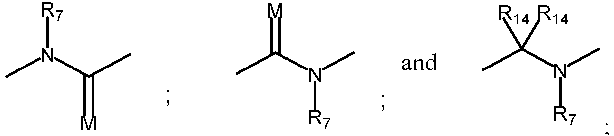
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
   wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
   wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R_{A1}, R_{A2}, R_{A3}, R_{B2}, and R_{B5} are each independently selected from the group consisting of hydrogen, halogen, hydroxyl, -SH, -SC₁-C₄ alkyl, -S(O)(C₁-C₄ alkyl), -S(O)₂(C₁-C₄ alkyl), cyano, C₁-C₄ alkyl, C₁-C₄ halogenoalkyl, C₁-C₄-alkoxy, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; -NH₂, -NH(C₁-C₄ alkyl), and -N(C₁-C₄ alkyl)₂;
R_{A4} and R_{B3} are independently selected from the group consisting of hydrogen, C₁-C₄ alkyl, and C₁-C₄ halogenoalkyl;
R_{B1} is independently selected from the group consisting of halogen, cyano, -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -N(C₁-C₄ alkyl)(4- to 7-membered heterocycloalkyl), -NH(4- to 7-membered heterocycloalkyl), -N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, - S(O)C₁-C₄ alkyl, -SO₂C₁-C₄ alkyl, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl having at least one nitrogen atom via which the 5-membered heteroaryl ring is connected to the rest of the molecule, and 6-membered heteroaryl having at least one nitrogen atom; each of the aryl, heterocycloalkyl, and heteroaryl rings in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B1} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, - NH(C₃-C₆ cycloalkyl), -N(C₁-C₄ alkyl)(C₃-C₆-cycloalkyl), -NHSO₂(C₁-C₄ alkyl), -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B1} may be optionally substituted with 1, 2 or 3 substituents independently selected from the group consisting of halogen, hydroxy, oxo, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B4} is selected from the group consisting of -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -N(C₁-C₄ alkyl)(4- to 7-membered heterocycloalkyl), -NH(4- to 7-membered heterocycloalkyl), -N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, -S(O)C₁-C₄ alkyl, -SO₂C₁-C₄ alkyl, - B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl, and 6-membered heteroaryl; each of the aryl, heterocycloalkyl, and heteroaryl rings in R_{B4} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B4} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B4} may be optionally substituted with 1, 2 or 3 substituents independently selected from the group consisting of halogen, hydroxy, -NH₂, oxo, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is selected from the group consisting of hydrogen, C₁-C₄ alkyl, and C₃-C₆ cycloalkyl optionally substituted with 1 to 5 halogen atoms, -C(H)O, C₂-C₄ alkenyl, C₂-C₄ alkynyl, C₁-C₄ halogenoalkyl, and C₁-C₄-alkoxy;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
R₁₀ is selected from the group consisting of hydrogen and C₁-C₄ alkyl;
R₁₁ is, each time selected, independently selected from the group consisting of hydrogen, halogen, hydroxyl, cyano, C₁-C₄ alkyl, C₁-C₄ halogenoalkyl, C₁-C₄-alkoxy, C₃-C₆ cycloalkyl, - NH₂, -NH(C₁-C₄ alkyl), and -N(C₁-C₄ alkyl)₂; and
Q is selected from the group consisting of
   (i) 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl;
   (ii) 5- to 10-membered heteroaryl having 1, 2, or 3 heteroatoms independently selected from the group O, S, and N and wherein the carbons of the 5- to 10-membered heteroaryl are optionally substituted with 1, 2, 3, 4, or 5 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, benzyloxy, -C(O)R₁₇, -NH₂, -NH(C₁-C₄ alkyl), -N(C₁-C₄ alkyl)₂, -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl, and any N in the heteroaryl, valency permitting, is optionally substituted with a substituent selected from the group consisting of hydrogen, C₁-C₄ alkyl, and C₃-C₆ cycloalkyl;
   (iii) 4- to 7-membered heterocycloalkyl having 1, 2, or 3 heteroatoms independently selected from the group O, S, N, wherein the heterocycloalkyl is optionally benzo-fused, wherein the carbons of the 4- to 7-membered heterocycloalkyl or optionally benzo-fused 4- to 7-membered heterocycloalkyl are optionally substituted with 1, 2, 3, or 4 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), and -N(C₁-C₄ alkyl)₂ and any N in the heterocycloalkyl is optionally substituted with a substituent selected from the group consisting of hydrogen, C₁-C₄ alkyl, and C₃-C₆ cycloalkyl;
   (iv) 6- or 10 membered aryloxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
   (v) 6- or 10 membered arylthio-oxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and
   (vi) 5- to 10-membered heteroaryloxy optionally substituted with 1, 2 or 3 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
R₁₃ is selected from the group consisting of hydroxy, C₁-C₄ alkoxy, and -NH₂;
R₁₄ is, each time selected, independently selected from the group consisting of hydrogen, halogen, cyano, nitro, hydroxyl, C₁-C₄ alkyl, C₃-C₆ cycloalkyl, C₁-C₄ halogenoalkyl, C₁-C₄ alkoxy, C₁-C₄ halogenalkoxy, -NH₂, -NH(C₁-C₄ alkyl), and -N(C₁-C₄ alkyl)₂; and
R₁₇ is, each time selected, independently selected from the group consisting of C₁-C₄ alkyl, C₃-C₆ cycloalkyl, C₁-C₄ halogenoalkyl, C₁-C₄ alkoxy, C₁-C₄ halogenalkoxy, -OH, -NH₂, - NH(C₁-C₄ alkyl), -N(C₁-C₄ alkyl)₂, -NH(C₃-C₆ cycloalkyl), and -N(C₁-C₄ alkyl)(C₃-C₆-cycloalkyl);
wherein, when J is 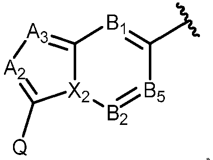 A₂ is N, and A₃, B₁, B₂, B₃, X₂, and Q are as defined above;
or a salt thereof.

The present invention also provides pharmaceutical compositions, comprising: a compound of formula (I) or a salt thereof and at least one an acceptable carrier, the composition optionally further comprising at least one additional active compound.

In one embodiment, the present invention also provides a method for treating parasites, comprising: administering to a subject in need thereof an effective amount of a compound of formula (I) or a salt thereof, the method optionally further comprising an effective amount of at least one additional active compound.

In one embodiment, the present invention also provides a method for controlling parasites, comprising: administering to a subject in need thereof an effective amount of a compound of formula (I) or a salt thereof, the method optionally further comprising an effective amount of at least one additional active compound.

In one embodiment, the present invention also provides a method for treating or controlling parasites, comprising: contacting a subject's environment with an effective amount of a compound of formula (I) or a salt thereof, the method optionally further comprising an effective amount of at least one additional active compound.

Thus, the invention provides for a use of the compounds of the invention as a medicament, including for the manufacture of a medicament. In one embodiment, the invention provides a manufacture of a medicament comprising a compound of formula (I) or a salt thereof for treating parasites. In one embodiment, the invention provides a manufacture of a medicament comprising a compound of formula (I) or a salt thereof for controlling parasites.

The present invention also provides processes from making compounds of the invention and intermediates thereof.

### DETAILED DESCRIPTION

In accordance with a first aspect, the present invention covers compounds of formula (I) 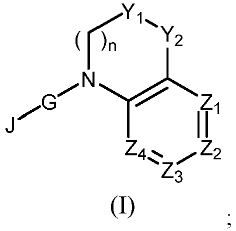 wherein
n is 0 or 1;
J is selected from the group consisting of 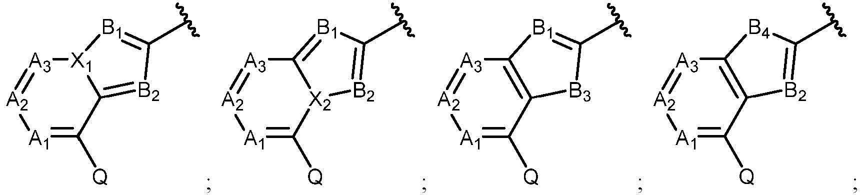 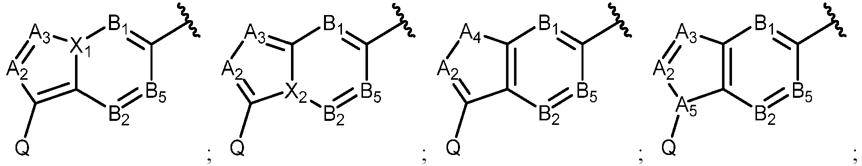 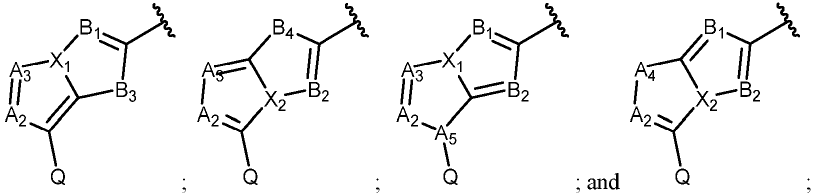
A₁ is selected from the group consisting of N and CR_{A1};
A₂ is selected from the group consisting of N and CR_{A2};
A₃ is selected from the group consisting of N and CR_{A3};
A₄ is selected from the group consisting of O, S, and NR_{A4};
As is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 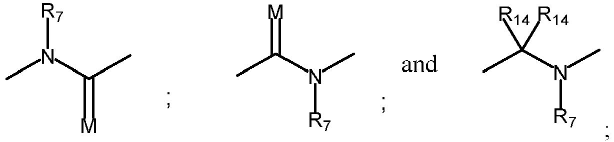
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
   wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
   wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R_{A1}, R_{A2}, R_{A3}, R_{B2}, and R_{B5} are each independently selected from the group consisting of hydrogen, halogen, hydroxyl, -SH, -SC₁-C₄ alkyl, -S(O)(C₁-C₄ alkyl), -S(O)₂(C₁-C₄ alkyl), cyano, C₁-C₄ alkyl, C₁-C₄ halogenoalkyl, C₁-C₄-alkoxy, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; -NH₂, -NH(C₁-C₄ alkyl), and -N(C₁-C₄ alkyl)₂;
R_{A4} and R_{B3} are independently selected from the group consisting of hydrogen, C₁-C₄ alkyl, and C₁-C₄ halogenoalkyl;
R_{B1} is selected from the group consisting of halogen, cyano, -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), - N(C₁-C₄ alkyl)(4- to 7-membered heterocycloalkyl), -NH(4- to 7-membered heterocycloalkyl), - N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, -S(O)C₁-C₄ alkyl, -SO₂C₁-C₄ alkyl, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl having at least one nitrogen atom via which the 5-membered heteroaryl ring is connected to the rest of the molecule, and 6-membered heteroaryl having at least one nitrogen atom; each of the aryl, heterocycloalkyl, and heteroaryl rings in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, - NH(C₃-C₆ cycloalkyl), -N(C₁-C₄ alkyl)(C₃-C₆-cycloalkyl), -NHSO₂(C₁-C₄ alkyl), -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B1} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, - NH(C₃-C₆ cycloalkyl), -N(C₁-C₄ alkyl)(C₃-C₆-cycloalkyl), -NHSO₂(C₁-C₄ alkyl), -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B1} may be optionally substituted with 1, 2 or 3 substituents independently selected from the group consisting of halogen, hydroxy, oxo, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B4} is selected from the group consisting of -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -N(C₁-C₄ alkyl)(4- to 7-membered heterocycloalkyl), -NH(4- to 7-membered heterocycloalkyl), -N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, -S(O)C₁-C₄ alkyl, -SO₂C₁-C₄ alkyl, - B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl, and 6-membered heteroaryl; each of the aryl, heterocycloalkyl, and heteroaryl rings in R_{B4} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B4} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B4} may be optionally substituted with 1, 2 or 3 substituents independently selected from the group consisting of halogen, hydroxy, oxo, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is selected from the group consisting of hydrogen, C₁-C₄ alkyl, and C₃-C₆ cycloalkyl optionally substituted with 1 to 5 halogen atoms, -C(H)O, C₂-C₄ alkenyl, C₂-C₄ alkynyl, C₁-C₄ halogenoalkyl, and C₁-C₄-alkoxy;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
R₁₀ is selected from the group consisting of hydrogen and C₁-C₄ alkyl;
R₁₁ is, each time selected, independently selected from the group consisting of hydrogen, halogen, hydroxyl, cyano, C₁-C₄ alkyl, C₁-C₄ halogenoalkyl, C₁-C₄-alkoxy, C₃-C₆ cycloalkyl, - NH₂, -NH(C₁-C₄ alkyl), and -N(C₁-C₄ alkyl)₂; and
Q is selected from the group consisting of
   (i) 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl;
   (ii) 5- to 10-membered heteroaryl having 1, 2, or 3 heteroatoms independently selected from the group O, S, and N and wherein the carbons of the 5- to 10-membered heteroaryl are optionally substituted with 1, 2, 3, 4, or 5 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, benzyloxy, -C(O)R₁₇, -NH₂, -NH(C₁-C₄ alkyl), -N(C₁-C₄ alkyl)₂, -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl, and any N in the heteroaryl, valency permitting, is optionally substituted with a substituent selected from the group consisting of hydrogen, C₁-C₄ alkyl, and C₃-C₆ cycloalkyl;
   (iii) 4- to 7-membered heterocycloalkyl having 1, 2, or 3 heteroatoms independently selected from the group O, S, N, wherein the heterocycloalkyl is optionally benzo-fused, wherein the carbons of the 4- to 7-membered heterocycloalkyl or optionally benzo-fused 4- to 7-membered heterocycloalkyl are optionally substituted with 1, 2, 3, or 4 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), and -N(C₁-C₄ alkyl)₂ and any N in the heterocycloalkyl is optionally substituted with a substituent selected from the group consisting of hydrogen, C₁-C₄ alkyl, and C₃-C₆ cycloalkyl;
   (iv) 6- or 10 membered aryloxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
   (v) 6- or 10 membered arylthio-oxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and
   (vi) 5- to 10-membered heteroaryloxy optionally substituted with 1, 2 or 3 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
R₁₃ is selected from the group consisting of hydroxy, C₁-C₄ alkoxy, and -NH₂;
R₁₄ is, each time selected, independently selected from the group consisting of hydrogen, halogen, cyano, nitro, hydroxyl, C₁-C₄ alkyl, C₃-C₆ cycloalkyl, C₁-C₄ halogenoalkyl, C₁-C₄ alkoxy, C₁-C₄ halogenalkoxy, -NH₂, -NH(C₁-C₄ alkyl), and -N(C₁-C₄ alkyl)₂; and
R₁₇ is, each time selected, independently selected from the group consisting of C₁-C₄ alkyl, C₃-C₆ cycloalkyl, C₁-C₄ halogenoalkyl, C₁-C₄ alkoxy, C₁-C₄ halogenalkoxy, -OH, -NH₂, - NH(C₁-C₄ alkyl), -N(C₁-C₄ alkyl)₂, -NH(C₃-C₆ cycloalkyl), and -N(C₁-C₄ alkyl)(C₃-C₆-cycloalkyl);
wherein, when J is 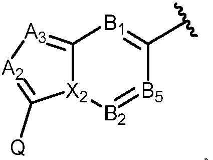 A₂ is N, and A₃, B₁, B₂, B₃, X₂, and Q are as defined above;
or a salt thereof.

A preferred embodiment provides compounds of formula (I), wherein
n is 0 or 1,
J is selected from the group consisting of 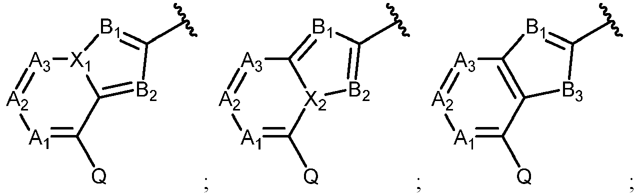 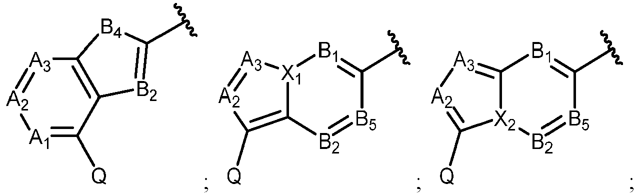 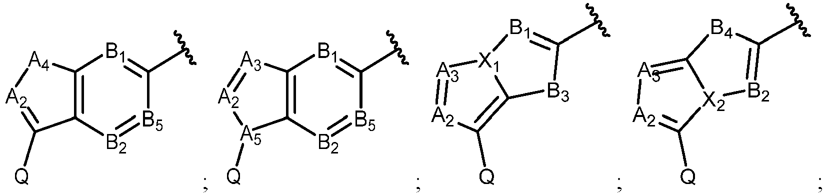 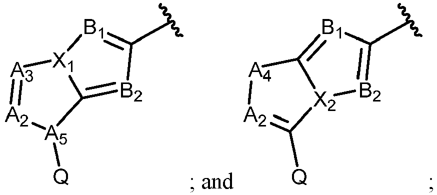
A₁ is selected from the group consisting of N and CR_{A1};
A₂ is selected from the group consisting of N and CR_{A2};
A₃ is selected from the group consisting of N and CR_{A3};
A₄ is selected from the group consisting of O, S, and NR_{A4};
A₅ is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 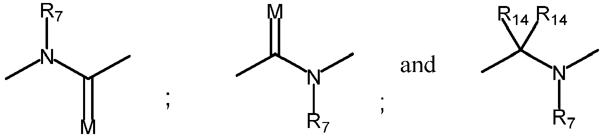
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
   wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
   wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
   R_{A1}, R_{A2}, R_{A3}, R_{B2}, and R_{B5} are each independently selected from the group consisting of hydrogen, halogen, hydroxyl, -SH, NH₂, C₁-C₄ alkyl, C₁-C₄ halogenoalkyl, C₁-C₄-alkoxy, and -SC₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   R_{A4} and R_{B3} are independently selected from the group consisting of hydrogen, halogen, hydroxyl, -SH, NH₂, C₁-C₄alkyl, C₁-C₄ halogenoalkyl, C₁-C₄-alkoxy, and -SC₁-C₄ alkyl, wherein each C₁-C₄ alkyl in R_{A4} and R_{B3} may be optionally substituted with 1, 2 or 3 substituents independently selected from the group consisting of halogen, hydroxy, - NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   R_{B1} is selected from the group consisting of halogen, hydroxyl, -SH, NH₂, C₁-C₄ alkyl, C₃-C₆ cycloalkyl, C₁-C₄ halogenoalkyl, C₁-C₄-alkoxy, -SC₁-C₄ alkyl, and -NH(C₁-C₄ alkyl), -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
   R_{B4} is selected from the group consisting of halogen, hydroxyl, -SH, NH₂, C₁-C₄ alkyl, C₃-C₆ cycloalkyl, C₁-C₄ halogenoalkyl, C₁-C₄-alkoxy, and -SC₁-C₄ alkyl,, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   R₇ is selected from the group consisting of hydrogen, C₁-C₄ alkyl, and C₃-C₆ cycloalkyl optionally substituted with 1 to 5 halogen atoms;
   R₈ is, each time selected, independently selected from the group consisting of hydrogen, fluoro, and C₁-C₄ alkyl;
   R₉ is, each time selected, independently selected from the group consisting of hydrogen, fluoro, and C₁-C₄ alkyl;
   R₁₀ is selected from the group consisting of hydrogen and C₁-C₄ alkyl;
   R₁₁ is selected from the group consisting of hydrogen, halogen, C₁-C₄ alkyl, C₁-C₄ halogenoalkyl, C₃-C₆ cycloalkyl;
   Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
   R₁₃ is selected from the group consisting of hydroxy, C₁-C₄ alkoxy, and -NH₂;
   R₁₄ is, each time selected, independently selected from the group consisting of hydrogen, and halogen; and
   R₁₇ is, each time selected, independently selected from the group consisting of C₁-C₄ alkyl, C₃-C₆ cycloalkyl, C₁-C₄ halogenoalkyl, C₁-C₄ alkoxy, C₁-C₄
halogenalkoxy, -OH, -NH₂, -NH(C₁-C₄ alkyl), -N(C₁-C₄ alkyl)₂, -NH(C₃-C₆ cycloalkyl), and -N(C₁-C₄ alkyl)(C₃-C₆-cycloalkyl);
wherein, when J is 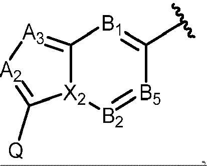 A₂ is N, and A₃, B₁, B₂, B₅, X₂, and Q are as defined above;
or a salt thereof.

A preferred embodiment provides compounds of formula (I), wherein
n is 0 or 1,
J is selected from the group consisting of 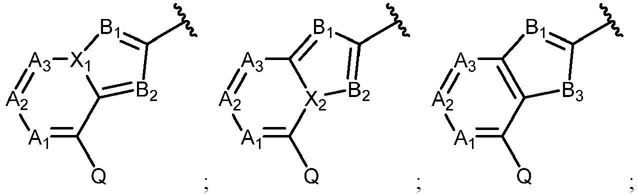 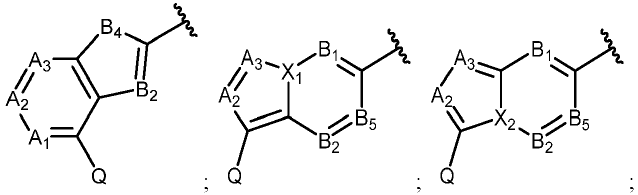 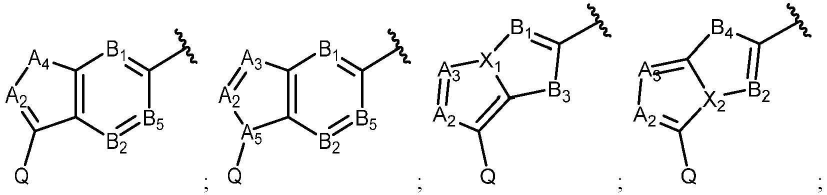 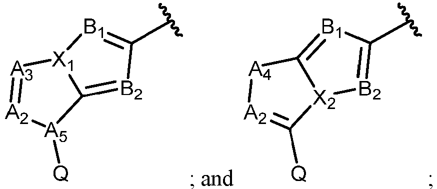
A₁ is selected from the group consisting of N and CR_{A1};
A₂ is selected from the group consisting of N and CR_{A2};
A₃ is selected from the group consisting of N and CR_{A3};
A₄ is selected from the group consisting of O, S, and NR_{A4};
A₅ is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 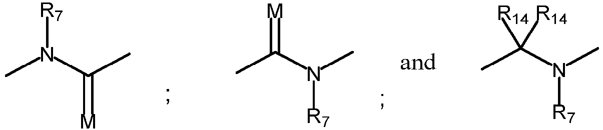
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
   wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
   wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
   R_{A1}, R_{A2}, R_{A3}, R_{B2}, and R_{B5} are each independently selected from the group consisting of hydrogen, halogen, and C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
   R_{A4} and R_{B3} are independently selected from the group consisting of hydrogen, C₁-C₄ alkyl, wherein each C₁-C₄ alkyl in R_{A4} and R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   R_{B1} is selected from the group consisting of C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
   R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   R₇ is hydrogen;
   R₈ is hydrogen;
   R₉ is hydrogen;
   R₁₀ is selected from the group consisting of hydrogen and C₁-C₄ alkyl;
   R₁₁ is hydrogen;
   Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
   R₁₃ is selected from the group consisting of hydroxy, C₁-C₄ alkoxy, and -NH₂;
   wherein, when J is 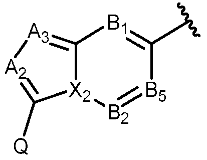 A₂ is N, and A₃, B₁, B₂, B₃, X₂, and Q are as defined above;
or a salt thereof.

A preferred embodiment provides compounds of formula (I), wherein
n is 1,
J is selected from the group consisting of 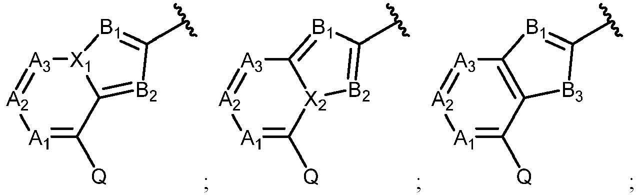 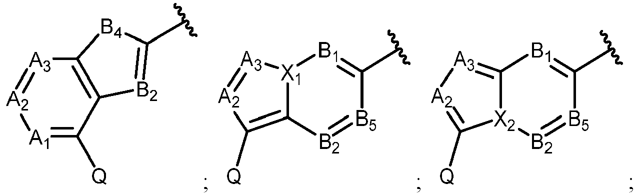 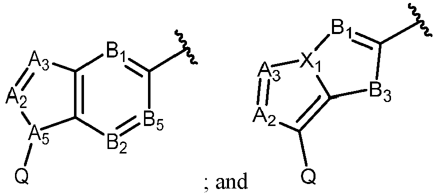
A₁ is selected from the group consisting of N and CR_{A1};
A₂ is selected from the group consisting of N and CR_{A2};
A₃ is selected from the group consisting of N and CR_{A3};
A₅ is selected from the group consisting of N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is selected from the group consisting of N;
X₂ is selected from the group consisting of N;
G is selected from the group consisting of 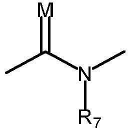
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B3} is selected from the group consisting of hydrogen, and C₁-C₄alkyl, wherein each C₁-C₄ alkyl in R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
R_{B1} is selected from the group consisting of
C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is hydrogen;
R₈ is hydrogen;
R₉ is hydrogen;
R₁₁ is hydrogen;
Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
wherein, when J is 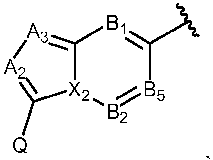 A₂ is N, and A₃, B₁, B₂, B₃, X₂, and Q are as defined above;
or a salt thereof.

A preferred embodiment provides compounds of formula (I), wherein
n is 1,
J is selected from the group consisting of 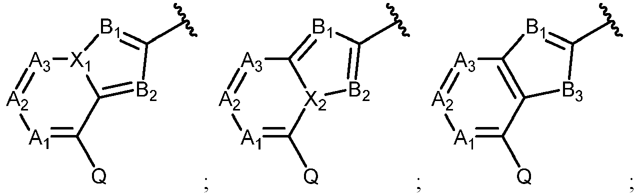 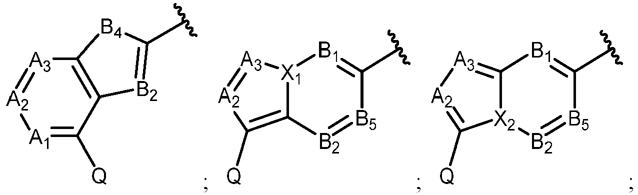 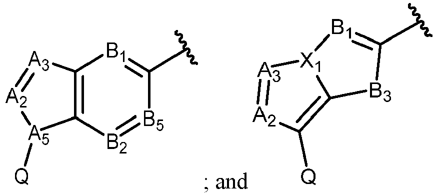
A₁ is selected from the group consisting of N and CR_{A1};
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is N;
X₂ is N;
G is 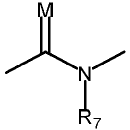
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, and C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B3} is selected from the group consisting of hydrogen, and C₁-C₄alkyl, wherein each C₁-C₄ alkyl in R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
R_{B1} is selected from the group consisting of
C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
   and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is hydrogen;
R₈ is hydrogen;
R₉ is hydrogen;
R₁₁ is hydrogen;
Q is phenyl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
wherein, when J is 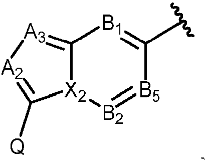 A₂ is N, and A₃, B₁, B₂, B₃, X₂, and Q are as defined above;
or a salt thereof.

A preferred embodiment provides compounds of formula (I), wherein
n is 1,
J is selected from the group consisting of 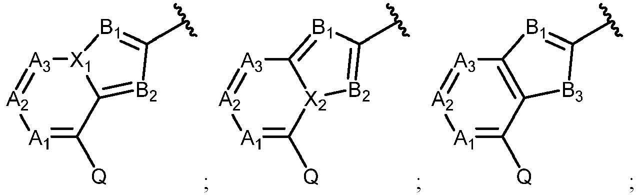 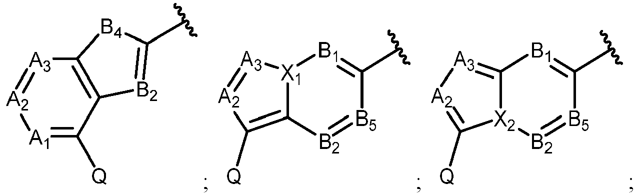 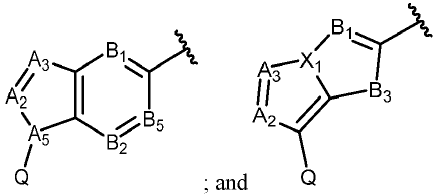
A₁ is selected from the group consisting of N and CR_{A1};
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is N;
X₂ is N;
G is 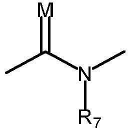
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, C₁-C₄ alkyl;
R_{B3} is selected from the group consisting of hydrogen, and C₁-C₄ alkyl;
R_{B1} is selected from the group consisting of
C₁-C₄ alkyl, -N(C₁-C₄ alkyl)₂, wherein each C₁-C₄-alkyl in R_{B1} may be optionally substituted with 1, 2 or 3 substituents of hydroxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents of halogen;
R_{B4} is C₁-C₄ alkyl;
R₇ is hydrogen;
R₈ is hydrogen;
R₉ is hydrogen;
R₁₁ is hydrogen;
Q is phenyl having 1 to 5 halogen atoms;
wherein, when J is 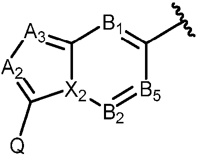 A₂ is N, and A₃, B₁, B₂, B₃, X₂, and Q are as defined above;
or a salt thereof.

A preferred embodiment provides compounds of formula (I), wherein
n is 1,
J is selected from the group consisting of 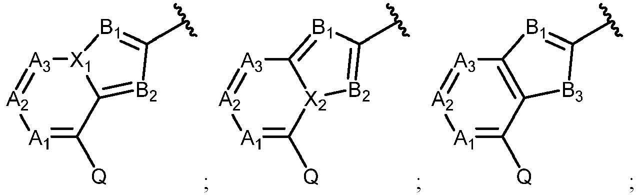 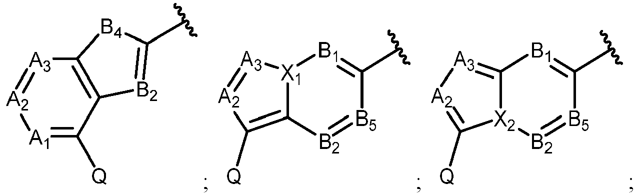 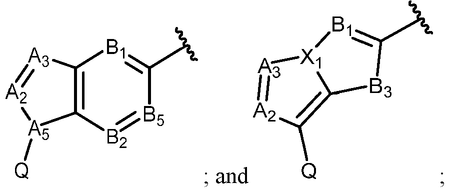
A₁ is selected from the group consisting of N and CR_{A1};
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is selected from the group consisting of N;
X₂ is selected from the group consisting of N;
G is selected from the group consisting of 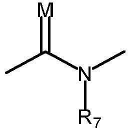
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, fluoro, and methyl;
R_{B3} are independently selected from the group consisting of hydrogen, and methyl;
R_{B1} is independently selected from the group consisting of 4-morpholino, isopropyl, 2-hydroxyisopropyl, 3-fluoroazetidinyl, and NMe₂;
R_{B4} is isopropyl;
R₇ is hydrogen;
R₈ is hydrogen;
R₉ is hydrogen;
R₁₁ is hydrogen;
Q is selected from the group consisting of 2,3,5-trifluorophenyl and 2,6-difluorophenyl;
wherein, when J is 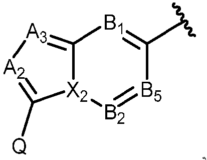 A₂ is N, and A₃, B₁, B₂, B₃, X₂, and Q are as defined above;
or a salt thereof.

A preferred embodiment provides compounds of formula (I), wherein
J is selected from the group consisting of 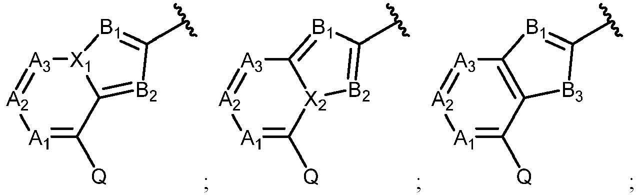 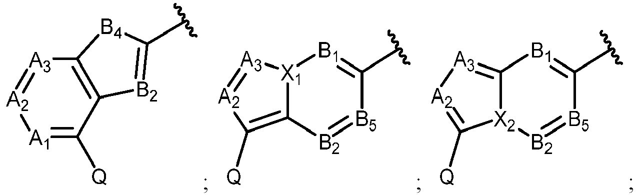 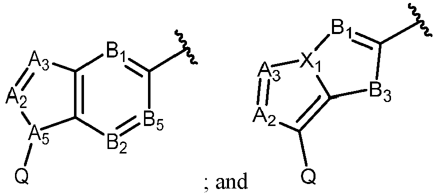
wherein, when J is 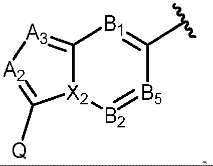 A₂ is N, and A₃, B₁, B₂, B₃, X₂, and Q are as defined herein;
or a salt thereof.

A preferred embodiment provides compounds of formula (I) having formula (Ia-1), or a salt thereof,

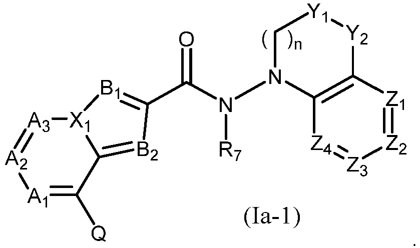

A preferred embodiment provides compounds of formula (I) having formula (Ia-1) or a salt thereof, wherein A₁ is CR_{A1}; A₂ is CR_{A2}; A₃ is CR_{A3}; B₁ is CR_{B1}; B₂ is N; and X₁ is N.

A preferred embodiment provides compounds of formula (I) having formula (Ia-1) or a salt thereof, wherein A₁ is N; A₂ is CR_{A2}; A₃ is CR_{A3}; B₁ is CR_{B1}; B₂ is N; and X₁ is N.

A preferred embodiment provides compounds of formula (I) having formula (Ia-2), or a salt thereof,

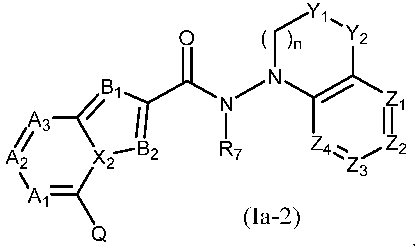

A preferred embodiment provides compounds of formula (I) having formula (Ia-2), or a salt thereof, wherein
A₁ is CR_{A1}; A₂ is CR_{A2}; A₃ is N; B₁ is CR_{B1}; B₂ is N; and X₂ is N.

A preferred embodiment provides compounds of formula (I) having formula (Ia-3), or a salt thereof

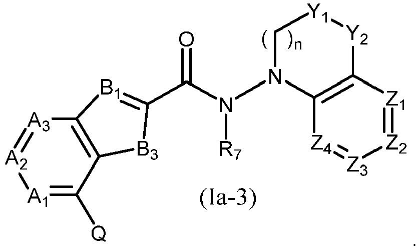

A preferred embodiment provides compounds of formula (I) having formula (Ia-3), or a salt thereof, wherein A₁ is CR_{A1}; A₂ is CR_{A2}; A₃ is CR_{A3}; B₁ is CR_{B1}; and B₃ is S.

A preferred embodiment provides compounds of formula (I) having formula (Ia-3), or a salt thereof, wherein A₁ is N; A₂ is CR_{A2}; A₃ is CR_{A3}; B₁ is CR_{B1}; and B₃ is S.

A preferred embodiment provides compounds of formula (I) having formula (Ia-3), or a salt thereof, wherein A₁ is CR_{A1}; A₂ is CR_{A2}; A₃ is N; B₁ is CR_{B1}; and B₃ is S.

A preferred embodiment provides compounds of formula (I) having formula (Ia-3), or a salt thereof, wherein R_{A1} is halogen, preferably fluoro; R_{A2} is hydrogen; R_{B1} is isopropyl or 2-hydroxyisopropyl; and Q is 2,3,5-trifluorophenyl.

A preferred embodiment provides compounds of formula (I) having formula (Ia-4), or a salt thereof

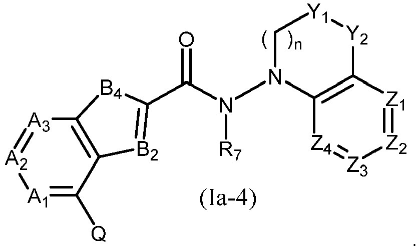

A preferred embodiment provides compounds of formula (I) having formula (Ia-4), or a salt thereof, wherein A₁ is CR_{A1}; A₂ is CR_{A2}; A₃ is CR_{A3}; B₂ is N; and B₄ is NR_{B4}.

A preferred embodiment provides compounds of formula (I) having formula (Ia-4), or a salt thereof, wherein A₁ is N; A₂ is CR_{A2}; A₃ is CR_{A3}; B₂ is N; and B₄ is NR_{B4}.

A preferred embodiment provides compounds of formula (I) having formula (Ia-5), or a salt thereof

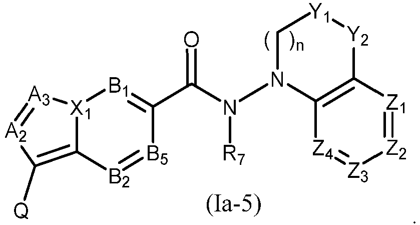

A preferred embodiment provides compounds of formula (I) having formula (Ia-5), or a salt thereof, wherein A₂ is CR_{A2}; A₃ is N; B₁ is CR_{B1}; B₂ is N; B₅ is CR_{B5}; and X₁ is N.

A preferred embodiment provides compounds of formula (I) having formula (Ia-5), or a salt thereof, wherein A₂ is CR_{A2}; A₃ is N; B₁ is CR_{B1}; B₂ is N; B₅ is N; and X₁ is N.

A preferred embodiment provides compounds of formula (I) having formula (Ia-5), or a salt thereof, wherein A₂ is N; A₃ is CR_{A3}; B₁ is CR_{B1}; B₂ is N; B₅ is CR_{B5}; and X₁ is N.

A preferred embodiment provides compounds of formula (I) having formula (Ia-6), or a salt thereof

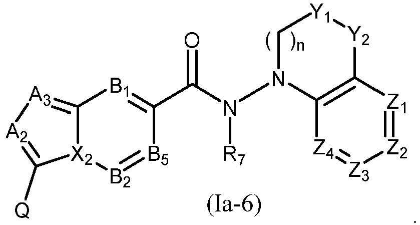

A preferred embodiment provides compounds of formula (I) having formula (Ia-6), or a salt thereof, wherein A₂ is N; A₃ is N; B₁ is CR_{B1}; B₂ is CR_{B2}; B₅ is CR_{B5}; and X₂ is N.

A preferred embodiment provides compounds of formula (I) having formula (Ia-7), or a salt thereof

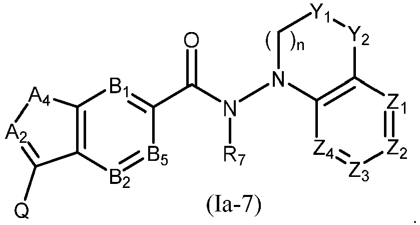

A preferred embodiment provides compounds of formula (I) having formula (Ia-8), or a salt thereof

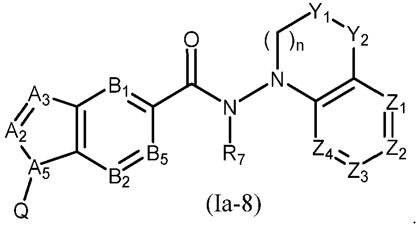

A preferred embodiment provides compounds of formula (I) having formula (Ia-8), or a salt thereof, wherein A₂ is N; A₃ is N; A₅ is N; B₁ is CR_{B1}; B₂ is CR_{B2}; and B₅ is CR_{B5}.

A preferred embodiment provides compounds of formula (I) having formula (Ia-8), or a salt thereof, wherein A₂ is N; A₃ is N; A₅ is N; B₁ is CR_{B1}; B₂ is N; and B₅ is CR_{B5}.

A preferred embodiment provides compounds of formula (I) having formula (Ia-8), or a salt thereof, wherein A₂ is N; A₃ is CR_{A3}; A₅ is N; B₁ is CR_{B1}; B₂ is N; and B₅ is CR_{B5}.

A preferred embodiment provides compounds of formula (I) having formula (Ia-9), or a salt thereof

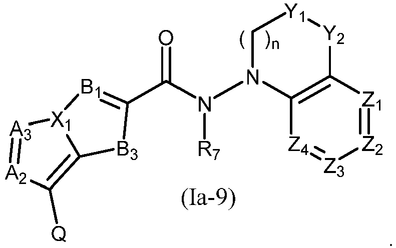

A preferred embodiment provides compounds of formula (I) having formula (Ia-9), or a salt thereof, wherein A₂ is CR_{A2}; A₃ is N; B₁ is CR_{B1}; B₃ is S; and X₁ is N.

A preferred embodiment provides compounds of formula (I) having formula (Ia-10), or a salt thereof

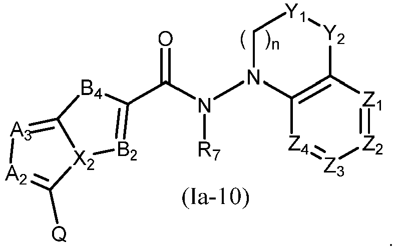

A preferred embodiment provides compounds of formula (I) having formula (Ia-11), or a salt thereof

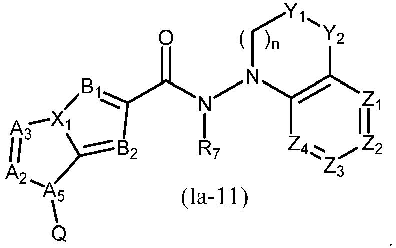

A preferred embodiment provides compounds of formula (I), wherein
G is 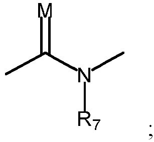 and
M is O;
or a salt thereof.

A preferred embodiment provides compounds of formula (I), wherein
G is 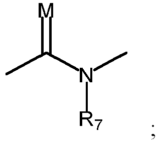
M is O; and
R₇ is hydrogen
or a salt thereof.

A preferred embodiment provides compounds of formula (I), wherein
G is 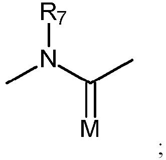 and
M is O;
or a salt thereof.

A preferred embodiment provides compounds of formula (I), wherein
G is 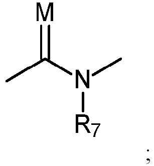 and
M is O;
or a salt thereof.

A preferred embodiment provides compounds of formula (I), wherein
Q is a 6- or 10 membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
or a salt thereof.

A preferred embodiment provides compounds of formula (I), wherein
Q is a 6-membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl.

A preferred embodiment provides compounds of formula (I), wherein
Q is 6-membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6-membered aryl is fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocyclalkyl is substituted with a substituent selected from the group consisting of hydrogen, C₁-C₄ alkyl, and C₃-C₆ cycloalkyl;
or a salt thereof.

A preferred embodiment provides compounds of formula (I), wherein
Q is a 5- to 10-membered heteroaryl having 1 or 2 heteroatoms selected from the group O, S, and N and wherein the carbons of the heteroaryl are optionally substituted with 1, 2 or 3 substituents independently selected from the group consisting of halogen, cyano, nitro, -OH, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), and -N(C₁-C₄ alkyl)₂ and any N in the heteroaryl is optionally substituted with a substituent selected from the group consisting of hydrogen, C₁-C₄ alkyl, and C₃-C₆ cycloalkyl;
or a salt thereof.

A preferred embodiment provides compounds of formula (I), wherein
Q is a 4- to 7-membered heterocycloalkyl having 1 or 2 heteroatoms selected from the group O, S, N, wherein the heterocycloalkyl is optionally benzo-fused, wherein the carbons of the heterocycloalkyl or optionally benzo-fused heterocycloalkyl are optionally substituted with 1, 2, 3, or 4 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), and -N(C₁-C₄ alkyl)₂ and any N in the heterocyclalkyl is optionally substituted with a substituent selected from the group consisting of hydrogen, C₁-C₄ alkyl, and C₃-C₆ cycloalkyl;
or a salt thereof.

A preferred embodiment provides compounds of formula (I), or a salt thereof, wherein Q is selected from:

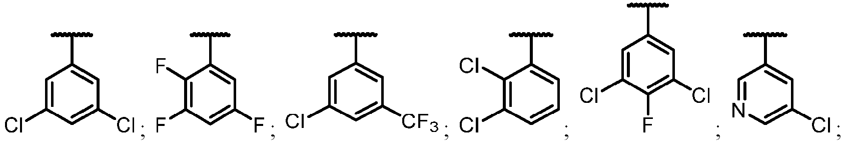

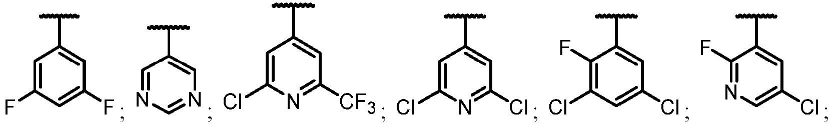

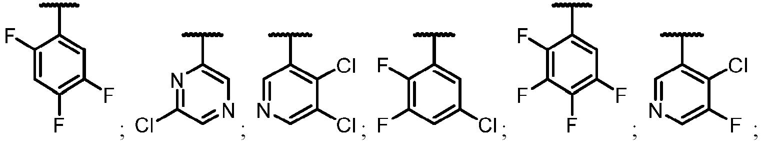

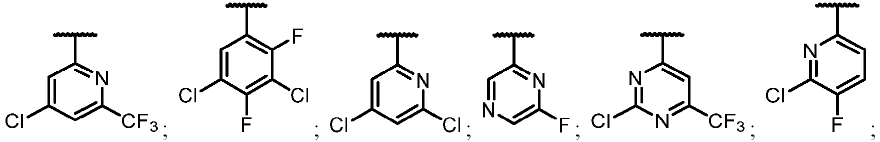

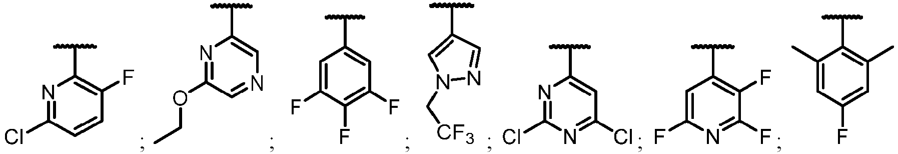

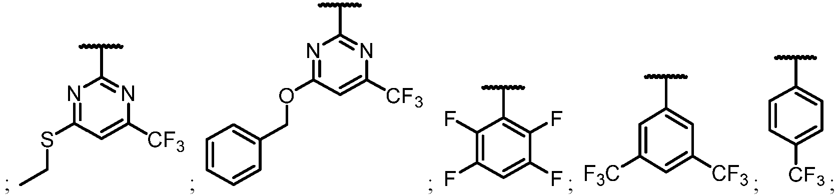

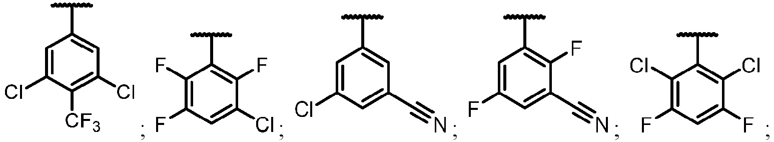

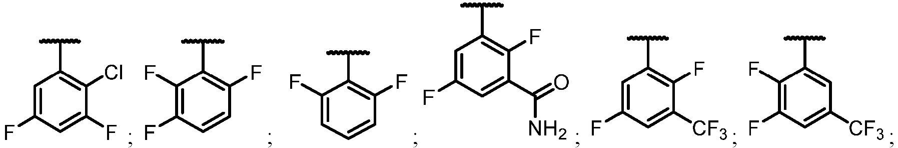

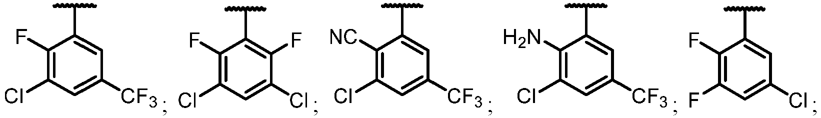

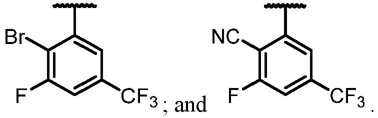

A preferred embodiment provides compounds of formula (I), or a salt thereof, wherein Q is

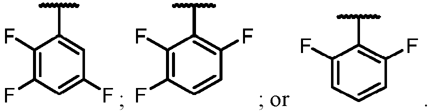

A preferred embodiment provides compounds of formula (I), or a salt thereof, wherein n is 1;

A preferred embodiment provides compounds of formula (I), wherein Y₁ is CR₈R₉ and Y₂ is O; or a salt thereof.

A preferred embodiment provides compounds of formula (I), wherein n is 1, Y₁ is CR₈R₉, Y₂ is O, Z₁ is CR₁₁, Z₂ is CR₁₁, Z₃ is CR₁₁, Z₄ is CR₁₁, or a salt thereof.

A preferred embodiment provides compounds of formula (I), wherein
Q is phenyl having 1 to 5 halogen atoms, preferably Q is 2,3,5-trifluorophenyl and 2,6-difluorophenyl;
n is 1, Y₁ is CR₈R₉, Y₂ is O, Z₁ is CR₁₁, Z₂ is CR₁₁, Z₃ is CR₁₁, Z₄ is CR₁₁, and
G is 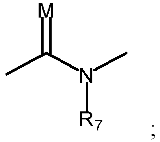
M is O; and
R₇ is hydrogen;
or a salt thereof.

A preferred embodiment provides compounds of formula (I), wherein R_{B1}, when present, is selected from the group consisting of C₁-C₄ alkyl, hydroxy-substituted C₁-C₄ alkyl, C₃-C₆ cycloalkyl, -N(C₁-C₄ alkyl)₂, and 4- to 7-membered heterocycloalkyl; or a salt thereof.

A preferred embodiment provides compounds of formula (I), or a salt thereof, wherein R_{B1}, when present, is 4-morpholino, isopropyl, or 2-hydroxyisopropyl.

A preferred embodiment provides compounds of formula (I), or a salt thereof, wherein R_{B1}, when present, is selected from:

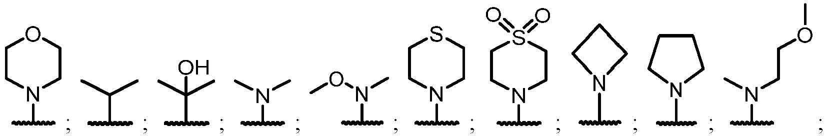

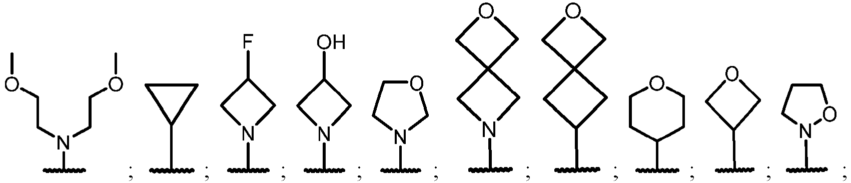

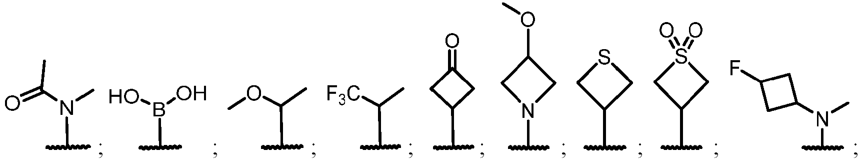

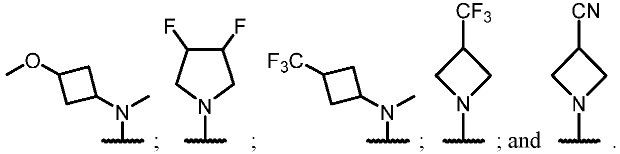

A preferred embodiment provides compounds of formula (I), wherein R_{B4}, when present, is selected from the group consisting of C₁-C₄ alkyl, hydroxy-substituted C₁-C₄ alkyl, C₃-C₆ cycloalkyl, -N(C₁-C₄ alkyl)₂, and 4- to 7-membered heterocycloalkyl; or a salt thereof.

A preferred embodiment provides compounds of formula (I), or a salt thereof, wherein R_{B4}, when present, is 4-morpholino, isopropyl, or 2-hydroxyisopropyl.

A preferred embodiment provides compounds of formula (I), or a salt thereof, wherein R_{B4}, when present, is isopropyl.

A preferred embodiment provides compounds of formula (I), wherein
R_{A1}, R_{A2}, R_{A3}, R_{B2}, and R_{B5} are each independently selected from the group consisting of hydrogen, halogen, C₁-C₄ alkyl; preferably R_{A1}, R_{A2}, R_{A3}, R_{B2}, and R_{B5} are each independently selected from the group consisting of hydrogen, fluoro, methyl;
R_{A4} and R_{B3} are independently selected from the group consisting of hydrogen, C₁-C₄ alkyl; preferably R_{A4} and R_{B3} are independently selected from the group consisting of hydrogen and methyl;
R_{B1} is independently selected from the group consisting of C₁-C₄ alkyl, -N(C₁-C₄ alkyl)₂, wherein each C₁-C₄-alkyl in R_{B1} may be optionally substituted with 1, 2 or 3 substituents of hydroxy; and a 4- to 7-membered heterocycloalkyl containing 1, 2, or 3 heteroatoms independently selected from N or O, wherein each heterocycloalkyl in R_{B1} is optionally substituted with 1, 2 or 3 substituents of halogen; preferably is R_{B1} independently selected from the group consisting of 4-morpholino, isopropyl, 2-hydroxyisopropyl, 3-fluoroazetidinyl, and NMe₂;
R_{B4} is selected from the group consisting of C₁-C₄ alkyl; preferably R_{B4} is isopropyl;
or a salt thereof.

A preferred embodiment provides compounds of formula (I), wherein the compound is selected from the group consisting of: 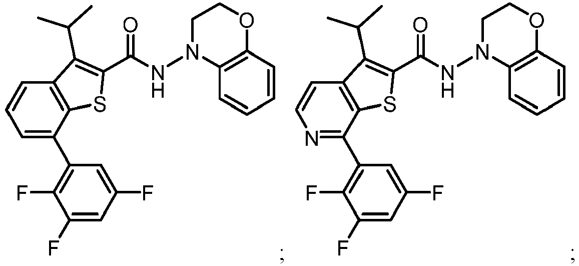 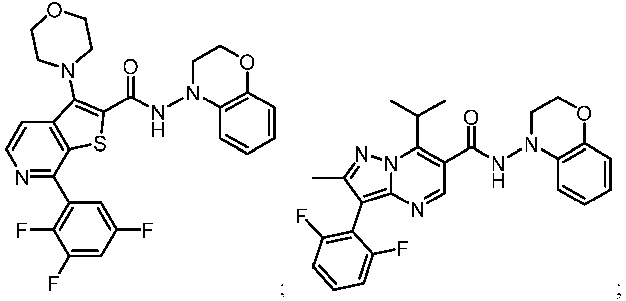 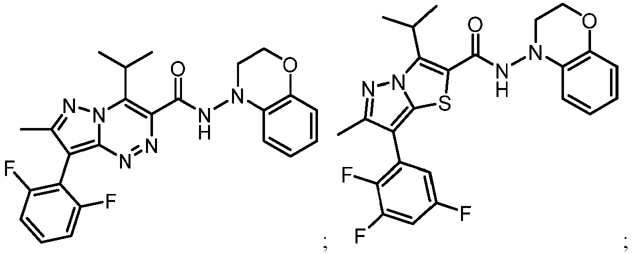 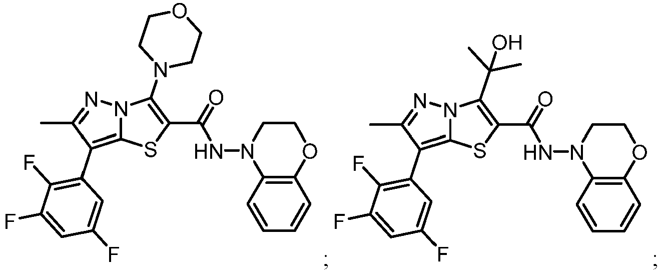 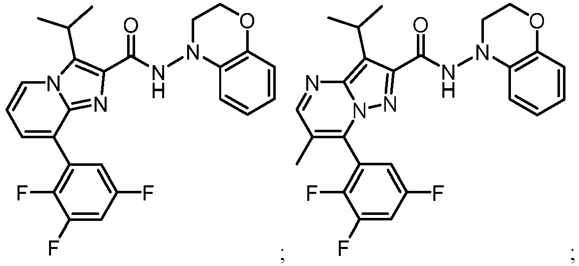 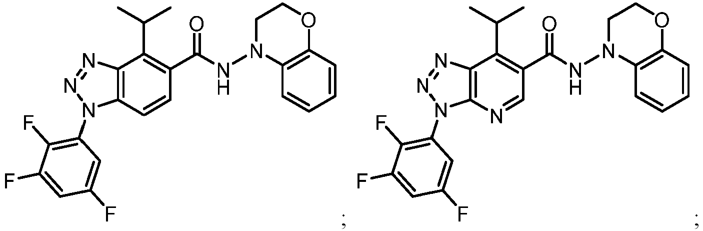 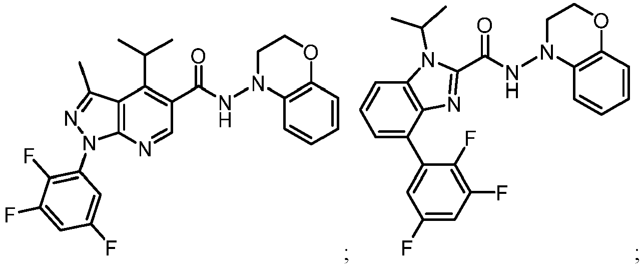 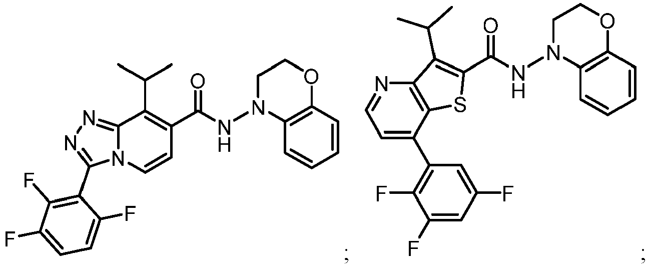 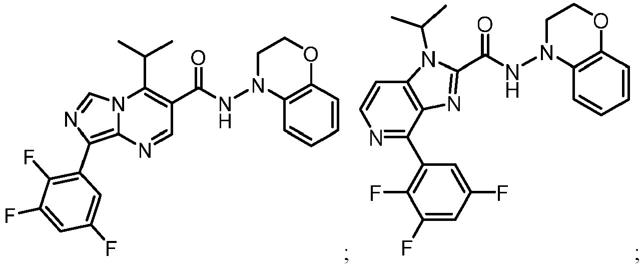 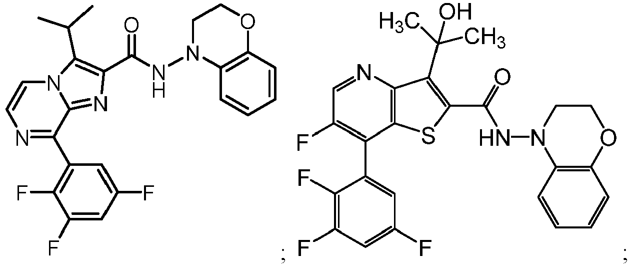 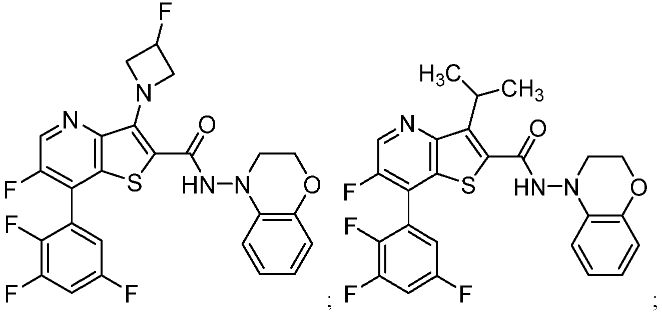 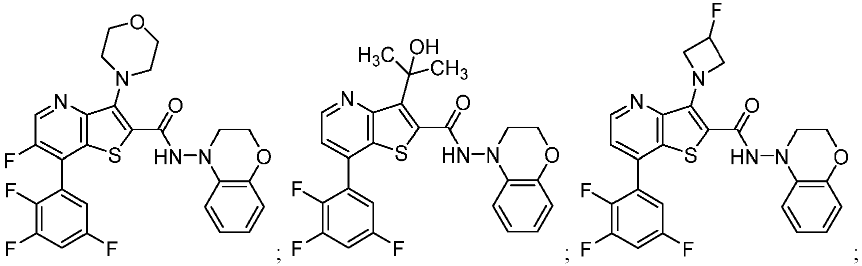 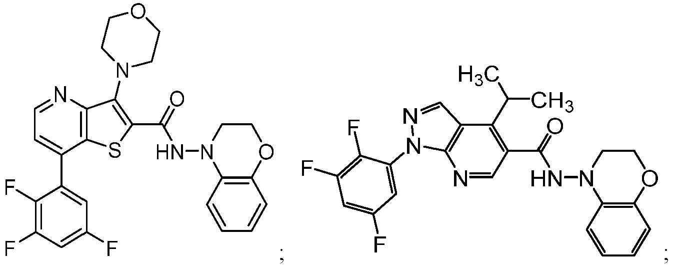 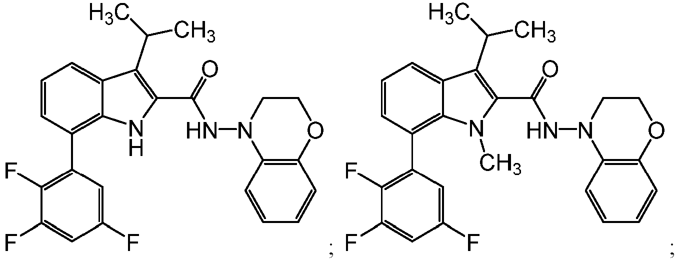 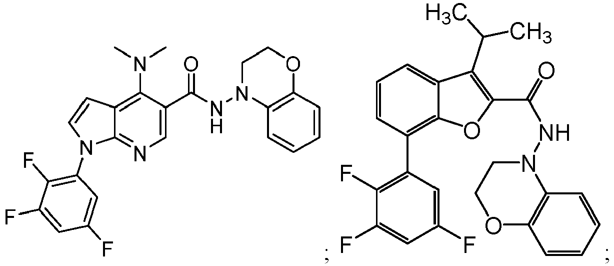 or a salt of any of the foregoing compounds.

In accordance with a further aspect, the present invention covers a pharmaceutical composition comprising a compound of formula (I), as described *supra,* or a salt thereof, and at least one acceptable carrier.

In accordance with a further aspect, the present invention covers a compound of formula (I), as described *supra,* or a pharmaceutical composition, as described *supra,* for use in the control, treatment and/or prevention of a disease.

In a preferred embodiment, the disease is an infection caused by endoparasites.

In a preferred embodiment, the disease is a helminthic infection.

In a preferred embodiment, the disease is a heartworm infection.

In accordance with a further aspect, the present invention covers a use of a compound of formula (I), as described *supra,* or a pharmaceutical composition, as described *supra,* for the control, treatment and/or prevention of a disease.

In accordance with a further aspect, the present invention covers a use of a compound of formula (I), as described *supra,* or a pharmaceutical composition, as described *supra,* for the preparation of a medicament for the control, treatment and/or prevention of a disease.

In a preferred embodiment, the disease is an infection caused by endoparasites.

In a preferred embodiment, the disease is a helminthic infection.

In a preferred embodiment, the disease is a heartworm infection.

In accordance with a further aspect, the present invention covers a method for controlling endoparasitic infections in humans and/or animals by administering an effective amount of at least one compound of formula (I), as described *supra,* to a human or an animal in need thereof.

In a preferred embodiment, the endoparasitic disease is a helminthic infection.

In a preferred embodiment, the endoparasitic disease is a heartworm infection.

In a particular further embodiment, the present invention covers combinations of two or more of the above mentioned embodiments.

The present invention covers any sub-combination within any embodiment or aspect of the present invention of compounds of formula (I), supra.

The term "C₁-C₄ alkyl" refers to a straight or branched alkyl chain having from one to four carbon atoms and includes methyl, ethyl, propyl, isopropyl, butyl, and the like.

The term "C₁-C₄ halogenoalkyl" refers to a straight or branched alkyl chain having from one to four carbon atoms and 1 to 5 halogen and includes fluoromethyl, difluoromethyl, trifluoromethyl, 2,2,2-trifluoroethyl, 1,2,2-trifluoroethyl, 3,3,3-trifluoropropyl, and the like.

The term "C₂-C₄ alkenyl" refers to a straight or branched alkenyl chain having from two to four carbon atoms and one carbon-carbon double bond, and includes ethylene, propylene, isopropylene, butylene, iso-butylene, sec-butylene, and the like.

The term "C₂-C₄ alkynyl" refers to a straight or branched alkynyl chain having from two to four carbon atoms and one carbon-carbon triple bond, and includes acetylene, propargyl, and the like.

The term "C₁-C₄ alkoxy" refers to a C₁-C₄ alkyl attached through an oxygen atom and includes methoxy, ethoxy, propoxy, isopropoxy, butoxy, and the like.

The term "C₃-C₆ cycloalkyl" refers to an alkyl ring of three to six carbon atoms, and includes cyclopropyl, cyclobutyl, cyclopentyl, and cyclohexyl.

The terms "halogen" and "halogeno" refers to a chloro, fluoro, bromo or iodo atom.

The term "C₆- or C₁₀- membered aryl" refers to phenyl or naphthyl.

The term "C₆- or C₁₀- membered aryloxy" refers to phenyl or naphthyl attached through an oxygen atom and includes phenoxy and naphtyloxy.

The term "C₆- or C₁₀- membered arylthio-oxy" refers to phenyl or naphthyl attached through an sulfur atom and includes phenthio-oxy and naphtylthio-oxy. Further it is understood that the term "C₆- or C₁₀- membered arylthio-oxy" also encompasses in which the sulfur is the -SO₂- and - S(O)-.

The term "4- to 7-membered heterocycloalkyl" refers to a 4 to 7 membered monocyclic saturated or partially (but not fully) unsaturated ring having one or more heteroatoms, preferably one, two, or three heteroatoms, selected from the group consisting of nitrogen, oxygen, and sulfur and the ring optionally includes a carbonyl to form a lactam or lactone. It is understood that where sulfur is included that the sulfur may be either -S-, -SO-, or -SO₂-. For example, but not limiting, the term includes azetidinyl, pyrrolidinyl, piperidinyl, piperazinyl, morpholinyl, thiomorpholinyl, oxetanyl, dioxolanyl, tetrahydropyranyl, tetrahydrothiopyranyl, tetrahydrofuryl, hexahydropyrimidinyl, tetrahydropyrimidinyl, dihydroimidazolyl, and the like.

The term "5-membered heteroaryl" refers to a five membered, monocyclic, fully unsaturated, ring with one to four carbon atoms and one to four heteroatoms selected from the group consisting of nitrogen, oxygen, and sulfur. For example, but not limiting, the term includes furyl, thienyl, pyrrolyl, imidazolyl, isothiazolyl, isoxazolyl, oxadiazolyl, oxazolyl, thiazolyl, thiadiazolyl, triazolyl, tetrazolyl, and the like. It is understood that a 5-membered heteroaryl can be attached as a substituent through a ring carbon or a ring nitrogen atom where such an attachment mode is available, for example for a pyrrolyl, imidazolyl, pyrazolyl, triazolyl, and the like.

The term "6-membered heteroaryl" refers to a six membered, monocyclic, fully unsaturated ring with one to five carbon atoms and one or more, typically one to four, heteroatoms selected from the group consisting of nitrogen, oxygen, and sulfur. For example, but not limiting, the term includes pyrazinyl, pyrazolyl, pyridazinyl, pyridyl, pyrimidyl, and the like. It is understood that a 6-membered heteroaryl can be attached as a substituent through a ring carbon or a ring nitrogen atom where such an attachment mode is available.

The term "5- to 10-membered heteroaryl" refers to a five to ten membered, monocyclic or polycyclic fully unsaturated, ring or ring system with one to nine carbon atoms and one or more heteroatoms, preferably one, two, or three heteroatoms, selected from the group consisting of nitrogen, oxygen, and sulfur. For example, but not limiting, the term includes furyl, thienyl, pyrrolyl, imidazolyl, isothiazolyl, isoxazolyl, oxadiazolyl, oxazolyl, thiazolyl, pyrazinyl, pyrazolyl, pyridazinyl, pyridyl, pyrimidyl, azepinyl, diazepinyl, benzofuryl, benzothienyl, indolyl, isoindolyl, benzimidazolyl, benzisothiazolyl, benzisoxazolyl, benzoxazolyl, benzopyrazinyl, benzopyrazolyl, quinazolyl, thienopyridyl, quinolyl, isoquinolyl, benzothiazolyl and the like. It is understood that a 5- to 10-membered heteroaryl having 1, 2, or 3 heteroatoms selected from the group O, S, and N can be attached as a substituent through a ring carbon or a ring nitrogen atom where such an attachment mode is available.

The term "5- to 10-membered heteroaryloxy" refers to a 5- to 10-membered heteroaryl having one or more heteroatoms, preferably 1, 2, or 3 heteroatoms, selected from the group O, S, and N, attached through an oxygen atom and includes imidazolyloxy, pyrazolyloxy, pyridyloxy, pyrimidyloxy, quinolyloxy, and the like.

The term "oxo" refers to an oxygen atom doubly bonded to the carbon to which it is attached to form the carbonyl of a ketone or aldehyde. For example, a pyridone radical is contemplated as an oxo substituted 6-membered heteroaryl.

The term "carboxyl" refers to the group below:

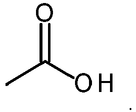

The term "carbamoyl" refers to the group below:

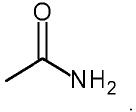

The term "C₁-C₄ alkoxy carbonyl" refers the group below: 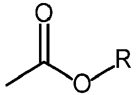 wherein R is a C₁-C₄ alkyl.

The term "nil" as used herein with reference to a group, substituent, moiety, or the like, indicates that that group, substituent, or moiety is not present. Wherein a group, substituent, or moiety is ordinarily bonded to two or more other groups, substituents, or moieties, the others are bonded together in lieu of the group, substituent, or moiety which is nil. For example, with a compound having the structure A-B-C; wherein B is nil, then A is directly bonded to C and the compound is A-C. As another example, with a compound having the structure A-B-C; wherein C is nil, then the compound is A-B.

The term "salt" refers to salts of veterinary or pharmaceutically acceptable organic acids and bases or inorganic acids and bases. Such salts are well known in the art and include those described in Journal of Pharmaceutical Science, 66, 2-19 (1977). An example is the hydrochloride salt.

The term "substituted," including when used in "optionally substituted" refers to one or more hydrogen radicals of a group being replaced with non-hydrogen radicals (substituent(s)). It is understood that the substituents may be either the same or different at every substituted position. Combinations of groups and substituents envisioned by this invention are those that are stable or chemically feasible. For compounds described herein, groups and substituents thereof may be selected in accordance with permitted valence of the atoms and the substituents, such that the selections and substitutions result in a stable compound, e.g., which does not spontaneously undergo transformation such as by rearrangement, cyclization, elimination, etc.

It is understood that when a cycloalkyl or heterocycloalkyl ring is substituted with a spiro group, the spiro group can be attached, valency permitting, to any position of the cycloalkyl or heterocycloalkyl, forming an additional ring such that the spiro group is attached to the cycloalkyl or heterocycloalkyl ring through a common atom. Examples of such spiro substituted rings include 2-oxa-6-azaspiro[3.3]heptane, 2-azaspiro[3.3]heptane, 2-azaspiro[3.4]octane, 6-oxa-2-azaspiro[3.4]octane, and the like.

The term "stable" refers to compounds that are not substantially altered when subjected to conditions to allow for their production. In a non-limiting example, a stable compound or chemically feasible compound is one that is not substantially altered when kept at a temperature of 40°C or less, in the absence of moisture or other chemically reactive conditions, for about a week.

It is understood that, where the terms defined herein mention a number of carbon atoms, that the mentioned number refers to the mentioned group and does not include any carbons that may be present in any optional substituent(s) thereon or any carbons that may be present as part of a fused ring, including a benzo-fused ring.

The skilled artisan will appreciate that certain of the compounds of the present invention exist as isomers. All stereoisomers of the compounds of the invention, including geometric isomers, enantiomers, and diastereomers, in any ratio, are contemplated to be within the scope of the present invention.

The skilled artisan will also appreciate that certain of the compounds of the present invention exist as tautomers. All tautomeric forms the compounds of the invention are contemplated to be within the scope of the present invention.

Compounds of the invention also include all isotopic variations, in which at least one atom of the predominant atom mass is replaced by an atom having the same atomic number, but an atomic mass different from the predominant atomic mass. Use of isotopic variations (e.g., deuterium, ²H) may afford greater metabolic stability. Additionally, certain isotopic variations of the compounds of the invention may incorporate a radioactive isotope (e.g., tritium, ³H, or ¹⁴C), which may be useful in drug and/or substrate tissue distribution studies. Substitution with positron emitting isotopes, such as ¹¹C, ¹⁸F, ¹⁵O and ¹³N, may be useful in Positron Emission Topography (PET) studies.

The terms "compounds of the invention" and "a compound of the invention" and "compounds of the present invention" and a like include the embodiment of formula (I) and the other more particular embodiments encompassed by formula (I) described herein and the exemplified compounds described herein and a salt of each of these embodiments.

The compound of formula (I) with G as defined has the formulae:

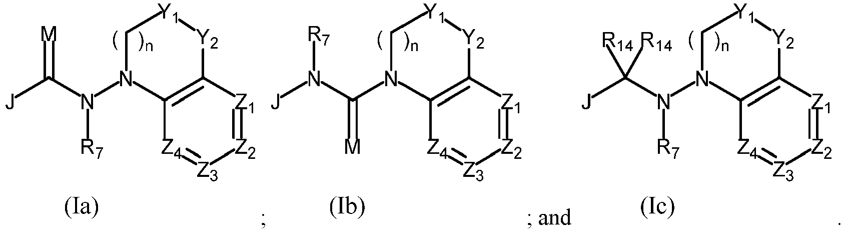

Another embodiment provides compounds of formulae: 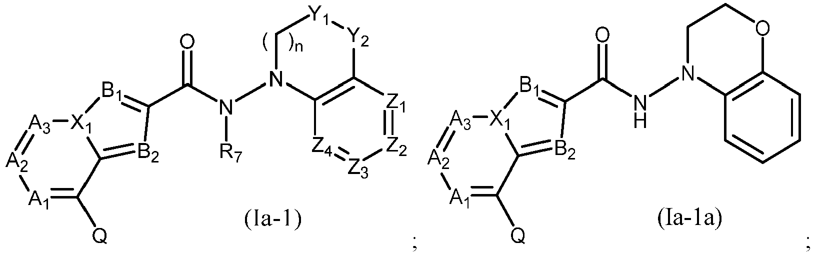 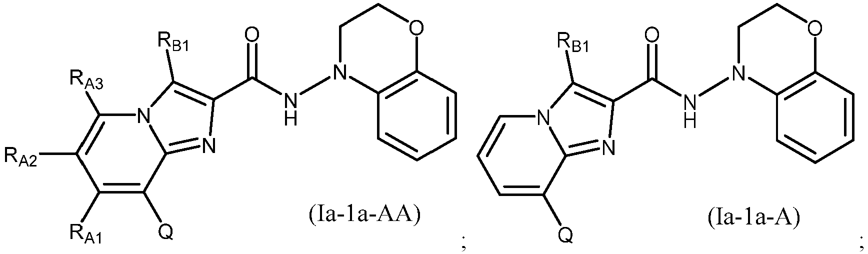 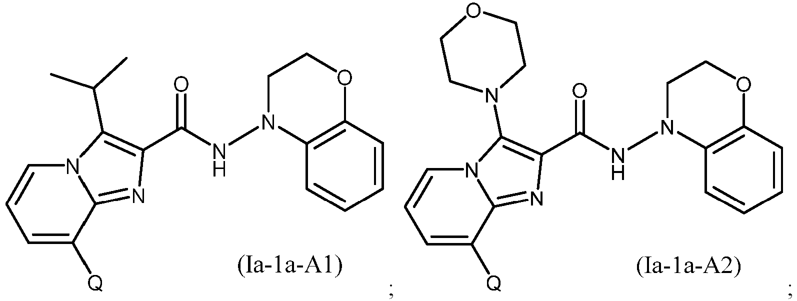 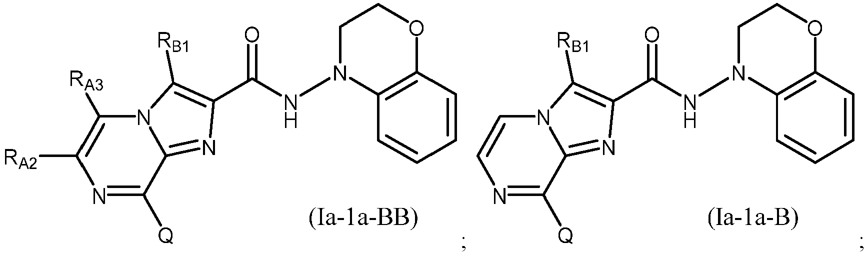 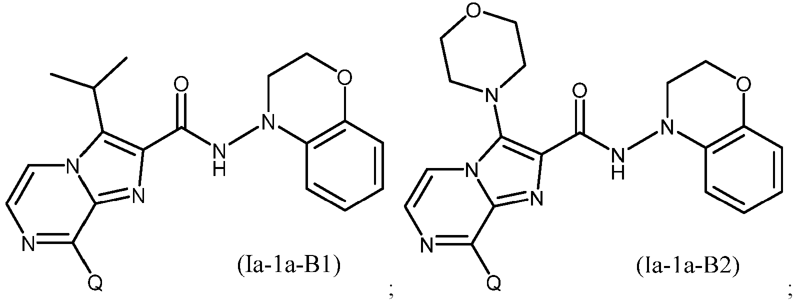 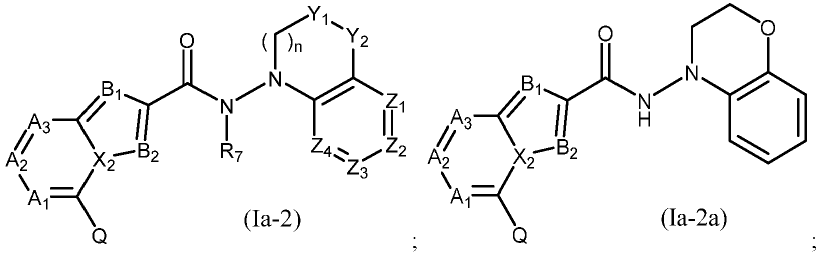 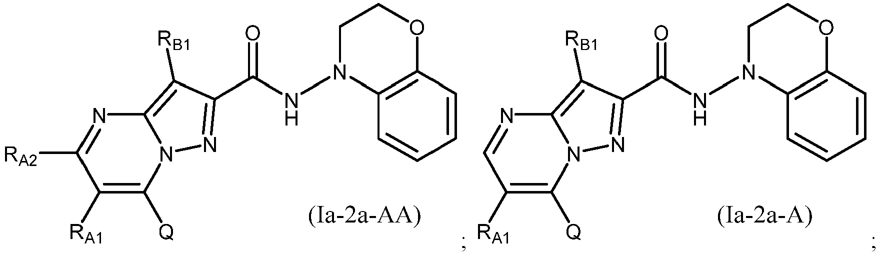 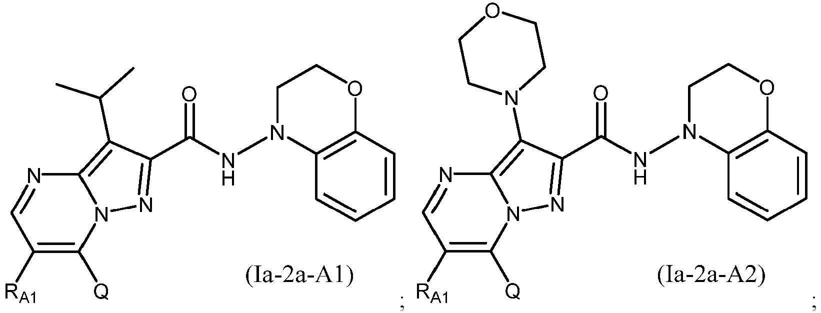 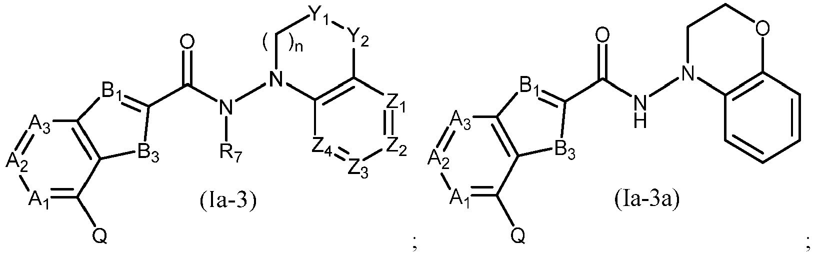 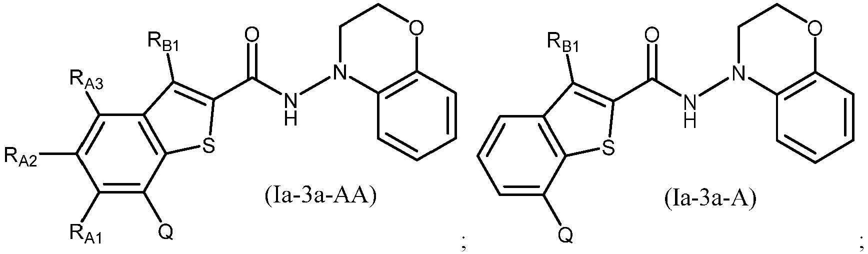 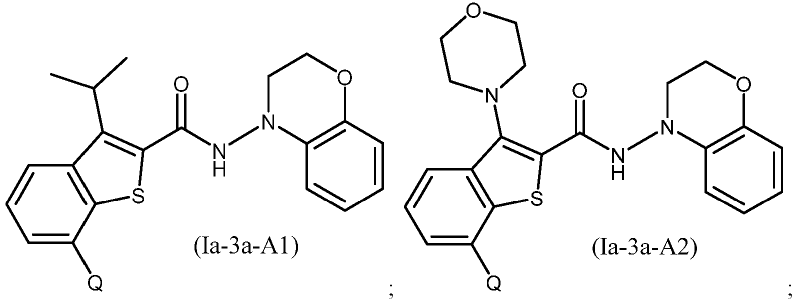 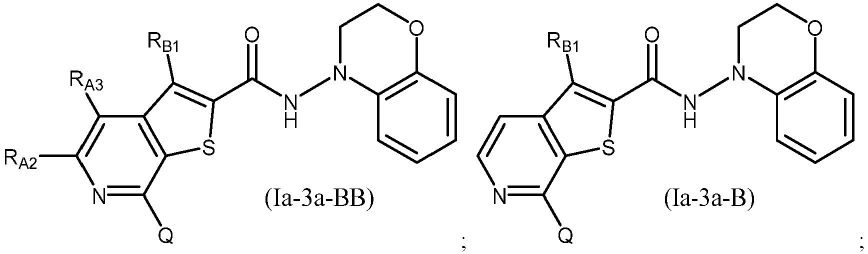 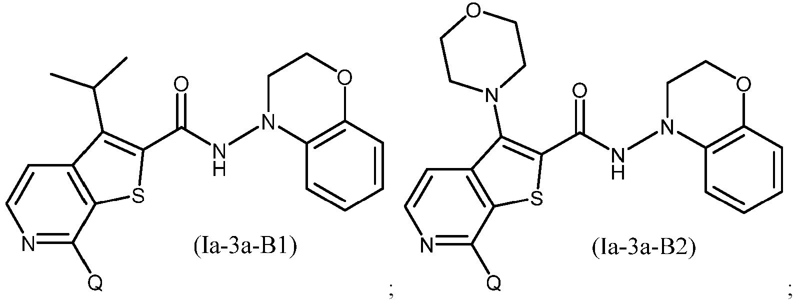 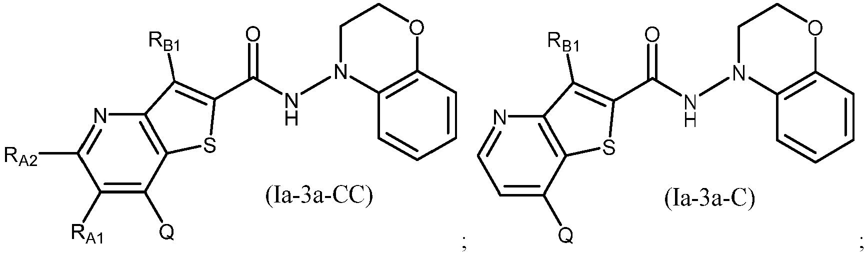 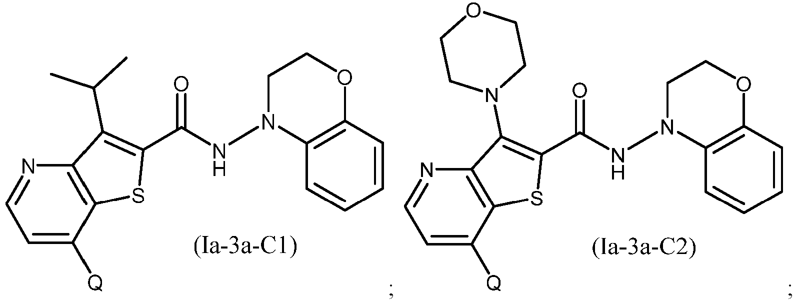 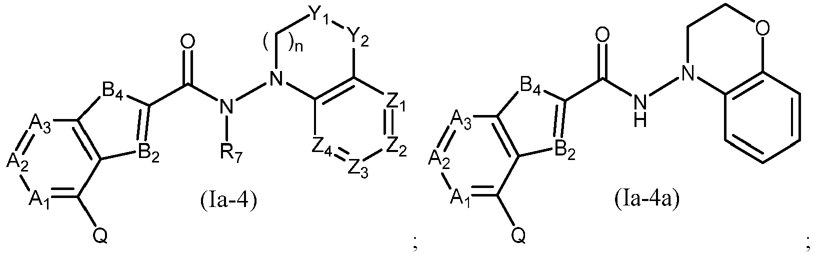 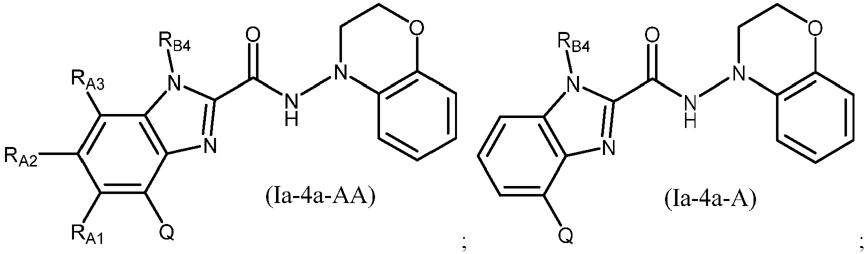 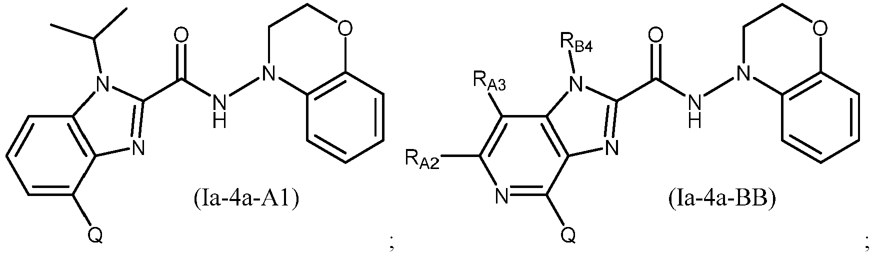 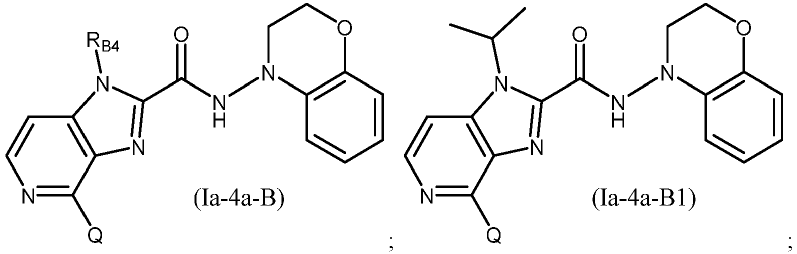 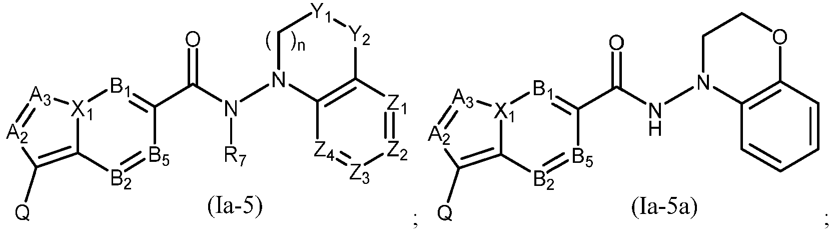 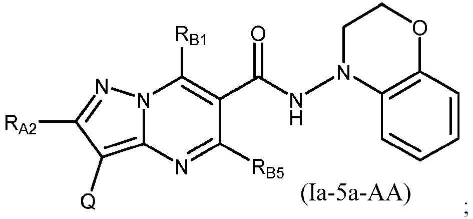 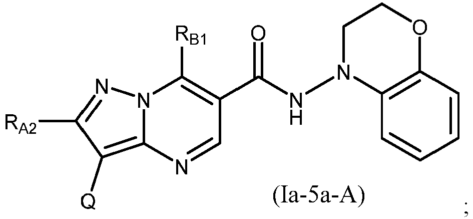 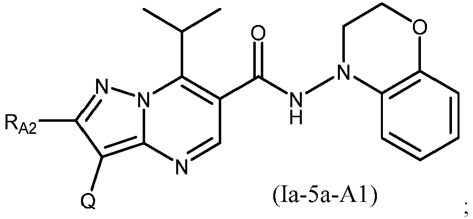 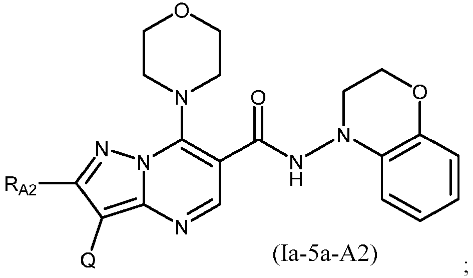 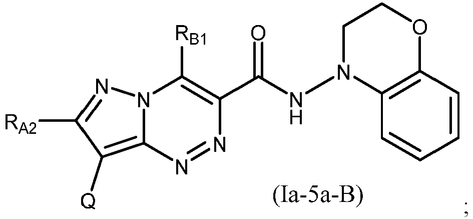 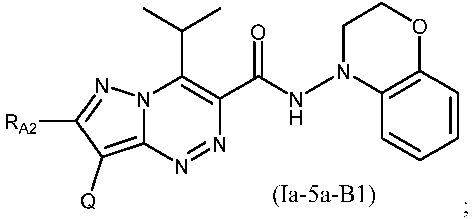 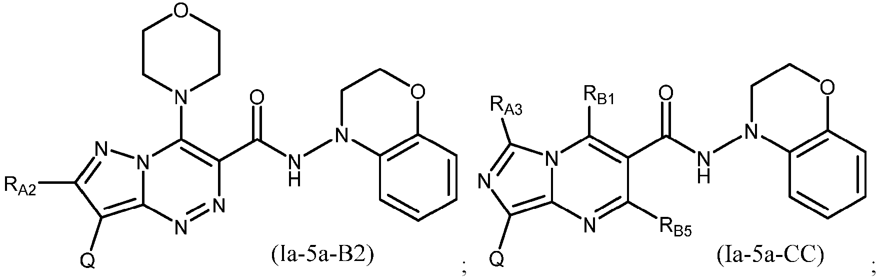 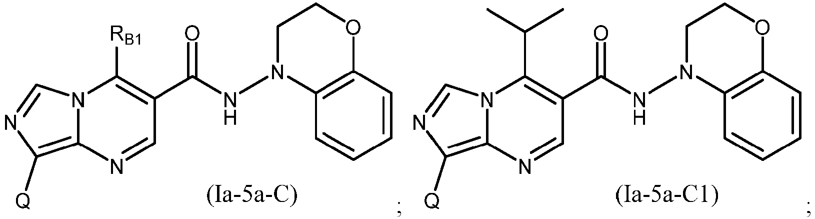 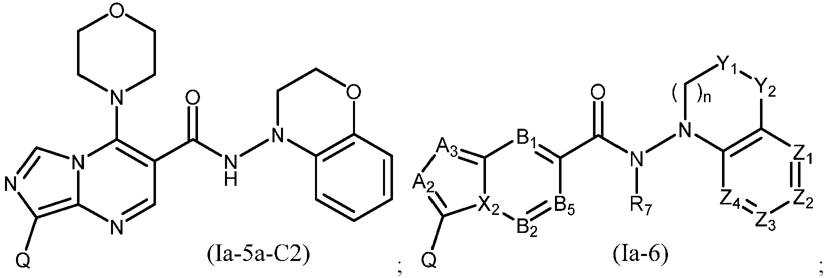 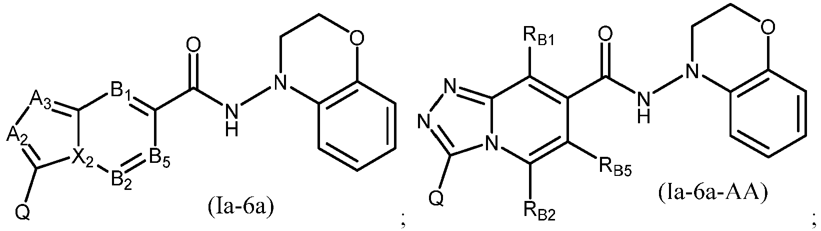 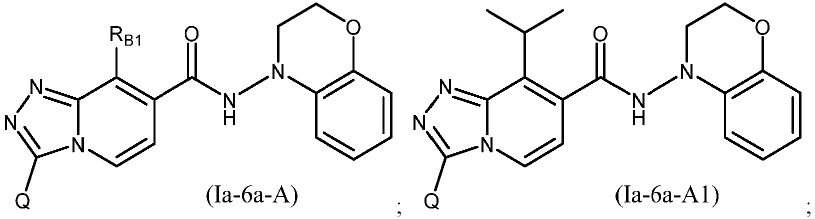 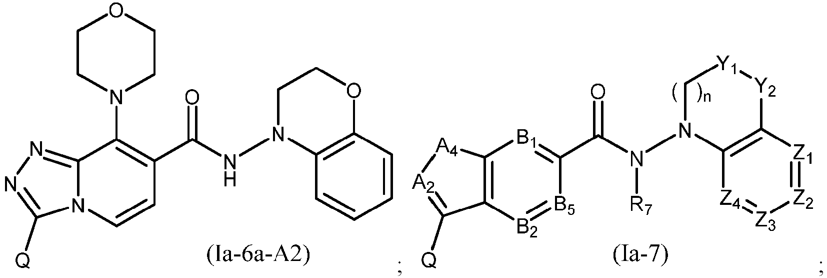 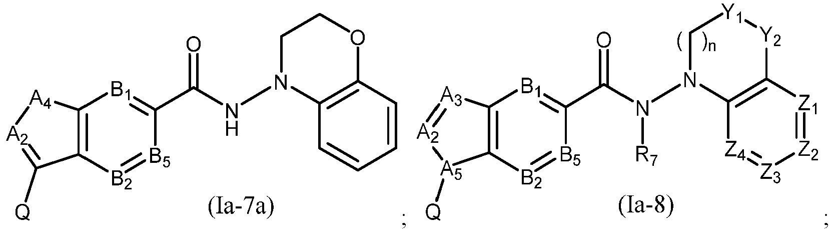 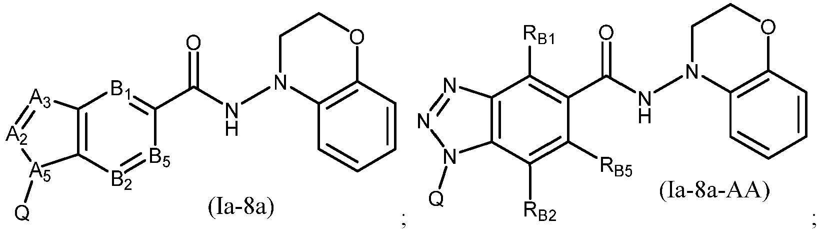 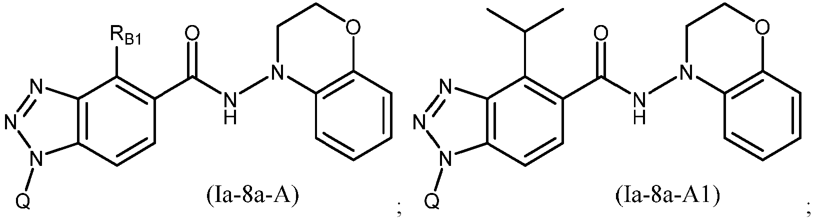 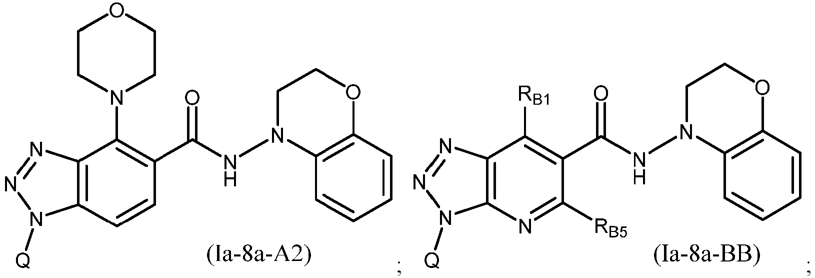 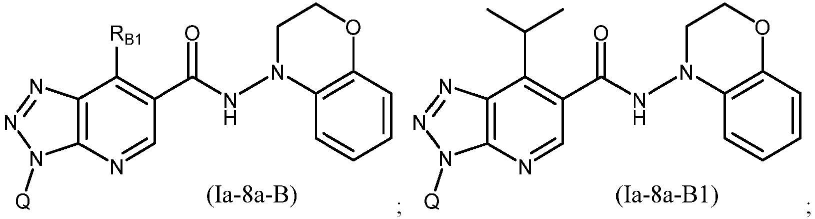 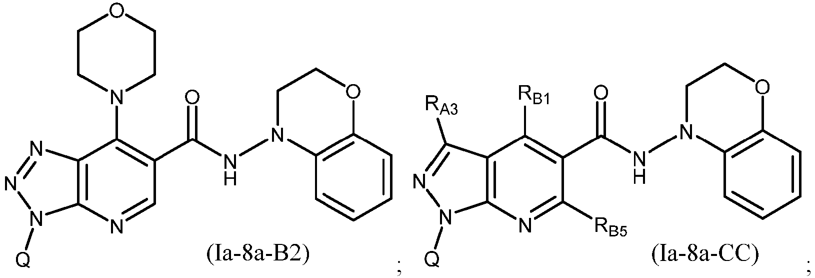 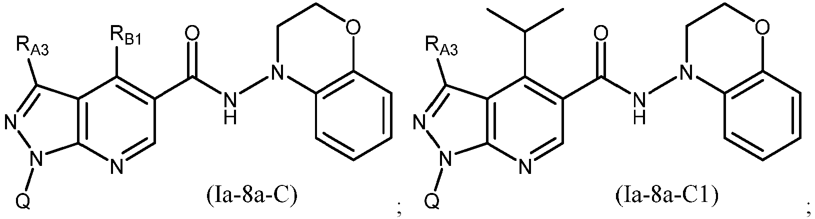 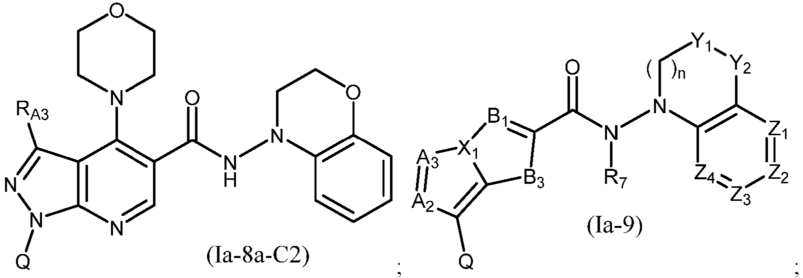 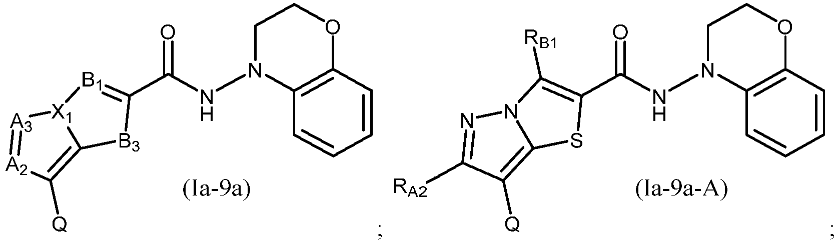 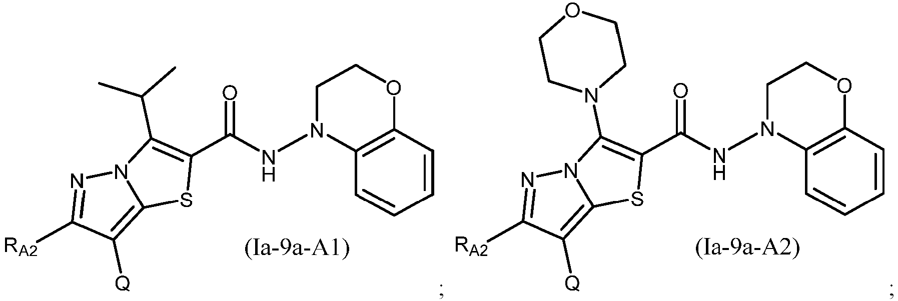 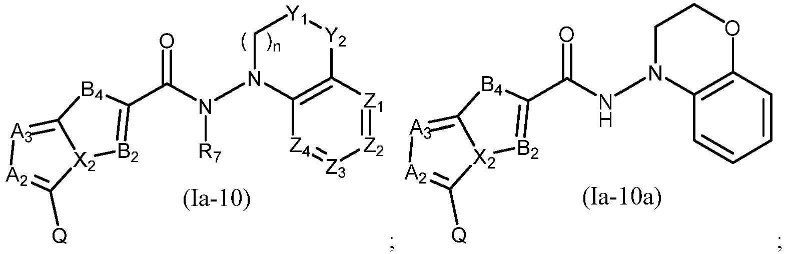 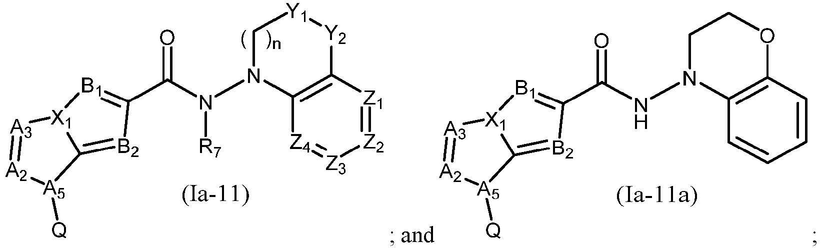
or a salt of any of the foregoing;
wherein A₁, A₂, A₃, A₄, As, B₁, B₂, B₃, B₄, B₅, X₁, X₂, Y₁, Y₂, Z₁, Z₂, Z₃, Z₄, R_{A1}, R_{A2}, R_{A3}, R_{B1}, R_{B2}, R_{B4}, R_{B5}, R₇, Q, and n are as defined in the Summary except for formula (Ia-6) and (Ia-6a), for which A₂ is N and A₁, A₃, A₄, A₃, B₁, B₂, B₃, B₄, B₃, X₁, X₂, Y₁, Y₂, Z₁, Z₂, Z₃, Z₄, R_{A1}, R_{A2}, R_{A3}, R_{B1}, R_{B2}, R_{B4}, R_{B5}, R₇, Q, and n are as defined in the Summary.

In another embodiment for formula (Ia-1) through (Ia-11a) [*i.e.,* formulae (Ia-1), (Ia-1a), (Ia-1a-AA), (Ia-1a-A), (Ia-1a-A1), (Ia-1a-A2), (Ia-1a-BB), (Ia-1a-B), (Ia-1a-B1), (Ia-1a-B2), (Ia-2), (Ia-2a), (Ia-2a-AA), (Ia-2a-A), (Ia-2a-A1), (Ia-2a-A2), (Ia-3), (Ia-3a), (Ia-3a-AA), (Ia-3a-A), (Ia-3a-A1), (Ia-3a-A2), (Ia-3a-BB), (Ia-3a-B), (Ia-3a-B1), (Ia-3a-B2), (Ia-3a-CC), (Ia-3a-C), (Ia-3a-C1), (Ia-3a-C2), (Ia-4), (Ia-4a), (Ia-4a-AA), (Ia-4a-A), (Ia-4a-A1), (Ia-4a-BB), (Ia-4a-B), (Ia-4a-B1), (Ia-5), (Ia-5a), (Ia-5a-AA), (Ia-5a-A), (Ia-5a-A1), (Ia-5a-A2), (Ia-5a-B), (Ia-5a-B1), (Ia-5a-B2), (Ia-5a-CC), (Ia-5a-C), (Ia-5a-C1), (Ia-5a-C2), (Ia-6), (Ia-6a), (Ia-6a-AA), (Ia-6a-A), (Ia-6a-A1), (Ia-6a-A2), (Ia-7), (Ia-7a), (Ia-8), (Ia-8a), (Ia-8a-AA), (Ia-8a-A), (Ia-8a-A1), (Ia-8a-A2), (Ia-8a-BB), (Ia-8a-B), (Ia-8a-B1), (Ia-8a-B2), (Ia-8a-CC), (Ia-8a-C), (Ia-8a-C1), (Ia-8a-C2), (Ia-9), (Ia-9a), (Ia-9a-A), (Ia-9a-A1), (Ia-9a-A2), (Ia-10), (Ia-10a), (Ia-11), and (Ia-11a)], R_{A1}, R_{A2}, R_{A3}, when present [*i.e.,* when specifically depicted in the formula], are each independently selected from hydrogen, C₁-C₄ alkyl, C₁-C₄ halogenoalkyl, halogen, and cyano. In another embodiment for formula (Ia-1) through (Ia-11a), R_{A1}, R_{A2}, R_{A3}, when present, are each independently selected from hydrogen and methyl.

In another embodiment for formula (Ia-1) through (Ia-11a), R_{B1} when present, is selected from

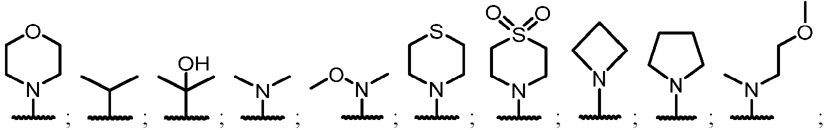

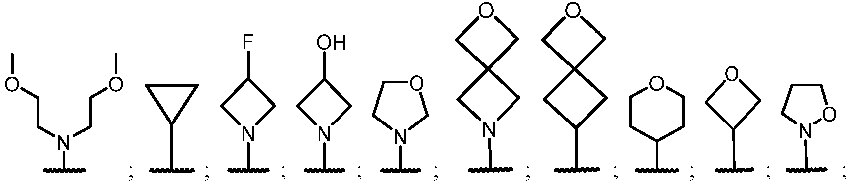

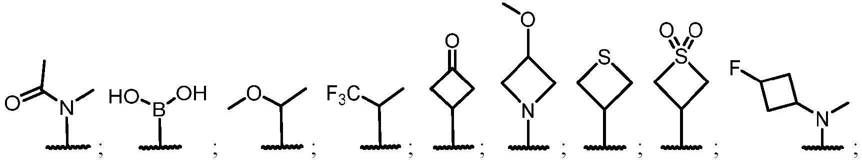

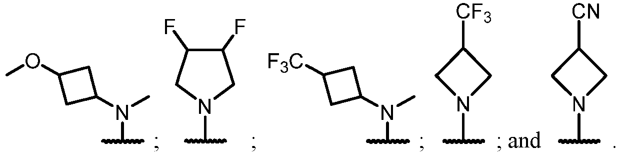

In another embodiment for formula (Ia-1) through (Ia-11a), R_{B1}, when present, is selected from:

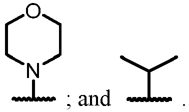

In another embodiment for formula (Ia-1) through (Ia-11a), R_{B4}, when present, is selected from:

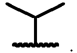

In another embodiment for formula (Ia-1) through (Ia-11a), R₇, when present, is hydrogen.

In another embodiment for formula (Ia-1) through (Ia-11a), Q is selected from a 6-membered aryl and a 5- or 6-membered heteroaryl having 1, 2, or 3 heteroatoms independently selected from N, O, and S, wherein the aryl and heteroaryl are optionally substituted by 1, 2, 3, 4, or 5 substituents independently selected from halogen, C₁-C₄ halogenoalkyl, and C₁-C₄ alkoxy. In another embodiment for formula (Ia-1) through (Ia-11a), Q is selected from a 6-membered aryl optionally substituted by 1, 2, 3, 4, or 5 substituents independently selected from halogen.

In another embodiment for formula (Ia-1) through (Ia-11a), Q is selected from:

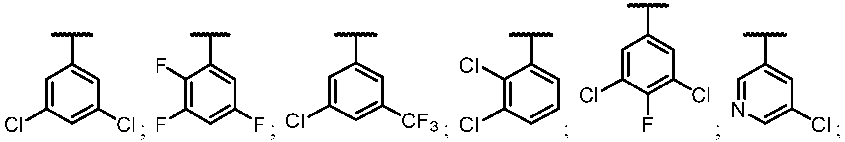

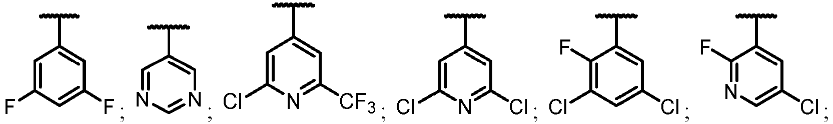

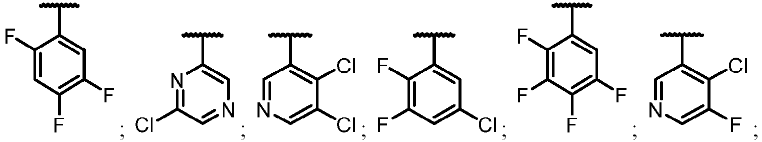

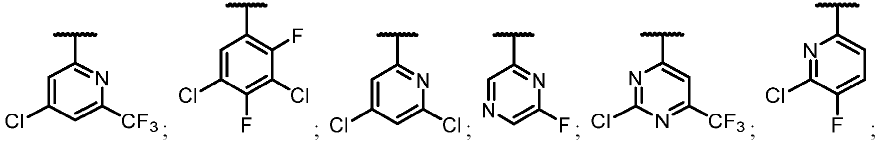

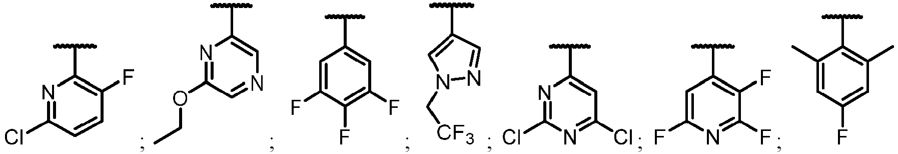

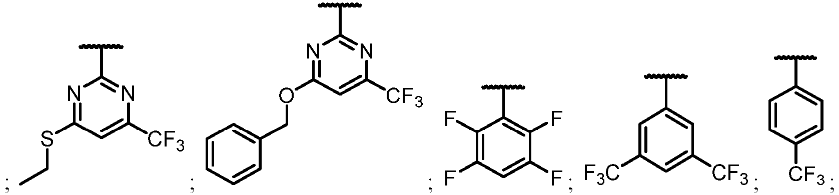

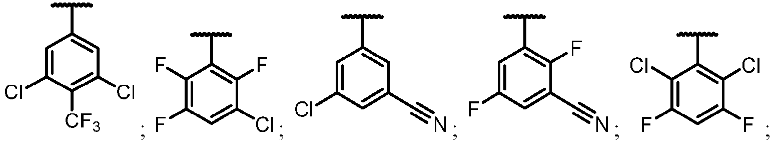

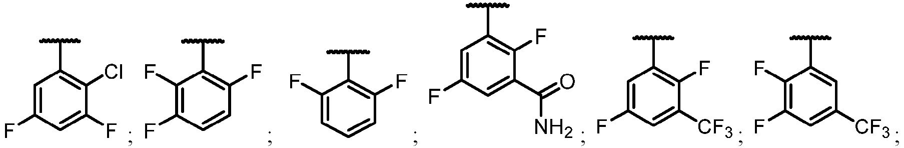

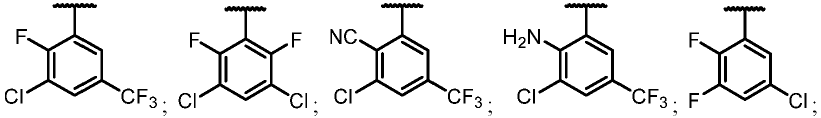

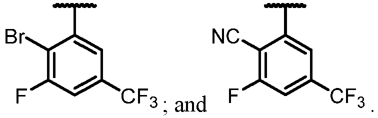

In another embodiment for formula (Ia-1) through (Ia-11a), Q is selected from:

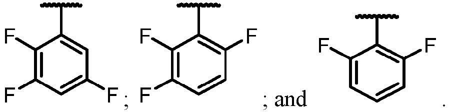

In another embodiment for formula (Ia-1) through (Ia-11a),
A₁, A₂, A₃, A₄, A₅, B₁, B₂, B₃, B₄, B₅, X₁, X₂, Y₁, Y₂, Z₁, Z₂, Z₃, Z₄, R_{B2}, R_{B5}, and n, when present, are as defined in the Summary;
R_{A1}, R_{A2}, R_{A3}, when present, are each independently selected from hydrogen and methyl;
R_{B1}, when present, is selected from: 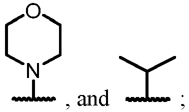
R_{B4}, when present, is selected from: 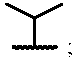
R₇, when present, is hydrogen; and
Q is selected from: 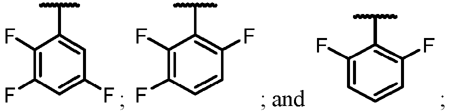
or a salt thereof.

The compounds of the invention can be prepared by a variety of procedures, some of which are described below. All substituents, unless otherwise indicated, are as previously defined.

The products of each step can be recovered by conventional methods including extraction, evaporation, precipitation, chromatography, filtration, trituration, crystallization, and the like. The procedures may require protection of certain groups, for example hydroxyl, thiol, amino, or carboxyl groups to minimize unwanted reactions. The selection, use, and removal of protecting groups are well known and appreciated as standard practice, for example T.W. Greene and P. G. M. Wuts in Protective Groups in Organic Chemistry (John Wiley and Sons, 1991).

As used herein: AcOH refers to acetic acid; aq. refers to aqueous, br refers to broad, CH₃CN refers to acetonitrile, CH₂Cl₂ refers to methylene chloride, d refers to doublet, dd refers to doublet of doublet, DIPEA refers to N-diisopropylethylamine, DMA refers to N,N-dimethylacetamide, DMF refers to N,N-dimethylformamide, DMSO refers to dimethylsulfoxide, ee: refers to enantiomeric excess, eq. refers to equivalent, ES refers to electrospray ionization, EtOAc refers to ethyl acetate, EtOH refers to ethanol, h refers to hour(s), H₂O refers to water, HATU refers to 1-[Bis(dimethylamino)methylene]-1*H*-1,2,3-triazolo[4,5-*b*]pyridinium 3-oxid hexafluorophosphate, HPLC refers to high performance liquid chromatography, iPrOH refers to isopropanol, J refers to coupling constant, KOAc refers to potassium acetate, K₂CO₃ refers to potassium carbonate, LCMS refers to liquid chromatography - mass spectrometry, m/z: refers to mass-to-charge ratio, M refers to molarity, m refers to multiplet, MeOH refers to methanol, min refers to minutes, NaHCO₃ refers to sodium bicarbonate, Na₂CO₃ refers to sodium carbonate, NEt₃ refers to triethylamine, NMR refers to nuclear magnetic resonance, NMP refers to N-methylpyrrolidone, PE refers to petroleum ether, PEG refers to polethyleneglycol, q refers to quartet, quint. refers to quintet, rt refers to room temperature, Rₜ refers to retention time, s refers to singlet, sat. refers to saturated, T refers to temperature, t refers to triplet, td refers to triplet of doublets, THF refers to tetrahydrofuran, wt refers to weight, and □ refers to chemical shift.

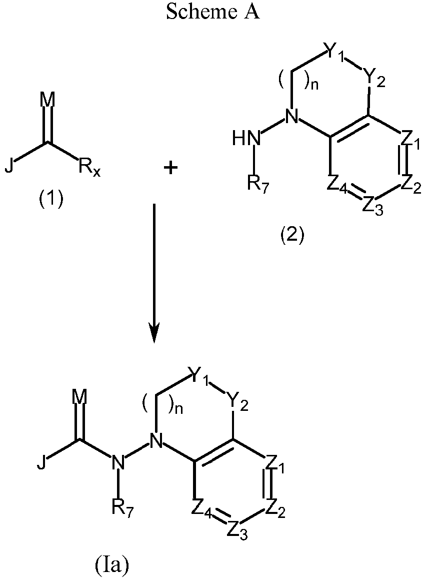

Scheme A depicts the reaction of a compound of formula (1) and a compound of formula (2) to give a compound of formula (Ia). The depicted compound of formula (1) is one in which the group Rₓ is a hydroxyl group, or an activating group as is discussed below, and J and M are as desired in the final compound of formula (Ia) or a group that gives rise to J and M as desired in the final compound of formula (Ia). The preparation of such compounds of formula (1) is readily appreciated in the art. A compound of formula (2) is one in which R₇, n, Y₁, Y₂, Z₁, Z₂, Z₃, and Z₄ are as desired in the final product of formula (Ia) or a group that gives rise to R₇, Y₁, Y₂, Z₁, Z₂, Z₃, and Z₄ as desired in the final product of formula (Ia). The preparation of such compounds of formula (2) is readily appreciated in the art.

As mentioned above, Scheme A depicts the reaction of a compound of formula (1) using a compound of formula (2) to give a compound of formula (Ia). Typical groups Rₓ are hydroxyl or a leaving group, such as chloro, bromo, or imidazolyl, an activating moiety, a mixed anhydride of another carboxylic acid, such as formic acid, acetic acid, or represents the other part of a symmetrical anhydride formed from two compounds of formula (1). For example, standard amide forming conditions can be used, such as those using coupling agents, including those used in peptide couplings, such as 2-(1H-7-azabenzotriazol-1-yl)- 1,1,3,3-tetramethyl uronium hexafluorophosphate methanaminium (HATU), dicyclohexylcarbodiimide (DCC), and 1-(3-dimethylaminopropyl)-3-ethylcarbodiimide-HCl. If necessary or desired, an additive such as 4-(dimethylamino)pyridine, 1-hydroxybenzotriazole, and the like may be used to facilitate the reaction. Such reactions are generally carried out using a base, such as N-methylmorpholine or NEt₃, in a wide variety of suitable solvents such as CH₂Cl₂, DMF, NMP, DMA, THF, and the like. Such reactions are well understood and appreciated in the art. As is well known, a compound of (Ia) in which M is O can be further elaborated to a compound in which M is S or in which M is NR₁₃.

It will be recognized by one of ordinary skill in the art that a compound of formula (Ia) can be elaborated in a variety of ways to give other compounds of formula (Ia). Such reactions include hydrolysis, oxidation, reduction, alkylation, arylation (including heteroaryl groups) amidations, sulfonations, and the like.

Also, in an optional step, not shown, the compounds of formula (Ia) can be converted to salts by methods well known and appreciated in the art.

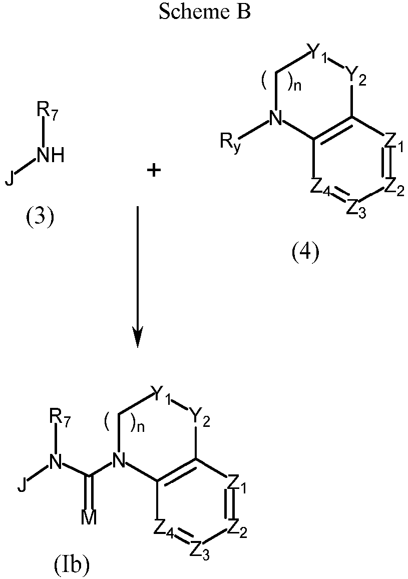

Scheme B depicts the reaction of a compound of formula (3) and a compound of formula (4) to give a compound of formula (Ib). In the depicted compound of formula (3), J and R₇ are as desired in the final compound of formula (Ib) or a group that gives rise to J and R₇ as desired in the final compound of formula (Ib). The preparation of such compounds of formula (3) is readily appreciated in the art. A compound of formula (4) is one in which the group R_{y} is a carboxy group, or an activating group as is discussed below, and n, Y₁, Y₂, Z₁, Z₂, Z₃, and Z₄ are as desired in the final product of formula (Ib) or a group that gives rise to Y₁, Y₂, Z₁, Z₂, Z₃, and Z₄ as desired in the final product of formula (Ib). The preparation of such compounds of formula (4) is readily appreciated in the art.

As mentioned above, Scheme B depicts the reaction of a compound of formula (3) in which using a compound of formula (4) to give a compound of formula (Ib). Typical groups R_{y} are carboxy or an acid chloride or acid bromide, or imidazide, an activating moiety, a mixed anhydride of another carboxylic acid, such as formic acid, acetic acid, or represents the other part of a symmetrical anhydride formed from two compounds of formula (4) in which R_{y} is carboxy derivative or another activated moiety. Such reactions are generally carried out using a base, such as N-methylmorpholine or triethylamine, in a wide variety of suitable solvents such as CH₂Cl₂, DMF, N-methylpyrrolidone (NMP), DMA, THF, and the like. As is well known, a compound of (Ib) in which M is O can be further elaborated to a compound in which M is S or in which M is NR₁₃.

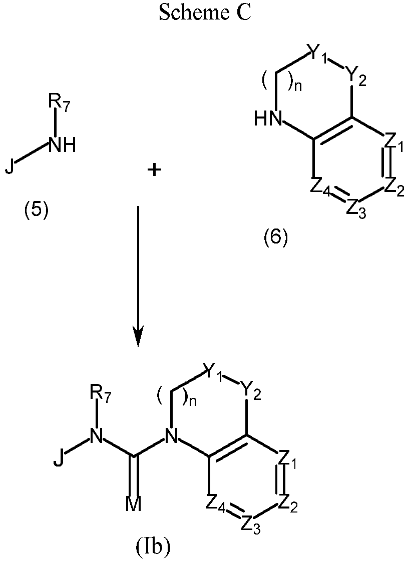

Scheme C depicts the reaction of a compound of formula (5) and a compound of formula (6) to give a compound of formula (Ib). The depicted compound of formula (5) is the same as the a compound of formula (3) described in Scheme B. A compound of formula (6) is one in which is one in which the depicted n, Y₁, Y₂, Z₁, Z₂, Z₃, and Z₄ are as desired in the final product of formula (Ib) or a group that gives rise to the depicted Y₁, Y₂, Z₁, Z₂, Z₃, and Z₄ as desired in the final product of formula (Ib). The preparation of such compounds of formula (6) is readily appreciated in the art. The formation of unsymmetrical ureas is well known using phosgene, carbonyldiimidazole, isopropenyl carbamates, and optionally substituted phenoxy carbonyl halides, such as p-nitrophenoxycarbonyl chloride.

Such reactions are generally carried out in a sequential manner by adding phosgene, carbonyldiimidazole, isopropenyl carbamates, and optionally substituted phenoxycarbonyl halides to either a compound of formula (5) or a compound of formula (6) using a base, such as N-methylmorpholine or triethylamine, in a wide variety of suitable solvents such as CH₂Cl₂, DMF, N-methylpyrrolidone (NMP), DMA, THF, and the like. Then the other of compound (5) or compound (6) is added.

It will be recognized by one of ordinary skill in the art that in Schemes B and C a compound of formula (Ib) can be elaborated in a variety of ways to give other compounds of formula (Ib). Such reactions include hydrolysis, oxidation, reduction, alkylation, arylation (including heteroaryl groups) amidations, sulfonations, and the like. As is well known, a compound of (Ib) in which M is O can be further elaborated to compound in M is S or in which M is NR₁₃.

Also, in an optional step, not shown, the compounds of formula (Ib) can be converted to salts by methods well known and appreciated in the art.

The following examples are intended to be illustrative and non-limiting, and represent specific embodiments of the present invention.

Method A: Analyses were carried out on a Waters XBridge BEH C18 of 50 mm length, 2.1 mm internal diameter and 2.5 µm particle size. The mobile phase used was: A1= Water with 0.1% formic acid / B1= CH₃CN with 0.1% formic acid. The run was performed at a temperature of 40 °C and a flow rate of 0.6 mL/min, with a gradient elution from 5% to 95% (B1) over 1.5 min followed by a *0.5* min hold at 95% (B1).

Method B1: Analyses were carried out on a SHIMADZU LCMS - UFLC 20-AD - LCMS 2020 MS detector; Column: CORTECS C18 2.7 µm, 50 x 2.1 mm; eluent A: H₂O + 0.1 vol % formic acid, eluent B: CH₃CN + 0.10 vol % formic acid; gradient: assigned for each compound; flow 1.2 mL/min; temperature: 40°C; PDA scan: 190 - 400 nm.

Method B2: Analyses were carried out on a SHIMADZU LCMS - UFLC 20-AD - LCMS 2020 MS detector; Column: Ascentis Express C18 2.7 µm, 50 x 3.0 mm; eluent A: H₂O + 0.05 vol % trifluoroacetic acid, eluent B: CH₃CN + 0.05 vol % trifluoroacetic acid; gradient: assigned for each compound; flow 1.5 mL/min; temperature: 40°C; PDA scan: 190 - 400 nm.

Method C: Analyses were carried out on a SHIMADZU LCMS - UFLC 20-AD - LCMS 2020 MS detector; Column: Infinity Lab Poroshell HPH-C18 2.7 µm, 50 x 3.0 mm; eluent A: H₂O + 0.05 vol % ammonium hydrogenocarbonate, eluent B: CH₃CN; gradient: assigned for each compound; flow 1.2 mL/min; temperature: 40°C; PDA scan: 190 - 400 nm.

Method D: Analyses were carried out on an Acquity UPLC BEH C18 column of 50 mm length, 2.1 mm internal diameter and 1.7 µm particle size. The mobile phase used was: A1= Water with 10 mM Ammonium acetate / B1= CH₃CN with 0.1% formic acid. The injection volume was 0.1µL. The run was performed at a temperature of 45 °C and a flow rate of 0.5 mL/min, with a gradient elution. Method info (Time (min) and A %): 0-98; 0.3-98; 3.2-2; 4.4-2; 4.7-98.

Method E1: Analyses were carried out on an SHIMADZU LCMS - UFLC 20-AD - LCMS 2020 MS detector; Column: Kinetex EVO C18 2.6 µm, 50x3.0 mm; eluent A: H₂O + 0.05 vol % ammonium hydrogenocarbonate, eluent B: CH₃CN; gradient: assigned for each compound; flow 1.5 mL/min; temperature: 40°C; PDA scan: 190 - 400 nm.

Method E2: Analyses were carried out on an SHIMADZU LCMS - UFLC 20-AD - LCMS 2020 MS detector; Column: Luna Omega 3.0 µm, 50 x 3.0 mm; eluent A: H₂O + 0.1 vol % Formic acid, eluent B: CH₃CN + 0.1 vol % Formic acid; gradient: assigned for each compound; flow 1.2 mL/min; temperature: 40°C; PDA scan: 190 - 400 nm.

Method F1: Analyses were carried out on an SHIMADZU LCMS - UFLC 20-AD - LCMS 2020 MS detector; Column: Cortecs-C18 2.7 µm, 50x2.1 mm; eluent A: H₂O + 0.09 vol % formic acid, eluent B: CH₃CN + 0.1 vol % formic acid; gradient: assigned for each compound; flow 1.0 mL/min; temperature: 40°C; PDA scan: 190 - 400 nm.

Method F2: Analyses were carried out on an SHIMADZU LCMS - UFLC 20-AD - LCMS 2020 MS detector; Column: Ascentis Express C18 2.7 µm, 50 x 3.0 mm; eluent A: H₂O + 0.05 vol % trifluoroacetic acid, eluent B: CH₃CN + 0.05 vol % trifluoroacetic acid; gradient: assigned for each compound; flow 1.2 mL/min; temperature: 40°C; PDA scan: 190 - 400 nm

Method G1: Analyses were carried out on an SHIMADZU LCMS - UFLC 20-AD - LCMS 2020 MS detector; Column: Kinetex EVO C18 2.6 µm, 50 x 3.0 mm; eluent A: H₂O + 0.05 vol % trifluoroacetic acid, eluent B: CH₃CN + 0.05 vol % trifluoroacetic acid; gradient: assigned for each compound; flow 1.2 mL/min; temperature: 40°C; PDA scan: 190 - 400 nm.

Method G2: SHIMADZU LCMS - UFLC 20-AD - LCMS 2020 MS detector; Column: Shim-pack XR-ODS, 2.2 µm, 3.0 × 50 mm; eluent A: H₂O + 0.05 vol % trifluoroacetic acid, eluent B: CH₃CN *+* 0.05 vol % trifluoroacetic acid; gradient: assigned for each compound; flow 1.5 mL/min; temperature: 40°C; PDA scan: 190 - 400 nm.

### Example 1.1

### N-(2,3-dihydro-1,4-benzoxazin-4-yl)-3-isopropyl-7-(2,3,5-trifluorophenyl)benzo-thiophene-2-carboxamide

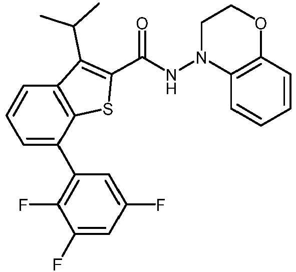

### Example 1.1(a)

### 1-(3-Bromo-2-fluoro-phenyl)-2-methyl-propan-1-one

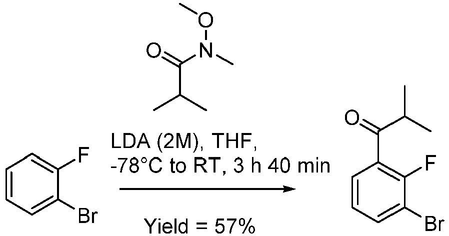

To a stirred solution of 1-bromo-2-fluoro-benzene (5 g, 28 mmol) in abs. THF (50 mL), LDA (21.4 mL, 2 M in abs. THF) was added dropwise at -78°C and the reaction mixture was stirred for 3 h at same temperature. After adding N-methoxy-N,2-dimethyl-propanamide (3.7 g, 28.6 mmol), the resulting reaction mixture was stirred at -78°C for 40 min. The reaction mixture was quenched by addition of saturated ammonium chloride solution (50 mL) and extracted with EtOAc (2 x 50 mL). The combined organic layers were washed with brine (50 mL), dried over anhydrous Na₂SO₄ and concentrated under reduced pressure. The crude compound was purified by silica gel column chromatography, eluting with 10% EtOAc in PE to obtain 1-(3-bromo-2-fluoro-phenyl)-2-methyl-propan-1-one.

### Example 1.1(b)

### Ethyl 7-bromo-3-isopropyl-benzothiophene-2-carboxylate

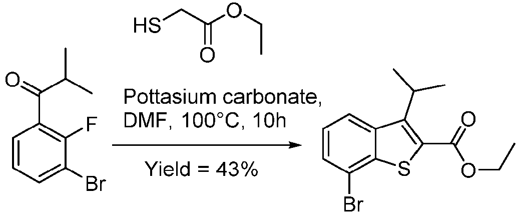

To a stirred solution of 1-(3-bromo-2-fluoro-phenyl)-2-methyl-propan-1-one (3.5 g, 14.3 mmol) and ethyl 2-sulfanylacetate (1.5 g, 12.4 mmol) in DMF (25 mL), potassium carbonate (5.9 g, 42.8 mmol) was added and the resulting reaction mixture was stirred at 100 °C for 10 h. The reaction mixture was quenched by addition of water (30 mL) and extracted with EtOAc (2 x 50 mL). The combined organic layers were washed with brine (30 mL), dried over anhydrous Na₂SO₄ and concentrated under reduced pressure. The crude compound was purified by silica gel column chromatography eluting with 20% EtOAc in PE to obtain ethyl 7-bromo-3-isopropyl-benzothiophene-2-carboxylate.

### Example 1.1(c)

### Ethyl 3-isopropyl-7-(2,3,5-trifluorophenyl)benzothiophene-2-carboxylate

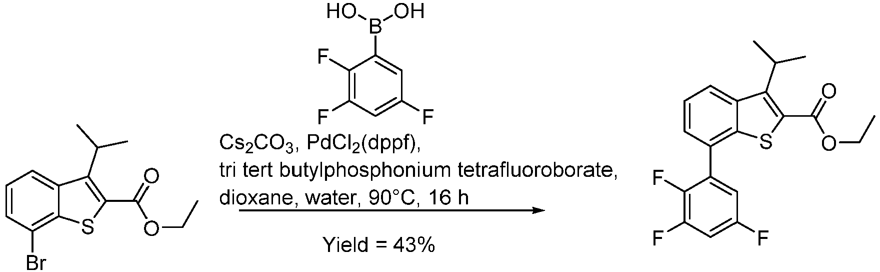

To a stirred solution of ethyl 7-bromo-3-isopropyl-benzothiophene-2-carboxylate (2 g, 6.13 mmol) and (2,3,5-trifluorophenyl)boronic acid (4.32 g, 24.5 mmol) in dioxane (40 mL)-water (10 mL) mixture, Cs₂CO₃ (6 g, 18.4 mmol) was added and the reaction mixture was degassed using N₂ for 10 min. Subsequently, PdCl₂(dppf) (448 mg, 0.613 mmol) and tri tert butylphosphonium tetrafluoroborate (355 mg, 1.23 mmol) were added and the resulting reaction mixture was stirred at 90 °C for 16 h. The reaction mixture was quenched by adding water (20 mL) and extracted with EtOAc (3 x 20 mL). The combined organic layers were washed with brine (15 mL), dried over anhydrous Na₂SO₄ and concentrated under reduced pressure. The crude compound was purified by silica gel column chromatography eluting with 20% EtOAc in PE to obtain ethyl 3-isopropyl-7-(2,3,5-trifluorophenyl)benzothiophene-2-carboxylate.

### Example 1.1(d)

### 3-Isopropyl-7-(2,3,5-trifluorophenyl)benzothiophene-2-carboxylic acid

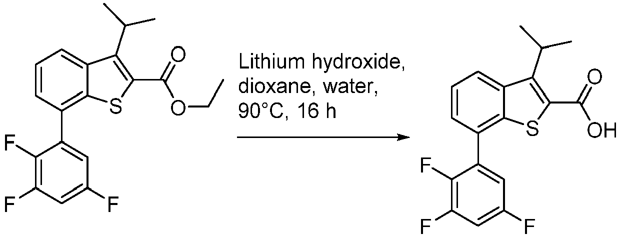

To a stirred solution of ethyl 3-isopropyl-7-(2,3,5-trifluorophenyl)benzothiophene-2-carboxylate (0.5 g, 1.32 mmol) in water (10 mL) and dioxane (10 mL), LiOH (166 mg, 3.967 mmol) was added. The reaction mixture was stirred for 16 h at 90 °C. The reaction mixture was quenched by adding HCl (5.0 mL, 2 N) and extracted with 5% methanol in CH₂Cl₂ solution (2 x 15 mL). The combined organic layers were washed with brine (15 mL), dried over anhydrous Na₂SO₄ and concentrated under reduced pressure. The product was purified by trituration with diethyl ether and pentane solution (1:1).

### Example 1.1(e)

### N-(2,3-Dihydro-1,4-benzoxazin-4-yl)-3-isopropyl-7-(2,3,5-trifluorophenyl)benzothiophene-2-carboxamide

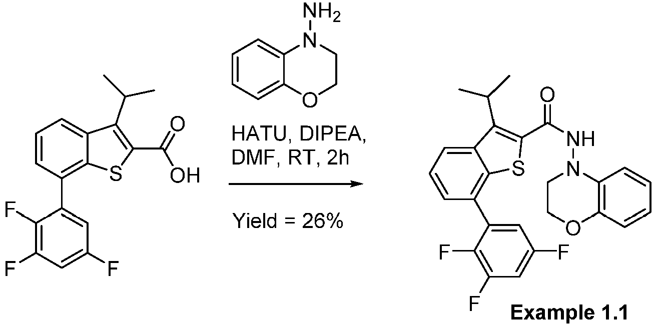

To a stirred solution of 3-isopropyl-7-(2,3,5-trifluorophenyl)benzothiophene-2-carboxylic acid (377 mg, 1.08 mmol) and 2,3-dihydro-1,4-benzoxazin-4-amine (242 mg, 1.62 mmol) in DMF (10.0 mL), HATU (613 mg, 1.615 mmol) was added and the reaction mixture was stirred at rt for 5 min. Subsequently, DIPEA (416 mg, 3.23 mmol) was added and the resulting reaction mixture was stirred for 3 h at rt. The reaction mixture was quenched by adding water (10 mL) and extracted with EtOAc (2 x 10 mL). The combined organic layers were washed with brine (15 mL), dried over anhydrous Na₂SO₄ and concentrated under reduced pressure. The crude compound was purified by reverse phase prep-HPLC to obtain title compound. ¹H NMR (400 MHz, DMSO) δ [ppm]: 10.52 (s, 1 H), 8.23 (d, J= 8 Hz, 1 H), 7.71-7.75 (m, 1 H), 7.63 (t, J= 7.6 Hz, 1 H), 7.56 (m, 1 H), 7.44 (br s, 1 H), 6.69-6.82 (m, 4 H), 4.33 (br s, 2 H), 3.81-3.87 (m, 1 H), 3.59 (br s, 2 H), 1.47 (d, J= 4 Hz, 6 H). LCMS (method D): Rₜ= 2.61 min, *m*/*z=* 481.20 [M+H]⁻.

### Example 2.1

### N-(2,3-Dihydro-1,4-benzoxazin-4-yl)-3-morpholino-7-(2,3,5-trifluorophenyl)thieno-[2,3-c]pyridine-2-carboxamide

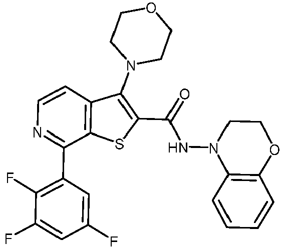

### Example 2.1(a)

### Ethyl 3-chloropyridine-4-carboxylate

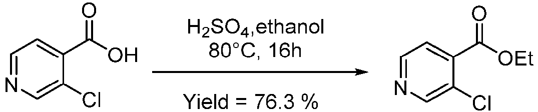

To a stirred solution of 3-chloropyridine-4-carboxylic acid (10 g, 63.5 mmol) in ethanol (100 mL) was added H₂SO₄ (10 mL) at 0°C and the resulting reaction mixture was heated at 80°C for 16 h. The reaction mixture was quenched by adding aq. sat. NaHCO₃ (500 mL) and extracted with EtOAc (3 x 100 mL). The combined organic layers were washed with brine (100 mL), dried over anhydrous Na₂SO₄ and concentrated under reduced pressure to afford ethyl 3-chloropyridine-4-carboxylate

### Example 2.1(b)

### Ethyl 3-hydroxythieno[2,3-c]pyridine-2-carboxylate

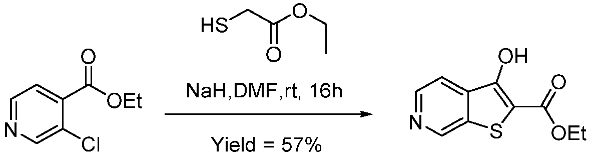

To a stirred solution of ethyl 3-chloropyridine-4-carboxylate (9.00 g, 48.5 mmol) and ethyl 2-sulfanylacetate (11.6 g, 97.0 mmol) in DMF (60 mL) was added NaH (2.87 g, 120 mmol) at 0°C and the resulting reaction mixture was stirred at rt for 16 h. The reaction mixture was quenched by adding acetic acid (300 mL) and extracted with EtOAc (2 x 150 mL). The combined organic layers were washed with brine (50 mL), dried over anhydrous Na₂SO₄ and concentrated *in vacuo*to afford ethyl 3-hydroxythieno[2,3-c]pyridine-2-carboxylate.

### Example 2.1(c)

### Ethyl 3-(trifluoromethylsulfonyloxy)thieno[2,3-c]pyridine-2-carboxylate

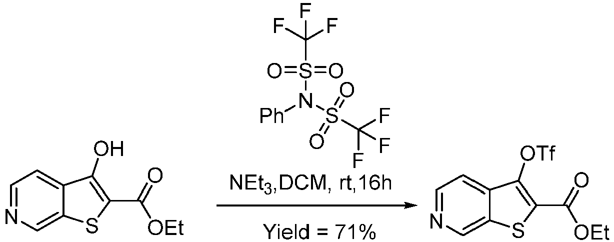

To a stirred solution of ethyl 3-hydroxythieno[2,3-c]pyridine-2-carboxylate (6.2 g, 28 mmol) and 1,1,1-trifluoro-N-phenyl-N-(trifluoromethylsulfonyl)methanesulfonamide (10.0 g, 28 mmol) in CH₂Cl₂ (60 mL) was added NEt₃ (6.83 g, 67.5 mmol) at rt. The reaction mixture was stirred at rt for 16 h. The reaction mixture was quenched by adding water (300 mL) and extracted with CH₂Cl₂ (2 x 50 mL). The combined organic layers were washed with brine (50 mL), dried over anhydrous Na₂SO₄ and concentrated under reduced pressure to afford the crude product. The crude compound was purified by silica gel column chromatography eluting with 0-5% EtOAc in PE to obtain ethyl 3-(trifluoromethylsulfonyloxy)thieno[2,3-c]pyridine-2-carboxylate.

### Example 2.1(d)

### Ethyl 3-morpholinothieno[2,3-c]pyridine-2-carboxylate

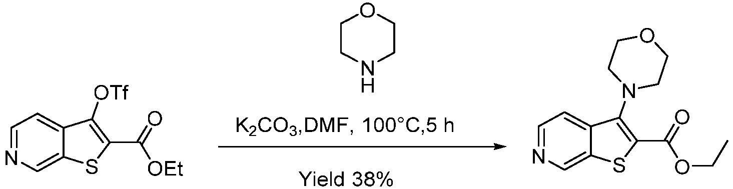

To a stirred solution of ethyl 3-(trifluoromethylsulfonyloxy)thieno[2,3-c]pyridine-2-carboxylate (3.5 g, 9.9 mmol) and morpholine (0.98 g, 11.3 mmol) in DMF (20 mL) was added K₂CO₃ (4.05 g, 29.3 mmol) at rt. The reaction mixture was heated at 100°C for 5 h. The reaction mixture was quenched by adding water (200 mL) and extracted with EtOAc (2 x 50 mL). The combined organic layers were washed with brine (40 mL), dried over anhydrous Na₂SO₄ and concentrated under reduced pressure. The crude compound was purified by silica gel column chromatography eluting with 0-26% EtOAc in PE to obtain ethyl 3-morpholinothieno[2,3-c]pyridine-2-carboxylate.

### Example 2.1(e)

### Ethyl 7-iodo-3-morpholino-thieno[2,3-c]pyridine-2-carboxylate

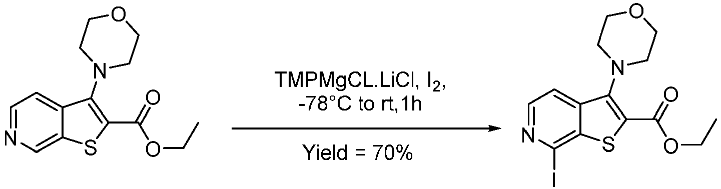

To a stirred solution of ethyl 3-morpholinothieno[2,3-c]pyridine-2-carboxylate (0.9 g, 3.08 mmol) in abs. THF (15mL) under nitrogen atmosphere at -78°C, was added TMPMgCl•LiC1 (1 M in abs. THF; 12.3 mL, 12.3 mmol) and the reaction mixture allowed to stir for 15 min while warming up to rt. The reaction mixture was again cooled to -78°C and a solution of iodine (dissolved in 15mL abs. THF, 3.9 g, 15.4 mmol) was added. The resulting solution was stirred at -78°C for 30 min. The reaction mixture was quenched by adding aq. sat. Na₂S₂O₃-solution (100 mL) and extracted with EtOAc (3 x 30 mL). The combined organic layers were washed with brine (35 mL), dried over anhydrous Na₂SO₄ and concentrated under reduced pressure to obtain the crude compound. The crude compound was purified by silica gel column chromatography eluting with 0-10% EtOAc in PE to obtain ethyl 7-iodo-3-morpholino-thieno[2,3-c]pyridine-2-carboxylate.

### Example 2.1(f)

### Ethyl 3-morpholino-7-(2,3,5-trifluorophenyl)thieno[2,3-c]pyridine-2-carboxylate

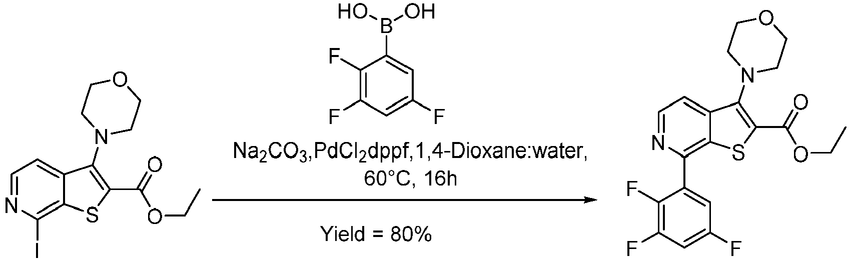

To a stirred solution of ethyl 7-iodo-3-morpholino-thieno[2,3-c]pyridine-2-carboxylate (0.7 g, 1.6 mmol) and (2,3,5-trifluorophenyl)boronic acid (589 mg, 3.34 mmol) in 1,4-dioxane (12 mL):water (3 mL) was added Na₂CO₃ (508 mg, 4.8 mmol). The reaction mixture was degassed with nitrogen gas for 10 min followed by the addition of PdCl₂(dppf) (234 mg, 0.32 mmol); the reaction mixture was heated to 60°C for 16 h. The reaction mixture was quenched by adding water (100 mL) and extracted with EtOAc (2 x 50 mL). The combined organic layers were washed with brine (40 mL), dried over anhydrous Na₂SO₄ and concentrated under reduced pressure. The crude compound was purified by silica gel column chromatography eluting with 12% EtOAc in PE to obtain ethyl 3-morpholino-7-(2,3,5-trifluorophenyl)thieno[2,3-c]pyridine-2-carboxylate.

### Example 2.1(g)

### 3-Morpholino-7-(2,3,5-trifluorophenyl)thieno[2,3-c]pyridine-2-carboxylic acid

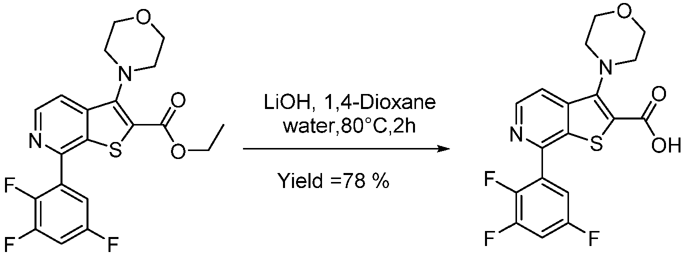

To a stirred solution of ethyl 3-morpholino-7-(2,3,5-trifluorophenyl)thieno[2,3-c]pyridine-2-carboxylate (0.45 g, 1.1 mmol) in 1,4-dioxane (5 mL) was added LiOH solution in water (5 mL, 138 mg, 3.3 mmol) at rt. The reaction mixture was heated to 80°C. The reaction mixture was cooled to rt and concentrated to remove solvents. The pH of the reaction mixture was adjusted to 2-3 by addition of aq. HCl (1 M) solution, extracted with EtOAc (2 x 50mL), washed with water (10 mL) and dried over anhydrous Na₂SO₄ and reduced to dryness under reduced pressure to afford 3-morpholino-7-(2,3,5-trifluorophenyl)thieno[2,3-c]pyridine-2-carboxylic acid.

### Example 2.1(h)

### N-(2,3-Dihydro-1,4-benzoxazin-4-yl)-3-morpholino-7-(2,3,5-trifluorophenyl)thieno[2,3-c]pyridine-2-carboxamide

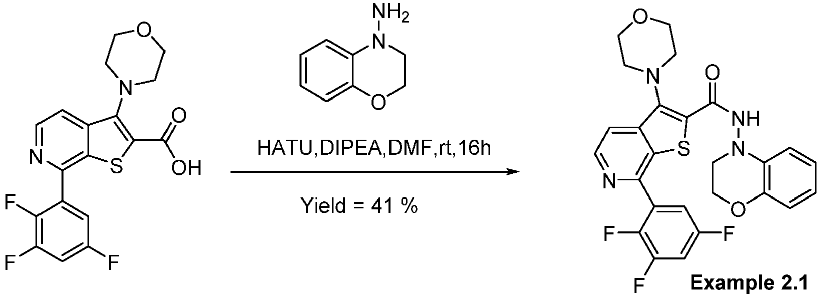

A solution of 3-morpholino-7-(2,3,5-trifluorophenyl)thieno[2,3-c]pyridine-2-carboxylic acid (0.33 g, 0.83 mmol), 2,3-dihydro-1,4-benzoxazin-4-amine (188 mg, 1.25 mmol) and HATU (473 mg, 1.25 mmol) in DMF (5 mL) was stirred for 15 min. Subsequently, DIPEA (321 mg, 2.49 mmol) was added and the reaction mixture was stirred at rt for 5 h. The reaction mixture was quenched by adding water (100 mL) and extracted with EtOAc (3 x 25 mL). The combined organic layers were washed with brine (30 mL), dried over anhydrous Na₂SO₄ and concentrated under reduced pressure. The crude compound was purified by silica gel column chromatography eluting with 0-20% EtOAc in PE to obtain N-(2,3-dihydro-1,4-benzoxazin-4-yl)-3-morpholino-7-(2,3,5-trifluorophenyl)thieno[2,3-c]pyridine-2-carboxamide. ¹H NMR (400 MHz, DMSO) δ [ppm]: 10.96 (s, 1 H), 8.70 (d, J= 5.6 Hz, 1 H), 8.09 (d, J= 5.6 Hz, 1 H), 7.80-7.86 (m, 1 H), 7.50 (br s, 1 H), 6.71-6.84 (m, 4 H), 4.38 (br s, 2 H), 3.80 (br s, 4 H), 3.62 (br s, 2 H), 3.33 (br s, 2 H). LCMS (method A): Rₜ= 2.48 min, *m*/*z=* 527.16 [M+H]⁺.

### Example 2.2

### N-(2,3-Dihydro-1,4-benzoxazin-4-yl)-3-isopropyl-7-(2,3,5-trifluorophenyl)-thieno[2,3-c]pyridine-2-carboxamide

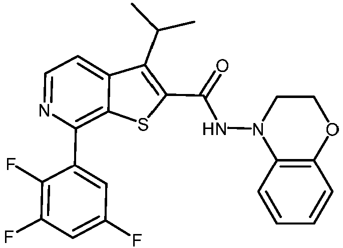

### Example 2.2 (a)

### Ethyl 3-isopropenylthieno[2,3-c]pyridine-2-carboxylate

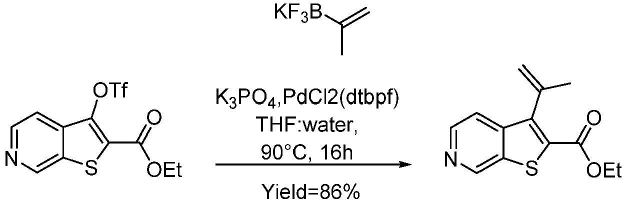

To a stirred solution of compound ethyl 3-(trifluoromethylsulfonyloxy)thieno[2,3-c]pyridine-2-carboxylate - see Example 2.1(c) (3.5 g, 9.85 mmol) and 2-methylprop-1-ene potassium trifluoroborane (1.74 g, 11.82 mmol) in THF (16 mL): water (4 mL) was added K₃PO₄ (4.18 g, 19.7 mmol). The reaction mixture was degassed for 10 min with nitrogen gas followed by the addition of PdCl₂(dtbpf) (319 mg, 0.49 mmol). The reaction mixture was heated to 90°C for 16 h.

The reaction mixture was quenched by adding water (200 mL) and extracted with EtOAc (2 x 50 mL). The combined organic layers were washed with brine (40 mL), dried over anhydrous Na₂SO₄ and concentrated under reduced pressure to obtain crude compound. The crude compound was purified by silica gel column chromatography eluting with 15% EtOAc in PE to obtain ethyl 3-isopropenylthieno[2,3-c]pyridine-2-carboxylate.

### Example 2.2(b)

### Ethyl 3-isopropylthieno[2,3-c]pyridine-2-carboxylate

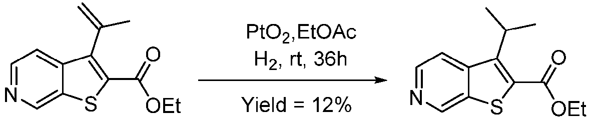

To a solution of ethyl 3-isopropenylthieno[2,3-c]pyridine-2-carboxylate (1.5 g, 6.06 mmol) in EtOAc (15 mL) was added PtO₂ (0.688 g, 3.03 mmol). The reaction mixture was stirred at rt for 36 h under hydrogen atmosphere. The mixture was then filtered through celite and washed with EtOAc (100 mL) and concentrated under reduced pressure to obtain the crude compound. The crude compound was purified by silica gel column chromatography eluting with 5% EtOAc in PE to obtain ethyl 3-isopropylthieno[2,3-c]pyridine-2-carboxylate.

### Example 2.2(c)

### Ethyl 7-iodo-3-isopropyl-thieno[2,3-c]pyridine-2-carboxylate

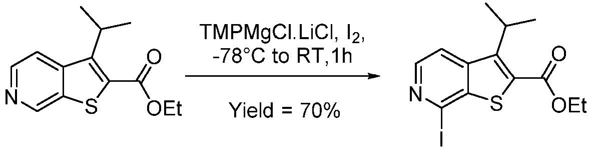

To a stirred solution of ethyl 3-isopropylthieno[2,3-c]pyridine-2-carboxylate (130 mg, 0.52 mmol) in abs. THF (3 mL) under nitrogen atmosphere at -78°C, was added TMPMgCl·LiCl (1 M in abs. THF, 2.08 mL, 2.08 mmol)) and the reaction mixture was allowed to stir for 15 min while warming up to rt. The reaction mixture was again cooled to -78°C and a solution of iodine (0.658 g, 2.6 mmol, dissolved in 3 mL abs. THF) was added. The resulting solution was stirred at -78°C for 30 min. The reaction mixture was quenched by adding aq. sat. Na₂S₂O₃ sodium thiosulfate (100 mL) and extracted with EtOAc (2x 50 mL). The combined organic layers were washed with brine (40mL), dried over anhydrous Na₂SO₄ and concentrated under reduced pressure. The crude compound was purified by silica gel column chromatography eluting with 0-4% EtOAc in petroleum ether to obtain ethyl 7-iodo-3-isopropyl-thieno[2,3-c]pyridine-2-carboxylate.

### Example 2.2(d)

### Ethyl 3-isopropyl-7-(2,3,5-trifluorophenyl)thieno[2,3-c]pyridine-2-carboxylate

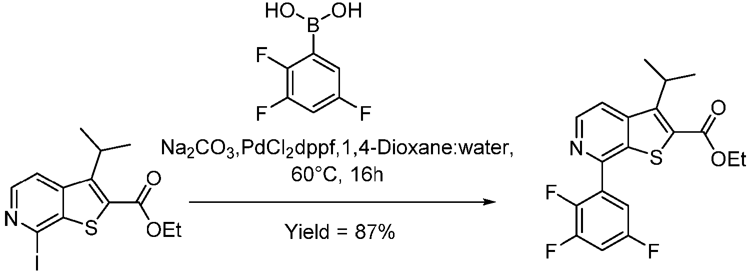

To a stirred solution of ethyl 7-iodo-3-isopropyl-thieno[2,3-c]pyridine-2-carboxylate (0.17 g, 0.45 mmol) and (2,3,5-trifluorophenyl)boronic acid (0.159 g, 0.9 mmol) in 1,4-dioxane (4 mL):water (1 mL) was added Na₂CO₃ (0.143 g, 1.35 mmol). The reaction mixture was degassed with nitrogen gas for 10 min before addition of PdCl₂(dppf) (0.065 g, 0.9 mmol) and then heated to 60°C for 16 h. The reaction mixture was then quenched by adding water (50 mL) and extracted with EtOAc (2 x 50 mL). The combined organic layers were washed with brine (40 mL), dried over anhydrous Na₂SO₄ and concentrated under reduced pressure to obtain crude compound. The crude compound was purified by silica gel column chromatography eluting with 10% EtOAc in PE to obtain ethyl 3-isopropyl-7-(2,3,5-trifluorophenyl)thieno[2,3-c]pyridine-2-carboxylate.

### Example 2.2 (e)

### 3-Isopropyl-7-(2,3,5-trifluorophenyl)thieno[2,3-c]pyridine-2-carboxylic acid

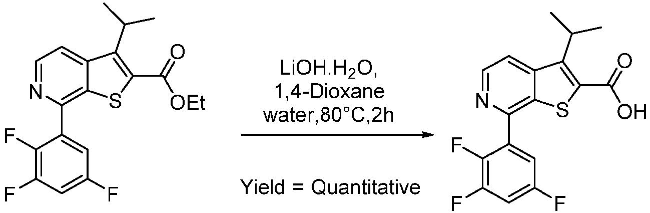

To a stirred solution of ethyl 3-isopropyl-7-(2,3,5-trifluorophenyl)thieno[2,3-c]pyridine-2-carboxylate (0.13 g, 0.34 mmol) in 1,4-dioxane (3 mL) was added a solution of LiOH·H₂O solution (43 mg, 1.02 mmol) in water (3 mL) at rt and the resulting mixture was heated to 80°C for 2h. The reaction mixture was then allowed to cool down to rt and solvents were evaporated under reduced pressure. H₂O was added, the pH was adjusted to 2-3 by addition of aq. HCl (1 M) and the aq. mixture was extracted with EtOAc (2 x 50mL). The combined organic layers were washed with H₂O (10 mL) and dried over anhydrous Na₂SO₄, filtered and evaporated to dryness to afford 3-isopropyl-7-(2,3,5-trifluorophenyl)thieno[2,3-c]pyridine-2-carboxylic acid.

### Example 2.2(f)

### N-(2,3-Dihydro-1,4-benzoxazin-4-yl)-3-morpholino-7-(2,3,5-trifluorophenyl)thieno[2,3-c]pyridine-2-carboxamide

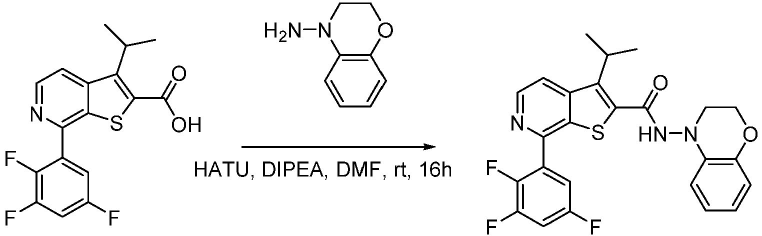

### Example 2.2

A solution of 3-isopropyl-7-(2,3,5-trifluorophenyl)thieno[2,3-c]pyridine-2-carboxylic acid (0.13 g, 0.37 mmol), 2,3-dihydro-1,4-benzoxazin-4-amine (0.083 g, 0.55 mmol) and HATU (0.209 g, 0.55 mmol) in DMF (3 mL) was stirred for 15 min. Subsequently, DIPEA was added and the reaction mixture was stirred at rt. The reaction mixture was quenched by adding water (30 mL) and extracted with EtOAc (3 x 25 mL). The combined organic layers were washed with brine (30 mL), dried over anhydrous Na₂SO₄ and concentrated under reduced pressure. The crude compound was purified by silica gel column chromatography eluting with 0-17% EtOAc in PE to obtain N-(2,3-dihydro-1,4-benzoxazin-4-yl)-3-morpholino-7-(2,3,5-trifluorophenyl)thieno[2,3-c]pyridine-2-carboxamide. ¹H NMR (400 MHz, DMSO) δ [ppm]: 10.8 (s, 1 H), 8.71 (d, J= 5.2 Hz, 1H), 8.20 (d, J= 5.6 Hz, 1 H), 7.78-7.85 (m, 1H), 7.51-7.53 (m, 1H), 6.69-6.87 (m, 4H), 4.34 (br s, 2H), 3.78 (quint, J= 6.8 Hz, 1H), 3.60 (br s, 2H), 1.48 (d, J= 7.2 Hz, 6H). LCMS (method D): Rₜ= 2.93 min, *m*/*z=* 484.16 [M+H]⁺.

### Example 3.1

### 3-(2,6-Difluorophenyl)-N-(2,3-dihydro-1,4-benzoxazin-4-yl)-7-isopropyl-2-methyl-pyrazolo[1,5-a]pyrimidine-6-carboxamide

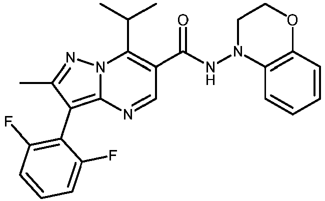

### Example 3.1(a)

3-(2,6-Difluorophenyl)-7-isopropyl-2-methyl-pyrazolo[1,5-a]pyrimidine-6-carboxylic acid was synthetized according to WO2017178416.

### Example 3.1(b)

### 3-(2,6-Difluorophenyl)-N-(2,3-dihydro-1,4-benzoxazin-4-yl)-7-isopropyl-2-methyl-pyrazolo[1,5-a]pyrimidine-6-carboxamide

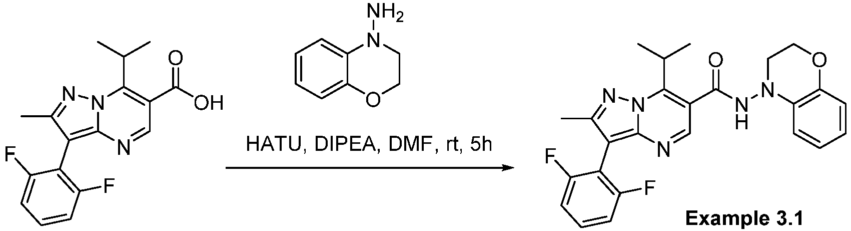

To a stirred solution of 3-(2,6-difluorophenyl)-7-isopropyl-2-methyl-pyrazolo[1,5-a]pyrimidine-6-carboxylic acid (0.12 g, 0.362 mmol), and 2,3-dihydro-1,4-benzoxazin-4-amine (0.065 g, 0.434 mmol), in DMF (5 mL) was added HATU (165 mg, 0.434 mmol) and the resulting mixture was stirred for 5 min, followed by addition of DIPEA (0.2 mL, 1.08 mmol). The reaction mixture was then stirred for 5 h at rt. The reaction was quenched by addition of H₂O (10 mL) and resulting precipitate was collected by filtration, washed with H₂O (20 mL) and dried *in vacuo* to get crude compound. The crude compound was purified by silica gel column chromatography eluting with 10-30 % EtOAc in PE to obtain 3-(2,6-difluorophenyl)-N-(2,3-dihydro-1,4-benzoxazin-4-yl)-7-isopropyl-2-methyl-pyrazolo[1,5-a]pyrimidine-6-carboxamide. ¹H NMR (400 MHz, DMSO) δ [ppm]: 10.71 (s, 1 H), 8.63 (s, 1 H), 7.52-7.60 (m, 1 H), 7.27 (t, J= 8 Hz, 1 H), 7.02 (d, J= 6.8 Hz, 1 H), 6.76-6.93 (m, 3 H), 4.37 (t, J= 4 Hz,2 H), 3.90-3.97 (m, 1 H), 3.66 (br s, 2 H), 2.36 (s, 3 H), 1.59 (d, J= 7.2 Hz, 6 H). LCMS (method D): Rₜ= 2.89 min, *m*/*z=* 462.14 [M+H]⁻.

### Example 4.1

### N-(2,3-Dihydro-1,4-benzoxazin-4-yl)-3-isopropyl-6-methyl-7-(2,3,5-trifluorophenyl)-pyrazolo[5,1-b]thiazole-2-carboxamide

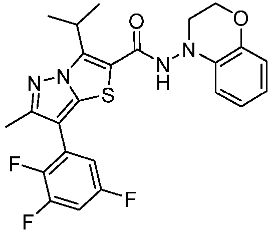

### Example 4.1(a)

### Ethyl 2-chloro-4-methyl-3-oxo-pentanoate

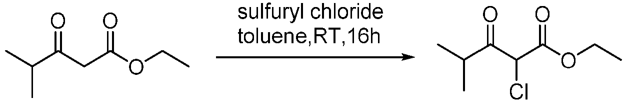

To an ice cooled stirred solution of sulfuryl chloride (2.55 g, 19.0 mmol) in toluene (10 mL) was added drop wise a solution of ethyl 4-methyl-3-oxo-pentanoate (3.00 g, 18.96 mmol) in toluene (10 mL) at 0°C and the resulting mixture was then allowed to stir at rt for 16h. The reaction mixture was quenched by adding aq. sat. NaHCO₃ solution (100 mL) and the aq. layer was extracted with EtOAc (2 x 50 mL). The combined organic layers were washed with brine (30 mL), dried over anhydrous Na₂SO₄ and concentrated under reduced pressure to afford ethyl 2-chloro-4-methyl-3-oxo-pentanoate.

### Example 4.1(b)

### 3-methyl-1,4-dihydropyrazole-5-thione

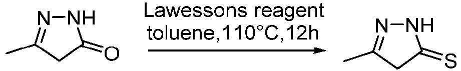

To a stirred solution of 3-methyl-1,4-dihydropyrazol-5-one (3 g, 30.58 mmol) in toluene (75 mL) was added Lawessons reagent (6.18 g, 15.29 mmol) at rt and the resulting mixture was stirred at 110°C for 12 h. A white precipitate was obtained upon cooling the reaction mixture was cooled to rt. The precipitate was filtered off, washed with EtOAc (50 mL) and dried *in vacuo* to afford 3-methyl-1,4-dihydropyrazole-5-thione.

### Example 4.1(c)

### Ethyl 3-isopropyl-6-methyl-pyrazolo[5,1-b]thiazole-2-carboxylate

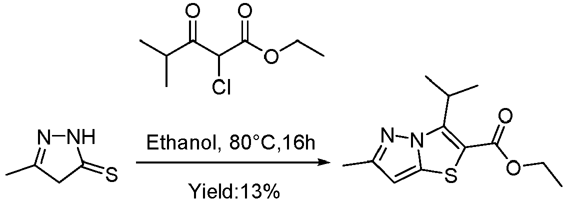

A mixture of ethyl 2-chloro-4-methyl-3-oxo-pentanoate (1.00 g, 5.19 mmol) and 3-methyl-1,4-dihydropyrazole-5-thione (594 mg, 5.19 mmol) in EtOH (10 mL) was heated at 80°C for 16 h. The reaction mixture was quenched by adding H₂O (100 mL) and extracted with EtOAc (2 x 50 mL). The combined organic layers were washed with brine (30 mL), dried over anhydrous Na₂SO₄ and concentrated under reduced pressure to afford crude compound. The crude compound was purified by silica gel column chromatography eluting with 0-1% EtOAc in PE to obtain ethyl 3-isopropyl-6-methyl-pyrazolo[5,1-b]thiazole-2-carboxylate.

### Example 4.1(d)

### Ethyl 7-bromo-3-isopropyl-6-methyl-pyrazolo[5,1-b]thiazole-2-carboxylate

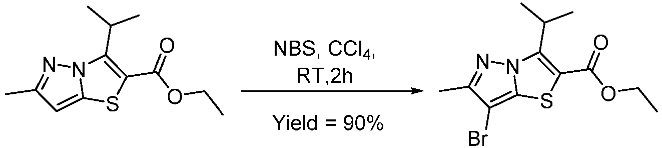

To an ice-cold stirred solution of ethyl 3-isopropyl-6-methyl-pyrazolo[5,1-b]thiazole-2-carboxylate (0.16 g, 0.63 mmol) in CCl₄ (5 mL) was added NBS (124 mg, 0.69 mmol), and the mixture was then stirred for 2 h and allowed to warm up to rt. The reaction mixture was quenched by adding H₂O (100 mL) and extracted with CH₂Cl₂ (2 x 50 mL). The combined organic layers were washed with brine (30 mL), dried over anhydrous Na₂SO₄ and concentrated under reduced pressure to obtain ethyl 7-bromo-3-isopropyl-6-methyl-pyrazolo[5,1-b]thiazole-2-carboxylate.

### Example 4.1(e)

### 7-Bromo-3-isopropyl-6-methyl-pyrazolo[5,1-b]thiazole-2-carboxylic acid

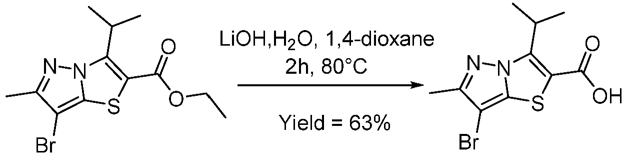

To a stirred solution of 7-bromo-3-isopropyl-6-methyl-pyrazolo[5,1-b]thiazole-2-carboxylate (0.19 g, 0.57 mmol) in 1,4-dioxane (2 mL) was added and aq. solution of LiOH·H₂O (2 ml) at rt and the resulting mixture was then heated at 80°C for 2h. The reaction mixture was allowed to cool down to rt and was concentrated to dryness under reduced pressure. The residue was diluted with cold H₂O and the pH of the reaction mixture was adjusted to 2-3 by addition of aq.HCl (1 M). The aq. solution was extracted with EtOAc (2 x 30mL), washed with H₂O (10 mL), dried over anhydrous Na₂SO₄ and concentrated under reduced pressure to obtain 7-bromo-3-isopropyl-6-methyl-pyrazolo[5,1-b]thiazole-2-carboxylic acid.

### Example 4.1(f)

### 7-Bromo-N-(2,3-dihydro-1,4-benzoxazin-4-yl)-3-isopropyl-6-methyl-pyrazolo[5,1-b]thiazole-2-carboxamide

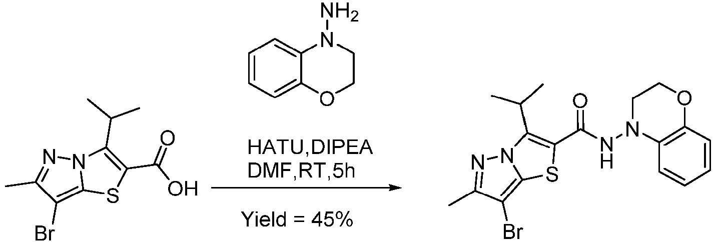

A solution of 7-bromo-3-isopropyl-6-methyl-pyrazolo[5,1-b]thiazole-2-carboxylic acid (0.40 g, 1.31 mmol), 2,3-dihydro-1,4-benzoxazin-4-amine (0.256 g, 1.70 mmol) and HATU (0.745 g, 1.96 mmol) in DMF (5 mL) was stirred at rt for 15 min. Subsequently, DIPEA (0.507 g, 3.93 mmol) was added and the reaction mixture was stirred at rt for 5 h. The reaction mixture was quenched by adding H₂O (30 mL) and extracted with EtOAc (3 x 25 mL). The combined organic layers were washed with brine (30 mL), dried over anhydrous Na₂SO₄ and concentrated under reduced pressure. The crude compound was purified by silica gel column chromatography eluting with 0-7% EtOAc in PE to obtain 7-bromo-N-(2,3-dihydro-1,4-benzoxazin-4-yl)-3-isopropyl-6-methyl-pyrazolo[5,1-b]thiazole-2-carboxamide.

### Example 4.1(g)

### N-(2,3-Dihydro-1,4-benzoxazin-4-yl)-3-isopropyl-6-methyl-7-(2,3,5-trifluorophenyl)pyrazolo[5,1-b]thiazole-2-carboxamide

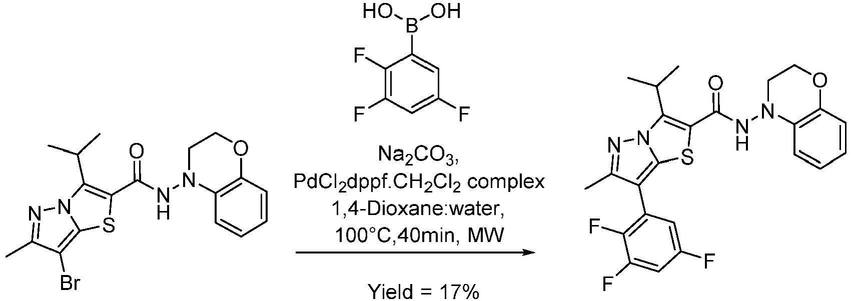

### Example 4.1

To a stirred solution of 7-bromo-N-(2,3-dihydro-1,4-benzoxazin-4-yl)-3-isopropyl-6-methyl-pyrazolo[5,1-b]thiazole-2-carboxamide (0.13 g, 0.29 mmol) and (2,3,5-trifluorophenyl)boronic acid (105 mg, 0.58 mmol) in 1,4-Dioxane (3 mL):H₂O (1 mL) was added Na₂CO₃ (0.092 g).The reaction mixture was degassed with nitrogengas for 10 min before PdCl₂(dppf)·CH₂Cl₂ complex (0.023 g, 0.029 mmol) was added. The reaction mixture was again degassed with nitrogen gas for 10 min and irradiated at 100°C in the microwave for 40 min. The reaction mixture was quenched by adding H₂O (100 mL) and extracted with EtOAc (2 x 50 mL). The combined organic layers were washed with brine (40 mL), dried over anhydrous Na₂SO₄ and concentrated under reduced pressure to obtain crude compound. The crude compound was purified by silica gel column chromatography eluting with 20% EtOAc in PE to obtain N-(2,3-dihydro-1,4-benzoxazin-4-yl)-3-isopropyl-6-methyl-7-(2,3,5-trifluorophenyl)pyrazolo[5,1-b]thiazole-2-carboxamide. ¹H-NMR (400 MHz, DMSO) δ [ppm] 9.86-10.51 (m, 1 H), 7.48-7.55 (m, 1 H), 7.25-7.35 (m, 1 H), 6.71-6.92 (m, 4 H), 4.1-4.8 (m, 3 H), 3.51-3.6 (m, 2 H), 2.31-2.48 (m, 3 H), 1.51 (d, J= 6.8 Hz, 6 H). LCMS (method A): Rₜ= 2.54; *m*/*z =* 487.12 [M+H]⁺.

### Example 4.2

### N-(2,3-Dihydro-4H-benzo[b][1,4]oxazin-4-yl)-6-methyl-3-morpholino-7-(2,3,5-trifluorophenyl)pyrazolo[5,1-b]thiazole-2-carboxamide

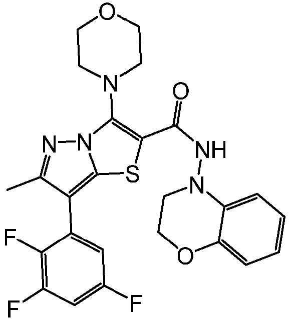

### Example 4.2 (a)

### 5-Methyl-2,4-dihydro-3H-pyrazole-3-thione

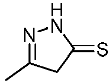

Under inert nitrogen atmosphere was placed a solution of 3-methyl-5-pyrazolone (10.0 g, 102 mmol) and Lawesson Reagent (20.2 g, 50.0 mmol) in toluene (200 mL). The resulting solution was stirred at 110°C for 24 h. After cooling down to rt, the resulting mixture was concentrated under reduced pressure. n-Hexane was added, and the precipitate was collected by filtration, washed with n-hexane, and then dried *in vacuo* to give 5-methyl-2,4-dihydropyrazole-3-thione.

### Example 4.2 (b)

### Ethyl 6-methylpyrazolo[5,1-b]thiazole-2-carboxylate

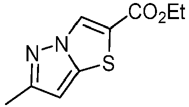

Under inert nitrogen atmosphere was placed a solution of 5-methyl-2,4-dihydropyrazole-3-thione (26.20 g, 91.79 mmol, 40% purity) and ethyl 2-chloro-3-oxopropanoate (20.7 g, 138 mmol) in EtOH (250 mL). The resulting mixture was stirred at 90°C overnight. After cooling down to rt, the resulting mixture was concentrated under reduced pressure. The residue was purified by silica gel column chromatography (PE: EtOAc = 5:1) to give ethyl 6-methylpyrazolo[3,2-b][1,3]thiazole-2-carboxylate.

### Example 4.2 (c)

### Ethyl 7-bromo-6-methylpyrazolo[5,1-b]thiazole-2-carboxylate

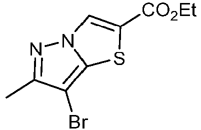

Under inert nitrogen atmosphere was placed a solution of ethyl 6-methylpyrazolo[3,2-b][1,3]thiazole-2-carboxylate (5.20 g, 24.73 mmol) in DMF (120 mL) at 0°C. Then, NBS (4.84 g, 27.19 mmol) was added at 0°C. The mixture was stirred for 2 h. The reaction mixture was poured into H₂O and extracted with EtOAc. The organic layers were combined, dried over anhydrous Na₂SO₄, filtered, and concentrated under reduced pressure. The residue was purified by silica gel column chromatography (EtOAc: PE = 0 ~ 10%) to obtain ethyl 7-bromo-6-methylpyrazolo[3,2-b][1,3]thiazole-2-carboxylate.

### Example 4.2 (d)

### Ethyl 6-methyl-7-(2,3,5-trifluorophenyl)pyrazolo[5,1-b] thiazole-2-carboxylate

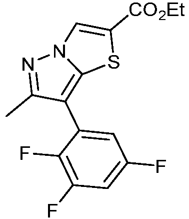

Under inert nitrogen atmosphere was placed a solution of ethyl 7-bromo-6-methylpyrazolo[3,2-b][1,3]thiazole-2-carboxylate (3.00 g, 10.38 mmol) in 1,4-dioxane/ H₂O (54 mL, v:v = 5/1), 2,3,5-trifluorophenylboronic acid (2.74 g, 15.58 mmol), xphos (890 mg, 1.87 mmol), XPhos Pd G3 (878 mg, 1.04 mmol) and K₂CO₃ (2.87 g, 20.77 mmol). The resulting mixture was stirred at 100°C with microwave irradiation for 1 h. After cooling down to rt, the reaction mixture was poured into H₂O and extracted with EtOAc. The organic layers were combined, dried over anhydrous Na₂SO₄, filtered, and concentrated under reduced pressure. The residue was purified by silica gel column chromatography (PE:EtOAc = 10:1) to give ethyl 6-methyl-7-(2,3,5-trifluorophenyl) pyrazolo[3,2-b][1,3]thiazole-2-carboxylate.

### Example 4.2 (e)

### Ethyl 3-iodo-6-methyl-7-(2,3,5-trifluorophenyl)pyrazolo[5,1-b]thiazole-2-carboxylate

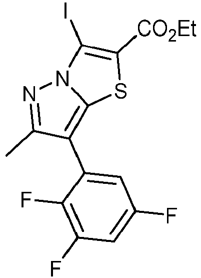

Under inert nitrogen atmosphere was placed (TMP)MgCl₂·LiCl (1.0 M) in abs. THF (6 mL, 6.0 mmol) at 0°C. Then, a ZnCl₂-solution (0.7 M) in abs. THF (4.3 mL, 3.0 mmol) was added slowly to the above solution. The resulting mixture was stirred at 0°C for 2 h to give a solution of (TMP)₂ZnCl₂. Under inert atmosphere, a solution of ethyl 6-methyl-7-(2,3,5-trifluorophenyl)pyrazolo[3,2-b][1,3]thiazole-2-carboxylate (1.02 g, 3.00 mmol) in abs. THF (10.00 mL) at 0°C was prepared. To this, the solution of (TMP)₂ZnCl₂ (3.00 mmol) was added slowly at 0°C. After 2 h, iodine (829 mg, 3.27 mmol) was added at 0°C and the mixture was stirred for further 2 h. The reaction was quenched by the addition of aq. Na₂SO₃ solution at 0°C. The resulting mixture was extracted with EtOAc. The combined organic layers were washed with brine, dried over anhydrous Na₂SO₄, filtered, and concentrated under reduced pressure. The residue was purified by silica gel column chromatography (PE:EtOAc = 5:1) to afford ethyl 3-iodo-6-methyl-7-(2,3,5-trifluorophenyl)pyrazolo[3,2-b][1,3]thiazole-2-carboxylate.

### Example 4.2 (f)

### 3-Acetyl-6-fluoro-7-(2,3,5-trifluorophenyl)thieno[3,2-b]pyridine-2-carboxylic acid

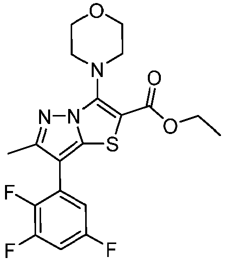

A solution of ethyl 3-chloro-6-methyl-7-(2,3,5-trifluorophenyl)pyrazolo[3,2-b] [1,3] thiazole-2-carboxylate (534.00 mg, 1.00 mmol, 70%), morpholine (173.80 mg, 2.00 mmol), and K₂CO₃ (413.55 mg, 2.99 mmol) in DMF (4.0 mL) was stirred for 6 h at 80°C. After cooling down to rt, the solution was diluted with H₂O and extracted with EtOAc. The organic layers were combined, dried over anhydrous Na₂SO₄, filtered, and concentrated under reduced pressure. The residue was purified by silica gel column chromatography (PE: EtOAc = 5: 1) to afford ethyl 6-methyl-3-(morpholin-4-yl)-7-(2,3,5-trifluorophenyl)pyrazolo[3,2-b][1,3]thiazole-2-carboxylate.

### Example 4.2 (g)

### Potassium 6-methyl-3-morpholino-7-(2,3,5-trifluorophenyl)pyrazolo[5,1-b] thiazole-2-carboxylate

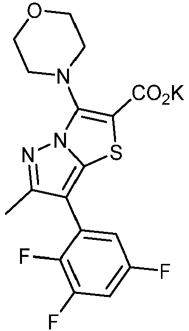

A solution of 3-acetyl-6-fluoro-7-(2,3,5-trifluorophenyl)thieno[3,2-b]pyridine-2-carboxylic acid (250 mg, 0.59 mmol, 68% purity), TMSOK (266 mg, 1.76 mmol) and abs. THF (8.0 mL) was stirred at rt overnight. Then the solution was concentrated *in vacuo* to afford potassium 6-methyl-3-morpholino-7-(2,3,5-trifluorophenyl)pyrazolo[5,1-b]thiazole-2-carboxylate (used directly in next step).

### Example 4.2 (h)

### N-(2,3-Dihydro-4H-benzo[b][1,4]oxazin-4-yl)-6-methyl-3-morpholino-7-(2,3,5-trifluorophenyl)-pyrazolo[5,1-b]thiazole-2-carboxamide

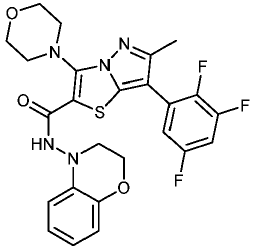

A solution of potassium 6-methyl-3-morpholino-7-(2,3,5-trifluorophenyl)pyrazolo[5,1-b] thiazole-2-carboxylate (180 mg, 0.41 mmol, 60% purity), HBTU (472 mg, 1.24 mmol), and DMF (5 mL) was stirred at rt for 1 h. Then, DIPEA (160 mg, 1.24 mmol), and 2,3-dihydro-4H-benzo[b][1,4]oxazin-4-amine (124 mg, 0.83 mmol) in abs. THF (3 mL) were added slowly at 0°C. The resulting solution was stirred overnight at rt. Then the solution was diluted with H₂O and extracted with EtOAc. The organic layers were combined, dried over anhydrous Na₂SO₄, filtered, and concentrated under reduced pressure. The crude product was further purified by prep-HPLC [Mobile Phase A: H₂O (10 mmol/L NH₄HCO₃), Mobile Phase B: CH₃CN; Flow rate: 60 mL/min; Gradient: 53% B to 83% B in 7 min] to afford N-(2,3-dihydro-4H-benzo[b][1,4]oxazin-4-yl)-6-methyl-3-morpholino-7-(2,3,5-trifluorophenyl)pyrazolo[5,1-b]thiazole-2-carboxamide.

¹H-NMR (400 MHz, 90°C, DMSO-d6) δ [ppm] 9.80 (s, 1H), 7.41-7.38 (m, 1H), 7.20-7.15 (m, 1H), 6.84-6.75 (m, 4H), 4.40-4.32 (m, 2H), 3.83-3.80 (m, 4H), 3.62-3.52 (m, 6H), 2.48 (s, 3H). LCMS (Method E1) Rt= 1.06 min; m/z= 530 (M+H)⁺.

### Example 4.3

### N-(2,3-Dihydro-1,4-benzoxazin-4-yl)-3-(2-hydroxypropan-2-yl)-6-methyl-7-(2,3,5-trifluorophenyl)pyrazolo [3,2-b][1,3]thiazole-2-carboxamide

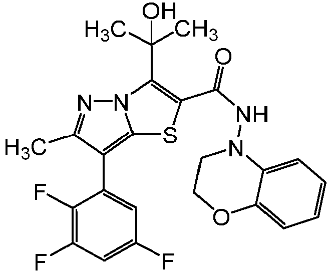

### Example 4.3 (a)

### 3-(1-Ethoxyethenyl)-6-methyl-7-(2,3,5-trifluorophenyl) pyrazolo [3,2-b] [1,3] thiazole-2-carboxylate

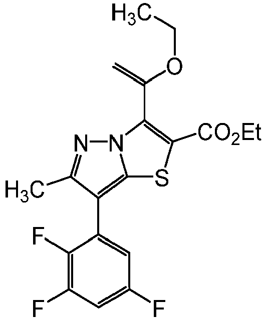

Under inert nitrogen atmosphere was placed ethyl 3-iodo-6-methyl-7-(2,3,5-trifluorophenyl)pyrazolo [3,2-b][1,3]thiazole-2-carboxylate (Example 4.2 (e); 150 mg, 0.322 mmol), tributyl(1-ethoxyethenyl)stannane (232 mg, 0.644 mmol), Pd(PPh₃)₄ (37 mg, 0.032 mmol) and 1,4-dioxane (2 mL). The mixture was stirred overnight at 90°C. After cooling down to rt, the solution was diluted with H₂O and extracted with EtOAc. The organic layers were combined, dried over anhydrous Na₂SO₄, filtered and concentrated under reduced pressure. The residue was purified by prep-TLC (PE: EtOAc=1:1) to afford ethyl 3-(1-ethoxyethenyl)-6-methyl-7-(2,3,5-trifluorophenyl) pyrazolo[3,2-b][1,3]thiazole-2-carboxylate.

### Example 4.3 (b)

### 3-(1-Ethoxyethenyl)-6-methyl-7-(2,3,5-trifluorophenyl)pyrazolo[3,2-b][1,3]thiazole-2-carboxylic acid

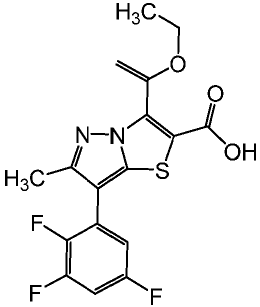

To a solution of ethyl 3-(1-ethoxyethenyl)-6-methyl-7-(2,3,5-trifluorophenyl)pyrazolo [3,2-b][1,3]thiazole-2-carboxylate (120 mg, 0.292 mmol) in THF/ H₂O (1 mL/1 mL) was added LiOH (21 mg, 0.876 mmol). The mixture was stirred for 2 h at rt. Upon completion of the reaction, THF was evaporated under reduced pressure, the pH value of the mixture was adjusted to 5 by addition of aq. HCl (1 M). The resulting mixture was extracted with EtOAc. The organic layers were combined, dried over anhydrous Na₂SO₄, filtered and concentrated *in vacuo* to afford 3-(1-ethoxyethenyl)-6-methyl-7-(2,3,5-trifluorophenyl) pyrazolo[3,2-b][1,3]thiazole-2-carboxylic acid.

### Example 4.3 (c)

### N-(2,3-Dihydro-1,4-benzoxazin-4-yl)-3-(1-ethoxyethenyl)-6-methyl-7-(2,3,5-trifluorophenyl)-pyrazolo[3,2-b][1,3]thiazole-2-carboxamide

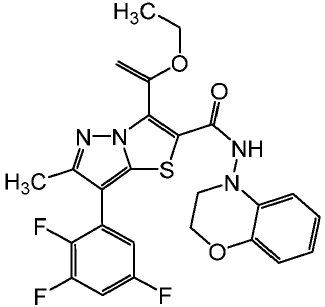

To a solution of 3-(1-ethoxyethenyl)-6-methyl-7-(2,3,5-trifluorophenyl)pyrazolo [3,2-b] [1,3]thiazole-2-carboxylic acid (95 mg, 0.248 mmol) in DMF (1 mL), was added 2,3-dihydro-1,4-benzoxazin-4-amine (45 mg, 0.298 mmol), HATU (113 mg, 0.298 mmol) and DIPEA (0.13 mL, 0.744 mmol). The mixture was stirred for 3 h at rt. Upon completion of the reaction, the resulting mixture was treated with H₂O and extracted with EtOAc (3x). The organic layers were combined, dried over anhydrous Na₂SO₄, filtered and concentrated under reduced pressure. The residue was purified by prep-TLC (PE: EtOAc= 1:1) to afford N-(2,3-dihydro-1,4-benzoxazin-4-yl)-3-(1-ethoxyethenyl)-6-methyl-7-(2,3,5-trifluorophenyl)pyrazolo [3,2-b] [1,3]thiazole-2-carboxamide.

### Example 4.3 (d)

### 3-Acetyl-N-(2,3-dihydro-1,4-benzoxazin-4-yl)-6-methyl-7-(2,3,5-trifluorophenyl) pyrazolo[3,2-b][1,3]thiazole-2-carboxamide

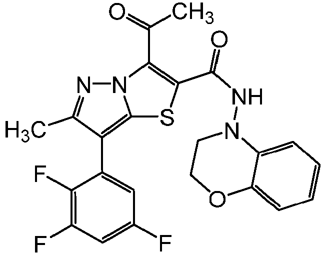

Under inert nitrogen atmosphere was placed N-(2,3-dihydro-1,4-benzoxazin-4-yl)-3-(1-ethoxyethenyl)-6-methyl-7-(2,3,5-trifluorophenyl)pyrazolo[3,2-b][1,3]thiazole-2-carboxamide (60 mg, 0.117 mmol) and HCl (6 N, 1 mL) in CH₃CN (1 mL). The mixture was stirred overnight at rt. Then the solution was concentrated under reduced pressure. The residue was purified by prep-HPLC (Mobile Phase A: H₂O (0.1%FA), Mobile Phase B: CH₃CN; Gradient: 55% B to 74% B in 8 min) to afford 3-acetyl-N-(2,3-dihydro-1,4-benzoxazin-4-yl)-6-methyl-7-(2,3,5-trifluorophenyl)pyrazolo [3,2-b][1,3]thiazole-2-carboxamide.

### Example 4.3 (e)

### N-(2,3-Dihydro-1,4-benzoxazin-4-yl)-3-(2-hydroxypropan-2-yl)-6-methyl-7-(2,3,5-trifluorophenyl)pyrazolo[3,2-b][1,3]thiazole-2-carboxamide

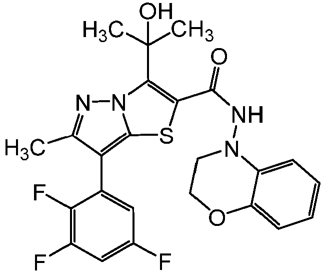

Under inert nitrogen atmosphere was placed a solution of 3-acetyl-N-(2,3-dihydro-1,4-benzoxazin-4-yl)-6-methyl-7-(2,3,5-trifluorophenyl)pyrazolo[3,2-b][1,3]thiazole-2-carboxamide (20 mg, 0.040 mmol) in abs. THF (1 mL). Then CH₃MgBr (0.06 mL, 0.060 mmol) was added at 0°C. The resulting solution was stirred for 2 h at 0°C. Upon completion of the reaction, the resulting mixture was treated with aq. sat. NH₄Cl-solution and extracted with EtOAc. The organic layers were combined, dried over anhydrous Na₂SO₄, filtered, and concentrated under reduced pressure. The residue was purified by prep-HPLC (Mobile Phase A: H₂O (0.1% FA), Mobile Phase B: CH₃CN; Gradient: 58% B to 86% B in 7 min) to afford of N-(2,3-dihydro-1,4-benzoxazin-4-yl)-3-(2-hydroxypropan-2-yl)-6-methyl-7-(2,3,5-trifluorophenyl) pyrazolo [3,2-b] [1,3] thiazole-2-carboxamide. ¹H-NMR (300 MHz, DMSO-d6) δ [ppm]: 10.84 (br s, 1 H), 7.55-7.52 (m, 1H), 7.38-7.34 (m, 1H), 6.96-6.94 (m, 1H), 6.79-6.71 (m, 3H), 4.40-4.32 (m, 2H), 3.58-3.57 (m, 2H), 2.45 (s, 3H), 1.81(s, 6H). LCMS (Method B1) Rt= 1.29 min; m/z= 502.95 (M+H)⁺.

### Example 5.1

### 8-(2,6-Difluorophenyl)-N-(2,3-dihydro-1,4-benzoxazin-4-yl)-4-isopropyl-7-methyl-pyrazolo[5,1-c][1,2,4]triazine-3-carboxamide

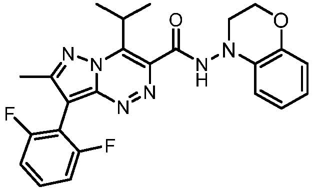

### Example 5.1(a)

### Ethyl 4-isopropyl-7-methyl-pyrazolo [5,1-c] [1,2,4]triazine-3-carboxylate

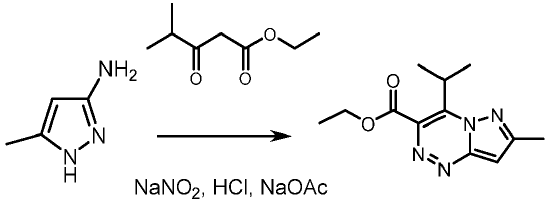

A solution of 3-amino-5-methylpyrazole (3.65 g, 37.55 mmol) in EtOH (40 mL) and H₂O (21 mL) was cooled to 0°C under nitrogen atmosphere, and a cooled solution (0°C) of sodium nitrite (3.91 g, 56.7 mmol) in H₂O (21 mL) was added. A solution of HCl (37% in H₂O, 9.5 mL, 120 mmol) in H₂O (7 mL) was added at 0°C. Then, ethyl isobutyryl acetate (6.80 mL, 42 mmol) in EtOH (7 mL) was added, followed by sodium acetate (9.25 g, 112.72 mmol). The resulting reaction mixture was stirred at 0°C for 30 min and was then heated at 50°C for 18 h. After this time, the mixture was concentrated *in vacuo* to half volume. The mixture was diluted with H₂O (100 mL) and then extracted with EtOAc (100 mL), and 10% MeOH in CH₂Cl₂ (100 mL). The combined organic and aq. layers were filtered to remove the brown precipitate and then separated. The aq. layer was extracted with CH₂Cl₂ (2x100 mL), the combined organic layers were dried over anhydrous Na₂SO₄ and concentrated under reduced pressure. The crude mixture was purified by silica gel column chromatography eluting with 10-25% EtOAc in cyclohexane to afford ethyl 4-isopropyl-7-methyl-pyrazolo[5,1-c][1,2,4]triazine-3-carboxylate.

### Example 5.1(b)

### Ethyl 8-bromo-4-isopropyl-7-methyl-pyrazolo[5,1-c][1,2,4]triazine-3-carboxylate

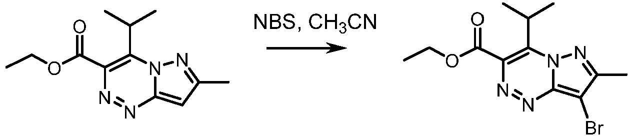

To a solution of ethyl 4-isopropyl-7-methyl-pyrazolo[5,1-c][1,2,4]triazine-3-carboxylate (3.29 g, 13.28 mmol) in CH₃CN (66 mL) under nitrogen atmosphere was added N-bromosuccinimide (2.40 g, 13.51 mmol). Ice cold H₂O (200 mL) was poured into the reaction mixture causing a yellow solid to precipitate. The solid was filtered off and dried *in vacuo* at 40°C overnight. The compound was used in the next step without further purification.

### Example 5.1(c)

### Ethyl 8-bromo-4-isopropyl-7-methyl-pyrazolo[5,1-c] [1,2,4]triazine-3-carboxylate

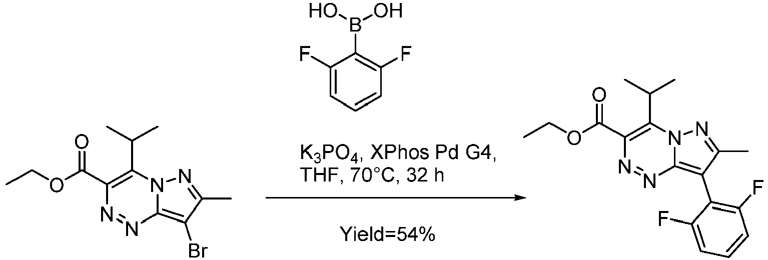

To a stirred solution of ethyl 8-bromo-4-isopropyl-7-methyl-pyrazolo[5,1-c][1,2,4]triazine-3-carboxylate (0.05 g, 0.15 mmol) and (2,6-difluorophenyl)-boronic acid (0.072 g, 0.458 mmol) in THF (2 mL) was added K₃PO₄ (0.9 mL, 0.458 mmol, 0.5 M in H₂O). The reaction mixture was degassed with nitrogen gas for 3 min, Xphos Pd G4 (0.013 g, 0.015 mmol) was added and the mixture was heated to 70°C for 32 h. The reaction mixture was quenched with H₂O (5 mL) and extracted with EtOAc (2 x 10 mL). The combined organic layers were washed with brine (10 mL), dried over anhydrous Na₂SO₄ and concentrated under reduced pressure. The crude compound was purified by silica gel column chromatography eluting with 0-20% EtOAc in PE to afford ethyl 8-bromo-4-isopropyl-7-methyl-pyrazolo[5,1-c][1,2,4]triazine-3-carboxylate.

### Example 5.1(d)

### 8-(2,6-Difluorophenyl)-4-isopropyl-7-methyl-pyrazolo[5,1-c][1,2,4]triazine-3-carboxylic acid

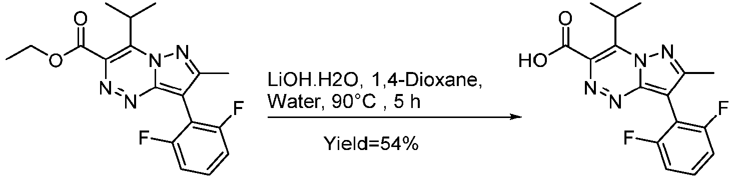

To a stirred solution of ethyl 8-bromo-4-isopropyl-7-methyl-pyrazolo[5,1-c][1,2,4]triazine-3-carboxylate (0.03g, 0.083 mmol) in 1,4-dioxane (0.5 mL), H₂O (0.5 mL) and LiOH·H₂O (0.01 g, 0.25 mmol) was added at rt and the mixture was heated at 90°C for 5 h. The mixture was cooled to rt and the solvents were evaporated under reduced pressure. The residues were dissolved in H₂O (1 mL), acidified to 2-3 pH by addition of aq. HCl (1 M) and extracted with EtOAc (2 x 5 mL). The combined organic layers were washed with brine (10 mL), dried over anhydrous Na₂SO₄ and concentrated under reduced pressure to afford 8-(2,6-difluorophenyl)-4-isopropyl-7-methyl-pyrazolo[5,1-c][1,2,4]triazine-3-carboxylic acid.

### Example 5.1(e)

### 8-(2,6-Difluorophenyl)-N-(2,3-dihydro-1,4-benzoxazin-4-yl)-4-isopropyl-7-methyl-pyrazolo[5,1-c] [1,2,4]triazine-3-carboxamide

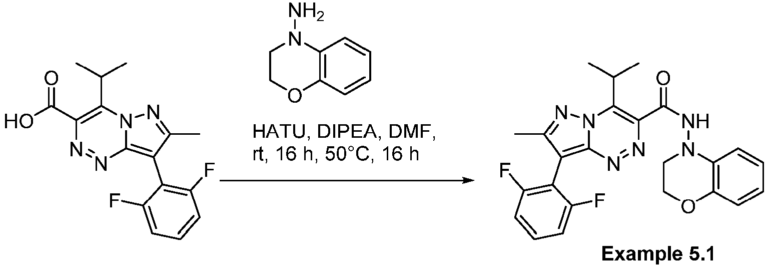

A solution of 8-(2,6-difluorophenyl)-4-isopropyl-7-methyl-pyrazolo[5,1-c][1,2,4]triazine-3-carboxylic acid (0.015 g, 0.045 mmol), 2,3-dihydro-1,4-benzoxazin-4-amine (0.020 g, 0.13 mmol) and HATU (0.03433 g, 0.09 mmol) in DMF (1 mL) was stirred for 15 min. Subsequently, DIPEA (0.023 g, 0.18 mmol) was added and the reaction mixture was stirred for 20 h at rt and then heated to 50°C for 16 h. The reaction was quenched by adding H₂O (2 mL) and extracted with EtOAc (2 x 10 mL). The combined organic layers were washed with brine (15 mL), dried over anhydrous Na₂SO₄ and concentrated under reduced pressure to obtain crude compound. The crude compound was purified by prep-HPLC. ¹H NMR (400 MHz, DMSO) δ [ppm]: 10.97 (s, 1 H), 7.63-7.66 (m, 1 H), 7.36 (t, J= 8 Hz, 1 H), 6.93 (d, J= 7.2 Hz, 1 H), 6.72-6.82 (m, 3 H), 4.39 (m, 2 H), 4.20 (t, J= 6.4 Hz, 1 H), 3.68 (br s, 2H), 3.1 (s, 3 H), 1.60 (d, J= 7.2 Hz, 6 H). LCMS (method A) Rt= 2.34; *m*/*z* = 465.46 [M+H]⁺.

### Example 6.1

### N-(2,3-Dihydro-4H-benzo[b][1,4]oxazin-4-yl)-6-fluoro-3-(2-hydroxypropan-2-yl)-7-(2,3,5-trifluorophenyl)thieno[3,2-b]pyridine-2-carboxamide

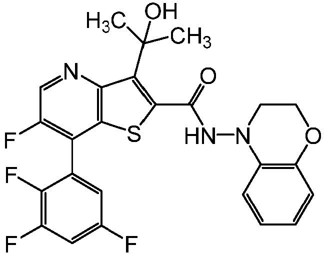

### Example 6.1 (a)

### 3,5-Difluoro-4-iodopicolinonitrile

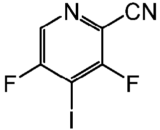

Under nitrogen atmosphere was placed a solution of diisopropylamine (44 mL, 394 mmol) in abs. THF (500 mL). Then a solution of n-BuLi (120 mL, 290 mmol) was added slowly at -78°C. After stirring for 1 h, a solution of 3,5-difluoropyridine-2-carbonitrile (40 g, 286 mmol) in abs. THF (100 mL) was added at -78°C. After 2 h, iodine (100 g, 394 mmol) was added at -78°C and the mixture was stirred for 2 h. The reaction was quenched by the addition of aq. Na₂SO₃-solution at 0°C. The resulting mixture was extracted with EtOAc. The combined organic layers were washed with brine, dried over anhydrous Na₂SO₄, filtered, and concentrated under reduced pressure. The residue was purified by silica gel column chromatography (eluting with PE: EtOAc = 5: 1) to afford 3,5-difluoro-4-iodopyridine-2-carbonitrile.

### Example 6.1 (b)

### 2-Ethynyl-3,5-difluoro-4-(2,3,5-trifluorophenyl)pyridine

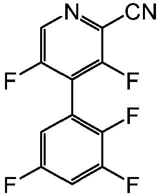

Under inert nitrogen atmosphere was placed a solution of 2-ethynyl-3,5-difluoro-4-iodopyridine (20 g, 75.47 mmol,) in 1,4-dioxane: H₂O (440 mL; 10: 1), 2,3,5-trifluorophenylboronic acid (19.91 g, 113.21 mmol), Pd(DtBPF)Cl₂ (4.92 g, 7.55 mmol) and K₂CO₃ (32.04 g, 150.94 mmol). The resulting mixture was stirred at 90°C overnight. After cooling down to rt, the mixture was filtered and extracted with EtOAc. The organic layers were combined, dried over anhydrous Na₂SO₄, filtered, and concentrated under reduced pressure. The residue was purified by silica gel column chromatography (EtOAc: PE = 0 to 5%) to afford 2-ethynyl-3,5-difluoro-4-(2,3,5-trifluorophenyl) pyridine.

### Example 6.1 (c)

### Methyl 3-amino-6-fluoro-7-(2,3,5-trifluorophenyl) thieno[3,2-b] pyridine-2-carboxylate

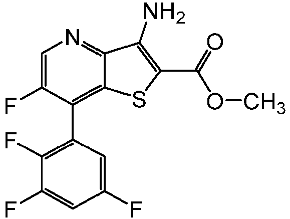

Under inert nitrogen atmosphere 3,5-difluoro-4-(2,3,5-trifluorophenyl)pyridine-2-carbonitrile (910 mg, 2.86 mmol) was dissolved in abs. THF (35 mL). NaH (126 mg, 3.15 mmol) was added portionwise at 0°C. The resulting mixture was then cooled to -50°C and a solution of methyl 2-mercaptoacetate (320 mg, 3.02 mmol) in abs. THF (5.00 mL) was added. The resulting mixture was stirred overnight at rt. The reaction was quenched with trace amount of cold H₂O. The mixture was dried over anhydrous Na₂SO₄, filtered, and concentrated. The residue was dissolved in CH₃CN (15.0 mL) and treated with K₂CO₃ (900 mg, 6.51 mmol). The resulting mixture was stirred for 2 h at 80°C. After cooling down to rt, the mixture was diluted with cold H₂O. The precipitated solids were collected by filtration and dried *in vacuo* to afford methyl 3-amino-6-fluoro-7-(2,3,5-trifluorophenyl) thieno [3,2-b] pyridine-2-carboxylate.

### Example 6.1 (d)

### Methyl 3-bromo-6-fluoro-7-(2,3,5-trifluorophenyl)thieno[3,2-b]pyridine-2-carboxylate

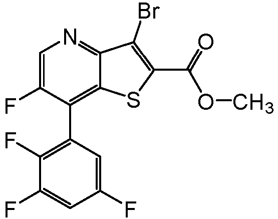

Under inert nitrogen atmosphere, methyl 3-amino-6-fluoro-7-(2,3,5-trifluorophenyl)-thieno[3,2-b]pyridine-2-carboxylate (1.5 g, 3.58 mmol), CuBr (539 mg, 3.76 mmol) and HBr solution (26 mL, 33% HBr in AcOH) were mixed. Then a solution of NaNO₂ (296 mg, 4.29 mmol) in H₂O (1.5 mL) was added dropwise at -5°C. The resulting mixture was stirred overnight at rt and was then extracted with EtOAc. The organic layers were combined, dried over anhydrous Na₂SO₄, filtered, and concentrated under reduced pressure. The residue was purified by prep-TLC (PE: EtOAc = 4: 1) to afford methyl 3-bromo-6-fluoro-7-(2,3,5-trifluorophenyl)thieno[3,2-b]pyridine-2-carboxylate.

### Example 6.1 (e)

### Methyl 3-acetyl-6-fluoro-7-(2,3,5-trifluorophenyl) thieno [3,2-b] pyridine-2-carboxylate

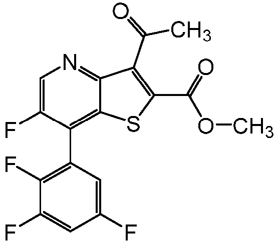

Under inert nitrogen atmosphere, methyl 3-bromo-6-fluoro-7-(2,3,5-trifluorophenyl)- thieno[3,2-b]pyridine-2-carboxylate (300 mg, 0.71 mmol), tributyl(1-ethoxyethenyl)-stannane (774 mg, 2.14 mmol) and Pd(PPh₃)₄ (83 mg, 0.07 mmol) were mixed in 1,4-dioxane (3 mL). The resulting mixture was stirred overnight at 100°C. After cooling down to rt, HCl (2 mL, 2 M) was added and the resulting solution was stirred for 3 h. Then, the solution was extracted with EtOAc. The organic layers were combined, dried over anhydrous Na₂SO₄, filtered, and concentrated under reduced pressure. The residue was purified by prep-TLC (PE: EtOAc = 2: 1) to afford methyl 3-acetyl-6-fluoro-7-(2,3,5-trifluorophenyl)thieno[3,2-b]pyridine-2-carboxylate.

### Example 6.1 (f)

### 3-Acetyl-6-fluoro-7-(2,3,5-trifluorophenyl)thieno[3,2-b]pyridine-2-carboxylic acid

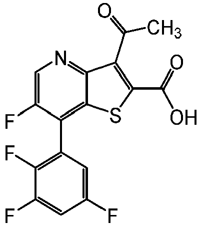

To a solution of methyl 3-(3-fluoroazetidin-1-yl)-7-(2,3,5-trifluorophenyl)thieno[3,2-b]pyridine-2-carboxylate (230 mg, 0.60 mmol) in THF (2.3 mL) and H₂O (2.3 mL) was added LiOH (72 mg, 3.00 mmol). The reaction mixture was stirred for 1 h at rt. The pH of the solution was adjusted to 5 by addition of aq. HCl (1 M). The mixture was extracted with EtOAc. The organic layers were combined, dried over anhydrous Na₂SO₄, filtered, and concentrated *in vacuo* to afford 3-(3-fluoroazetidin-1-yl)-7-(2,3,5-trifluorophenyl)- thieno[3,2-b]pyridine-2-carboxylic acid.

### Example 6.1 (g)

### 3-Acetyl-N-(2,3-dihydro-1,4-benzoxazin-4-yl)-6-fluoro-7-(2,3,5-trifluorophenyl)thieno- [3,2-b]pyridine-2-carboxamide

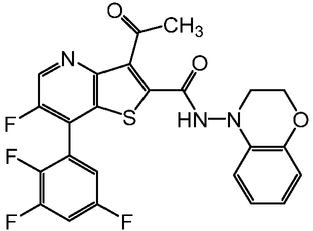

To a solution of 3-acetyl-6-fluoro-7-(2,3,5-trifluorophenyl)thieno[3,2-b]pyridine-2-carboxylic acid (210 mg, 0.57 mmol) and DMF (2 mL), was added HATU (359 mg, 0.68 mmol), DIPEA (220 mg, 1.71 mmol) and 2,3-dihydro-1,4-benzoxazin-4-amine (111 mg, 0.74 mmol). The resulting solution was stirred overnight at rt. Then the solution was diluted with H₂O and extracted with EtOAc. The organic layers were combined, dried over anhydrous Na₂SO₄, filtered, and concentrated under reduced pressure. The residue was purified by prep-TLC (PE: EtOAc = 1: 1) to afford 3-acetyl-N-(2,3-dihydro-1,4-benzoxazin-4-yl)-6-fluoro-7-(2,3,5-trifluorophenyl)thieno[3,2-b]pyridine-2-carboxamide

### Example 6.1 (h)

### N-(2,3-Dihydro-1,4-benzoxazin-4-yl)-6-fluoro-3-(2-hydroxypropan-2-yl)-7-(2,3,5-trifluorophenyl)thieno[3,2-b]pyridine-2-carboxamide

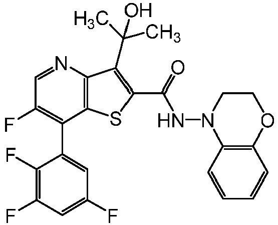

Under inert nitrogen atmosphere, 3-acetyl-N-(2,3-dihydro-1,4-benzoxazin-4-yl)-6-fluoro-7-(2,3,5-trifluorophenyl)thieno[3,2-b]pyridine-2-carboxamide (150 mg, 0.30 mmol) was dissolved in abs. THF (2 mL) and CH₃MgBr (1.5 mL, 1.50 mmol, 1M abs. THF) was added at 0°C. The resulting mixture was stirred for 2 h at 0°C. Then aq. sat. NH₄Cl-solution was added and the mixture was extracted with EtOAc. The organic layers were combined, dried over anhydrous Na₂SO₄, filtered, and concentrated under reduced pressure. The residue was purified by prep-HPLC [Mobile Phase A: H₂O (0.1% FA), Mobile Phase B: CH₃CN; Gradient: 55% B to 64% B in 11 min] to afford of N-(2,3-dihydro-1,4-benzoxazin-4-yl)-6-fluoro-3-(2-hydroxypropan-2-yl)-7-(2,3,5-trifluorophenyl)thieno[3,2-b]pyridine-2-carboxamide. ¹H-NMR (400 MHz, DMSO-d₆) δ [ppm] = 10.37 (s, 1H), 8.99 (s, 1H), 7.97-7.66 (m, 2H), 6.98 (dd, 4H), 5.69 (s, 1H), 4.34 (m, 2H), 3.59 (s, 2H), 1.80-1.67 (s, 6H). LCMS (method E1) Rt= 1.42; m/z= 518 [M+H]⁺.

### Example 6.2

### N-(2,3-dihydro-4H-benzo[b] [1,4]oxazin-4-yl)-6-fluoro-3-(3-fluoroazetidin-1-yl)-7-(2,3,5-trifluorophenyl)thieno[3,2-b]pyridine-2-carboxamide

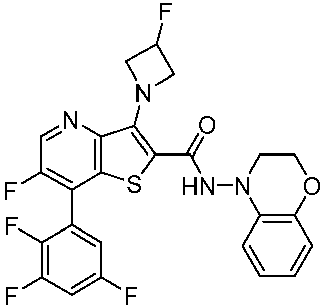

### Example 6.2 (a)

### Methyl 6-fluoro-3-(3-fluoroazetidin-1-yl)-7-(2,3,5-trifluorophenyl)thieno[3,2-b]pyridine-2-carboxylate

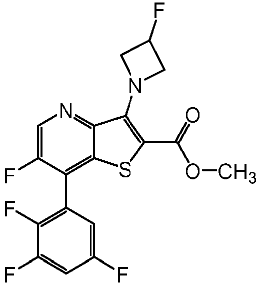

Under inert nitrogen atmosphere, to a solution of methyl 3-bromo-6-fluoro-7-(2,3,5-trifluorophenyl)thieno[3,2-b]pyridine-2-carboxylate (Example 6.1 (d), 200 mg, 0.48 mmol) in 1,4-dioxane (4 mL), was added Pd(PPh₃)₄ (55 mg, 0.05 mmol), K₃PO₄ (303 mg, 1.43 mmol), and 3-fluoroazetidine hydrochloride (85 mg, 0.76 mmol). The resulting mixture was stirred overnight at 100°C. After cooling down to rt, the reaction mixture was treated with H₂O and extracted with EtOAc. The organic layers were combined, dried over anhydrous Na₂SO₄, filtered, and concentrated under reduced pressure. The residue was purified by prep-TLC (EtOAc: PE = 1: 4) to afford methyl 6-fluoro-3-(3-fluoroazetidin-1-yl)-7-(2,3,5-trifluorophenyl)thieno[3,2-b]pyridine-2-carboxylate.

### Example 6.2 (b)

### 6-Fluoro-3-(3-fluoroazetidin-1-yl)-7-(2,3,5-trifluorophenyl)thieno[3,2-b]pyridine-2-carboxylic acid

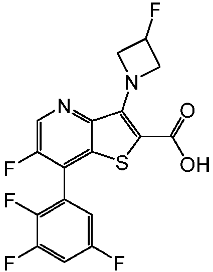

To a solution of methyl 6-fluoro-3-(3-fluoroazetidin-1-yl)-7-(2,3,5-trifluorophenyl)- thieno[3,2-b]pyridine-2-carboxylate (180 mg, 0.43 mmol) in THF (5 mL) was added a solution of LiOH (125 mg, 5.21 mmol) in H₂O (2 mL) at 0°C. The reaction mixture was stirred for 24 h at 65°C. The mixture was concentrated under reduced pressure and then diluted with H₂O (5 mL), acidified to pH 3~5 by addition of aq. HCl (1M) and extracted with EtOAc. The organic layers were combined, dried over anhydrous Na₂SO₄, filtered and concentrated *in vacuo* at 0 ~ 10°C to afford 6-fluoro-3-(3-fluoroazetidin-1-yl)-7-(2,3,5-trifluorophenyl) thieno [3,2-b]pyridine-2-carboxylic acid.

### Example 6.2 (c)

### N-(2,3-Dihydro-4H-benzo[b][1,4] oxazin-4-yl)-6-fluoro-3-(3-fluoroazetidin-1-yl)-7-(2,3,5-trifluorophenyl)thieno[3,2-b]pyridine-2-carboxamide

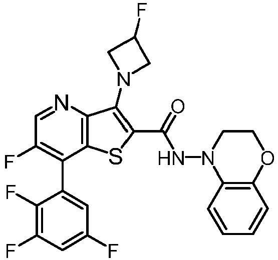

To a solution of 6-fluoro-3-(3-fluoroazetidin-1-yl)-7-(2,3,5-trifluorophenyl)thieno[3,2-b]pyridine-2-carboxylic acid (120 mg, 0.30 mmol) in DMF (4 mL) was added HATU (137 mg, 0.36 mmol), DIPEA (82 mg, 0.63 mmol) and 2,3-dihydro-1,4-benzoxazin-4-amine (59 mg, 0.39 mmol). The solution was stirred for 3 h at rt. H₂O was added and the mixture was extracted with EtOAc. The organic layers were combined, dried over anhydrous Na₂SO₄, filtered, and concentrated under reduced pressure. The residue was purified by prep-TLC (EtOAc: PE = 1: 4), and further purified by prep-HPLC (Mobile Phase A: H₂O (10 mmol/L NH₄HCO₃+ 0.1%NH₃·H₂O), Mobile Phase B: CH₃CN; Gradient: 53% B to 78% B in 9 min) to afford N-(2,3-dihydro-1,4-benzoxazin-4-yl)-6-fluoro-3-(3-fluoroazetidin-1-yl)-7-(2,3,5-trifluorophenyl)thieno[3,2-b]pyridine-2-carboxamide. ¹H-NMR (400 MHz, chloroform-d) δ [ppm] 8.65 (s, 1H), 7.13 (d, 2H), 6.86 (s, 4H), 5.49 (d, 1H), 4.89 (s, 2H), 4.58-4.76 (m, 2H), 4.47 (s, 2H), 3.66 (s, 2H). LCMS (method G1) Rt= 1.52; m/z= 533.15 [M+H]⁺.

### Example 6.3

### N-(2,3-Dihydro-4H-benzo[b][1,4]oxazin-4-yl)-6-fluoro-3-isopropyl-7-(2,3,5-trifluorophenyl)thieno[3,2-b]pyridine-2-carboxamide

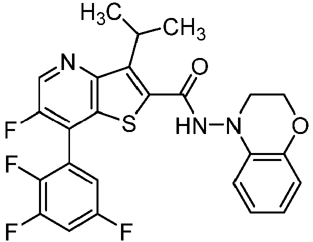

### Example 6.3 (a)

### Methyl 6-fluoro-3-(prop-1-en-2-yl)-7-(2,3,5-trifluorophenyl)thieno [3,2-b]pyridine-2-carboxylate

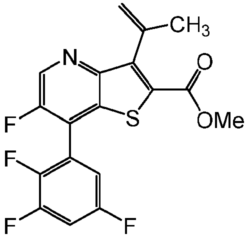

Under inert nitrogen atmosphere, methyl 3-bromo-6-fluoro-7-(2,3,5-trifluorophenyl)- thieno[3,2-b]pyridine-2-carboxylate (300 mg, 0.71 mmol), 4,4,5,5-tetramethyl-2-(prop-1-en-2-yl)-1,3,2-dioxaborolane (180 mg, 1.07 mmol), Pd(DtBPF)Cl₂ (47 mg, 0.07 mmol), and K₃PO₄ (303 mg, 1.428 mmol) were mixed in THF (10 mL) and H₂O (2 mL). The resulting mixture was stirred for 3 h at 90°C. After cooling down to rt, the reaction mixture was diluted with EtOAc, washed with H₂O, dried over anhydrous Na₂SO₄, filtered and concentrated under reduced pressure. The residue was purified by silica gel column chromatography (PE: EtOAc = 2: 1) to give methyl 6-fluoro-3-(prop-1-en-2-yl)-7-(2,3,5-trifluorophenyl)thieno[3,2-b]pyridine-2-carboxylate.

### Example 6.3 (b)

### Methyl 6-fluoro-3-isopropyl-7-(2,3,5-trifluorophenyl)thieno[3,2-b]pyridine-2-carboxylate

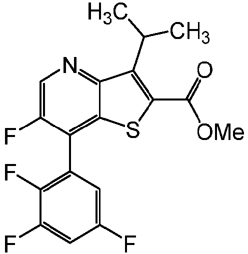

To a solution of methyl 6-fluoro-3-(prop-1-en-2-yl)-7-(2,3,5-trifluorophenyl)thieno[3,2-b]pyridine-2-carboxylate (265 mg, 0.67 mmol) in EtOAc (20 mL), was added PtO₂ (16 mg, 0.07 mmol). The reaction stirred for 2 h at rt under hydrogen gas (1 atm). The mixture was filtered and concentrated under reduced pressure. The residue was purified by silica gel column chromatography (PE: EtOAc = 2: 1) to give methyl 6-fluoro-3-isopropyl-7-(2,3,5-trifluorophenyl)thieno[3,2-b]pyridine-2-carboxylate.

### Example 6.3 (c)

### 6-Fluoro-3-isopropyl-7-(2,3,5-trifluorophenyl)thieno[3,2-b]pyridine-2-carboxylic acid

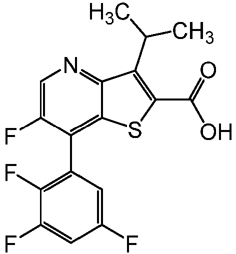

To a solution of methyl 6-fluoro-3-isopropyl-7-(2,3,5-trifluorophenyl)thieno[3,2-b] pyridine-2-carboxylate (242 mg, 0.63 mmol) in THF (41 mL) was added a solution of LiOH (81 mg, 3.39 mmol) in H₂O (10 mL). The resulting mixture was stirred overnight at rt. Upon completion of the reaction, THF was removed *in vacuo* and the pH value was adjusted to 4 by addition of aq. HCl (1 M). The mixture was extracted with EtOAc. The organic layers were combined, dried over anhydrous Na₂SO₄, filtered and concentrated *in vacuo* to give 6-fluoro-3-isopropyl-7-(2,3,5-trifluorophenyl) thieno [3,2-b] pyridine-2-carboxylic acid.

### Example 6.3 (d)

### N-(2,3-Dihydro-4H-benzo[b][1,4]oxazin-4-yl)-6-fluoro-3-isopropyl-7-(2,3,5-trifluorophenyl)-thieno[3,2-b]pyridine-2-carboxamide

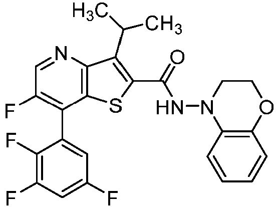

To a solution of 6-fluoro-3-isopropyl-7-(2,3,5-trifluorophenyl)thieno[3,2-b]pyridine-2-carboxylic acid (150 mg, 0.41 mmol) in DMF (5 mL), were added 2,3-dihydro-1,4-benzoxazin-4-amine (122 mg, 0.81 mmol), HATU (232 mg, 0.61 mmol,) and DIPEA (157 mg, 1.22 mmol). The resulting mixture was stirred at rt for 30 min. After this time, the mixture was treated with H₂O and extracted with EtOAc. The organic layers were combined, dried over anhydrous Na₂SO₄, filtered and concentrated under reduced pressure. The residue was purified by prep-HPLC [Mobile Phase A: H₂O (0.1% FA), Mobile Phase B: CH₃CN; Gradient: 58% B to 82% B in 8 min] to afford N-(2,3-dihydro-1,4-benzoxazin-4-yl)-6-fluoro-3-isopropyl-7-(2,3,5-trifluorophenyl)thieno[3,2-b]-pyridine-2-carboxamide. ¹H-NMR (300 MHz, DMSO-d6) δ [ppm] 10.65 (s, 1H), 9.01 (s, 1H), 7.96-7.86 (m, 1H), 7.68-7.65 (m, 1H), 6.86-6.68 (m, 4H), 4.36-4.33 (m, 2H), 3.96-3.87 (m, 1H), 3.62-3.59 (m, 2H), 1.52 (m, 6H). LCMS (method F2) Rt= 1.517; m/z= 502.00 [M+H]⁺.

### Example 6.4

### N-(2,3-Dihydro-4H-benzo[b][1,4]oxazin-4-yl)-6-fluoro-3-morpholino-7-(2,3,5-trifluorophenyl)thieno[3,2-b]pyridine-2-carboxamide

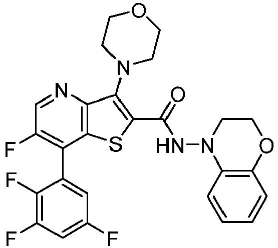

### Example 6.4 (a)

### Methyl 6-fluoro-3-morpholino-7-(2,3,5-trifluorophenyl)thieno[3,2-b]pyridine-2-carboxylate

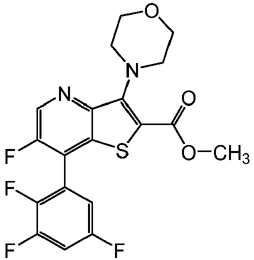

Under inert nitrogen atmosphere, K₂CO₃ (99 mg, 0.71 mmol) and morpholine (124 mg, 1.43 mmol) was added to a solution of methyl 3-bromo-6-fluoro-7-(2,3,5-trifluorophenyl) thieno[3,2-b]pyridine-2-carboxylate (150 mg, 0.36 mmol) in DMF (1.5 mL). The resulting mixture was stirred overnight at 80°C. Upon completion of the reaction, H₂O was added and the resulting mixture was extracted with EtOAc. The combined organic layers were dried over anhydrous Na₂SO₄, filtered and concentrated under reduced pressure. The residue was purified by prep-TLC (PE: EtOAc = 4: 1) to give methyl 6-fluoro-3-(morpholin-4-yl)-7-(2,3,5-trifluorophenyl) thieno [3,2-b] pyridine-2-carboxylate.

### Example 6.4 (b)

### 6-Fluoro-3-morpholino-7-(2,3,5-trifluorophenyl)thieno[3,2-b]pyridine-2-carboxylic acid

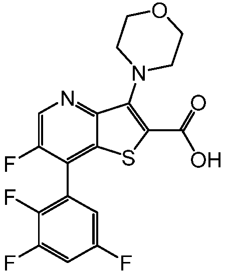

To a solution of methyl 6-fluoro-3-(morpholin-4-yl)-7-(2,3,5-trifluorophenyl)thieno[3,2-b]pyridine-2-carboxylate (50 mg, 0.10 mmol) in THF (0.9 mL) was added LiOH (15 mg, 0.61 mmol) and H₂O (0.9 mL). The resulting mixture was stirred overnight at rt. The reaction mixture was concentrated under reduced pressure and then diluted with H₂O (5 mL). The mixture was acidified to pH 3~5 by addition of aq. HCl (1 M). The aq. solution was then extracted with EtOAc. The combined organic layers were dried over anhydrous Na₂SO₄, filtered and concentrated under reduced pressure to afford 6-fluoro-3-(morpholin-4-yl)-7-(2,3,5-trifluorophenyl) thieno [3,2-b] pyridine-2-carboxylic acid.

### Example 6.4 (c)

### N-(2,3-Dihydro-4H-benzo[b][1,4]oxazin -4-yl)-6-fluoro-3-morpholino-7-(2,3,5-trifluorophenyl)thieno[3,2-b]pyridine-2-carboxamide

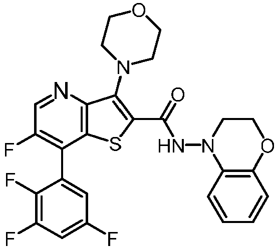

To a solution of 6-fluoro-3-(morpholin-4-yl)-7-(2,3,5-trifluorophenyl)thieno [3,2-b] pyridine-2-carboxylic acid (80 mg, 0.19 mmol) in DMF (3 mL) was added HATU (89 mg, 0.23 mmol), DIPEA (53 mg, 0.41 mmol) and 2,3-dihydro-1,4-benzoxazin-4-amine (38 mg, 0.25 mmol). The resulting mixture was stirred overnight at rt, then treated with H₂O, and extracted with EtOAc. The combined organic layers were washed with brine, dried over anhydrous Na₂SO₄, filtered, and concentrated under reduced pressure. The residue was purified by prep-TLC (PE: EtOAc = 4: 1) to afford N-(2,3-dihydro-1,4-benzoxazin-4-yl)-6-fluoro-3-(morpholin-4-yl)-7-(2,3,5-trifluorophenyl) thieno [3,2-b] pyridine-2-carboxamide. ¹H-NMR (400 MHz, chloroform-d) δ [ppm] 11.57 (s, 1H), 8.75 (d, 1H), 7.22-7.08 (m, 2H), 6.92-6.82 (m, 4H), 4.56-4.48 (m, 2H), 3.84 (t, 4H), 3.75-3.57 (m, 6H). LCMS (method G2) Rt= 1.60; m/z= 545.2 [M+H]⁺.

### Example 6.5

### N-(2,3-Dihydro-4H-benzo[b][1,4]oxazin-4-yl)-3-(2-hydroxypropan-2-yl)-7-(2,3,5-trifluorophenyl)thieno[3,2-b]pyridine-2-carboxamide

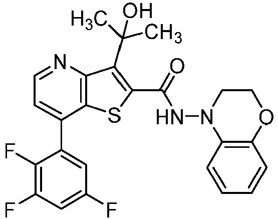

### Example 6.5 (a)

### 3-Fluoro-4-iodopicolinonitrile

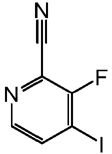

A solution of diisopropylamine (18.2 g, 180.18 mmol) in abs. THF (400 mL) was placed under inert nitrogen atmosphere. Then n-BuLi (68.8 mL, 172 mmol, 2.5 M in hexane) was added dropwise at -78°C. The resulting solution was stirred at -78°C for 30 min. Then 3-fluoropyridine-2-carbonitrile (20.0 g, 164 mmol) in abs. THF (20 mL) was added dropwise at -78°C. The resulting solution was stirred for 1 h at -78°C. Then, iodine (83.2 g, 328 mmol) was added. The resulting solution was stirred for 1 h at -78°C. The reaction was then quenched by the addition of H₂O and extracted with EtOAc. The combined organic layers were dried over anhydrous Na₂SO₄ and concentrated *in vacuo.* The residue was purified by silica gel column chromatography (EtOAc: PE = 0-1: 4) to give 3-fluoro-4-iodopyridine-2-carbonitrile.

### Example 6.5 (b)

### 3-Fluoro-4-(2,3,5-trifluorophenyl)picolinonitrile

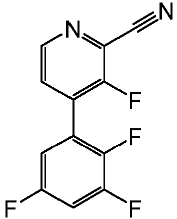

Under inert nitrogen atmosphere, Pd(dppf)Cl₂ (3.80 g, 4.03 mmol) and K₂CO₃ (11.15 g, 80.64 mmol) was added to a solution of 3-fluoro-4-iodopyridine-2-carbonitrile (10.0 g, 40.3 mmol) and 2,3,5-trifluorophenylboronic acid (8.50 g, 48.4 mmol) in 1,4-dioxane (200 m) and H₂O (50 mL). The resulting mixture was stirred overnight at 75°C. After cooling down to rt, EtOAc was added. The resulting mixture was washed with H₂O and dried over anhydrous Na₂SO₄, filtered and concentrated under reduced pressure. The residue was purified by silica gel column chromatography (EtOAc: PE = 0-1: 2) to give 3-fluoro-4-(2,3,5-trifluorophenyl)pyridine -2-carbonitrile.

### Example 6.5 (c)

### Methyl 3-amino-7-(2,3,5-trifluorophenyl)thieno[3,2-b]pyridine-2-carboxylate

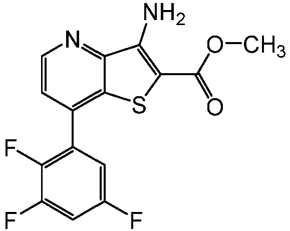

Under inert nitrogen atmosphere, methyl thioglycolate (6.15 g, 57.94 mmol) and K₂CO₃ (8.00 g, 57.89 mmol) was added to a solution of 3-fluoro-4-(2,3,5-trifluorophenyl) pyridine-2-carbonitrile (7.30 g, 28.95 mmol) in CH₃CN (50 mL). The resulting mixture was stirred for 3 h at 80°C. After cooling down to rt, the reaction mixture was treated with H₂O/ice. The solids were collected by filtration and dried *in vacuo* to give methyl 3-amino-7-(3,5-difluorophenyl)thieno[3,2-b]pyridine-2-carboxylate.

### Example 6.5 (d)

### Methyl 3-bromo-7-(2,3,5-trifluorophenyl) thieno [3,2-b] pyridine-2-carboxylate

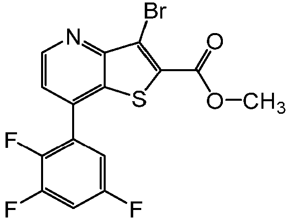

To methyl 3-amino-7-(3,5-difluorophenyl)thieno[3,2-b]pyridine-2-carboxylate (5.00 g, 15.61 mmol) was added HBr (50 mL, 30% HBr in AcOH) and CuBr (2.46 g, 17.17 mmol). Then a solution of NaNO₂ (1.18 g, 17.17 mmol) in H₂O (5 mL) was added dropwise at 0°C. The resulting solution was stirred overnight at rt. Upon completion of the reaction, H₂O/ice was added. The solids were collected by filtration and dried *in vacuo* to give methyl 3-bromo-7-(3,5-difluorophenyl) thieno [3,2-b] pyridine-2-carboxylate.

### Example 6.5 (e)

### Methyl 3-acetyl-7-(2,3,5-trifluorophenyl)thieno[3,2-b]pyridine-2-carboxylate

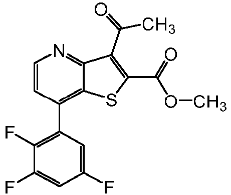

Under inert nitrogen atmosphere, to a solution of methyl 3-bromo-7-(2,3,5-trifluorophenyl)thieno[3,2-b]pyridine-2-carboxylate (500 mg, 1.24 mmol) in 1,4-dioxane (5 mL) was added tributyl(1-ethoxyethenyl)stannane (1.35 g, 3.73 mmol), Pd(PPh₃)₄ (144 mg, 0.12 mmol). The resulting mixture was stirred overnight at 100°C. After cooling down to rt, 1 M HCl (5 mL) was added and the resulting mixture was stirred for 2 h at rt. The mixture was extracted with EtOAc. The organic layers were combined, dried over anhydrous Na₂SO₄, filtered and concentrated under reduced pressure. The residue was purified by purified by prep-TLC (EtOAc: PE = 1 : 2) to afford methyl 3-acetyl-7-(2,3,5-trifluorophenyl)thieno[3,2-b]pyridine-2-carboxylate.

### Example 6.5 (f)

### 3-acetyl-7-(2,3,5-trifluorophenyl) thieno [3,2-b] pyridine-2-carboxylic acid

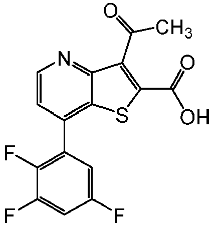

To a solution of methyl 3-acetyl-7-(2,3,5-trifluorophenyl)thieno[3,2-b]pyridine-2-carboxylate (430 mg, 1.18 mmol) in THF (4 mL) was added LiOH (141 mg, 5.89 mmol) and H₂O (4 mL). The resulting solution was stirred for 2 h at rt. The pH value of the solution was adjusted to 5 by addition of aq. HCl (1 M). The resulting solution was extracted with EtOAc. The organic layers were combined, dried over anhydrous Na₂SO₄, filtered and concentrated *in vacuo* to afford 3-acetyl-7-(2,3,5-trifluorophenyl) thieno [3,2-b] pyridine-2-carboxylic acid.

### Example 6.5 (g)

### 3-Acetyl-N-(2,3-dihydro-1,4-benzoxazin-4-yl)-7-(2,3,5-trifluorophenyl) thieno [3,2-b] pyridine-2-carboxamide

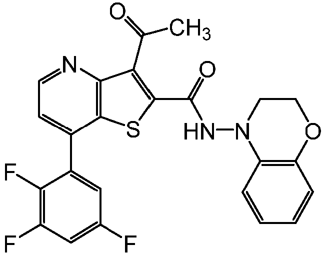

To a solution of 3-acetyl-7-(2,3,5-trifluorophenyl)thieno[3,2-b]pyridine-2-carboxylic acid (360 mg, 1.03 mmol) in DMF (4 mL) was added HATU (468 mg, 1.23 mmol), DIPEA (397 mg, 3.07 mmol), and 2,3-dihydro-1,4-benzoxazin-4-amine (200 mg, 1.33 mmol). The resulting solution was stirred overnight at rt. The mixture was then treated with H₂O and extracted with EtOAc. The combined organic layers were dried over anhydrous Na₂SO₄, filtered and concentrated under reduced pressure. The residue was purified by prep-TLC (EtOAc: PE = 1:4) to give 3-acetyl-N-(2,3-dihydro-1,4-benzoxazin-4-yl)-7-(2,3,5-trifluorophenyl)thieno[3,2-b]pyridine-2-carboxamide.

### Example 6.5 (h)

### N-(2,3-Dihydro-1,4-benzoxazin-4-yl)-3-(2-hydroxypropan-2-yl)-7-(2,3,5-trifluorophenyl) thieno[3,2-b]pyridine-2-carboxamide

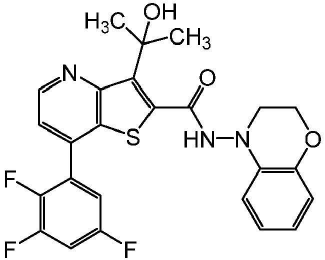

Under inert nitrogen atmosphere, CH₃MgBr (1.5 mL, 1.55 mmol, 1M abs. THF) was added to a solution of 3-acetyl-N-(2,3-dihydro-1,4-benzoxazin-4-yl)-6-fluoro-7-(2,3,5-trifluorophenyl)thieno[3,2-b]pyridine-2-carboxamide (150 mg, 0.31 mmol) in abs. THF (2 mL) at 0°C. The resulting solution was stirred for 2 h at 0°C. After this time, the mixture was treated with aq. sat. NH₄Cl-solution and extracted with EtOAc. The combined organic layers were dried over anhydrous Na₂SO₄, filtered and concentrated under reduced pressure. The residue was purified by prep-HPLC [Mobile Phase A: H₂O (0.1%FA), Mobile Phase B: CH₃CN; Gradient: 51% B to 68% B in 8 min] to afford N-(2,3-dihydro-1,4-benzoxazin-4-yl)-3-(2-hydroxypropan-2-yl)-7-(2,3,5-trifluorophenyl) thieno[3,2-b]pyridine-2-carboxamide. ¹H-NMR (400 MHz, DMSO-d₆) δ [ppm] 10.37 (s, 1H), 8.99 (s, 1H), 7.97-7.66 (m, 3H), 6.98 (dd, 4H), 5.69 (s, 1H), 4.34 (m, 2H), 3.59 (s, 2H), 1.80-1.67 (s, 6H). LCMS (method F1) Rt= 1.28; m/z= 502.00 [M+H]⁺.

### Example 6.6

### N-(2,3-dihydro-4H-benzo[b][1,4]oxazin-4-yl)-3-(3-fluoroazetidin-1-yl)-7-(2,3,5-trifluorophenyl)thieno[3,2-b]pyridine-2-carboxamide

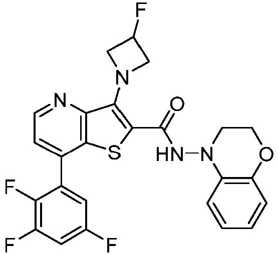

### Example 6.6 (a)

### Methyl 3-(3-fluoroazetidin-1-yl)-7-(2,3,5-trifluorophenyl)thieno[3,2-b]pyridine-2-carboxylate

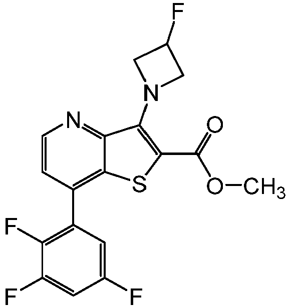

Under inert nitrogen atmosphere, 3-fluoroazetidine·HCl (70 mg, 0.93 mmol), K₃PO₄ (264 mg, 1.24 mmol) and tetrakis(triphenylphosphine)nickel(0) (69 mg, 0.06 mmol) was added to a solution of methyl 3-bromo-7-(2,3,5-trifluorophenyl)thieno[3,2-b]pyridine-2-carboxylate (250 mg, 0.62 mmol) in 1,4-dioxane (5.5 mL). The resulting mixture was stirred overnight at 100°C. After cooling down to rt, EtOAc was added. The resulting mixture was washed with H₂O, dried over anhydrous Na₂SO₄, filtered and concentrated under reduced pressure. The residue was purified by silica gel column chromatography (EtOAc: PE = 0- 1: 1) to give methyl 3-(3-fluoroazetidin-1-yl)-7-(2,3,5-trifluorophenyl)thieno[3,2-b]pyridine-2-carboxylate.

### Example 6.6 (b)

### 3-(3-Fluoroazetidin-1-yl)-7-(2,3,5-trifluorophenyl)thieno[3,2-b]pyridine-2-carboxylic acid

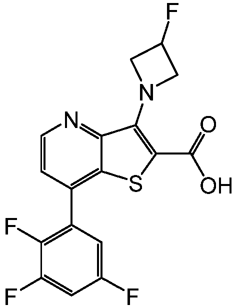

To a solution of methyl 3-(3-fluoroazetidin-1-yl)-7-(2,3,5-trifluorophenyl)thieno[3,2-b]pyridine-2-carboxylate (200 mg, 0.51 mmol) in THF (10 mL), LiOH (60 mg, 2.52 mmol) and H₂O (10 mL) were added. The resulting solution was stirred for 3 days at 60°C. After this time, THF was evaporated under reduced pressure, H₂O was added, and the pH value of the mixture was adjusted to 4 by addition of aq. HCl (1 M). The resulting solution was extracted with EtOAc. The combined organic layers were dried over anhydrous Na₂SO₄, filtered and concentrated *in vacuo* to give 3-(3-fluoroazetidin-1-yl)-7-(2,3,5-trifluorophenyl)thieno[3,2-b]pyridine-2-carboxylic acid.

### Example 6.6 (c)

### N-(2,3-Dihydro-4H-benzo[b][1,4]oxazin-4-yl)-3-(3-fluoroazetidin-1-yl)-7-(2,3,5-trifluorophenyl)thieno[3,2-b]pyridine-2-carboxamide

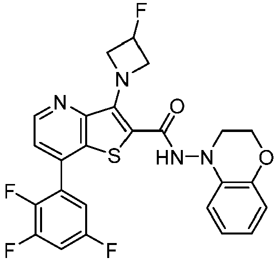

To a solution of 3-(3-fluoroazetidin-1-yl)-7-(2,3,5-trifluorophenyl)thieno[3,2-b]pyridine-2-carboxylic acid (160 mg, 0.42 mmol) in DMF (5 mL), was added 2,3-dihydro-1,4-benzoxazin-4-amine (75 mg, 0.50 mmol), HATU (239 mg, 0.63 mmol), and DIPEA (162 mg, 1.26 mmol). The resulting solution was stirred overnight at rt. Upon completion of the reaction, H₂O was added and the resulting solution was extracted with EtOAc. The combined organic layers were dried over anhydrous Na₂SO₄, filtered and concentrated under reduced pressure. The residue was purified by prep-HPLC [Mobile Phase A: H₂O (10 mmol/L NH₄HCO₃+ 0.1%NH₃·H₂O), Mobile Phase B: CH₃CN; Gradient: 48% B to 78% B in 7 min] to afford N-(2,3-dihydro-1,4-benzoxazin-4-yl)-3-

(3-fluoroazetidin-1-yl)-7-(2,3,5-trifluorophenyl)thieno[3,2-b]pyridine-2-carboxamide. ¹H-NMR (400 MHz, DMSO-d₆) δ [ppm] 10.29 (s, 1H), 8.82 (s, 1H), 7.83 (s, 1H), 7.68-7.48 (m, 2H), 6.75-6.369 (m, 4H), 5.48 (d, 1H), 4.72- 4.65 (m, 2H), 4.48-4.32 (m, 4H), 3.56-3.51 (m, 2H). LCMS (method E1) Rt= 143; m/z= 514.95 [M+H]⁺.

### Example 6.7

### N-(2,3-Dihydro-4H-benzo[b][1,4]oxazin-4-yl)-3-isopropyl-7-(2,3,5-trifluorophenyl)-thieno[3,2-b]pyridine-2-carboxamide

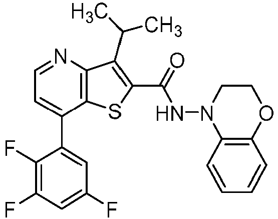

### Example 6.7 (a)

### Methyl 3-(prop-1-en-2-yl)-7-(2,3,5-trifluorophenyl)thieno[3,2-b]pyridine-2-carboxylate

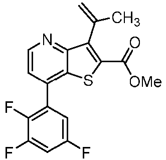

Under inert nitrogen atmosphere, to a solution of methyl 3-bromo-7-(2,3,5-trifluorophenyl)thieno[3,2-b]pyridine -2-carboxylate (500 mg, 1.24 mmol) in THF (4 mL) and H₂O (2 mL) was added 4,4,5,5-tetramethyl-2-(prop-1-en-2-yl)-1,3,2-dioxaborolane (313 mg, 1.87 mmol), Pd(DtBPF)Cl₂ (81 mg, 0.12 mmol) and K₃PO₄ (528 mg, 2.49 mmol). The mixture was stirred for 3 h at 90°C. After cooling down to rt, the reaction mixture was diluted with EtOAc, washed with H₂O, dried over anhydrous Na₂SO₄, filtered and concentrated under reduced pressure. The residue was purified by silica gel column chromatography (EtOAc: PE = 0- 2: 1) to give methyl 3-(prop-1-en-2-yl)-7- (2,3,5-trifluorophenyl)thieno[3,2-b]pyridine-2-carboxylate.

### Example 6.7 (b)

### Methyl 3-isopropyl-7-(2,3,5-trifluorophenyl)thieno[3,2-b]pyridine-2-carboxylate

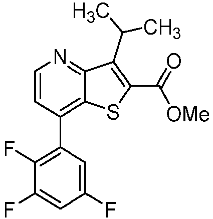

To a solution of methyl 3-(prop-1-en-2-yl)-7-(2,3,5-trifluorophenyl)thieno[3,2-b]- pyridine-2-carboxylate (342 mg, 0.94 mmol) in EtOAc (20 mL), was added PtO₂ (21 mg, 0.09 mmol). The reaction was stirred for 2 h at rt under hydrogen atmosphere. The reaction mixture was filtered and concentrated under reduced pressure. The residue was purified by silica gel column chromatography (EtOAc: PE = 0- 2: 1) to give methyl 3-isopropyl-7-(2,3,5-trifluorophenyl)thieno[3,2-b]pyridine-2-carboxylate.

### Example 6.7 (c)

### 3-Isopropyl-7-(2,3,5-trifluorophenyl)thieno[3,2-b]pyridine-2-carboxylic acid

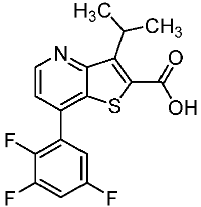

To a solution of methyl 3-isopropyl-7-(2,3,5-trifluorophenyl)thieno[3,2-b]pyridine-2-carboxylate (260 mg, 0.71 mmol) in THF (10 mL) was added LiOH (85 mg, 3.56 mmol) and H₂O (10 mL). The resulting mixture was stirred overnight at rt. Upon completion of the reaction, THF was evaporated under reduced pressure, H₂O was added, and the pH was adjusted to 4 by addition of aq. HCl (1 M). The resulting mixture was extracted with EtOAc. The combined organic layers were dried over anhydrous Na₂SO₄, filtered and concentrated *in vacuo* to give 3-isopropyl-7-(2,3,5-trifluorophenyl)thieno[3,2-b]pyridine-2-carboxylic acid.

### Example 6.7 (d)

### N-(2,3-Dihydro-4H-benzo[b][1,4]oxazin-4-yl)-3-isopropyl-7-(2,3,5-trifluorophenyl)-thieno[3,2-b]pyridine-2-carboxamide

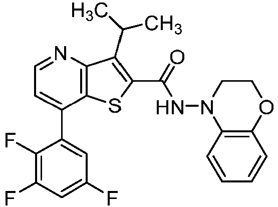

To a solution of 3-isopropyl-7-(2,3,5-trifluorophenyl)thieno[3,2-b]pyridine-2-carboxylic acid (150 mg, 0.43 mmol) in DMF (5 mL) was added 2,3-dihydro-1,4-benzoxazin-4-amine (128 mg, 0.85 mmol), HATU (243 mg, 0.64 mmol) and DIPEA (166 mg, 1.28 mmol). The resulting mixture was stirred at rt for 30 min. Upon completion of the reaction, H₂O was added, and the resulting solution was extracted with EtOAc. The combined organic layers were dried over anhydrous Na₂SO₄, filtered and concentrated under reduced pressure. The residue was purified by prep-HPLC [Mobile Phase A: H₂O (0.1% FA), Mobile Phase B: CH₃CN; Gradient: 57% B to 73% B in 8 min] to afford N-(2,3-dihydro-1,4-benzoxazin-4-yl) -3-isopropyl-7-(2,3,5-trifluorophenyl)thieno[3,2-b]pyridine-2-carboxamide. ¹H-NMR (300 MHz, DMSO-d₆) δ [ppm] 10.65 (s, 1H), 8.92 (d, 1H), 7.88-7.79 (m, 1H), 7.64-7.63 (m, 1H), 7.58-7.53 (m, 1H), 6.87-6.68 (m, 4H), 4.36-4.34 (m, 2H), 3.97-3.88 (m, 1H), 3.63-3.59 (m, 2H), 1.53 (d, 6H). LCMS (method F2) Rt= 1.32; m/z= 484.00 [M+H]⁺.

### Example 6.8

### N-(2,3-Dihydro-4H-benzo[b][1,4]oxazin-4-yl)-3-morpholino-7-(2,3,5-trifluorophenyl)thieno[3,2-b]pyridine-2-carboxamide

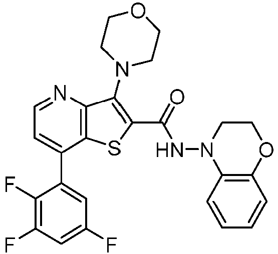

### Example 6.8 (a)

### Methyl 3-morpholino-7-(2,3,5-trifluorophenyl)thieno[3,2-b]pyridine-2-carboxylate

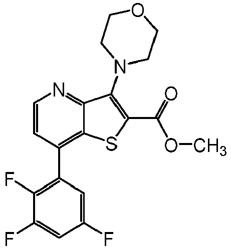

Under inert nitrogen atmosphere, methyl 3-amino-7-(2,3,5-trifluorophenyl)thieno[3,2-b]- pyridine-2-carboxylate (200 mg, 0.59 mmol), 1-bromo-2-(2-bromoethoxy)ethane (165 mg, 0.71 mmol), and Cs₂CO₃ (385 mg, 1.18 mmol) were mixed in DMF (5 mL). The resulting mixture was stirred overnight at 100°C. After cooling down to rt, the resulting mixture was treated with H₂O and extracted with EtOAc. The organic layers were combined, dried over anhydrous Na₂SO₄, filtered and concentrated under reduced pressure. The residue was purified by silica gel column chromatography (EtOAc: PE = 0-2: 1) to afford methyl 3-(morpholin-4-yl)-7-(2,3,5-trifluorophenyl)thieno[3,2-b]pyridine-2-carboxylate.

### Example 6.8 (b)

### 3-Morpholino-7-(2,3,5-trifluorophenyl) thieno [3,2-b] pyridine-2-carboxylic acid

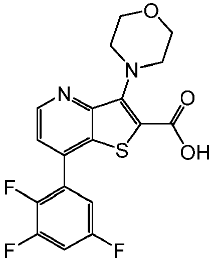

To a solution of methyl 3-(morpholin-4-yl)-7-(2,3,5-trifluorophenyl)thieno[3,2-b]- pyridine-2-carboxylate (140 mg, 0.34 mmol) in THF (10 mL) was added LiOH (41 mg, 1.71 mmol) and H₂O (10 mL). The resulting solution was stirred overnight at rt. Upon completion of the reaction, THF was evaporated under reduced pressure, H₂O was added, and the pH value of the mixture was adjusted to 4 by addition of aq. HCl (1 M). The resulting solution was extracted with EtOAc. The combined organic layers were dried over anhydrous Na₂SO₄ and concentrated *in vacuo* to give 3-(morpholin-4-yl)-7-(2,3,5-trifluorophenyl)thieno[3,2-b]pyridine-2-carboxylic acid.

### Example 6.8 (c)

### N-(2,3-Dihydro-4H-benzo[b][1,4]oxazin-4-yl)-3-morpholino-7-(2,3,5-trifluorophenyl) thieno[3,2-b]pyridine-2-carboxamide

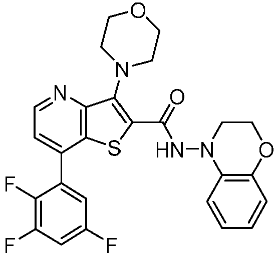

To a solution of 3-(morpholin-4-yl)-7-(2,3,5-trifluorophenyl)thieno[3,2-b]pyridine-2-carboxylic acid (135 mg, 0.34 mmol) in DMF (10 mL) was added HATU (195 mg, 0.51 mmol), DIPEA (133 mg, 1.03 mmol) and 2,3-dihydro-1,4-benzoxazin-4-amine (56 mg, 0.38 mmol). The resulting solution was stirred overnight at rt. Upon completion of the reaction, H₂O was added, and the resulting mixture was extracted with EtOAc. The combined organic layers were dried over anhydrous Na₂SO₄ and concentrated under reduced pressure. The residue was purified by prep-HPLC [Mobile Phase A: H₂O (10 mmol/L NH₄HCO₃ + 0.1% NH₃·H₂O), Mobile Phase B: CH₃CN; Gradient: 48% B to 78% B in 7 min] to afford N-(2,3-dihydro-1,4-benzoxazin-4-yl)-3-(morpholin-4-yl)-7-(2,3,5-trifluorophenyl)thieno[3,2-b]pyridine-2-carboxamide. ¹H-NMR (400 MHz, DMSO-d₆) δ [ppm] 10.16 (s, 1H), 8.68 (s, 1H), 8.35-8.39 (m, 1H), 7.70-7.77 (m, 2H), 7.47-7.51 (m, 1H), 7.40-7.42 (m, 1H), 7.29-7.31 (m, 1H), 7.13-7.15 (m, 1H), 6.90-6.92 (m, 1H), 6.80-6.82 (m, 1H), 4.38-4.40 (m, 1H), 4.17-4.20 (m, 1H), 4.05-4.08 (m, 1H), 3.11-3.15 (m, 2H), 2.22-2.32 (m, 2H), 1.20-1.23 (m, 3H). LCMS (method G2) Rₜ= 1.83; m/z= 527.30 [M+H]⁺.

### Example 7.1

### N-(2,3-dihydro-4H-benzo[b][1,4]oxazin-4-yl)-3-isopropyl-8-(2,3,5-trifluorophenyl)imidazo[1,2-a]pyridine-2-carboxamide

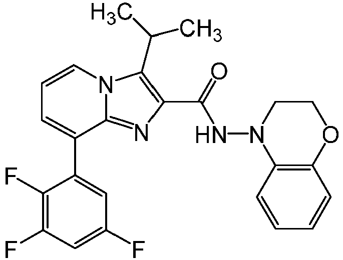

### Example 7.1 (a)

### Ethyl 8-(2,3,5-trifluorophenyl)imidazo[1,2-a]pyridine-2-carboxylate

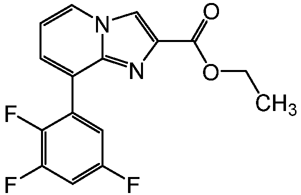

Under inert nitrogen atmosphere, ethyl 8-bromoimidazo[1,2-a]pyridine-2-carboxylate (1.00 g, 3.72 mmol), 2,3,5-trifluorophenylboronic acid (784 mg, 4.46 mmol), K₂CO₃ (1.03 g, 7.43 mmol) and Pd(PPh₃)₄ (315 mg, 0.27 mmol) were mixed in 1,4-dioxane (20 mL) and H₂O (4 mL). The resulting solution was stirred overnight at 75°C. After cooling down to rt, the reaction mixture was diluted with EtOAc, washed with H₂O, dried over anhydrous Na₂SO₄, filtered and concentrated under reduced pressure. The residue was purified by silica gel column chromatography (PE: EtOAc= 3: 1) to give ethyl 8-(2,3,5-trifluorophenyl) imidazo[1,2-a]pyridine-2-carboxylate.

### Example 7.1 (b)

### Ethyl 3-bromo-8-(2,3,5-trifluorophenyl)imidazo[1,2-a]pyridine-2-carboxylate

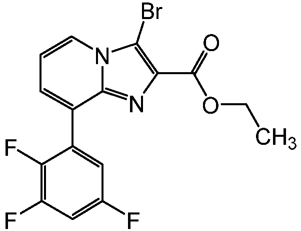

A solution of ethyl 8-(2,3,5-trifluorophenyl)imidazo[1,2-a]pyridine-2-carboxylate (650 mg, 2.03 mmol), CH₂Cl₂ (20 mL), and NBS (542 mg, 3.04 mmol) was stirred for 1 h at rt. Upon completion of the reaction, the resulting solution was extracted with CH₂Cl₂. The organic layers were combined, dried over anhydrous Na₂SO₄, filtered and concentrated under reduced pressure. The residue was purified by silica gel column chromatography (PE: EtOAc = 4: 1) to give ethyl 3-bromo-8-(2,3,5-trifluorophenyl)imidazo[1,2-a] pyridine-2-carboxylate.

### Example 7.1 (c)

### Ethyl 3-(prop-1-en-2-yl)-8-(2,3,5-trifluorophenyl)imidazo[1,2-a]pyridine-2-carboxylate

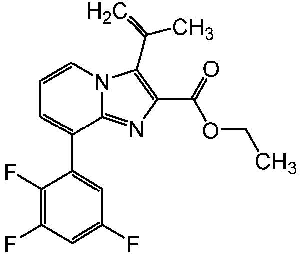

Under inert nitrogen atmosphere, ethyl 3-bromo-8-(2,3,5-trifluorophenyl)imidazo[1,2-a] pyridine-2-carboxylate (650.00 mg, 1.63 mmol), 4,4,5,5-tetramethyl-2-(prop-1-en-2-yl)-1,3,2-dioxaborolane (328 mg, 1.95 mmol), Pd(dppf)Cl₂·CH₂Cl₂ (132 mg, 0.16 mmol), and K₂CO₃ (450 mg, 3.26 mmol) were mixed in 1,4-dioxane (20 mL) and H₂O (4 mL). The resulting solution was stirred overnight at 80°C. After cooling down to rt, the mixture was diluted with EtOAc, washed with H₂O, dried over anhydrous Na₂SO₄, filtered and concentrated under reduced pressure. The residue was purified by silica gel column chromatography (PE: EtOAc = 3: 1) to give ethyl 3-(prop-1-en-2-yl)-8-(2,3,5-trifluorophenyl)imidazo[1,2-a]pyridine-2-carboxylate.

### Example 7.1 (d)

### Ethyl 3-isopropyl-8-(2,3,5-trifluorophenyl)imidazo[1,2-a]pyridine-2-carboxylate

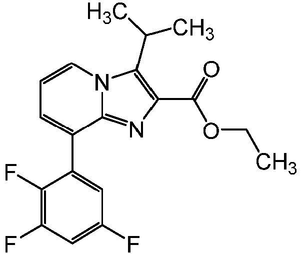

A mixture of ethyl 3-(prop-1-en-2-yl)-8-(2,3,5-trifluorophenyl)imidazo[1,2-a]pyridine-2-carboxylate (500 mg, 1.44 mmol) and Pd/C (15 mg, 0.14 mmol) in EtOH (20 mL) was stirred under hydrogen gas (1 atm) at rt for 30 min. After this time, the mixture was filtered and concentrated *in vacuo* to give ethyl 3-isopropyl-8-(2,3,5-trifluorophenyl)imidazo[1,2-a]pyridine-2-carboxylate.

### Example 7.1 (e)

### 3-Isopropyl-8-(2,3,5-trifluorophenyl)imidazo[1,2-a]pyridine-2-carboxylic acid

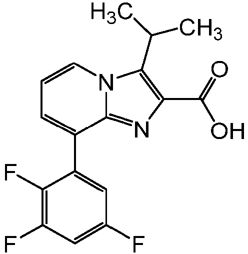

A solution of ethyl 3-isopropyl-8-(2,3,5-trifluorophenyl)imidazo[1,2-a]pyridine-2-carboxylate (150 mg, 0.41 mmol), THF (2 mL), MeOH (2 mL), H₂O (2 mL) and LiOH (49.57 mg, 2.07 mmol) was stirred for 2 h at rt. Upon completion of the reaction, THF was evaporated under reduced pressure, the pH value of the mixture was adjusted to 5 by addition of aq. HCl (1 M). The resulting mixture was extracted with EtOAc. The organic layers were combined, dried over anhydrous Na₂SO₄, filtered and concentrated *in vacuo* to give 3-isopropyl-8-(2,3,5-trifluorophenyl)imidazo[1,2-a]pyridine-2-carboxylic acid.

### Example 7.1 (f)

### N-(2,3-dihydro-4H-benzo[b][1,4] oxazin-4-yl)-3-isopropyl-8-(2,3,5-trifluorophenyl)-imidazo[1,2-a]pyridine-2-carboxamide

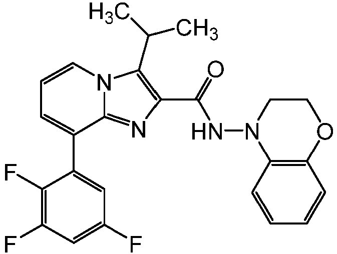

A solution of 3-isopropyl-8-(2,3,5-trifluorophenyl)imidazo[1,2-a]pyridine-2-carboxylic acid (130 mg, 0.39 mmol), 2,3-dihydro-1,4-benzoxazin-4-amine (88 mg, 0.58 mmol), HATU (222 mg, 0.58 mmol) and DIPEA (151 mg, 1.17 mmol) ) in DMF (5.00 mL) was stirred overnight at rt. After this time, the resulting mixture was treated with H₂O and extracted with EtOAc. The organic layers were combined, dried over anhydrous Na₂SO₄, filtered and concentrated under reduced pressure. The residue was purified by silica gel column chromatography (PE: EtOAc = 2: 1) to give crude product. The crude product (90 mg) was further purified by prep-HPLC [Mobile Phase A: H₂O (10 mmol/L NH₄HCO₃+0.1%NH₃·H₂O), Mobile Phase B: CH₃CN; Gradient: 53% B to 73% B in 10 min] to give N-(2,3-dihydro-4H-benzo[b][1,4]oxazin-4-yl)-3-isopropyl-8-(2,3,5-trifluorophenyl)imidazo[1,2-a]pyridine-2-carboxamide. ¹H-NMR (400 MHz, DMSO-d₆) δ [ppm] 9.40 (s, 1H), 8.34 (d, 1H), 7.44 (d, 1H), 7.39- 7.31 (m, 1H), 7.12-7.01 (m, 2H), 6.91 (d, 1H), 6.88-6.73 (m, 3H), 4.58-4.51 (m, 1H), 4.47-4.45 (m, 2H), 3.78-3.72 (m, 2H), 1.56 (d, 6H). LCMS (Method B1) Rₜ = 1.33 min; m/z= 467 (M+H)⁺ .

### Example 8.1

### N-(2,3-Dihydro-4H-benzo[b][1,4]oxazin-4-yl)-3-isopropyl-8-(2,3,5-trifluorophenyl)-imidazo[1,2-a]pyrazine-2-carboxamide

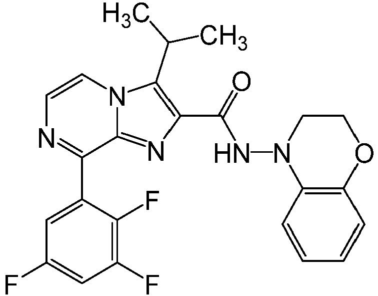

### Example 8.1 (a)

### Ethyl 8-chloroimidazo[1,2-a]pyrazine-2-carboxylate

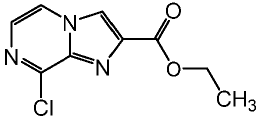

A solution of 3-chloropyrazin-2-amine (3.00 g, 23.2 mmol), ethyl 3-bromo-2-oxo-propanoate (4.52 g, 23.16 mmol) and NaHCO₃ (5.84 g, 69.52 mmol) in CH₃CN (60 mL) was stirred overnight at 80°C. After cooling down to rt, the mixture was filtered and concentrated under reduced pressure. The residue was purified by silica gel column chromatography (PE: EtOAc = 1: 1) to give ethyl 8-chloroimidazo[1,2-a]pyrazine-2-carboxylate.

### Example 8.1 (b)

### Ethyl 8-(2,3,5-trifluorophenyl)imidazo[1,2-a]pyrazine-2-carboxylate

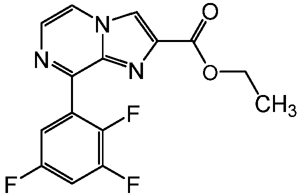

Under inert nitrogen atmosphere, a mixture of ethyl 8-chloroimidazo[1,2-a]pyrazine-2-carboxylate (1.00 g, 4.43 mmol), 2,3,5-trifluorophenylboronic acid (940 mg, 5.34 mmol), K₂CO₃ (1.2 g, 8.86 mmol) and Pd(PPh₃)₄ (512 mg, 0.44 mmol) in dioxane (20 mL) and H₂O (4 mL) was stirred overnight at 80°C. After cooling down to rt, the reaction mixture was diluted with EtOAc, washed with H₂O, dried over anhydrous Na₂SO₄, filtered and concentrated under reduced pressure. The residue was purified by silica gel column chromatography (PE: EtOAc = 3: 1) to give ethyl 8-(2,3,5-trifluorophenyl)imidazo[1,2-a] pyrazine-2-carboxylate.

### Example 8.1 (c)

### Ethyl 3-bromo-8-(2,3,5-trifluorophenyl)imidazo[1,2-a]pyrazine-2-carboxylate

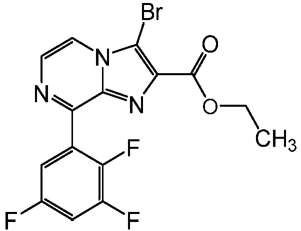

A solution of ethyl 8-(2,3,5-trifluorophenyl)imidazo[1,2-a]pyrazine-2-carboxylate (800 mg, 2.49 mmol), NBS (886 mg, 4.98 mmol) in CH₂Cl₂ (15 mL) was stirred overnight at 50°C. Upon completion of the reaction, the resulting mixture was extracted with CH₂Cl₂, washed with H₂O, dried over anhydrous Na₂SO₄, filtered and concentrated under reduced pressure. The residue was purified by silica gel column chromatography (PE: EtOAc = 3: 1) to give ethyl 3-bromo-8-(2,3,5-trifluorophenyl)imidazo[1,2-a]pyrazine-2-carboxylate.

### Example 8.1 (d)

### Ethyl 3-(prop-1-en-2-yl)-8-(2,3,5-trifluorophenyl)imidazo[1,2-a]pyrazine-2-carboxylate

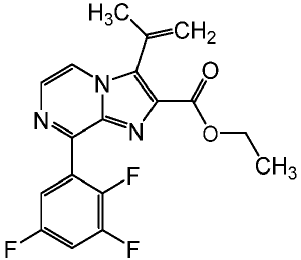

Under inert nitrogen atmosphere, a mixture of ethyl 3-bromo-8-(2,3,5-trifluorophenyl)-imidazo[1,2-a]pyrazine-2-carboxylate (500 mg, 1.25 mmol), 4,4,5,5-tetramethyl-2-(prop-1-en-2-yl)-1,2-oxaborolane (249 mg, 1.50 mmol), Pd(dppf)Cl₂·CH₂Cl₂ (102 mg, 0.13 mmol) and K₂CO₃ (345 mg, 2.50 mmol) in 1,4-dioxane (10 mL) and H₂O (2 mL) was stirred overnight at 80°C. After cooling down to rt, the reaction mixture was diluted with EtOAc, washed with H₂O, dried over anhydrous Na₂SO₄, filtered and concentrated under reduced pressure. The residue was purified by silica gel column chromatography (PE: EtOAc = 3: 1) to give ethyl 3-(prop-1-en-2-yl)-8-(2,3,5-trifluorophenyl)imidazo[1,2-a]pyrazine-2-carboxylate.

### Example 8.1 (e)

### Ethyl 3-isopropyl-8-(2,3,5-trifluorophenyl)imidazo[1,2-a]pyrazine-2-carboxylate

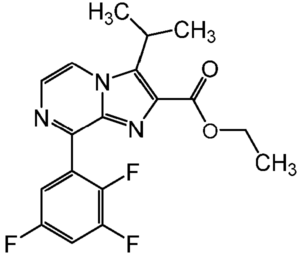

A mixture of ethyl 3-(prop-1-en-2-yl)-8-(2,3,5-trifluorophenyl)imidazo[1,2-a]pyrazine-2-carboxylate (360 mg, 1.00 mmol) and Pd/C (11 mg, 0.10 mmol) in EtOH (5 mL) was stirred under hydrogen atmosphere at rt for 30 min. The mixture was filtered and concentrated *in vacuo* to give ethyl 3-isopropyl-8-(2,3,5-trifluorophenyl)imidazo[1,2-a]pyrazine-2-carboxylate.

### Example 8.1 (f)

### Ethyl 3-isopropyl-8-(2,3,5-trifluorophenyl)imidazo[1,2-a]pyrazine-2-carboxylic acid

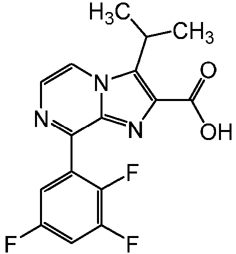

A solution of ethyl 3-isopropyl-8-(2,3,5-trifluorophenyl)imidazo[1,2-a]pyrazine-2-carboxylate (250 mg, 0.69 mmol) and LiOH (82 mg, 3.44 mmol) in MeOH (2 mL), THF (2 mL) and H₂O (2 mL) was stirred overnight at rt. Upon completion of the reaction, THF was evaporated under reduced pressure, the pH value of the mixture was adjusted to 5 by addition of aq. HCl (1 M). The resulting mixture was extracted with EtOAc. The organic layers were combined, dried over anhydrous Na₂SO₄, filtered and concentrated *in vacuo* to give 3-isopropyl-8-(2,3,5-trifluorophenyl)imidazo[1,2-a]pyrazine-2-carboxylic acid.

### Example 8.1 (g)

### N-(2,3-dihydro-4H-benzo[b][1,4] oxazin-4-yl)-3-isopropyl-8-(2,3,5-trifluorophenyl)-imidazo[1,2-a]pyrazine-2-carboxamide

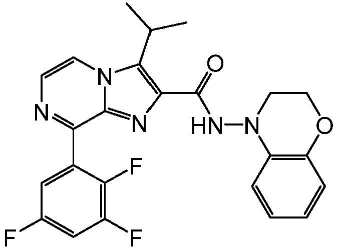

A solution of 3-isopropyl-8-(2,3,5-trifluorophenyl)imidazo[1,2-a]pyridine-2-carboxylic acid (200 mg, 0.60 mmol), 2,3-dihydro-1,4-benzoxazin-4-amine (135 mg, 0.90 mmol), HATU (341 mg, 0.90 mmol) and DIPEA (232 mg, 1.79 mmol) in DMF (5 mL) was stirred overnight at rt. Upon completion of the reaction, the resulting mixture was treated with H₂O and extracted with EtOAc. The organic layers were combined, dried over anhydrous Na₂SO₄, filtered and concentrated under reduced pressure. The crude product was purified by prep-HPLC [Mobile Phase A: H₂O (10 mmol/L NH₄HCO₃), Mobile Phase B: CH₃CN; Gradient: 41% B to 71% B in 7 min] to give N-(2,3-dihydro-4H-benzo[b][1,4]oxazin-4-yl)-3-isopropyl-8-(2,3,5-trifluorophenyl)imidazo[1,2-a]pyrazine-2-carboxamide. ¹H-NMR (400 MHz, DMSO-d₆) δ [ppm] 9.01 (s, 1H), 8.21 (d, 1H), 8.07 (d, 1H), 7.41-7.35 (m, 1H), 7.22-7.07 (m, 1H), 6.94- 6.74 (m, 4H), 4.56-4.40 (m, 3H), 3.79-3.70 (m, 2H), 1.57 (d, 6H). LCMS (Method B1) Rt= 1.27 min; m/z= 468 (M+H)⁺.

### Example 9.1

### N-(2,3-Dihydro-4H-benzo[b][1,4] oxazin-4-yl)-3-isopropyl-6-methyl-7-(2,3,5-trifluorophenyl)pyrazolo[1,5-a]pyrimidine-2-carboxamide

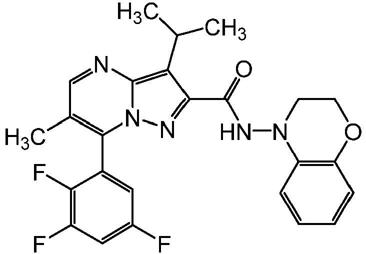

### Example 9.1 (a)

### 1-(2,3,5-Trifluorophenyl) propan-1-one

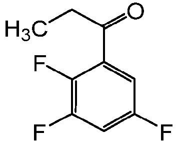

Under inert nitrogen atmosphere, isopropylmagnesium chloride (2.0 M solution in abs. THF, 36 mL, 71.1 mmol) was added to a solution of 1-bromo-2,3,5-trifluorobenzene (10 g, 47.4 mmol) in abs. THF (150 mL) at 0°C and the resulting mixture was stirred at 0°C for 30 min. Then, N-methoxy-N-methylpropanamide (5.55 g, 47.4 mmol) was added at 0°C and the reaction mixture was further stirred for 1 h at rt. After this time, the reaction was quenched with aq. sat. NH₄Cl-solution and extracted with EtOAc. The combined organic layers were dried over anhydrous Na₂SO₄, filetered and concentrated under reduced pressure. The residue was purified by silica gel column chromatography (PE: EtOAc= 4:1) to afford 1-(2,3,5-trifluorophenyl)propan-1-one.

### Example 9.1 (b)

### (E)-3-(Dimethylamino)-2-methyl-1-(2,3,5-trifluorophenyl)prop-2-en-1-one

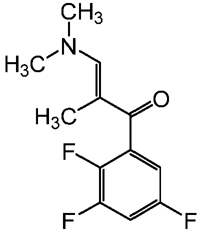

Under inert nitrogen atmosphere, a solution of 1-(2,3,5-trifluorophenyl)propan-1-one (6.9 g, 36.7 mmol) and DMF-DMA (17.48 g, 146.7 mmol) in DMF (110 mL) was stirred for 2 h at 120°C. After cooling down to rt, the reaction mixture was treated with H₂O and extracted with EtOAc. The combined organic layers were washed with brine and dried over anhydrous Na₂SO₄. After filtration, the filtrate was concentrated under reduced pressure. The residue was purified by silica gel column chromatography (PE: EtOAc= 4:1) to afford (2E)-3-(Dimethylamino)-2-methyl-1-(2,3,5-trifluorophenyl)prop-2-en-1-one.

### Example 9.1 (c)

### Ethyl 6-methyl-7-(2,3,5-trifluorophenyl)pyrazolo[1,5-a]pyrimidine-2-carboxylate

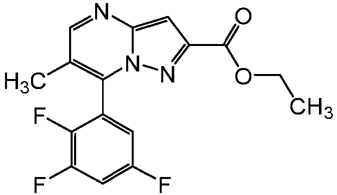

Under inert nitrogen atmosphere, (2E)-3-(dimethylamino)-2-methyl-1-(2,3,5-trifluorophenyl)prop-2-en-1-one (3.6 g, 14.8 mmol), AcOH (50 mL), piperidine (2.52 g, 29.6 mmol) and ethyl 5-amino-1H-pyrazole-3-carboxylate (2.30 g, 14.801 mmol) were mixed and stirred for 16 h at 80°C. After cooling down to rt, the reaction mixture was quenched with H₂O and extracted with EtOAc. The combined organic layers were washed with brine, dried over anhydrous Na₂SO₄, filtered and concentrated under reduced pressure. The residue was purified by silica gel column chromatography (PE: EtOAc= 2:1) to afford ethyl 6-methyl-7-(2,3,5-trifluorophenyl)pyrazolo[1,5-a]pyrimidine-2-carboxylate.

### Example 9.1 (d)

### Ethyl 3-bromo-6-methyl-7-(2,3,5-trifluorophenyl)pyrazolo[1,5-a]pyrimidine-2-carboxylate

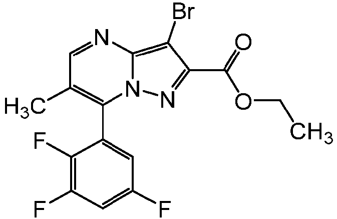

To a solution of ethyl 6-methyl-7-(2,3,5-trifluorophenyl)pyrazolo[1,5-a]pyrimidine-2-carboxylate (3.9 g, 11.6 mmol) in DMF (60 mL) was added NBS (3.11 g, 17.5 mmol). The reaction mixture was stirred for 3 h at rt. Upon completion of the reaction, the resulting mixture was quenched with H₂O and extracted with EtOAc. The combined organic layers were washed with brine, dried over anhydrous Na₂SO₄. After filtration, the filtrate was concentrated under reduced pressure and the residue was purified by silica gel column chromatography (PE: EtOAc= 2:1) to afford ethyl 3-bromo-6-methyl-7-(2,3,5-trifluorophenyl)pyrazolo[1,5-a]pyrimidine-2-carboxylate.

### Example 9.1 (e)

### Ethyl 6-methyl-3-(prop-1-en-2-yl)-7-(2,3,5-trifluorophenyl)pyrazolo[1,5-a]pyrimidine-2-carboxylate

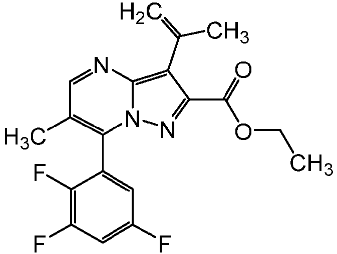

Under inert nitrogen atmosphere, ethyl 3-bromo-6-methyl-7-(2,3,5-trifluorophenyl) pyrazolo[1,5-a]pyrimidine-2-carboxylate (2 g, 4.83 mmol), prop-1-en-2-ylboronic acid (0.62 g, 7.24 mmol), K₂CO₃ (2.00 g, 14.5 mmol) and Pd(dppf)Cl₂ (353 mg, 0.483 mmol) were mixed in 1,4-dioxane (30 mL) and H₂O (6 mL). The reaction mixture was stirred for 3 h at 80°C under nitrogen atmosphere. After cooling down to rt, the resulting mixture was treated with H₂O and extracted with EtOAc. The combined organic layers were dried over anhydrous Na₂SO₄. After filtration, the filtrate was concentrated under reduced pressure and the residue was purified by silica gel column chromatography (PE: EtOAc= 2: 1) to afford ethyl 6-methyl-3-(prop-1-en-2-yl)-7-(2,3,5-trifluorophenyl)pyrazolo[1,5-a] pyrimidine-2-carboxylate.

### Example 9.1 (f)

### Ethyl 3-isopropyl-6-methyl-7-(2,3,5-trifluorophenyl) pyrazolo [1,5-a] pyrimidine-2-carboxylate

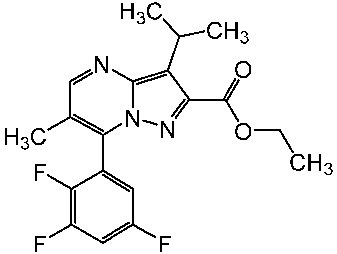

To a solution of ethyl 6-methyl-3-(prop-1-en-2-yl)-7-(2,3,5-trifluorophenyl)pyrazolo[1,5-a]pyrimidine-2-carboxylate (400 mg, 1.07 mmol) in MeOH (8 mL) was added Pd/C (40 mg, 10%).) The reaction mixture was stirred for 30 min under hydrogen gas (1 atm). The mixture was filtered and concentrated *in vacuo* to afford ethyl 3-isopropyl-6-methyl-7-(2,3,5-trifluorophenyl) pyrazolo[1,5-a]pyrimidine-2-caroxylate.

### Example 9.1 (g)

### 3-Isopropyl-6-methyl-7-(2,3,5-trifluorophenyl)pyrazolo[1,5-a]pyrimidine-2-carboxylic acid

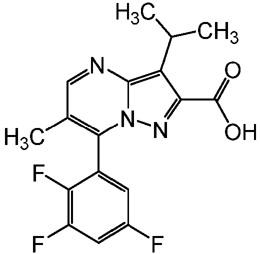

To a solution of ethyl 3-isopropyl-6-methyl-7-(2,3,5-trifluorophenyl)pyrazolo[1,5-a] pyrimidine-2-carboxylate (150 mg, 0.40 mmol) in THF (1.5 mL) and MeOH (1.5 mL) was added LiOH (4 7.60 mg, 1.99 mmol) and H₂O (1.5 mL). The reaction mixture was stirred for 16 h at rt. Upon completion of the reaction, the mixture was concentrated under reduced pressure, the pH value of the mixture was adjusted to 5 by addition of aq. HCl (1 M). The resulting mixture was extracted with EtOAc. The organic layers were combined, dried over anhydrous Na₂SO₄, filtered and concentrated *in vacuo* to afford 3-isopropyl-6-methyl-7-(2,3,5-trifluorophenyl)pyrazolo[1,5-a]pyrimidine-2-carboxylic acid.

### Example 9.1 (h)

### N-(2,3-Dihydro-4H-benzo[b][1,4]oxazin-4-yl)-3-isopropyl-6-methyl-7-(2,3,5-trifluorophenyl)-pyrazolo[1,5-a]pyrimidine-2-carboxamide

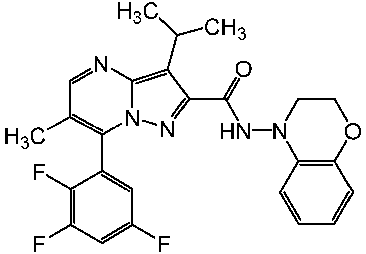

To a solution 3-isopropyl-6-methyl-7-(2,3,5-trifluorophenyl)pyrazolo[1,5-a]pyrimidine-2-carboxylic acid (150 mg, 0.43 mmol) in DMF (5 mL, 64.6 mmol), HATU (212 mg, 0.56 mmol), DIPEA (222 mg, 1.72 mmol) and 2,3-dihydro-1,4-benzoxazin-4-amine (64 mg, 0.43 mmol) were added. The reaction mixture was stirred for 1 h at rt. After this time, the mixture was treated with H₂O and extracted with EtOAc. The organic layers were combined, dried over anhydrous Na₂SO₄, filtered and concentrated under reduced pressure. The residue was purified by silica gel column chromatography (PE: EtOAc =2: 1) to give crude product. The crude product was re-purified by prep-HPLC [Mobile Phase A: H₂O (10 mmol/L NH₄HCO₃), Mobile Phase B: CH₃CN; Gradient: 49% B to 79% B in 7 min) to afford N-(2,3-dihydro-4H-benzo[b][1,4]oxazin-4-yl)-3-isopropyl-6-methyl-7-(2,3,5-tri-fluorophenyl)pyrazolo[1,5-a]pyrimidine-2-carboxamide. ¹H NMR (300 MHz, DMSO-d₆) δ ppm: 10.22 (s, 1H), 8.66 (s, 1H), 7.92-7.83 (m, 1H), 7.60-7.56 (m, 1H), 6.75-6.63 (m, 4H), 4.32-4.30 (m, 2H), 3.88-3.79 (m, 1H), 3.59-3.56 (m, 2H), 2.23 (s, 3H), 1.44-1.32 (m, 6H). LCMS (Method G2) Rₜ=1.27 min; m/z= 482 (M+H)⁺.

### Example 10.1

### N-(2,3-Dihydro-4H-benzo[b][1,4]oxazin-4-yl)-4-isopropyl-1-(2,3,5-trifluorophenyl)-1H-pyrazolo[3,4-b]pyridine-5-carboxamide

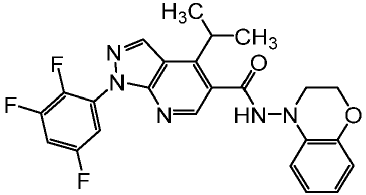

### Example 10.1 (a)

### (2,3,5-Trifluorophenyl)hydrazine hydrochloride

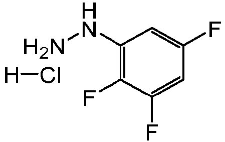

To a solution of 2,3,5-trifluoroaniline (5.00 g, 33.99 mmol) and conc. HCl (90 mL) was added NaNO₂ (2.81 g, 40.8 mmol) in H₂O (10 mL, 555 mmol) dropwise at -10°C. The resulting mixture was stirred at -10°C for 20 min. Then, SnCl₂ (9.67 g, 51.0 mmol) in conc. HCl (10 mL) was added dropwise at -10°C. The resulting mixture was stirred for 1 h at rt. The precipitated solids were collected by filtration and washed with concentrated HCl and dried *in vacuo* to give (2,3,5-trifluorophenyl) hydrazine HCl salt.

### Example 10.1 (b)

### 1-(2,3,5-Trifluorophenyl)-1H-pyrazol-5-amine

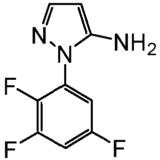

Under inert nitrogen atmosphere, (2,3,5-trifluorophenyl)hydrazine (3.2 g, 19.74 mmol), (2E)-3-methoxyprop-2-enenitrile (1.64 g, 19.74 mmol) and TFA (30 mL) were mixed. The resulting mixture was stirred for 3 h at 100°C. After cooling down to rt, the resulting mixture was concentrated under reduced pressure and extracted with EtOAc. The organic layers were combined, dried over anhydrous Na₂SO₄, filtered and concentrated under reduced pressure. The residue was purified by silica gel column chromatography (PE: EtOAc = 4: 1) to afford 1-(2,3,5-trifluorophenyl) pyrazol-3-amine.

### Example 10.1 (c)

### Diethyl 2-(((1-(2,3,5-trifluorophenyl)-1H-pyrazol-5-yl)amino)methylene)malonate

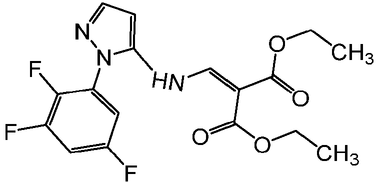

Under inert nitrogen atmosphere, a solution of 2-(2,3,5-trifluorophenyl)pyrazol-3-amine (1 g, 4.69 mmol) and diethyl(ethoxymethylene)malonate (1.22 g, 5.63 mmol) in toluene (10 mL) was stirred for 5 h at 100°C. After cooling down to rt, the resulting mixture was treated with H₂O and extracted with EtOAc. The organic layers were combined, dried over anhydrous Na₂SO₄, filtered and concentrated under reduced pressure. The residue was purified by silica gel column chromatography (PE: EtOAc = 4: 1) to give diethyl 2-(((1-(2,3,5-trifluorophenyl)-1H-pyrazol-5-yl)amino)methylene)malonate.

### Example 10.1 (d)

### Ethyl 4-chloro-1-(2,3,5-trifluorophenyl)-1H-pyrazolo[3,4-b] pyridine-5-carboxylate

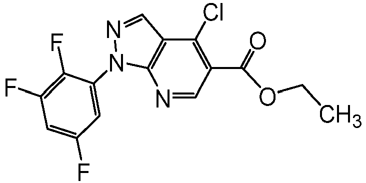

1,3-Diethyl 2-(((1-(2,3,5-trifluorophenyl)-1H-pyrazol-5-yl)amino)methylene)malonate (640 mg, 1.67 mmol) was treated with POCl₃ (6 mL). The resulting mixture was stirred overnight at 80°C. After cooling down to rt, the reaction mixture was concentrated and treated with ice H₂O and extracted EtOAc. The combined organic extracts were washed with H₂O, dried over anhydrous Na₂SO₄, filtered and concentrated under reduced pressure. The residue was purified by silica gel column chromatography (PE: EtOAc = 3: 1) to give ethyl 4-chloro-1-(2,3,5-trifluorophenyl)pyrazolo[3,4-b]pyridine-5-carboxylate.

### Example 10.1 (e)

### Ethyl 4-(prop-1-en-2-yl)-1-(2,3,5-trifluorophenyl)-1H-pyrazolo[3,4-b]pyridine-5-carboxylate

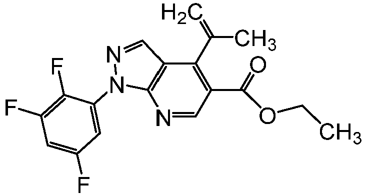

Under inert nitrogen atmosphere, a mixture of ethyl 4-chloro-1-(2,3,5-trifluorophenyl) pyrazolo[3,4-b]pyridine-5-carboxylate (308 mg, 0.87 mmol), 4,4,5,5-tetramethyl-2-(prop-1-en-2-yl)-1,3,2-dioxaborolane (175 mg, 1.04 mmol), Pd(dppf)Cl₂·CH₂Cl₂ (70.5 mg, 0.09 mmol) and K₂CO₃ (239 mg, 1.73 mmol) in dioxane (6 mL) and H₂O (1.5 mL) was stirred for 3 h at 80°C. After cooling down to rt, the reaction mixture was diluted with EtOAc, washed with H₂O, dried over anhydrous Na₂SO₄, filtered and concentrated under reduced pressure. The residue was purified by silica gel column chromatography (PE: EtOAc= 3: 1) to give ethyl 4-(prop-1-en-2-yl)-1-(2,3,5-trifluorophenyl)-1H-pyrazolo[3,4-b]pyridine-5-carboxylate.

### Example 10.1 (f)

### Ethyl 4-isopropyl-1-(2,3,5-trifluorophenyl)-1H-pyrazolo[3,4-b]pyridine-5-carboxylate

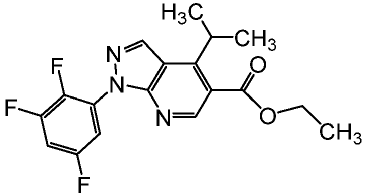

A mixture of ethyl 4-(prop-1-en-2-yl)-1-(2,3,5-trifluorophenyl)pyrazolo[3,4-b]pyridine-5-carboxylate (160 mg, 0.44 mmol) and Pd/C (16 mg) in EtOH (3 mL) was stirred under hydrogen gas (1 atm) for 3 h at rt. After this time, the mixture was filtered and concentrated *in vacuo* to give ethyl 4-isopropyl-1-(2,3,5-trifluorophenyl)-1H-pyrazolo[3,4-b]pyridine-5-carboxylate.

### Example 10.1 (g)

### 4-Isopropyl-1-(2,3,5-trifluorophenyl)-1H-pyrazolo [3,4-b]pyridine-5-carboxylic acid

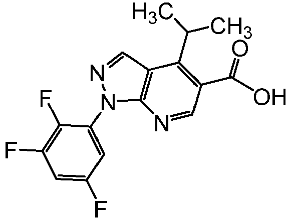

Ethyl 4-isopropyl-1-(2,3,5-trifluorophenyl)pyrazolo[3,4-b]pyridine-5-carboxylate (140 mg, 0.38 mmol) was treated with LiOH (46.1 mg, 1.93 mmol) in MeOH (1 mL), THF (1 mL) and H₂O (1 mL). The resulting solution was stirred for 3 h at rt. Upon completion of the reaction, the mixture was concentrated under reduced pressure, the pH value of the mixture was adjusted to 4 by addition of aq. HCl (1M). The resulting mixture was extracted with EtOAc. The organic layers were combined, dried over anhydrous Na₂SO₄, filtered and concentrated *in vacuo* to give 4-isopropyl-1-(2,3,5trifluorophenyl)-1H-pyrazolo[3,4-b]- pyridine-5-carboxylic acid.

### Example 10.1 (h)

### N-(2,3-Dihydro-4H-benzo[b][1,4]oxazin-4-yl)-4-isopropyl-1-(2,3,5-trifluorophenyl)-1H-pyrazolo[3,4-b]pyridine-5-carboxamide

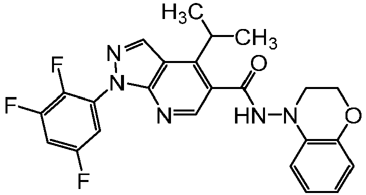

To a solution of 4-isopropyl-1-(2,3,5-trifluorophenyl)pyrazolo[3,4-b]pyridine-5-carboxylic acid (120 mg, 0.36 mmol) in DMF (3 mL) was added 2,3-dihydro-1,4-benzoxazin-4-amine (59 mg, 0.39 mmol), HATU (177 mg, 0.47 mmol) and DIPEA (139 mg, 1.07 mmol). The resulting solution was stirred for 2 h at rt. Upon completion of the reaction, the resulting mixture was treated with H₂O and extracted with EtOAc. The organic layers were combined, dried over anhydrous Na₂SO₄, filtered and concentrated under reduced pressure. The residue was purified by prep-HPLC [Mobile Phase A: H₂O (10 mmol/L NH₄HCO₃), Mobile Phase B: CH₃CN; Gradient: 37% B to 79% B in 10 min] with the following conditions to afford N-(2,3-dihydro-4H-benzo[b][1,4]oxazin-4-yl)-4-isopropyl-1-(2,3,5-trifluorophenyl)-1H-pyrazolo[3,4-b]pyridine-5-carboxamide. ¹H-NMR (300 MHz, 90°C, CD₃OD) δ [ppm]: 8.73 (s, 1H), 8.63 (s, 1H), 7.46-7.36 (m, 2H), 6.97-6.94 (d, 1H), 6.88-6.83 (m, 1H), 6.81-6.77 (d, 2H), 4.45-4.42 (m, 2H), 3.72-3.62 (m, 3H), 1.62-1.57 (d, 6H). LCMS (Method E1) Rt= 1.205 min; m/z= 468 (M+H)⁺.

### Example 10.2

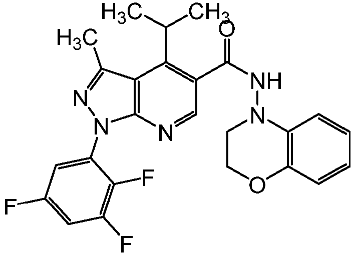

### Example 10.2 (a)

### (2,3,5-Trifluorophenyl)hydrazine hydrochloride

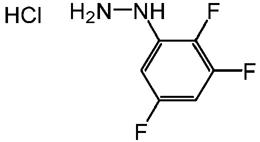

To a solution of 2,3,5-trifluoroaniline (6.00 g, 40.79 mmol) in conc. HCl (80 mL) was added NaNO₂ (3.38 g, 49.0 mmol) in H₂O (10 mL) dropwise at -10°C. The resulting mixture was stirred for 20 min at -10°C. To the above mixture was added SnCl₂ (11.6 g, 61.2 mmol) in conc. HCl (10 mL) dropwise at -10°C. The resulting mixture was stirred for additional 1 h at rt. The precipitated solids were collected by filtration and washed with conc. HCl.

### Example 10.2 (b)

### 3-Methyl-1-(2,3,5-trifluorophenyl)-1H-pyrazol-5-amine

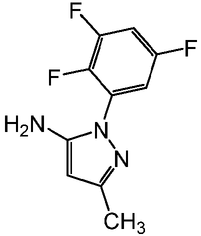

To a solution of (2,3,5-trifluorophenyl)hydrazine HCl salt (5.8 g, 35.8 mmol) in HCl (60 mL, 1 M), was added (2Z)-3-aminobut-2-enenitrile (2.94 g, 35.8 mmol). The resulting mixture was stirred overnight at 80°C under nitrogen atmosphere. After cooling down to rt, the reaction mixture was diluted with EtOAc, dried over anhydrous Na₂SO₄, filtered and concentrated under vacuum. The residue was purified by silica gel column chromatography (PE: EtOAc = 2: 1) to afford 5-methyl-2-(2,3,5-trifluorophenyl)pyrazol-3-amine.

### Example 10.2 (c)

### 4-Isopropyl-3-methyl-1-(2,3,5-trifluorophenyl)-1H-pyrazolo[3,4-b] pyridine-5-carboxylic acid

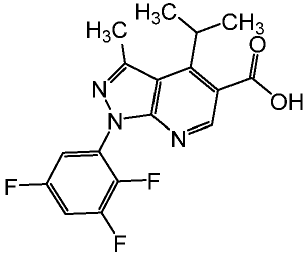

To a solution of 5-methyl-2-(2,3,5-trifluorophenyl)pyrazol-3-amine (1.00 g, 4.40 mmol) in diphenyl ether (6 mL), was added ethyl (2Z)-2-(ethoxymethylene)-4-methyl-3-oxo-pentanoate (1.89 g, 8.80 mmol). The resulting mixture was stirred for 1 h at 75°C and then 2 h at 250°C under nitrogen atmosphere. The reaction mixture was allowed to cool down to rt. To the resulting mixture was added H₂O. The aq. layer was separated and extracted with EtOAc, dried over anhydrous Na₂SO₄, filtered and concentrated under vacuum. The residue was purified by silica gel coloumn chromatography (CH₃CN: H₂O (0.05% NH₄HCO₃) = 2: 1) to afford 4-isopropyl-3-methyl-1-(2,3,5-trifluorophenyl) pyrazolo[3,4-b] pyridine-5-carboxylic acid.

### Example 10.2 (d)

### N-(2,3-Dihydro-4H-benzo[b][1,4]oxazin-4-yl)-4-isopropyl-3-methyl-1-(2,3,5-trifluorophenyl)-1H-pyrazolo[3,4-b]pyridine-5-carboxamide

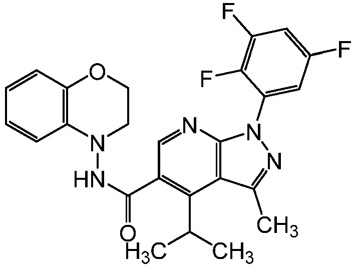

To a solution of 4-isopropyl-3-methyl-1-(2,3,5-trifluorophenyl)pyrazolo[3,4-b]pyridine-5-carboxylic acid (500 mg, 1.43 mmol) in DMF (6 mL), were added 2,3-dihydro-1,4-benzoxazin-4-amine (215 mg, 1.43 mmol), DIPEA (922 mg, 7.15 mmol) and HATU (815 mg, 2.15 mmol). The reaction mixture was stirred for 16 h at rt. Upon completion of the reaction, the resulting mixture was treated with H₂O and extracted with EtOAc. The organic layers were combined, dried over anhydrous Na₂SO₄, filtered and concentrated under vacuum. The residue purified by silica gel column chromatography (PE: EtOAc = 3: 1) and further purified by Prep-HPLC (Mobile Phase A: H₂O (10 mmol/L NH₄HCO₃), Mobile Phase B: CH₃CN; Gradient: 62% B to 92% B in 7 min) to afford N-(2,3-dihydro-1,4-benzoxazin-4-yl)-4-isopropyl-3-methyl-1-(2,3,5-trifluorophenyl)-pyrazolo[3,4-b]pyridine-5-carboxamide. ¹H-NMR (400 MHz, DMSO-D6): δ [*ppm*] = 8.65 (br s, 1H), 7.53-7.45 (m, 1H), 7.37-7.35 (m, 1H), 6.90-6.72 (m, 4H), 4.33-4.20 (m, 2H), 3.60-3.30 (m, 3H), 2.52 (s, 3H), 0.99-0.94 (m, 6H). LCMS (Method B2) Rₜ= 1.374 min; m/z= 482.15 (M+H)⁺.

### Example 11.1

### N-(2,3-Dihydro-4H-benzo[b][1,4]oxazin-4-yl)-4-isopropyl-8-(2,3,5-trifluoro-phenyl)-imidazo[1,5-a]pyrimidine-3-carboxamide

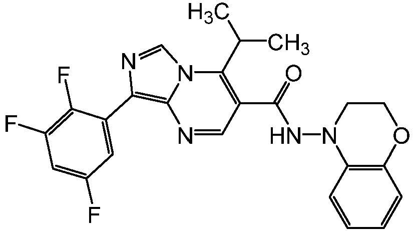

### Example 11.1 (a)

### 5-Nitro-4-(2,3,5-trifluorophenyl)-1H-imidazole

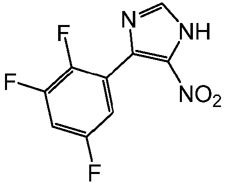

Under inert nitrogen atmosphere, a solution of 4-bromo-5-nitro-1H-imidazole (1.6 g, 8.34 mmol), 2,3,5-trifluorophenylboronic acid (2.20 g, 12.50 mmol), XPhos Pd G3 (0.71 g, 0.83 mmol), XPhos (0.79 g, 1.67 mmol) and K₃PO₄ (5.31 g, 25.0 mmol) in 1,4-dioxane (25 mL) and H₂O (25 mL) was stirred at 110°C for 24 h. After cooling down to rt, the reaction mixture was filtered and extracted with EtOAc. The organic layers were combined, dried over anhydrous Na₂SO₄, filtered, and concentrated under reduced pressure. The residue was purified by silica gel column chromatography (PE: EtOAc= 1: 5) to give 4-nitro-5-(2,3,5-trifluorophenyl)-3H-imidazole.

### Example 11.1 (b)

### 4-(2,3,5-Trifluorophenyl)-1H-imidazol-5-amine

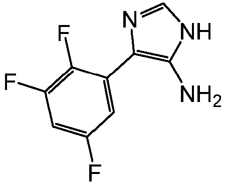

A mixture of 4-nitro-5-(2,3,5-trifluorophenyl)-3H-imidazole (240 mg, 0.74 mmol) and Pd/C (15 mg, 0.14 mmol) in EtOH (10 mL) was stirred under hydrogen gas (1 atm) for 4 h at rt. The resulting mixture was filtered, and the filtrate was concentrated under reduced pressure to give the 5-(2,3,5-trifluorophenyl)-3H-imidazol-4-amine.

### Example 11.1 (c)

### Ethyl 2-(ethoxymethylene)-4-methyl-3-oxopentanoate

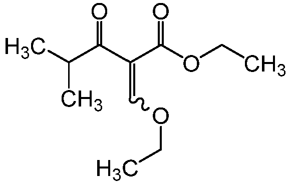

Under inert nitrogen atmosphere, a solution of ethyl 4-methyl-3-oxopentanoate (1.00 g, 6.32 mmol) and CH(OCH₃)₃ (2.01 g, 18.94 mmol) in Ac₂O (15 mL) was stirred overnight at 130°C. Then, the resulting mixture was concentrated under reduced pressure to give ethyl 2-(ethoxymethylidene)-4-methyl-3 -oxopentanoate.

### Example 11.1 (d):

### Ethyl 4-isopropyl-8-(2,3,5-trifluorophenyl)imidazo[1,5-a]pyrimidine-3-carboxylate

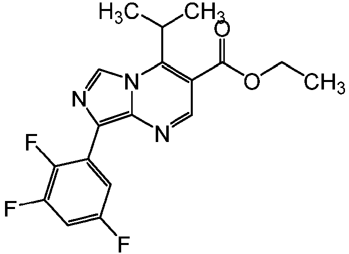

Under inert nitrogen atmosphere, a solution of 5-(2,3,5-trifluorophenyl)-4,5-dihydro-3H-imidazol-4-amine (210 mg, 60% purity, 0.59 mmol), ethyl 2-(ethoxymethylidene)-4-methyl-3-oxopentanoate (250 mg, 1.17 mmol) and Et₃N (300 mg, 2.97 mmol) in EtOH (6 mL) was stirred overnight at 100°C. After this time, the resulting mixture was concentrated under reduced pressure. The residue was purified by prep-TLC (PE: EtOAc= 5: 1) to afford the ethyl 4-isopropyl-8-(2,3,5-trimethylphenyl)imidazo[1,5-a]pyrimidine-3-carboxylate.

### Example 11.1 (e)

### 4-Isopropyl-8-(2,3,5-trifluorophenyl)imidazo[1,5-a]pyrimidine-3-carboxylic acid

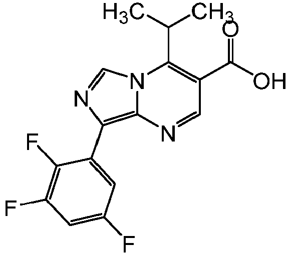

To a solution of ethyl 4-isopropyl-8-(2,3,5-trifluorophenyl)imidazo[1,5-a]pyrimidine-3-carboxylate (72 mg, 0.15 mmol) in THF (1.5 mL) was added LiOH (18 mg, 0.75 mmol) in MeOH (1.5 mL) and H₂O (1.5 mL). The resulting solution was stirred for 3 h at rt. Upon completion of the reaction, THF was evaporated under recued pressure and the pH value of the solution was adjusted to 5 by addition of aq. HCl (1 M). The mixture was extracted with EtOAc, the organic layers were combined, dried over anhydrous Na₂SO₄, filtered, and concentrated *in vacuo* to afford 4-isopropyl-8-(2,3,5-trifluorophenyl) imidazo[1,5-a]pyrimidine-3-carboxylic acid.

### Example 11.1 (f)

### N-(2,3-dihydro-4H-benzo[b][1,4]oxazin-4-yl)-4-isopropyl-8-(2,3,5-trifluorophenyl)-imidazo[1,5-a]pyrimidine-3-carboxamide

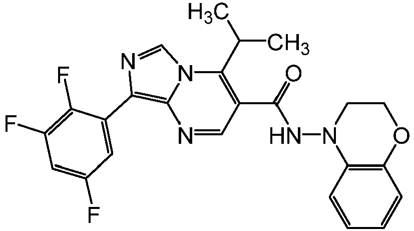

Under inert nitrogen atmosphere, a solution of 4-isopropyl-8-(2,3,5-trifluorophenyl) imidazo[1,5-a]pyrimidine-3-carboxylic acid (50 mg, 0.13 mmol), HATU (73 mg, 0.19 mmol), and 2,3-dihydro-1,4-benzoxazin-4-amine (25 mg, 0.17 mmol) in DMF (3.0 mL) was stirred for 4 h at rt. Then the solution was diluted with H₂O and extracted with EtOAc. The organic layers were combined, dried over anhydrous Na₂SO₄, filtered, and concentrated under reduced pressure. The residue was purified by prep-TLC (PE: EtOAc= 1: 1). The product was further purified by prep-HPLC [Column: XBridge Prep C18 OBD Column, 30* 100 mm, 5µm; Mobile Phase A: H₂O (10 mmol/L NH₄HCO₃), Mobile Phase B: CH₃CN; Gradient: 35% B to 60% B in 9 min] to afford N-(2,3-dihydro-1,4-benzoxazin-4-yl)-4-isopropyl-8-(2,3,5-trifluorophenyl)imidazo[1,5-a]pyrimidine-3-carboxamide. ¹H-NMR (400 MHz, 90°C, DMSO-d6) δ [ppm]: 10.49 (s, 1H), 8.82 (s, 1H), 8.43 (s, 1H), 7.74 (s, 1H), 7.35 (s, 1H), 6.91-6.72 (m, 4H), 4.36 (t, 2H), 3.68 (m, 1H), 3.67 (t, 2H), 1.55 (d, 6H). LCMS (Method E1) Rt= 1.38 min; m/z= 468 (M+H)⁺.

### Example 12.1

### N-(2,3-Dihydro-4H-benzo[b][1,4]oxazin-4-yl)-1-isopropyl-4-(2,3,5-trifluorophenyl)-1H-imidazo[4,5-c]pyridine-2-carboxamide

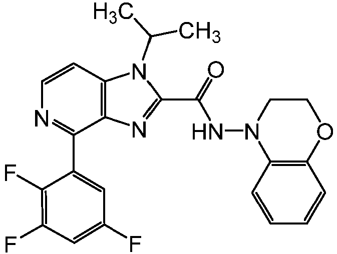

### Example 12.1 (a)

### 2-Chloro-N-isopropyl-3-nitropyridin-4-amine

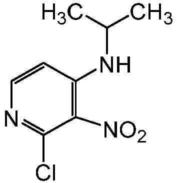

Under inert nitrogen atmosphere, isopropylamine (6.43 g, 108.82 mmol) was added slowly to a solution of 2,4-dichloro-3-nitropyridine (20 g, 103.64 mmol) and Et₃N (33.13 mL, 327.43 mmol) in DMF (200 mL). The resulting mixture was stirred overnight at rt. After this time, the mixture was diluted with ice H₂O and extracted with CH₂Cl₂. The organic layers were combined, dried over anhydrous Na₂SO₄, filtered and concentrated under reduced pressure. The residue was purified by silica gel column chromatography (PE: EtOAc= 2:1) to afford 2-chloro-N-isopropyl-3-nitropyridin-4-amine.

### Example 12.1 (b)

### 2-Chloro-N4-isopropylpyridine-3,4-diamine

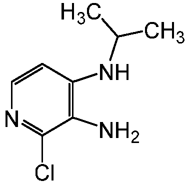

A mixture of 2-chloro-N-isopropyl-3-nitropyridin-4-amine (20.00 g, 92.78 mmol) and PtO₂ (211 mg, 0.93 mmol) in EtOH (200 mL) was stirred under hydrogen gas (1.5 atm) overnight at rt. After this time, the solution was filtered and concentrated under reduced pressure. The residue was purified by silica gel column chromatography (PE: EtOAc= 1:1) to afford 2-chloro-N4-isopropylpyridine-3,4-diamine.

### Example 12.1 (c)

### 4-Chloro-1-isopropylimidazo[4,5-c]pyridine

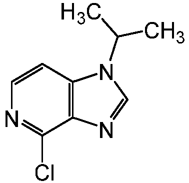

Under inert nitrogen atmosphere, 2-chloro-N4-isopropylpyridine-3,4-diamine (12.1 g, 65.2 mmol) was stirred in triethyl orthopropionate (120 mL) and conc. HCl (2.4 mL) overnight at rt. Then the solution was diluted with cooled sat. NaHCO₃-solution and extracted with CH₂Cl₂. The organic layers were combined, dried over anhydrous Na₂SO₄, filtered and concentrated under reduced pressure. The residue was purified by silica gel column chromatography (PE: EtOAc= 1:3) to afford 4-chloro-1-isopropylimidazo[4,5-c]pyridine.

### Example 12.1 (d)

### 4-Chloro-1-isopropylimidazo[4,5-c] pyridine-2-carboxylic acid

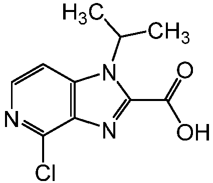

Under inert nitrogen atmosphere, 4-chloro-1-isopropylimidazo[4,5-c]pyridine (2.00 g, 10.2 mmol) was dissolved in abs. THF (20 mL). Then n-BuLi (1.44 mL, 2.5 M in abs. THF, 15.3 mmol) was added slowly and the mixture was stirred for 0.5 h at -78°C. Then, CO₂ gas (1.5 atm) was introduced, and the resulting mixture was allowed to warm to rt over 3 h. Then the solution was diluted with cooled sat. NH₄Cl-solution and extracted with EtOAc. The organic layers were combined, dried over anhydrous Na₂SO₄, filtered and concentrated *in vacuo* to afford 4-chloro-1-isopropylimidazo[4,5-c] pyridine-2-carboxylic acid.

### Example 12.1 (e)

### 4-chloro-N-(2,3-dihydro-4H-benzo[b][1,4]oxazin-4-yl)-1-isopropyl-1H-imidazo[4,5-c]-pyridine-2-carboxamide

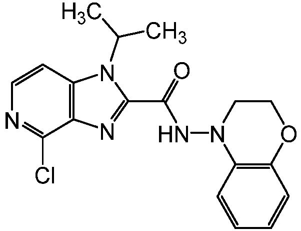

To a solution of 4-chloro-1-isopropylimidazo[4,5-c]pyridine-2-carboxylic acid (400 mg, 1.67 mmol) in CH₂Cl₂ (10 mL), oxalyl chloride (2.1 g, 16.7 mmol) was added at 0°C. The mixture was allowed to stir and warm up to rt over 2 h. After this time, the mixture was concentrated under reduced pressure. The residue was dissolved in CH₂Cl₂ (10 mL) and 2,3-dihydro-1,4-benzoxazin-4-amine (301 mg, 2.00 mmol) was added in portions at 0°C. The resulting mixture was stirred for 2 h at rt and was then concentrated under reduced pressure. The residue was purified by prep-TLC (PE: EtOAc= 3: 1) to afford 4-chloro-N-(2,3-dihydro-1,4-benzoxazin-4-yl)-1-isopropylimidazo[4,5-c]pyridine-2-carboxamide.

### Example 12.1 (f)

### N-(2,3-Dihydro-4H-benzo[b][1,4] oxazin-4-yl)-1-isopropyl-4-(2,3,5-trifluorophenyl)-1H-imidazo[4,5-c]pyridine-2-carboxamide

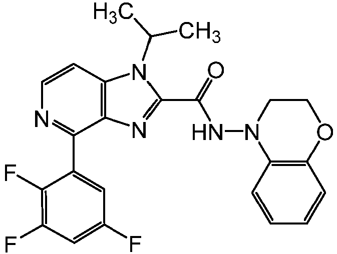

Under inert nitrogen atmosphere, 4-chloro-N-(2,3-dihydro-1,4-benzoxazin-4-yl)-1-isopropylimidazo[4,5-c]pyridine-2-carboxamide (50.0 mg, 0.13 mmol), 2,3,5-trifluorophenylboronic acid (28 mg, 0.16 mmol), K₃PO₄ (57 mg, 0.27 mmol), and XPhos Pd G3 (11.0 mg, 0.013 mmol) were mixed in 1,4-dioxane (1 mL) and H₂O (0.2 mL) and the resulting mixture was stirred overnight at 75°C. After cooling down to rt, the mixture was diluted with H₂O and extracted with EtOAc. The organic layers were combined, dried over anhydrous Na₂SO₄, filtered and concentrated under reduced pressure. The residue was purified by prep-TLC (PE: EtOAc= 1: 1) and further purified by prep-HPLC [Mobile Phase A: H₂O (10 mmol/L NH₄HCO₃), Mobile Phase B: MeOH; Gradient: 67% B to 70% B in 11 min) to afford N-(2,3-dihydro-1,4-benzoxazin-4-yl)-1-isopropyl-4-(2,3,5-trifluoro-phenyl)imidazo[4,5-c]pyridine-2-carboxamide. ¹H-NMR (400 MHz, DMSO-d6) δ [ppm]: 11.01 (s, 1H), 8.56 (d, 1H), 8.08 (d, 1H), 7.79-7.52 (m, 2H), 6.90-6.62 (m, 4H), 5.65-5.58 (m, 1H), 4.37 (m, 2H), 3.64 (t, 2H), 1.66 (d, 6H). LCMS (Method E1) Rₜ= 1.58 min; m/z= 466 (M-H)⁻.

### Example 13.1

### N-(2,3-Dihydro-4H-benzo[b][1,4]oxazin-4-yl)-1-isopropyl-4-(2,3,5-trifluorophenyl)-1H-benzo[d]imidazole-2-carboxamide

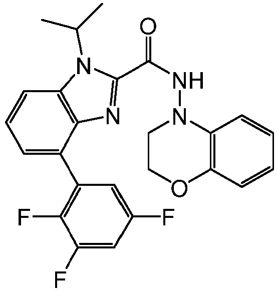

### Example 13.1 (a)

### 3-Chloro-N-isopropyl-2-nitroaniline

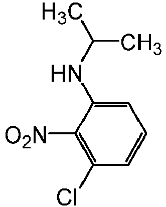

Under an inert atmosphere of nitrogen, 2,6-dichloro-1-nitrobenzene (6.00 g, 31.3 mmol), DMF (50 mL), isopropylamine (1.85 g, 31.3 mmol), and Cs₂CO₃ (20.4 g, 62.5 mmol) were mixed. The resulting mixture was stirred overnight at 80°C. After cooling down to rt, the resulting mixture was diluted with H₂O and extracted with EtOAc, dried over anhydrous Na₂SO₄, filtered, and concentrated under vacuum. The residue was purified by silica gel column chromatography (PE: EtOAc =12: 1) to afford 3-chloro-N-isopropyl-2-nitroaniline.

### Example 13.1 (b)

### 3-Chloro-N1-isopropylbenzene-1,2-diamine

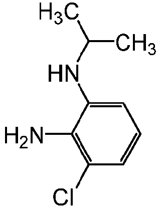

3-Chloro-N-isopropyl-2-nitroaniline (2.1 g, 9.78 mmol), NH₄Cl (20 mL, 97.8 mmol, 5 M) and iron powder (2.60 g, 46.6 mmol) were mixed in EtOH (20 mL). The resulting solution was stirred overnight at 60°C. After cooling down to rt, the resulting mixture was filtered. The filter cake was washed with EtOH. The filtrate was concentrated under reduced pressure and extracted with EtOAc, dried over anhydrous Na₂SO₄, filtered, and concentrated under vacuum. The crude product (1.8 g) was used in the next step directly without further purification.

### Example 13.1 (c)

### Ethyl 4-chloro-1-isopropyl-1H-benzo[d]imidazole-2-carboxylate

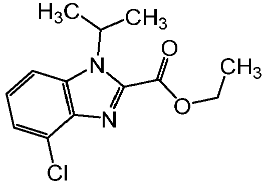

Under an inert atmosphere of nitrogen, were mixed 3-chloro-N1-isopropylbenzene-1,2-diamine (1.8 g, 9.75 mmol), methyl 2-oxoacetate (0.84 g, 9.53 mmol), Na₂S₂O₄ (8.49 g, 48.7 mmol), EtOH (15 mL) and DMSO (3 mL). The resulting solution was stirred overnight at 80°C. After cooling down to rt, the resulting mixture was diluted with H₂O and extracted with EtOAc, dried over anhydrous Na₂SO₄, filtered, and concentrated under vacuum. The residue was purified by silica gel column chromatography (PE: EtOAc = 3:1) to afford ethyl 4-chloro-1-isopropyl-1,3-benzodiazole-2-carboxylate.

### Example 13.1 (d)

### Ethyl 1-isopropyl-4-(2,3,5-trifluorophenyl)-1,3-benzodiazole-2-carboxylate

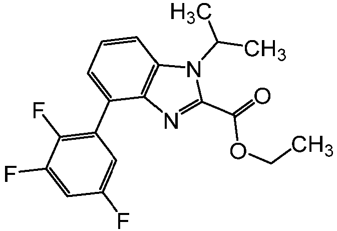

Under an inert atmosphere of nitrogen, were mixed ethyl 4-chloro-1-isopropyl-1,3-benzodiazole-2-carboxylate (600 mg, 2.25 mmol), 2,3,5-trifluorophenylboronic acid (475 mg, 2.7 mmol), XPhos Pd G3 (190 mg, 0.225 mmol), XPhos (214 mg, 0.45 mmol), K₃PO₄ (955 mg, 4.5 mmol), 1,4-dioxane (6 mL) and H₂O (1.2 mL). The resulting solution was stirred overnight at 80°C. After cooling down to rt, the solution was diluted with H₂O and extracted with EtOAc. The organic layers were combined, dried over anhydrous Na₂SO₄, filtered and concentrated under vacuum. The residue was purified by silica gel chromatography (PE: EtOAc=3: 1) to afford ethyl 1-isopropyl-4-(2,3,5-trifluorophenyl)-1,3-benzodiazole-2-carboxylate.

### Example 13.1 (e)

### Lithium 1-isopropyl-4-(2,3,5-trifluorophenyl)-1H-benzo[d]imidazole-2-carboxylate

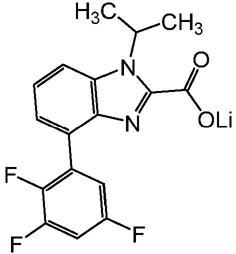

To a solution of ethyl 1-isopropyl-4-(2,3,5-trifluorophenyl)-1,3-benzodiazole-2-carboxylate (200 mg, 0.55 mmol) in THF (2 mL) was added LiOH (39.7 mg, 1.65 mmol), MeOH (2 mL) and H₂O (2 mL). The mixture was stirred for 1 h at rt. Upon completion of the reaction, the solution was blown to dry by nitrogen gas to afford lithium 1-isopropyl-4-(2,3,5-trifluorophenyl)-1H-benzo[d]imidazole-2-carboxylate.

### Example 13.1 (f)

### N-(2,3-dihydro-1,4-benzoxazin-4-yl)-1-isopropyl-4-(2,3,5-trifluorophenyl)-1,3-benzo-diazole-2-carboxamide

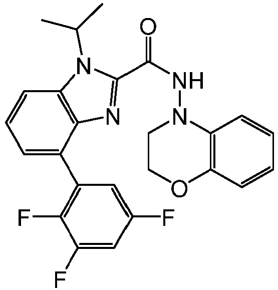

To a solution of lithium 1-isopropyl-4-(2,3,5-trifluorophenyl)-1H-benzo[d]imidazole-2-carboxylate (140 mg, 0.41 mmol) in DMF (2 mL), was added HATU (172 mg, 0.45 mmol), 2,3-dihydro-1,4-benzoxazin-4-amine (68.0 mg, 0.45 mmol) and DIPEA (53.18 mg, 0.41 mmol). The resulting solution was stirred for 30 min at rt. Upon completion of the reaction, the solution was diluted with H₂O and extracted with EtOAc. The organic layers were combined, dried over anhydrous Na₂SO₄, filtered, and concentrated under vacuum. The residue was purified by prep-HPLC (Column: XSelect CSH C18 OBD Column 30*150mm 5µm, n; Mobile Phase A: H₂O (0.1%FA), Mobile Phase B: CH₃CN; Gradient: 63% B to 80% B in 8 min, 80% B) to afford N-(2,3-dihydro-1,4-benzoxazin-4-yl)-1-isopropyl-4-(2,3,5-trifluorophenyl)-1,3-benzodiazole-2-carboxamide. ¹H-NMR (400 MHz, DMSO-d6) δ [ppm] 10.84 (s, 1H), 8.04-8.00 (m, 1H), 7.65-7.56 (m, 2H), 7.52-7.48 (m, 2H), 6.82-6.76 (m, 3H), 6.72-6.68 (m, 1H), 5.68-5.61 (m, 1H), 4.37-4.34 (m, 2H), 3.64-3.63 (m, 2H), 1.65 (d, 6H). LC-MS (Analytical Method E) Rt = 1.501 min; MS (ESIpos); m/z = 467 (M+H)⁺ . LCMS (Method F2) Rt= 1.50 min; m/z= 467 (M-H)⁻.

### Example 14.1

### N-(2,3-Dihydro-4H-benzo[b][1,4]oxazin-4-yl)-7-isopropyl-3-(2,3,5-trifluorophenyl)-3H-[1,2,3]triazolo[4,5-b]pyridine-6-carboxamide

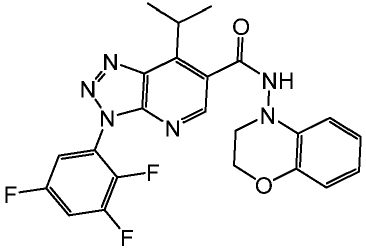

### Example 14.1 (a)

### Ethyl 6-chloro-5-nitro-4-(prop-1-en-2-yl) nicotinate

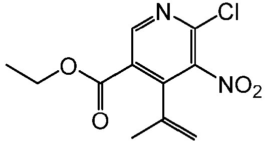

To a solution of ethyl 4,6-dichloro-5-nitro-pyridine-3-carboxylate (5.00 g, 18.9 mmol) in 1,4-dioxane (40 mL) and H₂O (10 mL) were added isopropenyl boronic acid (1.94 g, 22.6 mmol), cesium fluoride (5.73 g, 37.7 mmol, 1.39 mL) and palladium (0) tetrakis(triphenylphosphine) (2.18 g, 1.89 mmol). The mixture was stirred at 80°C under nitrogen atmosphere for overnight. After cooling down to rt, the organic solvent was evaporated and H₂O was added. The aq. layer was separated and extracted with EtOAc. The organic phase was washed with H₂O, brine and dried over anhydrous Na₂SO₄. After filtration, the filtrate was concentrated under reduced pressure and the residue was purified by silica gel column chromatography to afford ethyl 6-chloro-4-isopropenyl-5-nitro-pyridine-3-carboxylate.

### Example 14.1 (b)

### Ethyl 5-nitro-4-(prop-1-en-2-yl)-6-((2,3,5-trifluorophenyl)amino)nicotinate

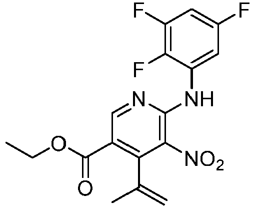

To a solution of ethyl 6-chloro-4-isopropenyl-5-nitro-pyridine-3-carboxylate (1.00 g, 3.69 mmol) in toluene (10 mL) were added 2,3,5-trifluoroaniline (1.09 g, 7.39 mmol), Cs₂CO₃ (2.41 g, 7.39 mmol), BINAP (460.10 mg, 738.91 µmol) and Pd(OAc)₂ (83.7 mg, 369.5 µmol). The mixture was stirred at 100°C under nitrogen atmosphere for 2 h. After cooling down to rt, H₂O was added, and the resulting mixture was extracted with EtOAc. The organic layer was washed with brine and dried over anhydrous Na₂SO₄. After filtration, the filtrate was concentrated under reduced pressure and the residue was purified by silica gel column chromatography to afford ethyl 4-isopropenyl-5-nitro-6-(2,3,5-trifluoroanilino) pyridine-3-carboxylate.

### Example 14.1 (c)

### Ethyl 5-amino-4-isopropyl-6-((2,3,5-trifluorophenyl)amino)nicotinate

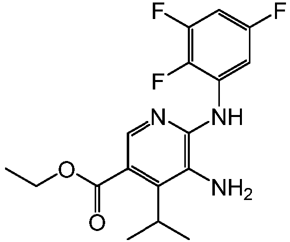

To a solution of ethyl 4-isopropenyl-5-nitro-6-(2,3,5-trifluoroanilino)pyridine-3-carboxylate (650 mg, 1.70 mmol) in MeOH (10 mL) was added palladium, 10% on carbon (145 mg, 1.36 mmol). The mixture was stirred at rt under hydrogen atmosphere for 2 h. The catalyst was removed by filtration and the filtrate was concentrated under reduced pressure to afford ethyl 5-amino-4-isopropyl-6-(2,3,5-trifluoroanilino)pyridine-3-carboxylate.

### Example 14.1 (d)

### Ethyl 7-isopropyl-3-(2,3,5-trifluorophenyl)-3H-[1,2,3] triazolo [4,5-b]pyridine-6-carboxylate

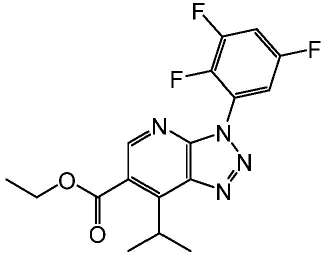

To a solution of ethyl 5-amino-4-isopropyl-6-(2,3,5-trifluoroanilino)pyridine-3-carboxylate (635 mg, 1.80 mmol) in H₂O (3 mL), EtOH (3 mL) and acetic acid (3 mL) was added sodium nitrite (161 mg, 2.34 mmol) at 0°C. The resulting mixture was stirred at rt for overnight and then, concentrated under reduced pressure. H₂O was added, and the mixture was extracted with EtOAc. The organic layer was washed with brine and dried over anhydrous Na₂SO₄. After filtration, the filtrate was concentrated under reduced pressure and the residue was purified by silica gel column chromatography to afford ethyl 7-isopropyl-3-(2,3,5-trifluorophenyl)triazolo[4,5-b]pyridine-6-carboxylate.

### Example 14.1 (e)

### 7-Isopropyl-3-(2,3,5-trifluorophenyl)-3H-[1,2,3]triazolo[4,5-b]pyridine-6-carboxylic acid

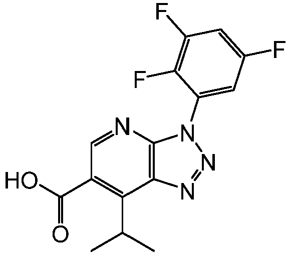

To a solution of ethyl 7-isopropyl-3-(2,3,5-trifluorophenyl)triazolo[4,5-b]pyridine-6-carboxylate (320 mg, 878 µmol) in EtOH (2 mL) and THF (6 mL) was added LiOH (63 mg, 2.64 mmol, in 3 mL H₂O). The reaction mixture was stirred at 50°C for overnight. After this time, the mixture was concentrated under reduced pressure. The residue was diluted with H₂O and the pH was adjusted to 5-6 by addition of aq. HCl (1 M). Then the resulting solution was extracted with EtOAc. The organic layer was washed with H₂O, brine and dried over anhydrous Na₂SO₄. After filtration, the filtrate was concentrated under reduced pressure to afford 7-isopropyl-3-(2,3,5-trifluorophenyl)triazolo[4,5-b]pyridine-6-carboxylic acid.

### Example 14.1 (f)

### N-(2,3-Dihydro-4H-benzo[b][1,4]oxazin-4-yl)-7-isopropyl-3-(2,3,5-trifluorophenyl)-3H-[1,2,3]triazolo[4,5-b]pyridine-6-carboxamide

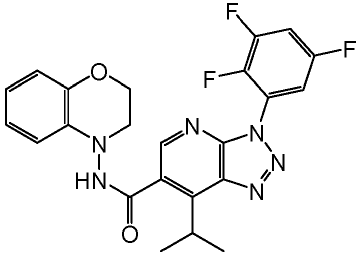

To a solution of 7-isopropyl-3-(2,3,5-trifluorophenyl)triazolo[4,5-b]pyridine-6-carboxylic acid (331 mg, 984 µmol) in N,N-dimethylformamide (10 mL) was added HATU (562 mg, 1.48 mmol), N,N-diisopropylethylamine (382 mg, 2.95 mmol, 514 µL) and 2,3-dihydro-4H-benzo[b][1,4]oxazin-4-amine (296 mg, 1.97 mmol). Then the resulting mixture was stirred at rt for 2 h. H₂O was added, and the resulting mixture was extracted with EtOAc. The organic layer was washed with H₂O, brine and dried over anhydrous Na₂SO₄. After filtration, the filtrate was concentrated under reduced pressure. The crude product (200 mg) was purified by Prep-HPLC with the following conditions (Column: XBridge Prep C18 OBD Column, 30*100 mm, 5µm; Mobile Phase A: H₂O (10 mmol/L NH₄HCO₃), Mobile Phase B: CH₃CN; Flow rate: 60 mL/min; Gradient: 44% B to 66% B in 9 min, 66% B) to afford N-(2,3-dihydro-1,4-benzoxazin-4-yl)-7-isopropyl-3-(2,3,5-trifluorophenyl)triazolo-[4,5-b]pyridine-6-carboxamide. 1H-NMR (400 MHz, DMSO-d6): δ [ppm] 10.96 (s, 1H), 9.00 (s, 1H), 7.96-7.75 (m, 2H), 6.72 (m, 4H), 4.32 (t, 2H), 4.09-3.98 (m, 1H), 3.51-3.45 (t, 2H), 1.50 (d, 6H). LCMS (Analytical Method B2): Rₜ = 1.195 min; MS (ESIpos): m/z = 469 (M+H)⁺.

### Example 15.1

### N-(2,3-Dihydro-1,4-benzoxazin-4-yl)-8-isopropyl-3-(2,3,6-trifluorophenyl)- [1,2,4] triazolo [4,3-a]pyridine-7-carboxamide

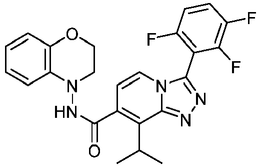

### Example 15.1 (a)

### 3-Chloro-2-hydrazino-pyridine-4-carboxylic acid

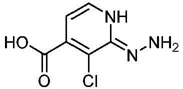

To a solution of 2,3-dichloropyridine-4-carboxylic acid (10 g, 52.1 mmol) in anhydrous iPrOH (50 mL) was added hydrazine monohydrate (15.2 mL, 312 mmol). The resulting mixture was stirred overnight at 85°C. After cooling down to rt, the mixture was concentrated and then slurried with iPrOH/EtOAc (1:10) to give 3-chloro-2-hydrazino-pyridine-4-carboxylic acid.

### Example 15.1 (b)

### 8-Chloro-3-(2,3,6-trifluorophenyl)-[1,2,4]triazolo[4,3-a]pyridine-7-carboxylic acid

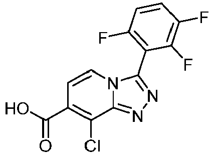

To a solution of (2Z)-3-chloro-2-hydrazinylidene-1H-pyridine-4-carboxylic acid (7.00 g, 37.3 mmol) and 2,3,6-trifluorobenzaldehyde (5.03 mL, 44.8 mmol) in i-PrOH (194 mL) was added 1,3,5-trichloro-1,3,5-triazinane-2,4,6-trione (10.5 mL, 93.3 mmol). The mixture was stirred for 2 h at rt. The reaction mixture was concentrated and washed with EtOAc to afford 8-chloro-3-(2,3,6-trifluorophenyl)-[1,2,4]triazolo[4,3-a]pyridine-7-carboxylic acid.

### Example 15.1 (c)

### Methyl 8-chloranyl-3-[2,3,6-tris(fluoranyl)phenyl]-[1,2,4]triazolo[4,3-a]pyridine-7-carboxylate

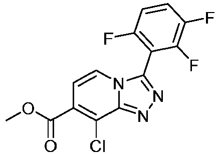

To a mixture of 8-chloranyl-3-[2,3,6-tris(fluoranyl)phenyl]-[1,2,4]triazolo[4,3-a]pyridine-7-carboxylic acid (2.00 g, 6.10 mmol) in i-PrOH (20 mL) at 0°C was added diazomethyl-tri(methyl)silane (2 M, 12.2 mL). The resulting mixture was allowed to warm to rt overnight. After this time, the reaction was quenched with AcOH, poured into H₂O and extracted with EtOAc. The organic layers were combined, dried over anhydrous Na₂SO₄, filtered and concentrated under vacuum. The residue was purified by silica gel column choromatography (eluting with EtOAc/PE = 50/50) to afford methyl 8-chloranyl-3-[2,3,6-tris(fluoranyl)phenyl]-[1,2,4]triazolo[4,3-a]pyridine-7-carboxylate.

### Example 15.1 (d)

### Methyl 8-isopropenyl-3-(2,3,6-trifluorophenyl)-[1,2,4]triazolo[4,3-a]pyridine-7-carboxylate

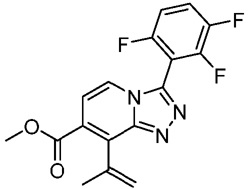

A mixture of methyl 8-chloro-3-(2,3,6-trifluorophenyl)-[1,2,4]triazolo[4,3-a]pyridine-7-carboxylate (800 mg, 2.34 mmol), 2-isopropenyl-4,4,5,5-tetramethyl-1,3,2-dioxaborolane (590 mg, 3.51 mmol), CsF (938 mg, 4.68 mmol) and Pd(PPh₃)₄ (271 mg, 234 µmol) in 1,4-dioxane (15 mL), H₂O (5 mL) was stirred at 80°C overnight. The reaction mixture was concentrated and purified by silica gel column chromatography (EtOAc/PE) to afford methyl 8-isopropenyl-3-(2,3,6-trifluorophenyl)-[1,2,4]triazolo[4,3-a]pyridine-7-carboxylate.

### Example 15.1 (e)

### Methyl 8-(1-methylethyl)-3-[2,3,6-tris(fluoranyl)phenyl]-[1,2,4]triazolo[4,3-a]pyridine-7-carboxylate

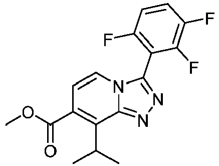

To a solution of methyl 8-(1-methylvinyl)-3-[2,3,6-tris(fluoranyl)phenyl]-[1,2,4] triazolo[4,3-a]pyridine-7-carboxylate (800 mg, 2.30 mmol) in EtOAc (20 mL) was added PtO₂ (40 mg, 176 µmol). The mixture was then stirred overnight at rt under H₂ gas. The precipitated solids were collected by diatomite and washed with EtOAc. The reaction mixture was concentrated and purified by silica gel column chromatography (EtOAc/PE) to afford methyl 8-(1-methylethyl)-3-[2,3,6-tris(fluoranyl)phenyl]-[1,2,4]triazolo[4,3-a]pyridine-7-carboxylate.

### Example 15.1 (f)

### 8-(1-Methylethyl)-3-[2,3,6-tris(fluoranyl)phenyl]-[1,2,4]triazolo[4,3-a]pyridine-7-carboxylic acid

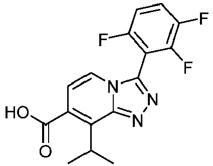

A mixture of methyl 8-(1-methylethyl)-3-[2,3,6-tris(fluoranyl)phenyl]-[1,2,4] triazolo[4,3-a]pyridine-7-carboxylate (200 mg, 572.56 µmol) and LiOH (69 mg, 2.86 mmol) in THF (1 mL), H₂O (1 mL) and MeOH (1 mL) was stirred for overnight at rt under nitrogen atmosphere. The reaction was quenched with aq. HCI (6M in H₂O) at 0°C. The aq. layer was extracted with EtOAc (3 x 5 mL). The resulting mixture was concentrated under vacuum to afford 8-(1-methylethyl)-3-[2,3,6-tris(fluoranyl)phenyl]-[1,2,4]triazolo[4,3-a]pyridine-7-carboxylic acid.

### Example 15.1 (g)

### N-(2,3-dihydro-1,4-benzoxazin-4-yl)-8-isopropyl-3-(2,3,6-trifluorophenyl)-[1,2,4]-triazolo[4,3-a]pyridine-7-carboxamide

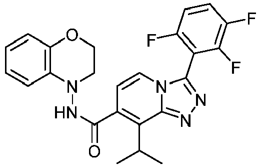

A mixture of 8-isopropyl-3-(2,3,6-trifluorophenyl)-[1,2,4]triazolo[4,3-a]pyridine-7-carboxylic acid (165 mg, 492 µmol) and HATU (206 mg, 541 µmol) in DMF was stirred for 30 min at rt under nitrogen atmosphere. To the above mixture was added DIPEA (200 mg, 984 µmol) and 2,3-dihydro-1,4-benzoxazin-4-amine (111 mg, 738 µmol) dropwise at rt. The resulting mixture was further stirred overnight at rt and then, concentrated under vacuum to afford N-(2,3-dihydro-1,4-benzoxazin-4-yl)-8-isopropyl-3-(2,3,6-trifluorophenyl)-[1,2,4]triazolo[4,3-a]pyridine-7-carboxamide. The residue was purified by prep-HPLC [Mobile Phase A: H₂O (10 mmol/L NH₄HCO₃+0.1%NH₃·H₂O), Mobile Phase B: CH₃CN; Flow rate: 60 mL/min; Gradient: 29% B to 55% B in 8 min] to afford of N-(2,3-dihydro-1,4-benzoxazin-4-yl)-8-isopropyl-3-(2,3,6-trifluorophenyl)-[1,2,4]triazolo[4,3-a]pyridine-7-carboxamide. ¹H-NMR (400 MHz, DMSO-d6): δ [ppm] = 10.36 (y, 1H), 8.24 (d, 1H), 7.80 (t, 1H), 7.43 (t, 1H), 7.06 (d, 1H), 6.88-6.75 (m, 4H), 4.35 (t, 2H), 3.67-3.66 (m, 3H), 1.63-1.61 (m, 6H). LCMS (Analytical Method E1): Rₜ = 1.045 min; MS (ESIpos): m/z = 468.05 (M+H)⁺.

### Example 16.1

### N-(2,3-Dihydro-4H-benzo[b][1,4]oxazin-4-yl)-4-isopropyl-1-(2,3,5-trifluorophenyl)-1H-benzo[d][1,2,3]triazole-5-carboxamide

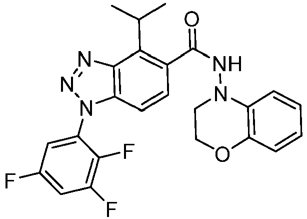

### Example 16.1 (a)

### Methyl 2-hydroxy-3-nitro-4-((2,3,5-trifluorophenyl)amino)benzoate

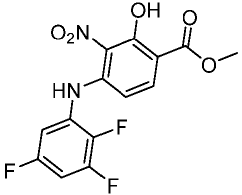

To a solution of methyl 4-fluoranyl-3-nitro-2-oxidanyl-benzoate (600 mg, 2.79 mmol) in iPrOH (10 mL) was added 2,3,5-tris(fluoranyl)aniline (615 mg, 4.18 mmol) and diisopropylethylamine (1.46 mL, 8.37 mmol) and the resulting mixture was stirred under nitrogen atmosphere for 16 h at 110°C. After this time, the mixture was concentrated under reduced pressure. To the residue was added H₂O (10 mL) and the aq. layer was extracted with EtOAc. The combined organic layers were washed with brine, dried over anhydrous Na₂SO₄. After filtration, the filtrate was concentrated under reduced pressure. The residue was purified by silica gel column chromatography, eluting with PE / EtOAc (24: 1) to afford methyl 2-hydroxy-3-nitro-4-(2,3,5-trifluoroanilino) benzoate.

### Example 16.1 (b)

### Ethyl 3-nitro-2-(((trifluoromethyl) sulfonyl)oxy)-4-((2,3,5-trifluorophenyl)-amino)-benzoate

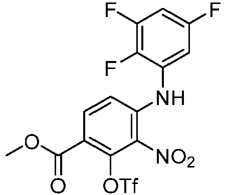

To a solution of methyl 2-hydroxy-3-nitro-4-(2,3,5-trifluoroanilino)benzoate (246 mg, 719 µmol) in CH₂Cl₂ (1.84 mL) was added pyridine (719 µmol, 58.1 µL). At 0°C, was then added trifluoromethanesulfonic anhydride (719 µmol, 121 µL) and the resulting mixture was stirred under nitrogen atmosphere for 16 h at rt. After this time, H₂O (10 mL) was added and the mixture extracted with CH₂Cl₂ (3 x 20 mL). The organic layers were combined, washed with brine, and dried over anhydrous Na₂SO₄. After filtration, the filtrate was concentrated under reduced pressure to afford propyl 3-nitro-4-(2,3,5-trifluoroanilino)-2-(trifluoromethylsulfonyloxy)benzoate.

### Example 16.1 (c)

### Methyl 3-nitro-2-(prop-1-en-2-yl)-4-((2,3,5-trifluorophenyl)amino)benzoate

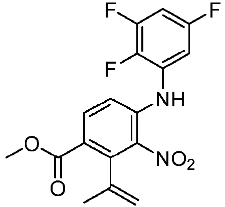

To a solution of propyl 3-nitro-4-(2,3,5-trifluoroanilino)-2-(trifluoromethylsulfonyloxy) benzoate (298 mg, 593 µmol) in 1,4-dioxane (4 mL) and H₂O (1 mL) was added Pd(dppf)Cl₂ (48.4 mg, 59.3 µmol) and potassium carbonate (246 mg, 1.78 mmol, 107 µL).The mixture was kept stirring under nitrogen atmosphere for 16 h at 80°C. To the residue was added H₂O (10 mL) and the mixture was extracted with EtOAc (3 x). The combined organic layers were dried over anhydrous Na₂SO₄. After filtration, the filtrate was concentrated under reduced pressure. The residue was purified by silica gel column chromatography, eluting with PE / EtOAc (49: 1) to afford methyl 2-isopropenyl-3-nitro-4-(2,3,5-trifluoroanilino).

### Example 16.1 (d)

### Methyl 3-amino-2-isopropyl-4-((2,3,5-trifluorophenyl)amino)benzoate

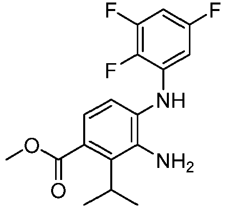

To a solution of methyl 2-isopropenyl-3-nitro-4-(2,3,5-trifluoroanilino)benzoate (80.0 mg, 218 µmol) in MeOH (4 mL) was added palladium, 10% on carbon (23.2 mg, 218 µmol). The mixture was then stirred under hydrogen gas for 4 h at rt. The reaction was filtered with MeOH and the filtrate was concentrated under reduced pressure to afford propyl 3-azanyl-2-(1-methylethyl)-4-[2,3,5-tris(fluoranyl)anilino]benzoate.

### Example 16.1 (e)

### Methyl 4-isopropyl-1-(2,3,5-trifluorophenyl)-1H-benzo[d][1,2,3]triazole-5-carboxylate

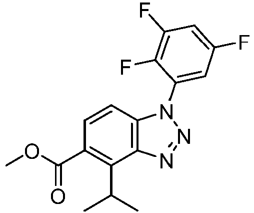

To a solution of propyl 3-azanyl-2-(1-methylethyl)-4-[2,3,5-tris(fluoranyl)anilino]-benzoate (80 mg, 218 µmol) in H₂O (2 mL):EtOH (2 mL):acetic acid (2 mL) was added sodium nitrite (19.6 mg, 284 µmol, 9.03 µL). The resulting mixture was stirred for 16 h at rt. The reaction was then concentrated under reduced pressure. To the residue was added H₂O (10 mL) and the mixture was extracted with EtOAc (3 x). The combined organic layers were dried over anhydrous Na₂SO₄. After filtration, the filtrate was concentrated under reduced pressure. The residue was purified by silica gel column chromatography, eluting with PE / EtOAc (24: 1) to afford propyl 4-(1-methylethyl)-1-[2,3,5-tris(fluoranyl)phenyl] benzotriazole-5-carboxylate.

### Example 16.1 (f)

### 4-Isopropyl-1-(2,3,5-trifluorophenyl)-1H-benzo[d][1,2,3]triazole-5-carboxylic acid

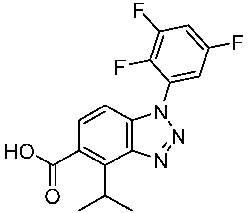

To a solution of ethyl 7-isopropyl-3-(2,3,5-trifluorophenyl)triazolo[4,5-b]pyridine-6-carboxylate (50 mg, 0.14 mmol) in EtOH (2 mL): THF (6 mL): H₂O (3 mL) was added LiOH (30 mg, 0.71 mmol). The mixture was stirred for 16 h at 50°C and was then concentrated under reduced pressure. The residue was diluted with H₂O and the pH was adjusted to 5-6 by addition of aq. HCl (1 M). Then the resulting solution was extracted with EtOAc. The organic layer was washed with H₂O, brine and dried over anhydrous Na₂SO₄. After filtration, the filtrate was concentrated under reduced pressure to afford 4-isopropyl-1-(2,3,5-trifluorophenyl)-1H-benzo[d][1,2,3]triazole-5-carboxylic acid.

### Example 16.1 (g)

### N-(2,3-dihydro-4H-benzo[b][1,4] oxazin-4-yl)-4-isopropyl-1-(2,3,5-trifluorophenyl)-1H-benzo-[d][1,2,3]triazole-5-carboxamide

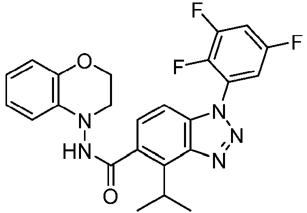

To a solution of 4-(1-methylethyl)-1-[2,3,5-tris(fluoranyl)phenyl]benzotriazole-5-carboxylic acid (37 mg, 110 µmol) and 4-(1-methylethyl)-1-[2,3,5-tris(fluoranyl)phenyl] benzotriazole-5-carboxylic acid (37 mg, 110 µmol) in DMF (5 mL) was added HATU (62.9 mg, 165 µmol) and DIPEA (331 µmol, 57.7 µL) at 0°C. The reaction mixture was slowly warmed to rt and stirred for 4 h. After this time, the reaction mixture was concentrated under reduced pressure and the residue was purified by prep-HPLC with following conditions: Column XBridge Prep C18 OBD Column, 30*100 mm, 5µm; Mobile Phase A: H₂O(10 mmol/L NH₄HCO₃), Mobile Phase B: CH₃CN; Flow rate: 60 mL/min; Gradient: 45% B to 64% B in 10 min, 64% B; Wave Length: 254/220 nm; Rₜ (min): 9.23; to get N-(2,3-dihydro-1,4-benzoxazin-4-yl)-4-(1-methylethyl)-1-[2,3,5-tris(fluoranyl)-phenyl] benzotriazole-5-carboxamide. 1H-NMR (400 MHz, DMSO-d6): δ [ppm] = 10.48 (s, 1H), 8.023-7.93 (m, 1H), 7.88-7.82 (m, 1H), 7.76-7.67 (m, 2H), 6.92-6.66 (m, 4H), 4.37 (t, J = 5.6 Hz, 2H), 3.68-3.57 (m, 3H), 1.66 (d, J = 5.6 Hz, 6H). LCMS (Analytical Method E1): Rt = 1.214 min; m/z = 468 (M+H)⁺.

### Example 17.1

### N-(2,3-dihydro-1,4-benzoxazin-4-yl)-3-(1-methylethyl)-7-[2,3,5-tris(fluoranyl)-phenyl]-1H-indole-2-carboxamide

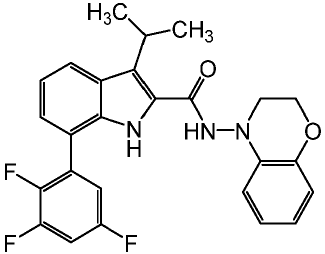

### Example 17.1 (a)

### Ethyl 7-[2,3,5-tris(fluoranyl)phenyl]-1H-indole-2-carboxylate

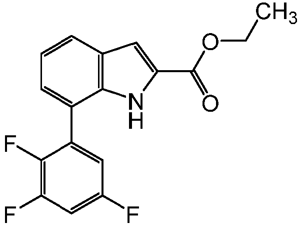

To a solution of ethyl 7-bromanyl-1H-indole-2-carboxylate (3.20 g, 11.9 mmol) in toluene (80 mL), was added [2,3,5-tris(fluoranyl)phenyl] boronic acid (2.10 g, 11.9 mmol), Pd(dppf)Cl₂ (974 mg, 1.19 mmol) and Cs₂CO₃ (7.76 g, 23.9 mmol). The resulting reaction mixture was stirred overnight at 100°C under nitrogen atmosphere. After cooling down to rt, the mixture was treated with H₂O and extracted with EtOAc (3 x). The combined organic layers were dried over anhydrous Na₂SO₄. After filtration, the filtrate was concentrated under reduced pressure and the residue was purified by silica gel column chromatography (EtOAc: PE = 0- 20%) to afford ethyl 7-[2,3,5-tris(fluoranyl)phenyl]-1H-indole-2-carboxylate.

### Example 17.1 (b)

### Ethyl 4-iodanyl-7-[2,3,5-tris(fluoranyl)phenyl]-1H-indole-2-carboxylate

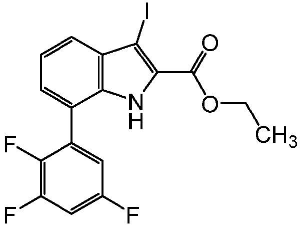

To a solution of ethyl 7-[2,3,5-tris(fluoranyl)phenyl]-1H-indole-2-carboxylate (2.95 g, 1.85 mmol) in CH₂Cl₂ (50 mL) was added NIS (624 mg, 2.78 mmol) at rt. The reaction mixture was stirred overnight at rt under nitrogen atmosphere. Upon completion of the reaction, the resulting mixture was treated with H₂O and extracted with EtOAc (3 x). The combined organic layers were dried over anhydrous Na₂SO₄. After filtration, the filtrate was concentrated under reduced pressure and the residue was purified by silica gel column chromatography (EtOAc: PE = 0- 20%) to afford to ethyl 4-iodanyl-7-[2,3,5-tris(fluoranyl)phenyl]-1H-indole-2-carboxylate.

### Example 17.1 (c)

### Ethyl 3-(1-methylvinyl)-7-[2,3,5-tris(fluoranyl)phenyl]-1H-indole-2-carboxylate

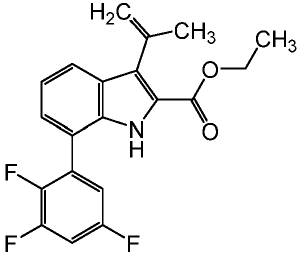

To a solution of ethyl 3-iodanyl-7-[2,3,5-tris(fluoranyl)phenyl]-1H-indole-2-carboxylate (1.00 g, 2.25 mmol) in 1,4-dioxane (16 mL), was added 4,4,5,5-tetra(methyl)-2-(1-methylvinyl)-1,3,2-dioxaborolane (567 mg, 3.37 mmol), Cs₂CO₃ (1.46 g, 4.50 mmol), Pd(dppf)Cl₂ (184 mg, 225 µmol) and H₂O (4 mL). The mixture was stirred overnight at 100°C under nitrogen atmosphere. After cooling down to rt, the mixture was treated with H₂O and extracted with EtOAc (3x). The combined organic layers were dried over anhydrous Na₂SO₄, filtered, and concentrated under reduced pressure. The residue was purified by silica gel column chromatography (EtOAc: PE = 0-20%) to give ethyl 3-(1-methylvinyl)-7-[2,3,5-tris(fluoranyl)phenyl]-1H-indole-2-carboxylate.

### Example 17.1 (d)

### Ethyl 3-(1-methylethyl)-7-[2,3,5-tris(fluoranyl)phenyl]-1H-indole-2-carboxylate

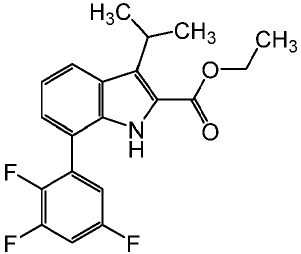

To a solution of ethyl 3-(1-methylvinyl)-7-[2,3,5-tris(fluoranyl)phenyl]-1H-indole-2-carboxylate (1.60 g, 3.70 mmol) in EtOAc (20 mL), was added Pd/C (180 mg, 1.69 mmol). To the above, hydrogen gas (1~2 atm) was introduced. The resulting mixture was stirred overnight at rt. The mixture was filtered and concentrated under vacuum. The residue was purified by silica gel chromatography (PE: EtOAc = 0-20%) to afford ethyl 3-(1-methylethyl)-7-[2,3,5-tris(fluoranyl)phenyl]-1H-indole-2-carboxylate.

### Example 17.1 (e)

### 3-(1-Methylethyl)-7-[2,3,5-tris(fluoranyl)phenyl]-1H-indole-2-carboxylic acid

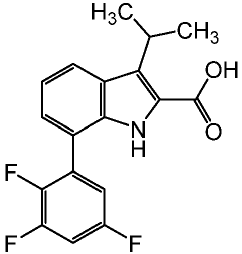

To a solution of ethyl 3-(1-methylethyl)-7-[2,3,5-tris(fluoranyl)phenyl]-1H-indole-2-carboxylate (400 mg, 1.11 mmol) in THF (3 mL), was added MeOH (3 mL) and LiOH (79.75 mg, 3.33 mmol) in H₂O (3 mL). The resulting mixture was stirred for 6 h at rt. Upon completion of the reaction, the solvents were removed *in vacuo* and the pH value of the mixture was adjusted to 4 by addition of aq. HCl (1 N). The resulting mixture was extracted with EtOAc (3 x). The organic layers were combined, dried over anhydrous Na₂SO₄, filtered and concentrated under vacuum to afford 3-(1-methylethyl)-7-[2,3,5-tris(fluoranyl)phenyl]-1H-indole-2-carboxylic acid.

### Example 17.1 (f)

### N-(2,3-Dihydro-1,4-benzoxazin-4-yl)-3-(1-methylethyl)-7-[2,3,5-tris(fluoranyl)phenyl]-1H-indole-2-carboxamide

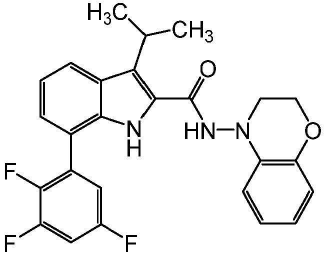

To a solution of 3-(1-methylethyl)-7-[2,3,5-tris(fluoranyl)phenyl]-1H-indole-2-carboxylic acid (300 mg, 900 µmol) in DMF (8 mL) was added 2,3-dihydro-1,4-benzoxazin-4-amine (203 mg, 1.35 mmol), DIPEA (232 mg, 1.80 mmol) and HATU (376 mg, 990 µmol). The resulting mixture was stirred at rt for 2 h. Upon completion of the reaction, the mixture was diluted with H₂O and extracted with EtOAc (3 x). The organic layers were combined, dried over anhydrous Na₂SO₄, filtered and concentrated under vacuum. The residue was purified by TLC (EtOAc: PE = 1: 10). The oil was repurified by Prep-HPLC (Mobile Phase A: H₂O (10 mmol/L NH₄HCO₃), Mobile Phase B: CH₃CN; Gradient: 57% B to 85% B in 7 min) to afford N-(2,3-dihydro-1,4-benzoxazin-4-yl)-3-(1-methylethyl)-7-[2,3,5-tris(fluoranyl)-phenyl]-1H-indole-2-carboxamide. ¹H-NMR (400 MHz, DMSO-d6): δ [ppm] = 7.92 (d, 1H), 7.68-7.65 (m, 1H), 7.36-7.15 (m, 3H), 6.84-6.65 (m, 4H), 4.35 (t, 2H), 3.96-3.93 (m, 1H), 3.63-3.68 (m, 2H), 1.64 (d, 6H). LCMS (Analytical Method E2): Rₜ = 2.327 min; m/z = 466.2 (M+H)⁺.

### Example 17.2

### N-(2,3-Dihydro-4H-benzo[b][1,4] oxazin-4-yl)-3-isopropyl-1-methyl-7-(2,3,5-trifluorophenyl)-1H-indole-2-carboxamide

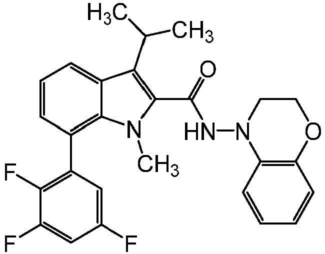

### Example 17.2 (a)

### Ethyl 3-isopropyl-1-methyl-7-(2,3,5-trifluorophenyl)-1H-indole-2-carboxylate

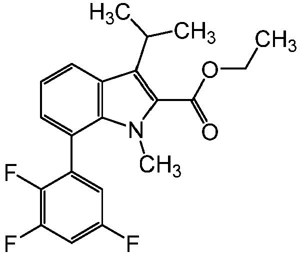

To a solution of ethyl 3-(1-methylethyl)-7-[2,3,5-tris(fluoranyl)phenyl]-1H-indole-2-carboxylate (Example 17.1 (d), 400 mg, 1.11 mmol) in DMF (8 mL) was added NaH (32 mg, 1.33 mmol, 60%) at 0°C. After 10 min at this temperature, MeI (174 mg, 1.22 mmol) was added to the solution and the resulting mixture was further stirred for 2 h at rt. Upon completion of the reaction, the reaction mixture was quenched with H₂O and extracted with EtOAc (3 x). The organic layers were combined, dried over anhydrous Na₂SO₄, filtered, and concentrated under vacuum. The residue was purified by Prep-TLC (EtOAc: PE = 1:1) to afford ethyl 3-isopropyl-1-methyl-7-(2,3,5-trifluorophenyl)-1H-indole-2-carboxylate.

### Example 17.2 (b)

### 3-Isopropyl-1-methyl-7-(2,3,5-trifluorophenyl)-1H-indole-2-carboxylic acid

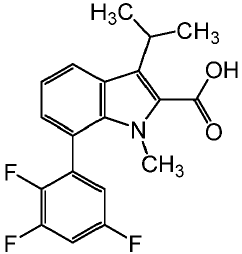

To a solution of ethyl 1-methyl-3-(1-methylethyl)-7-[2,3,5-tris(fluoranyl)phenyl]indole-2-carboxylate (280 mg, 477 µmol) in MeOH (2 mL) was added THF (2 mL) and LiOH (34 mg, 1.43 mmol) in H₂O (2 mL). The resulting mixture was stirred overnight at rt. Upon completion of the reaction, the solvents were removed in vacuo and H₂O was added, the pH value of the mixture was adjusted to 4 by addition of aq. HCl (1 M). The resulting mixture was extracted with EtOAc. The organic layers were combined (3 x), dried over anhydrous Na₂SO₄, filtered, and concentrated under vacuum to afford 1-methyl-3-(1-methylethyl)-7-[2,3,5-tris(fluoranyl)phenyl]indole-2-carboxylic acid.

### Example 17.2 (c)

### N-(2,3-Dihydro-4H-benzo[b][1,4] oxazin-4-yl)-3-isopropyl-1-methyl-7-(2,3,5-trifluorophenyl)-1H-indole-2-carboxamide

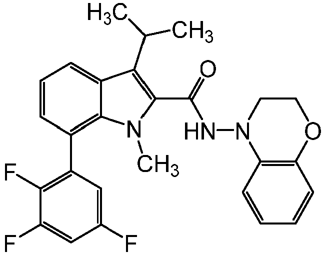

To a solution of 1-methyl-3-(1-methylethyl)-7-[2,3,5-tris(fluoranyl)phenyl]indole-2-carboxylic acid (80 mg, 230 µmol) in DMF (2 mL), was added 2,3-dihydro-1,4-benzoxazin-4-amine (52 mg, 345 µmol), HATU (96.3 mg, 253 µmol) and DIEA (59.4 mg, 461 µmol). The resulting mixture was stirred at rt for 2 h. Upon completion of the reaction, the mixture was diluted with H₂O and extracted with EtOAc (3 x). The organic layers were combined, dried over anhydrous Na₂SO₄, filtered and concentrated under vacuum. The residue was purified by TLC (EtOAc: PE = 1:2) to afford N-(2,3-dihydro-4H-benzo[b][1,4]oxazin-4-yl)-3-isopropyl-1-methyl-7-(2,3,5-trifluorophenyl)-1H-indole-2-carboxamide as a yellow oil. The oil was further-purified by Prep-HPLC [Mobile Phase A: H₂O (10 mmol/L NH₄HCO₃+0.1% NH₃-H₂O), Mobile Phase B: MeOH; Gradient: 73% B to 85% B in 10 min] to afford N-(2,3-dihydro-1,4-benzoxazin-4-yl)-1-methyl-3-(1-methylethyl)-7-[2,3,5-tris(fluoranyl)phenyl]indole-2-carboxamide. ¹H-NMR (400 MHz, DMSO-d6): δ [ppm] = 10.53 (s, 1H), 7.91 (d, 1H), 7.61-7.78 (m, 1H), 7.23-7.35 (m, 1H), 7.08-7.23 (m, 2H), 6.76-6.82 (m, 4H), 4.36 (t, 2H), 3.65-3.60 (m, 2H), 3.30-3.37 (m, 4H), 1.46 (t, 6H). LCMS (Analytical Method E2): Rₜ = 1.35 min; m/z = 502 (M+Na)⁺ .

### Example 18.1

### N-(2,3-Dihydro-4H-benzo[b][1,4] oxazin-4-yl)-4-(dimethylamino)-1-(2,3,5-trifluorophenyl)-1H-pyrrolo[2,3-b]pyridine-5-carboxamide

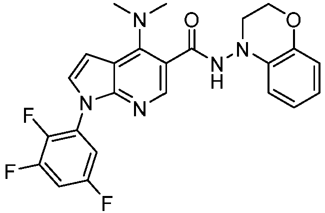

### Example 18.1 (a)

### Methyl 4-(dimethylamino)-1H-pyrrolo[2,3-b] pyridine-5-carboxylate

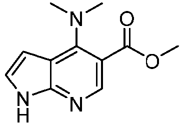

To a solution of methyl 4-chloranyl-1H-pyrrolo[2,3-b]pyridine-5-carboxylate (0.95 g, 4.51 mmol) in 1-butanol (20 mL) were added N-methylmethanamine (1.02 g, 22.6 mmol, 1.31 mL) and DIPEA (5.83 g, 45.1 mmol), then the resulting mixture was stirred at 130°C for 3 h under nitrogen atmosphere. After cooling down to rt, the mixture was concentrated under reduced pressure and treated with H₂O. The mixture was extracted with EtOAc, and the organic extracts were dried over anhydrous Na₂SO₄. After filtration, the filtrate was concentrated under reduced pressure. The residue was purified by silica gel column chromatography, eluting with PE/EtOAc (1:1) to afford methyl 4-[di(methyl)amino]-1H-pyrrolo[2,3-b] pyridine-5-carboxylate.

### Example 18.1 (b)

### Methyl 4-(dimethylamino)-1-(2,3,5-trifluorophenyl)-1H-pyrrolo[2,3-b]pyridine-5-carboxylate

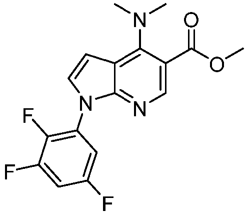

To a solution of methyl 4-[di(methyl)amino]-1H-pyrrolo[2,3-b]pyridine-5-carboxylate (1.00 g, 4.56 mmol) in 1,4-dioxane (20 mL) were added 1-bromanyl-2,3,5-tris(fluoranyl)benzene (1.92 g, 9.12 mmol, 1.09 mL), cuprous iodide (86.9 mg, 456 µmol, 15.46 µL), 1,2-diaminocyclohexane (104 mg, 912 µmol) and potassium phosphate tribasic (1.94 g, 9.12 mmol). The resulting mixture was stirred at 110°C for 16 h under the nitrogen atmosphere. After cooling down to rt, H₂O was added and the resulting mixture was extracted with EtOAc. The organic layer was dried over anhydrous Na₂SO₄. After filtration, the filtrate was concentrated under reduced pressure. The residue was purified by silica gel column chromatography, eluting with PE/EtOAc (49: 1) to afford to methyl 4-[di(methyl)amino]-1-[2,3,5-tris(fluoranyl)phenyl]pyrrolo[2,3-b]pyridine-5-carboxylate.

### Example 18.1 (c)

### 4-(Dimethylamino)-1-(2,3,5-trifluorophenyl)-1H-pyrrolo[2,3-b]pyridine-5-carboxylic acid

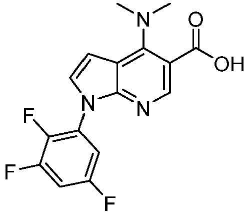

To a solution of methyl 4-[di(methyl)amino]-1-[2,3,5-tris(fluoranyl)phenyl]pyrrolo[2,3-b] pyridine-5-carboxylate (1.74 g, 4.98 mmol) in THF: H₂O: MeOH (15 mL, v: v: v = 1:1:1) was added LiOH monohydrate (627 mg, 14.9 mmol, 415 µL). The mixture was stirred at 60°C for 16 h. After cooling down to rt, the solvent were evaporated under reduced pressure. H₂O was added, and the resulting mixture was extracted with EtOAc. The aq. layer was adjusted to pH = 5 by addition of aq. HCl (1 M), and then extracted with EtOAc (3 x). The combined organic layers were dried over anhydrous Na₂SO₄. After filtration, the filtrate was concentrated under reduced pressure to afford 4-[di(methyl)amino]-1-[2,3,5-tris(fluoranyl)phenyl]pyrrolo[2,3-b]pyridine-5-carboxylic acid.

### Example 18.1 (d)

### N-(2,3-Dihydro-4H-benzo[b][1,4]oxazin-4-yl)-4-(dimethylamino)-1-(2,3,5-trifluoro-phenyl)-1H-pyrrolo[2,3-b]pyridine-5-carboxamide

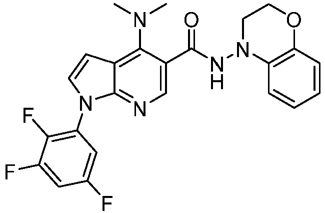

To a solution of 4-[di(methyl)amino]-1-[2,3,5-tris(fluoranyl)phenyl]pyrrolo[2,3-b] pyridine-5-carboxylic acid (250 mg, 745.65 µmol) in DMF (5 mL) was added HATU (340 mg, 894 µmol), DIPEA (482 mg, 3.73 mmol, 649 µL) and 2,3-dihydro-1,4-benzoxazin-4-amine (168 mg, 1.12 mmol). Then the resulting mixture was stirred at 70°C for 16 h. After cooling down to rt, H₂O was added and the resulting mixture was extracted with EtOAc (3 x). The combined organic layers were washed with H₂O, brine, dried over anhydrous Na₂SO₄ and then concentrated under reduced pressure. The residue was purified by prep-HPLC: [Column: XSelect CSH Fluoro Phenyl, 30* 150 mm, 5µm; Mobile Phase A: H₂O (0.1%FA), Mobile Phase B: CH₃CN; Flow rate: 60 mL/min; Gradient: 26% B to 56% B in 7 min] to afford N-(2,3-dihydro-1,4-benzoxazin-4-yl)-4-[di(methyl)amino]-1-[2,3,5-tris(fluoranyl)phenyl]pyrrolo[2,3-b]pyridine-5-carboxamide. ¹H-NMR (400 MHz, DMSO-d6): δ [ppm] = 10.38 (s, 1H), 8.17 (s, 1H), 7.77-7.70 (m, 1H), 7.69-7.53 (m, 2H), 7.04 (s, 1H), 6.89 (d, J = 8.8 Hz, 1H), 6.81-6.65 (m, 3H), 4.35 (t, J = 5.6 Hz, 2H), 3.64 (t, J = 5.6 Hz, 2H), 3.20 (s, 6H). LCMS (Analytical Method E1): Rt = 1.333 min; LCMS m/z = 468 (M+H)⁺.

### Example 19.1

### N-(2,3-Dihydro-1,4-benzoxazin-4-yl)-3-(1-methylethyl)-7-[2,3,5-tris(fluoranyl)-phenyl] benzofuran-2-carboxamide

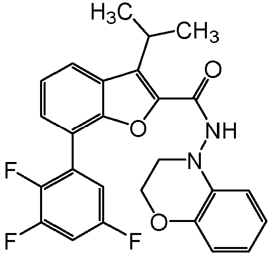

### Example 19.1 (a)

### Methyl 7-bromanylbenzofuran-2-carboxylate

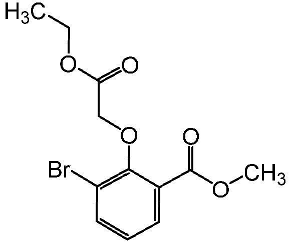

To a solution of methyl 3-bromanyl-2-oxidanyl-benzoate (5.00 g, 21.6 mmol) in acetone (50 mL), was added ethyl 2-bromanylacetate (3.61 g, 21.6 mmol) and K₂CO₃ (8.97 g, 64.9 mmol). The reaction mixture was stirred overnight at 60°C under nitrogen atmosphere. After cooling down to rt, the resulting mixture was filtered and concentrated under vacuum. The residue was purified by silica gel column chromatography (EtOAc: PE = 0-20%) to afford methyl 7-bromanylbenzofuran-2-carboxylate.

### Example 19.1 (b)

### Methyl 7-bromanyl-3-oxidanyl-benzofuran-2-carboxylate

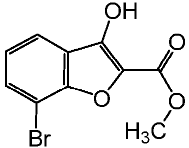

To a solution of methyl 3-bromanyl-2-(2-ethoxy-2-oxidanylidene-ethoxy) benzoate (7.00 g, 22.1 mmol) in MeOH (50 mL) was added sodium methanolate (2.38 g, 44.1 mmol) at rt. The reaction mixture was stirred overnight at rt under nitrogen atmosphere. After completion of the reaction, the reaction mixture was diluted with EtOAc, filtered, and then concentrated under reduced pressure. The residue was purified by silica gel column chromatography (EtOAc: PE = 0-20%) to afford to methyl 7-bromanyl-3-oxidanyl-benzofuran-2-carboxylate.

### Example 19.1 (c)

### Methyl 3-oxidanyl-7-[2,3,5-tris(fluoranyl)phenyl]benzofuran-2-carboxylate

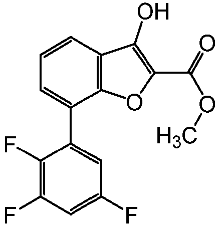

To a solution of methyl 7-bromanyl-3-oxidanyl-benzofuran-2-carboxylate (6.00 g, 22.1 mmol) in toluene (70 mL) was added [2,3,5-tris(fluoranyl)phenyl] boronic acid (7.79 g, 44.2 mmol), K₂CO₃ (44.3 g, 44.3 mmol), X-Phos (1.06 g, 2.21 mmol) and XPhos Pd G3 (1.87 g, 2.21 mmol). The resulting mixture was stirred overnight at 100°C under nitrogen atmosphere. After cooling down to rt, the mixture was treated with H₂O and extracted with EtOAc (3x). The combined organic layers were dried over anhydrous Na₂SO₄. After filtration, the filtrate was concentrated under reduced pressure. The residue was purified by silica gel column chromatography (EtOAc: PE = 0-20%) to given methyl 3-oxidanyl-7-[2,3,5-tris(fluoranyl)phenyl]benzofuran-2-carboxylate.

### Example 19.1 (d)

### Methyl 3-[tris(fluoranyl)methylsulfonyloxy]-7-[2,3,5-tris(fluoranyl)phenyl] benzofuran-2-carboxylate

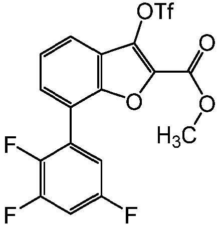

To a solution of methyl 3-oxidanyl-7-[2,3,5-tris(fluoranyl)phenyl]benzofuran-2-carboxylate (382 mg, 1.19 mmol) in CH₂Cl₂ (8 mL), was added NEt₃ (240 mg ,2.37 mmol) and Tf₂O (334 mg, 1.19 mmol) at 0°C. The resulting mixture was stirred overnight, where it was allowed to warm from 0°C to rt. Then, the mixture was filtered and concentrated. The residue was purified by silica gel column chromatography (PE: EtOAc = 0-20%) to afford methyl 3-[tris(fluoranyl)methylsulfonyloxy]-7-[2,3,5-tris(fluoranyl)phenyl] benzofuran-2-carboxylate.

### Example 19.1 (e)

### Methyl 3-(1-methylvinyl)-7-[2,3,5-tris(fluoranyl)phenyl]benzofuran-2-carboxylate

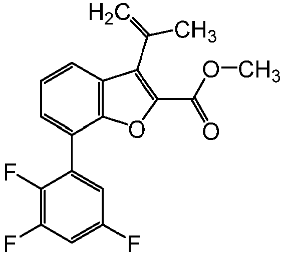

To a solution of methyl 3-[tris(fluoranyl)methylsulfonyloxy]-7-[2,3,5-tris(fluoranyl)-phenyl]benzofuran-2-carboxylate (482 mg, 1.06 mmol) in THF (5 mL), was added 4,4,5,5-tetra(methyl)-2-(1-methylvinyl)-1,3,2-dioxaborolane(214 mg, 1.27 mmol), Pd(dtbpf)Cl₂ (38.82 mg, 59.56 µmol) and K₃PO₄ (450 mg, 2.12 mmol) in H₂O (1.25 mL). The resulting mixture was stirred overnight at 90°C under nitrogen atmosphere. After cooling down to rt, the resulting mixture was treated with H₂O and extracted with EtOAc (3 x). The organic layers were combined, dried over anhydrous Na₂SO₄, filtered and concentrated under vacuum to afford methyl 3-(1-methylvinyl)-7-[2,3,5-tris(fluoranyl)phenyl]benzofuran-2-carboxylate.

### Example 19.1 (f)

### Methyl 3-(1-methylethyl)-7-[2,3,5-tris(fluoranyl)phenyl]benzofuran-2-carboxylate

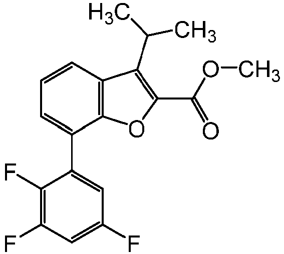

To a solution of methyl 3-(1-methylvinyl)-7-[2,3,5-tris(fluoranyl)phenyl]benzofuran-2-carboxylate (247 mg, 713 µmol) in EtOAc (5 mL), was added Pd/C (33 mg, 310 µmol). To the above hydrogen gas (1~2 atm) was introduced. The resulting mixture was stirred overnight at rt. The mixture was filtered and concentrated under vacuum. The residue was purified by silica gel column chromatography (PE: EtOAc = 0-20%) to afford methyl 3-(1-methylethyl)-7-[2,3,5-tris(fluoranyl)phenyl] benzofuran-2-carboxylate.

### Example 19.1 (g)

### 3-(1-Methylethyl)-7-[2,3,5-tris(fluoranyl)phenyl]benzofuran-2-carboxylic acid

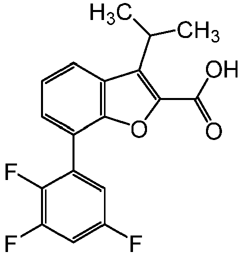

To a solution of methyl 3-(1-methylethyl)-7-[2,3,5-tris(fluoranyl)phenyl]benzofuran-2-carboxylate (150 mg, 431 µmol) in THF (1.5 mL), was added MeOH (1.5 mL) and LiOH (51.6 mg, 2.15 mmol) in H₂O (1.5 mL). The resulting mixture was stirred for 2 h at rt. Upon completion of the reaction, the solvents were removed in vacuo and the pH value was adjusted to 4 by addition of aq. HCl (1 M). The resulting mixture was extracted with EtOAc (3 x). The organic layers were combined, dried over anhydrous Na₂SO₄, filtered and concentrated under vacuum to afford 3-(1-methylethyl)-7-[2,3,5-tris(fluoranyl)phenyl] benzofuran-2-carboxylic acid. The product was used in the next step without further purification.

### Example 19.1 (h)

### N-(2,3-Dihydro-1,4-benzoxazin-4-yl)-3-(1-methylethyl)-7-[2,3,5-tris(fluoranyl)phenyl]-benzofuran-2-carboxamide

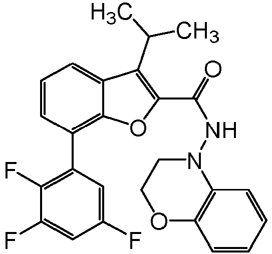

To a solution of 3-(1-methylethyl)-7-[2,3,5-tris(fluoranyl)phenyl]benzofuran-2-carboxylic acid (370 mg) in DMF (5 mL), was added 2,3-dihydro-1,4-benzoxazin-4-amine (249 mg, 1.66 mmol), DIPEA (286 mg, 2.21 mmol) and HATU (631 mg, 1.66 µmol). The resulting mixture was stirred at rt overnight. After this time, the reaction mixture was diluted with H₂O and extracted with EtOAc (3 x). The organic layers were combined, dried over anhydrous Na₂SO₄, filtered and concentrated under vacuum. The residue was purified by prep-TLC (EtOAc: PE = 1:10) to afford as a yellow oil. The oil was further purified by Prep-HPLC (Mobile Phase A: H₂O (10 mmol/L NH₄HCO₃ + 0.1%NH₃-H₂O), Mobile Phase B: CH₃CN; Gradient: 60% B to 78% B in 10 min) to afford N-(2,3-dihydro-1,4-benzoxazin-4-yl)-3-(1-methylethyl)-7-[2,3,5-tris(fluoranyl)phenyl]benzofuran-2-carbox-amide. ¹H-NMR (400 MHz, DMSO-d6): δ [ppm] = 10.54 (s, 1H), 8.09 (d, 1H), 7.71-7.68 (m, 1H), 7.61 (d, 1H), 7.53-7.46 (m, 2H), 6.77-6.69 (m, 4H), 4.36-4.34 (m, 2H), 4.12-4.08 (m, 1H), 3.62-3.60 (m, 2H), 1.42 (d, 6H). LCMS (Analytical Method E1): Rₜ = 2.065 min; m/z = 467 (M+H)⁺.

The compounds of formula (I) of the present invention are useful for the treatment and/or control, in particular helminths, in which the endoparasitic nematodes and trematodes may be the cause of serious diseases of mammals and poultry. Typical nematodes of this indication are: *Filariidae, Setariidae, Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris.* The trematodes include, in particular, the family of *Fasciolideae,* especially *Fasciola hepatica.*

Certain parasites of the species *Nematodirus, Cooperia* and *Oesophagostonum* infest the intestinal tract of the host animal, while others of the species *Haemonchus* and *Ostertagia* are parasitic in the stomach and those of the species *Dictyocaulus* are parasitic in the lung tissue. Parasites of the families and may be found in the internal cell tissue and in the organs, *e.g.* the heart, the blood vessels, the lymph vessels and the subcutaneous tissue. A particularly notable parasite is the heartworm of the dog, *Dirofilaria iminitis.* The parasites which may be treated and/or controlled by the compounds of formula (I) also include those from the class of *Cestoda* (tapeworms), *e.g.* the families *Mesocestoidae,* especially of the genus *Mesocestoides,* in particular *M. lineatus; Dipylidiidae,* especially *Dipylidium caninum, Joyeuxiella* spp., in particular *Joyeuxiella pasquali,* and *Diplopylidium* spp., and *Taeniidae,* especially *Taenia pisformis, Taenia cervi, Taenia ovis, Taeneia hydatigena, Taenia multiceps,Taenia taeniaeformis, Taenia serialis,* and *Echinococcus* spp., most particularly *Taneia hydatigena, Taenia ovis, Taenia multiceps, Taenia serialis; Echinococcus granulosus* and *Echinococcus multilocularis.*

Furthermore, the compounds of formula (I) are suitable for the treatment and/or control of human pathogenic parasites. Of these, typical representatives that appear in the digestive tract are those of the genus *Ancylostoma, Necator, Ascaris, Strongyloides, Trichinella, Capillaria, Trichuris* and *Enterobius.* The compounds of the present invention are also against parasites of the genus *Wuchereria, Brugia, Onchocerca* and *Loa* from the family of *Dracunculus* and parasites of the genus *Strongyloides* and *Trichinella,* which infect the gastrointestinal tract in particular.

A particular parasite to be treated and/or and controlled by the compounds of the invention is the heartworm *(Dirofilaria immitis).* Particular subjects for such treatment are dogs and cats.

The compounds of the invention can be administered alone or in the form of a composition. In practice, the compounds of the invention are usually administered in the form of compositions, that is, in admixture with at least one acceptable excipient. The proportion and nature of any acceptable excipient(s) are determined by the properties of the selected compound of the invention, the chosen route of administration, and standard practice as in the veterinary and pharmaceutical fields.

In one embodiment, the present invention provides compositions comprising: a compound of invention and at least one acceptable excipient.

In effecting such treatment and/or control, a compound of the invention can be administered in any form and route which makes the compound bioavailable. The compounds of the invention can be administered by a variety of routes, including orally, in particularly by tablets and capsules. The compounds of the invention can be administered parenteral routes, more particularly by inhalation, subcutaneously, intramuscularly, intravenously, intraarterially, transdermally, intranasally, rectally, vaginally, occularly, topically, sublingually, and buccally, intraperitoneally, intraadiposally, intrathecally and via local delivery for example by catheter or stent.

One skilled in the art can readily select the proper form and route of administration depending upon the particular characteristics of the compound selected, the disorder or condition to be treated, the stage of the disorder or condition, and other relevant circumstances. The pharmaceutical compositions of the invention may be administered to the subject, for example, in the form of tablets, capsules, cachets, papers, lozenges, wafers, elixirs, ointments, transdermal patches, aerosols, inhalants, suppositories, drenches, solutions, and suspensions.

The term "acceptable excipient" refers to those typically used in preparing veterinary and pharmaceutical compositions and should be pure and non-toxic in the amounts used. They generally are a solid, semi-solid, or liquid material which in the aggregate can serve as a vehicle or medium for the active ingredient. Some examples of acceptable excipients are found in Remington's Pharmaceutical Sciences and the Handbook of Pharmaceutical Excipients and include diluents, vehicles, carriers, ointment bases, binders, disintegrates, lubricants, glidants, sweetening agents, flavoring agents, gel bases, sustained release matrices, stabilizing agents, preservatives, solvents, suspending agents, buffers, emulsifiers, dyes, propellants, coating agents, and others.

In one embodiment, the composition is adapted for oral administration, such as a tablet or a capsule or a liquid formulation, for example, a solution or suspension, adapted for oral administration. In one embodiment, the composition is adapted for oral administration, such as chewable formulation, adapted for oral administration. In still another embodiment, the composition is a liquid or semi-solid formulation, for example, a solution or suspension or a paste, adapted for parenteral administration.

Particular compositions for usage on subjects in the treatment and/or control of nematodes/ helminths comprise solutions; emulsions including classical emulsions, microemulsions and self-emulsifying compositions, that are waterless organic, preferably oily, compositions which form emulsions, together with body fluids, upon addition to the subject's body; suspensions (drenches); pour-on formulations; food additives; powders; tablets including effervescent tablets; boli; capsules including micro-capsules; and chewable treats. Particularly composition forms are tablets, capsules, food additives or chewable treats.

The compositions of the present invention are prepared in a manner well known in the veterinary and pharmaceutical art and include at least one of the compounds of the invention as the active ingredient. The amount of a compound of the present invention may be varied depending upon its particular form and may conveniently be between 1% to about 50% of the weight of the unit dose form. The present pharmaceutical compositions are preferably formulated in a unit dose form, each dose typically containing from about 0.5 mg to about 100 mg of a compounds of the invention. One or more unit dose form(s) may be taken to affect the treatment dosage.

In one embodiment, the present invention also provides a method for treating parasites, comprising: administering to a subject in need thereof an effective amount of a compound of formula (I) or a salt thereof, the method optionally further comprising an effective amount of at least one additional active compound.

In one embodiment, the present invention also provides a method for controlling parasites, comprising: administering to a subject in need thereof an effective amount of a compound of formula (I) or a salt thereof, the method optionally further comprising an effective amount of at least one additional active compound.

In one embodiment, the present invention also provides a method for treating or controlling parasites, comprising: contacting a subject's environment with an effective amount of a compound of formula (I) or a salt thereof, the method optionally further comprising an effective amount of at least one additional active compound.

Thus, the invention provides for the use of the compounds of the invention as a medicament, including for the manufacture of a medicament. In one embodiment, the invention provides the manufacture of a medicament comprising a compound of formula (I) or a salt thereof for treating parasites. In one embodiment, the invention provides the manufacture of a medicament comprising a compound of the invention or a salt thereof for controlling parasites.

The terms "treating", "to treat", "treated", or "treatment", include without limitation restraining, slowing, stopping, reducing, ameliorating, reversing the progression or severity of an existing symptom, or preventing a disorder, condition, or disease. For example, an adult heartworm infection would be treated by administering a compound of the invention. A treatment may be applied or administered therapeutically.

The terms "control", "controlling" or "controlled" refers to include without limitation decreasing, reducing, or ameliorating the risk of a symptom, disorder, condition, or disease, and protecting an animal from a symptom, disorder, condition, or disease. Controlling may refer to therapeutic, prophylactic, or preventative administration. It is well understood that a larvae or immature heartworm infection may be asymptomatic and infection by mature parasites is symptomatic and/or debilitating. Therefore, for example, a heartworm infection would be controlled by acting on the larvae or immature parasite preventing the infection from progressing to an infection by mature parasites.

Thus, the use of the compounds of the invention in the treatment and/or control of parasites, in particular helminths, in which the endoparasitic nematodes and trematodes refers to the use of the compounds of the invention to act on the various forms of the parasites throughout its life cycle, independent of whether a subject is manifesting a symptom, including morbidity or mortality, and independently of the phase(s) of the parasitic challenge.

As used herein, "administering to a subject" includes but is not limited to cutaneous, subcutaneous, intramuscular, mucosal, submucosal, transdermal, oral or intranasal administration. Administration could include injection or topical administration.

The terms "subject" and "patient" refers includes humans and non-human mammalian animals, such as dogs, cats, mice, rats, guinea pigs, rabbits, ferrets, cows, horses, sheep, goats, and pigs. It is understood that a more particular subject is a human. Also, a more particular subject are mammalian pets or companion animals, such as dogs and cats and also mice, guinea pigs, ferrets, and rabbits.

The term "effective amount" refers to an amount which gives the desired benefit to the subject and includes administration for both treatment and control. The amount will vary from one individual subject to another and will depend upon a number of factors, including the overall physical condition of the subject and the severity of the underlying cause of the condition to be treated, concomitant treatments, and the amount of compound of the invention used to maintain desired response at a beneficial level.

An effective amount can be readily determined by the attending diagnostician, as one skilled in the art, by the use of known techniques and by observing results obtained under analogous circumstances. In determining the effective amount, the dose, a number of factors are considered by the attending diagnostician, including, but not limited to: the species of patient; its size, age, and general health; the specific condition, disorder, infection, or disease involved; the degree of or involvement or the severity of the condition, disorder, or disease, the response of the individual patient; the particular compound administered; the mode of administration; the bioavailability characteristics of the preparation administered; the dose regimen selected; the use of concomitant medication; and other relevant circumstances. An effective amount of the present invention, the treatment dosage, is expected to range from 0.5 mg to 100 mg. Specific amounts can be determined by the skilled person. Although these dosages are based on a subject having a mass of about 1 kg to about 20 kg, the diagnostician will be able to determine the appropriate dose for a subject whose mass falls outside of this weight range. An effective amount of the present invention, the treatment dosage, is expected to range from 0.1 mg/kg to 10 mg/kg of the subject. The dosing regimen is expected to be daily, weekly, or monthly administration.

The compounds of the invention may be combined with one or more other active compounds or therapies for the treatment of one or more disorders, diseases or conditions, including the treatment of parasites, for which it is indicated. The compounds of the invention may be administered simultaneously, sequentially or separately in combination with one or more compounds or therapies for treating parasites and other disorders.

For example, when used to treat parasites, including heartworm, a compound of the invention may be combined with a macrocyclic lactone such as ivermectin, moxidectin, or milbemycin oxime, or with imidacloprid. Particular combinations for treating parasites include a compound of the invention and ivermectin. Another particular combination for treating parasites include a compound of the invention and milbemycin oxime.

Thus, it is understood that the compositions and methods of the present invention optionally include comprising an effective amount of at least one additional active compound.

The activity of compounds as parasiticides may be determined by a variety of methods, including in vitro and in vivo methods.

### EXPERIMENTAL SECTION - BIOLOGICAL ASSAYS

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

### Animal parasitic nematodes in vitro

The in vitro activity of the compounds of the present invention can be demonstrated in the following assays:

### In vitro assay 1: C. elegans Slo-1a - Action at a recombinant C. elegans cell line

### Generation of a stable C. elegans CHO cell line

A CHO cell line was obtained from ATCC, code ATCC CRL-9096. For transfection with plasmid DNA to express *C. elegans* Slo-1a (accession number AAL28102) CHO cells were passaged to 40% confluence before adding the transfection solution to the cell culture. The transfection solution included 300 µL OptiMEM (Life Technologies, Nr.: 31985), 2 µL (= 6 µg) of plasmid DNA containing the *C. elegans* Slo-1a gene and 9µL FugeneHD (Promega, Nr.: E2311), and was added to the cells prior to incubation for 48 hours at 37°C, 5% CO₂. The transfection medium was exchanged for the selection medium which contains additional G418 (2 mg/ml, Invitrogen, Nr.: 10131) and the cells were seeded into 384 well plates (300 cells/well). After a few weeks, the remaining surviving cells were tested with a voltage sensitive dye (Membrane Potential Assay Kit, Molecular Devices Nr.: R8034) for K+ channel expression. Positive cell clones were purified by the limited dilution technique. For this the clone with the highest and most robust signal in the voltage sensitive dye assay was further subcloned (incubated) in 384 well plates (0.7 cells/well) to obtain clonal purity. This generated a final stable CHO cell line expressing the *C. elegans* Slo-1a.

### Cell culture conditions

Cells were cultured at 37 °C and 5% CO₂ in MEMalpha with Gutamax I (Invitrogen, Nr.: 32571), supplemented with 10% (v/v) heat inactivated fetal bovine serum (Invitrogen, Nr.: 10500), G418 (1 mg/ml, Invitrogen, Nr.: 10131). Cells were detached using Accutase (Sigma, Nr.: A6964).

### Membrane potential measurements

Laboratory compound testing was performed on 384-well microtiter plates (MTPs, Greiner, Nr.: 781092). 8000 cells/well were plated onto 384-well MTPs and cultured for 20 to 24 hours at 37 °C and 5% CO₂. After removal of the cell culture medium, the cells were washed once with tyrode (150 mM NaCl, 0.3 mM KCl, 2 mM CaCl₂, 1m M MgCl₂, 0.8 mM NaH₂PO₄, 5 mM Glucose, 28 mM Hepes, pH 7.4) and then loaded with the voltage sensitive dye of the Membrane Potential Assay Kit diluted in tyrode for 1 h at room temperature. After starting the measurement of fluorescence using a FLIPR Tetra (Molecular Devices, Exc. 510-545 nm, Emm. 565-625 nm), test compounds were added followed by the addition of KCl tyrode (final assay concentration: 70 mM KCl, 2 mM CaCl₂, 1 mM MgCl₂, 0.8 mM NaH₂PO₄, 5 mM Glucose, 28 mM Hepes, pH 7.4, including the voltage sensitive dye). The measurement was completed after 7 minutes.

### Statistics

EC₅₀ values were calculated using a four-parameter plotting by the Scilligence ELN /Regmol Software Tool, Bioassay.

For the following examples, EC₅₀ of < 0.1 µM has been found for: 1.1, 2.2, 3.1, 4.1, 4.2, 4.3, 6.1, 6.3, 6.4, 6.5, 6.7, 6.8.

For the following examples, EC₅₀ > 0.1 µM to < 1 µM has been found for: 2.1, 5.1, 6.2, 6.6, 10.1, 11.1, 13.1, 18.1.

For the following examples, EC₅₀ > 1 µM has been found for: 7.1, 8.1, 9.1, 10.2, 12.1, 14.1, 15.1, 16.1, 17.1, 17.2.

### In vitro assay 2: D. immitis Slo-1 - Action at a recombinant D. immitis cell line

### Generation of a stable D. immitis Slo-1 CHO cell line

A CHO cell line was obtained from ATCC, code ATCC CRL-9096. For transfection with plasmid DNA to express *D. immitis* Slo-1 (based on Protein sequence JQ730003, codon optimized for hamster) CHO cells were passaged to 40% confluence before adding the transfection solution to the cell culture. The transfection solution included 300 µL OptiMEM (Life Technologies, Nr.: 31985), 2 µL (= 6 µg) of plasmid DNA containing the *D. immitis* Slo-1 gene and 9µL FugeneHD (Promega, Nr.: E2311), and was added to the cells prior to incubation for 48 hours at 37°C, 5% CO₂. The transfection medium was exchanged for the selection medium which contains additional G418 (2 mg/ml, Invitrogen, Nr.: 10131) and the cells were seeded into 384 well plates (300 cells/well). After a few weeks, the remaining surviving cells were tested with a voltage sensitive dye (Membrane Potential Assay Kit, Molecular Devices Nr.: R8034) for K+ channel expression. Positive cell clones were purified by the limited dilution technique. For this the clone with the highest and most robust signal in the voltage sensitive dye assay was further subcloned (incubated) in 384 well plates (0.7 cells/well) to obtain clonal purity. This generated a final stable CHO cell line expressing the *D. immitis* Slo-1.

### Cell culture conditions

Cells were cultured at 37 °C and 5% CO₂ in MEMalpha with Gutamax I (Invitrogen, Nr.: 32571), supplemented with 10% (v/v) heat inactivated fetal bovine serum (Invitrogen, Nr.: 10500), G418 (1 mg/ml, Invitrogen, Nr.: 10131). Cells were detached using Accutase (Sigma, Nr.: A6964).

### Membrane potential measurements

Laboratory compound testing was performed on 384-well microtiter plates (MTPs, Greiner, Nr.: 781092). 8000 cells/well were plated onto 384-well MTPs and cultured for 20 to 24 hours at 37 °C and 5% CO₂. After removal of the cell culture medium, the cells were washed once with tyrode (150 mM NaCl, 0.3 mM KCl, 2 mM CaCl₂, 1 mM MgCl₂, 0.8 mM NaH₂PO₄, 5mM Glucose, 28 mM Hepes, pH 7.4) and then loaded with the voltage sensitive dye of the Membrane Potential Assay Kit diluted in tyrode for 1 h at room temperature. After starting the measurement of fluorescence using a FLIPR Tetra (Molecular Devices, Exc. 510-545 nm, Emm. 565-625 nm), test compounds were added followed by the addition of KCl tyrode (final assay concentration: 70 mM KCl, 2 mM CaCl₂, 1 mM MgCl₂, 0.8 mM NaH₂PO₄, 5 mM Glucose, 28 mM Hepes, pH 7.4, including the voltage sensitive dye). The measurement was completed after 7 minutes.

### Statistics

EC₅₀ values were calculated using a four-parameter plotting by the Scilligence ELN / Regmol Software Tool, Bioassay.

For the following examples, EC₅₀ of < 0.1 µM has been found for: 6.1, 6.3, 6.5, 6.7.

For the following examples, EC₅₀ > 0.1 µM to < 1 µM has been found for: 2.1, 2.2, 3.1, 4.3, 6.4, 6.8.

For the following examples, EC₅₀ > 1 µM has been found for: 1.1, 4.1, 4.2, 5.1, 6.2, 6.6, 7.1, 8.1, 9.1, 10.1, 10.2, 11.1, 12.1, 13.1, 14.1, 15.1, 16.1, 17.1, 17.2, 18.1.

### In vitro assay 3: Dirofilaria immitis microfilariae (DIROIM L1)

≥ 250 Dirofilaria immitis microfilariae, which were freshly purified from blood, were added to wells of a microtitre plate containing a nutrient medium and the test compound in DMSO. Compounds were tested in concentration-response assay in duplicate. Larvae exposed to DMSO and no test compounds were used as negative controls. Larvae were evaluated after 72 h of incubation with the compound. Efficacy was determined as the reduction of motility in comparison to the negative control. Based on the evaluation of a wide concentration range, concentration-response curves as well as EC₅₀-values were calculated.

For the following examples, EC₅₀ of < 0.1 ppm has been found for: 1.1, 2.2, 3.1, 4.2, 4.3, 5.1, 6.1, 6.2, 6.3, 6.4, 6.5, 6.6, 6.7, 6.8, 9.1, 14.1, 17.1.

For the following examples, EC₅₀ > 0.1 ppm to < 1 ppm has been found for: 2.1, 4.1, 7.1, 8.1, 10.1, 10.2, 11.1, 12.1, 13.1, 15.1.

For the following examples, EC₅₀ > 1 ppm has been found for: 16.1.

### In vitro assay 4: Dirofilaria immitis (DIROIML4)

*10 Dirofilaria immitis* third-stage larvae, which were freshly isolated from their vector (intermediate host), were added to wells of a microtitre plate containing a nutrient medium and the test compound in DMSO. Compounds were tested in concentration-response assay in duplicate. Larvae exposed to DMSO and no test compounds were used as negative controls. Larvae were evaluated after 72 h of incubation with the compound. Within these 72 h of incubation the majority of larvae in negative control moult to fourth-stage larvae. Efficacy was determined as the reduction of motility in comparison to the negative control. Based on the evaluation of a wide concentration range, concentration-response curves as well as EC₅₀-values were calculated.

For the following examples, EC₅₀ of < 0.1 ppm has been found for: 1.1, 2.2, 3.1, 4.1, 4.2, 4.3, 6.1, 6.3, 6.4, 6.5, 6.7, 6.8, 10.1, 11.1, 12.1, 18.1, 19.1.

For the following examples, EC₅₀ > 0.1 ppm to < 1 ppm has been found for: 2.1, 5.1, 6.2, 6.6, 7.1, 8.1, 9.1, 13.1.

For the following examples, EC₅₀ > 1 ppm has been found for: 10.2, 14.1, 15.1, 16.1, 17.1, 17.2.

### In vitro assay 5: Nippostrongylus brasiliensis (NIPOBR)

Adult *Nippostrongylus brasiliensis* were washed with saline buffer containing 100 U/ml penicillin, 0.1 mg/ml streptomycin and 2.5 µg/ml amphotericin B. Test compounds were dissolved in DMSO and worms were incubated in medium in a final concentration of 10 µg/ml (10 ppm), 1 µg/ml (1 ppm) and 0.1 µg/ml (0.1 ppm) respectively. An aliquot of the medium was used to determine the acetylcholine esterase activity in comparison to a negative control. The principle of measuring acetylcholine esterase as readout for anthelmintic activity was described in Rapson et al (1986) and Rapson et al (1987).

Based on the evaluation of a wide concentration range, concentration-response curves as well as EC₅₀-values were calculated.

For the following examples, EC₅₀ of < 0.1 ppm has been found for: 6.1, 6.3, 6,7.

For the following examples, EC₅₀ > 0.1 ppm to < 1 ppm has been found for: 1.1, 2.2, 4.2, 4.3, 6.4, 6.5, 19.1.

For the following examples, EC₅₀ > 1 ppm has been found for: 2.1, 3.1, 4.1, 5.1, 6.2, 6.6, 6.8, 7.1, 8.1, 9.1, 10.1, 10.2, 11.1, 12.1, 13.1, 14.1, 15.1, 16.1, 17.1, 17.2, 18.1.

### Animal parasitic nematodes in vivo

### Haemonchus contortus I Trichostrongylus colubriformis in gerbil

Gerbils, experimentally infected with *Haemonchus* and/or *Trichostrongylus,* were treated once during late prepatency. Test compounds were formulated as solutions or suspensions and applied orally or intraperitoneally. For both applications the same service formulation was used. The volume of the application amounted to normally 20 ml/kg at a maximum. By way of example, a gerbil with 40 g body weight was treated with 0.200 mL of the formulation of formulation example Fl. This corresponded to a treatment with 20 mg/kg body weight.

Efficacy was determined per group as reduction of worm count in stomach and small intestine, respectively, after necropsy compared to worm count in an infected and placebo-treated control group.

The following examples were tested and had an activity of ≥ 60% or higher at the given treatment: 6.1 and 18.1

### Formulation Example

Exemplary formulations consisted of the active substance in 10% Transcutol, 10% Cremophor EL and 80% isotonic saline solution. First the active substance was dissolved in Transcutol. After solution in Transcutol, Cremophor and isotonic saline solution were added. These formulations were used as service formulations in the following *in vivo* assay.

An example for a formulation according to the present invention is the following formulation Example Fl . Therein, the active substance was dissolved in Transcutol to form a stock solution A. Then 0.100 mL of this stock solution A were taken and 0.100 mL Cremophor EL and 0.800 mL isotonic saline solution were added. The resulting liquid formulation (formulation example Fl) had a volume of 1 mL.

### Stock solution A:

4.0 mg compound,
0.100 mL Transcutol.

### Formulation example F1:

0.100 mL stock solution A,
0.100 mL Cremophor EL, and
0.S00 mL isotonic saline solution.

## Claims

1. A compound of formula (I) 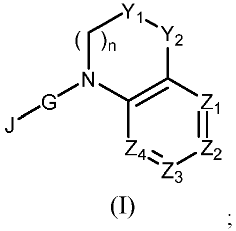 wherein
n is 0 or 1;
J is selected from the group consisting of 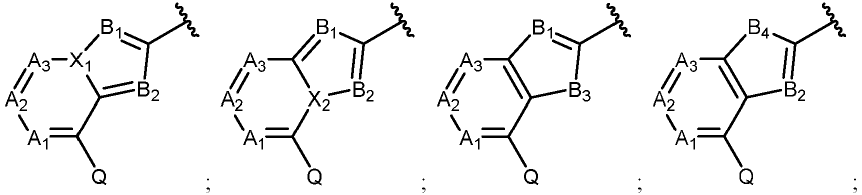 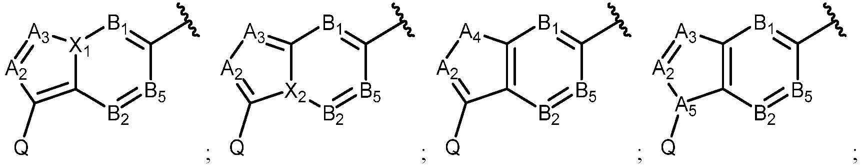 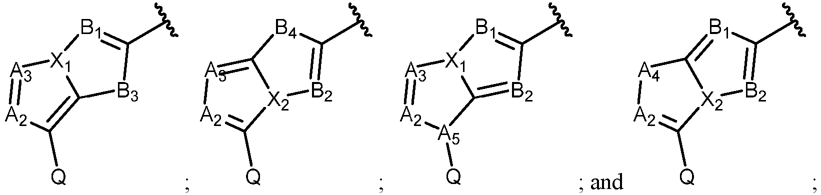
A₁ is selected from the group consisting of N and CR_{A1}
A₂ is selected from the group consisting of N and CR_{A2};
A₃ is selected from the group consisting of N and CR_{A3};
A₄ is selected from the group consisting of O, S, and NR_{A4};
A₅ is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 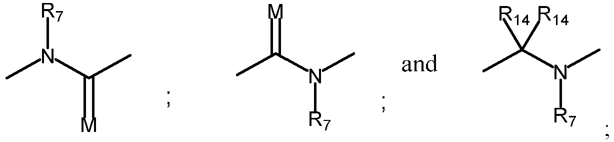
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R_{A1}, R_{A2}, R_{A3}, R_{B2}, and R_{B5} are each independently selected from the group consisting of hydrogen, halogen, hydroxyl, -SH, -SC₁-C₄ alkyl, -S(O)(C₁-C₄ alkyl), -S(O)₂(C₁-C₄ alkyl), cyano, C₁-C₄ alkyl, C₁-C₄ halogenoalkyl, C₁-C₄-alkoxy, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; -NH₂, -NH(C₁-C₄ alkyl), and -N(C₁-C₄ alkyl)₂;
R_{A4} and R_{B3} are independently selected from the group consisting of hydrogen, C₁-C₄ alkyl, and C₁-C₄ halogenoalkyl;
R_{B1} is selected from the group consisting of halogen, cyano, -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), - N(C₁-C₄ alkyl)(4- to 7-membered heterocycloalkyl), -NH(4- to 7-membered heterocycloalkyl), - N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, -S(O)C₁-C₄ alkyl, -SO₂C₁-C₄ alkyl, -B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl having at least one nitrogen atom via which the 5-membered heteroaryl ring is connected to the rest of the molecule, and 6-membered heteroaryl having at least one nitrogen atom; each of the aryl, heterocycloalkyl, and heteroaryl rings in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, - NH(C₃-C₆ cycloalkyl), -N(C₁-C₄ alkyl)(C₃-C₆-cycloalkyl), -NHSO₂(C₁-C₄ alkyl), -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B1} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), -N(C₁-C₄ alkyl)₂, - NH(C₃-C₆ cycloalkyl), -N(C₁-C₄ alkyl)(C₃-C₆-cycloalkyl), -NHSO₂(C₁-C₄ alkyl), -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B1} may be optionally substituted with 1, 2 or 3 substituents independently selected from the group consisting of halogen, hydroxy, oxo, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B4} is selected from the group consisting of -CHO, hydroxyl, C₁-C₄ alkyl, C₂-C₄ alkenyl, C₂-C₄ alkynyl, C₃-C₆ cycloalkyl, C₁-C₄ halogenoalkyl, C₁-C₄-alkoxy substituted-C₁-C₄ alkyl, benzyl optionally substituted with 1 to 5 halogen atoms, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -N(C₁-C₄ alkyl)(4- to 7-membered heterocycloalkyl), -NH(4- to 7-membered heterocycloalkyl), -N(C₁-C₄ alkyl)( C₁-C₄ alkoxy), -C(O)NH(C₁-C₄ alkyl), -C(O)N(C₁-C₄ alkyl)₂, -C(O)N(C₁-C₄ alkyl)(4- to 7-membered heterocycloalkyl), -NHSO₂(C₁-C₄ alkyl), -SC₁-C₄ alkyl, -S(O)C₁-C₄ alkyl, -SO₂C₁-C₄ alkyl, - B(OR₁₅)(OR₁₆) wherein R₁₅ is, each time taken, selected from the group consisting of hydrogen, C₁-C₄ alkyl, and C₃-C₆ cycloalkyl, R₁₆ is, each time taken, selected from the group consisting of hydrogen, C₁-C₄ alkyl, and C₃-C₆ cycloalkyl, or R₁₅ and R₁₆ together with the oxygen atoms to which they are attached form a 5- to 7- membered ring which is optionally substituted with 1 to 4 C₁-C₄ alkyl; 6- or 10 membered aryl; a monocyclic heterocycle selected from the group of 4- to 7-membered heterocycloalkyl, 5-membered heteroaryl, and 6-membered heteroaryl; each of the aryl, heterocycloalkyl, and heteroaryl rings in R_{B4} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; wherein the C₃-C₆ cycloalkyl and the heterocycloalkyl rings in R_{B4} are optionally substituted with a spiro group, wherein said spiro group is a 3- to 6-membered cycloalkyl or 4- to 6-membered heterocycloalkyl containing 1, 2, or 3 heteroatoms independently selected from N, S or O, wherein said spiro group is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and wherein each C₁-C₄ alkyl, C₃-C₆ cycloalkyl and C₁-C₄ alkoxy in R_{B4} may be optionally substituted with 1, 2 or 3 substituents independently selected from the group consisting of halogen, hydroxy, oxo, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is selected from the group consisting of hydrogen, C₁-C₄ alkyl, and C₃-C₆ cycloalkyl optionally substituted with 1 to 5 halogen atoms, -C(H)O, C₂-C₄ alkenyl, C₂-C₄ alkynyl, C₁-C₄ halogenoalkyl, and C₁-C₄-alkoxy;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
R₁₀ is selected from the group consisting of hydrogen and C₁-C₄ alkyl;
R₁₁ is, each time selected, independently selected from the group consisting of hydrogen, halogen, hydroxyl, cyano, C₁-C₄ alkyl, C₁-C₄ halogenoalkyl, C₁-C₄-alkoxy, C₃-C₆ cycloalkyl, - NH₂, -NH(C₁-C₄ alkyl), and -N(C₁-C₄ alkyl)₂; and
Q is selected from the group consisting of
(i) 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl;
(ii) 5- to 10-membered heteroaryl having 1, 2, or 3 heteroatoms independently selected from the group O, S, and N and wherein the carbons of the 5- to 10-membered heteroaryl are optionally substituted with 1, 2, 3, 4, or 5 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, benzyloxy, -C(O)R₁₇, -NH₂, -NH(C₁-C₄ alkyl), -N(C₁-C₄ alkyl)₂, -SC₁-C₄ alkyl, - S(O)C₁-C₄ alkyl, -SO₂C₁-C₄ alkyl, -S(O)C₁-C₄-halogenoalkyl and -SO₂C₁-C₄ halogenoalkyl, and any N in the heteroaryl, valency permitting, is optionally substituted with a substituent selected from the group consisting of hydrogen, C₁-C₄ alkyl, and C₃-C₆ cycloalkyl;
(iii) 4- to 7-membered heterocycloalkyl having 1, 2, or 3 heteroatoms independently selected from the group O, S, N, wherein the heterocycloalkyl is optionally benzo-fused, wherein the carbons of the 4- to 7-membered heterocycloalkyl or optionally benzo-fused 4- to 7-membered heterocycloalkyl are optionally substituted with 1, 2, 3, or 4 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), and -N(C₁-C₄ alkyl)₂ and any N in the heterocycloalkyl is optionally substituted with a substituent selected from the group consisting of hydrogen, C₁-C₄ alkyl, and C₃-C₆ cycloalkyl;
(iv) 6- or 10 membered aryloxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
(v) 6- or 10 membered arylthio-oxy optionally substituted with 1, 2 or 3 substituents independently selected from the group consisting of halogen, cyano, nitro, hydroxyl, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl; and
(vi) 5- to 10-membered heteroaryloxy optionally substituted with 1, 2 or 3 substituents independently selected from the group consisting of halogen, cyano, nitro, hydroxyl, oxo, C₁-C₄ alkyl, C₃-C₆ cycloalkyl, C₁-C₄ halogenoalkyl, C₁-C₄ alkoxy, -C(O)R₁₇, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
R₁₃ is selected from the group consisting of hydroxy, C₁-C₄ alkoxy, and -NH₂;
R₁₄ is, each time selected, independently selected from the group consisting of hydrogen, halogen, cyano, nitro, hydroxyl, C₁-C₄ alkyl, C₃-C₆ cycloalkyl, C₁-C₄ halogenoalkyl, C₁-C₄ alkoxy, C₁-C₄ halogenalkoxy, -NH₂, -NH(C₁-C₄ alkyl), and -N(C₁-C₄ alkyl)₂; and
R₁₇ is, each time selected, independently selected from the group consisting of C₁-C₄ alkyl, C₃-C₆ cycloalkyl, C₁-C₄ halogenoalkyl, C₁-C₄ alkoxy, C₁-C₄ halogenalkoxy, -OH, -NH₂, - NH(C₁-C₄ alkyl), -N(C₁-C₄ alkyl)₂, -NH(C₃-C₆ cycloalkyl), and -N(C₁-C₄ alkyl)(C₃-C₆-cycloalkyl);
wherein, when J is 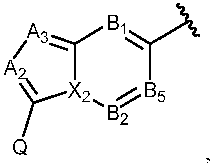 A₂ is N, and A₃, B₁, B₂, B₅, X₂, and Q are as defined above;
or a salt thereof.

2. The compound according to claim 1, wherein
n is 0 or 1,
J is selected from the group consisting of 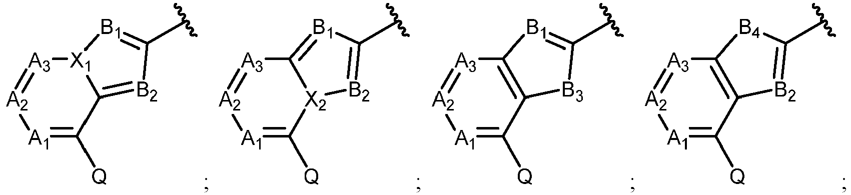 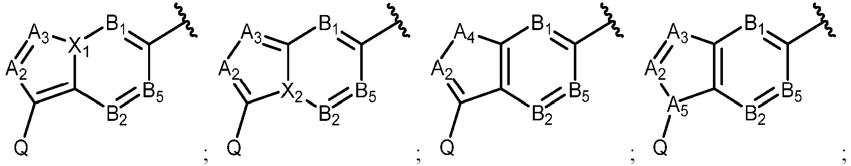 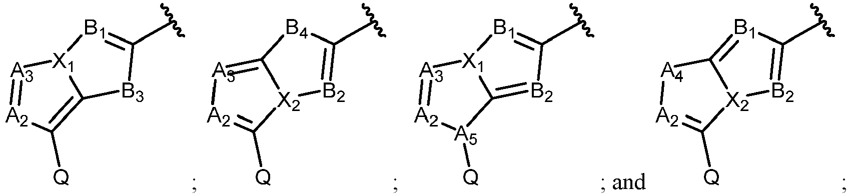
A₁ is selected from the group consisting of N and CR_{A1}
A₂ is selected from the group consisting of N and CR_{A2};
A₃ is selected from the group consisting of N and CR_{A3};
A₄ is selected from the group consisting of O, S, and NR_{A4};
A₅ is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 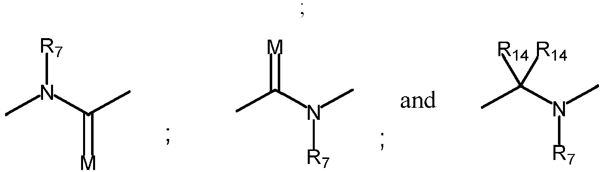
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R_{A1}, R_{A2}, R_{A3}, R_{B2}, and R_{B5} are each independently selected from the group consisting of hydrogen, halogen, hydroxyl, -SH, NH₂, C₁-C₄ alkyl, C₁-C₄ halogenoalkyl, C₁-C₄-alkoxy, and -SC₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{A4} and R_{B3} are independently selected from the group consisting of hydrogen, halogen, hydroxyl, -SH, NH₂, C₁-C₄alkyl, C₁-C₄ halogenoalkyl, C₁-C₄-alkoxy, and -SC₁-C₄ alkyl, wherein each C₁-C₄ alkyl in R_{A4} and R_{B3} may be optionally substituted with 1, 2 or 3 substituents independently selected from the group consisting of halogen, hydroxy, - NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B1} is selected from the group consisting of halogen, hydroxyl, -SH, NH₂, C₁-C₄ alkyl, C₃-C₆ cycloalkyl, C₁-C₄ halogenoalkyl, C₁-C₄-alkoxy, -SC₁-C₄ alkyl, and -NH(C₁-C₄ alkyl), -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is selected from the group consisting of halogen, hydroxyl, -SH, NH₂, C₁-C₄ alkyl, C₃-C₆ cycloalkyl, C₁-C₄ halogenoalkyl, C₁-C₄-alkoxy, and -SC₁-C₄ alkyl,, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is selected from the group consisting of hydrogen, C₁-C₄ alkyl, and C₃-C₆ cycloalkyl optionally substituted with 1 to 5 halogen atoms;
R₈ is, each time selected, independently selected from the group consisting of hydrogen, fluoro, and C₁-C₄ alkyl;
R₉ is, each time selected, independently selected from the group consisting of hydrogen, fluoro, and C₁-C₄ alkyl;
R₁₀ is selected from the group consisting of hydrogen and C₁-C₄ alkyl;
R₁₁ is selected from the group consisting of hydrogen, halogen, C₁-C₄ alkyl, C₁-C₄ halogenoalkyl, C₃-C₆ cycloalkyl;
Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R₁₃ is selected from the group consisting of hydroxy, C₁-C₄ alkoxy, and -NH₂;
R₁₄ is, each time selected, independently selected from the group consisting of hydrogen, and halogen; and
R₁₇ is, each time selected, independently selected from the group consisting of C₁-C₄ alkyl, C₃-C₆ cycloalkyl, C₁-C₄ halogenoalkyl, C₁-C₄ alkoxy, C₁-C₄ halogenalkoxy, -OH, -NH₂, -NH(C₁-C₄ alkyl), -N(C₁-C₄ alkyl)₂, -NH(C₃-C₆ cycloalkyl), and -N(C₁-C₄ alkyl)(C₃-C₆-cycloalkyl);
wherein, when J is 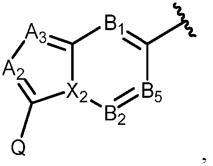 A₂ is N, and A₃, B₁, B₂, B₅, X₂, and Q are as defined above;
or a salt thereof.

3. The compound according to claim 1 or 2, wherein
n is 0 or 1,
J is selected from the group consisting of 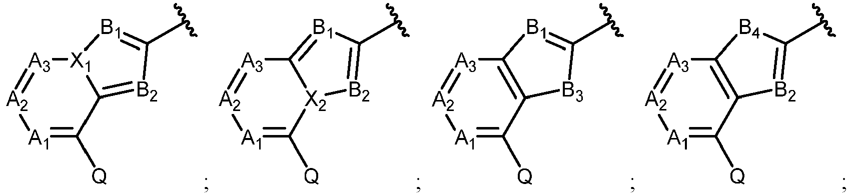 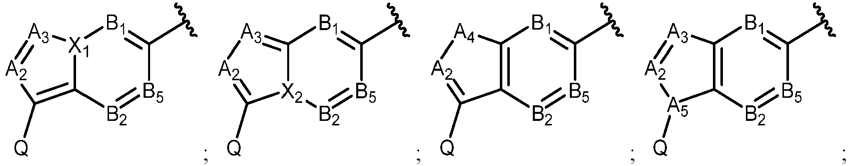 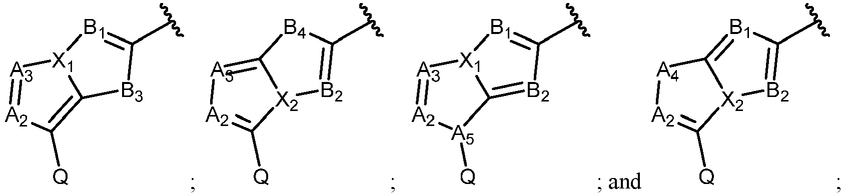
A₁ is selected from the group consisting of N and CR_{A1}
A₂ is selected from the group consisting of N and CR_{A2};
A₃ is selected from the group consisting of N and CR_{A3};
A₄ is selected from the group consisting of O, S, and NR_{A4};
As is selected from the group consisting of N;
B₁ is selected from the group consisting of N and CR_{B1};
B₂ is selected from the group consisting of N and CR_{B2};
B₃ is selected from the group consisting of O, S, and NR_{B3};
B₄ is selected from the group consisting of O, S, and NR_{B4};
B₅ is selected from the group consisting of N and CR_{B5};
X₁ is selected from the group consisting of N;
X₂ is selected from the group consisting of N;
G is selected from the group consisting of 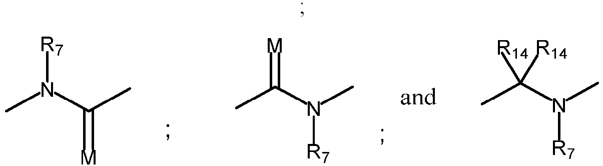
M is selected from the group consisting of N-R₁₃, O, and S;
Y₁ is selected from the group consisting of CR₈R₉, O, S, and NR₁₀;
Y₂ is selected from the group consisting of CR₈R₉, O, S, and NR₁₀;
wherein at least one of the groups Y₁ or Y₂ is CR₈R₉;
Z₁ is selected from the group consisting of N, O, S, and CR₁₁;
Z₂ is selected from the group consisting of nil, N, and CR₁₁;
Z₃ is selected from the group consisting of nil, N and CR₁₁;
Z₄ is selected from the group consisting of N, O, S, and CR₁₁;
wherein no more than 2 of Z₁, Z₂, Z₃, and Z₄ are N and wherein only one of Z₁ and Z₄ is O or S, Z₂ is nil only when Z₁ is O or S, and Z₃ is nil only when Z₄ is O or S;
R_{A1}, R_{A2}, R_{A3}, R_{B2}, and R_{B5} are each independently selected from the group consisting of hydrogen, halogen, and C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
R_{A4} and R_{B3} are independently selected from the group consisting of hydrogen, C₁-C₄ alkyl, wherein each C₁-C₄ alkyl in R_{A4} and R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B1} is selected from the group consisting of C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is hydrogen;
R₈ is hydrogen;
R₉ is hydrogen;
R₁₀ is selected from the group consisting of hydrogen and C₁-C₄ alkyl;
R₁₁ is hydrogen;
Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, - NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R₁₃ is selected from the group consisting of hydroxy, C₁-C₄ alkoxy, and -NH₂;
wherein, when J is 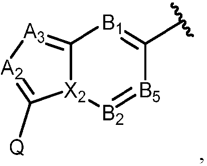 A₂ is N, and A₃, B₁, B₂, B₅, X₂, and Q are as defined above;
or a salt thereof.

4. The compound according to any one of claims 1 to 3, wherein
n is 1,
J is selected from the group consisting of 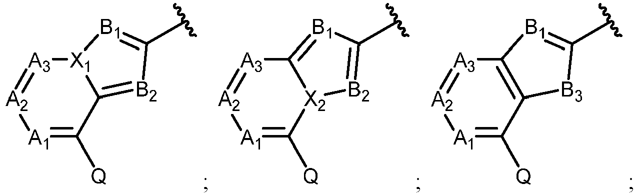 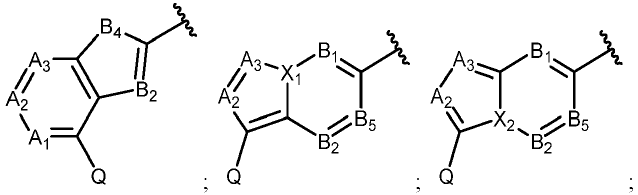 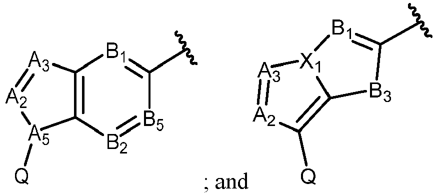
A₁ is selected from the group consisting of N and CR_{A1}
A₂ is selected from the group consisting of N and CR_{A2};
A₃ is selected from the group consisting of N and CR_{A3};
A₅ is selected from the group consisting of N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is selected from the group consisting of N;
X₂ is selected from the group consisting of N;
G is selected from the group consisting of 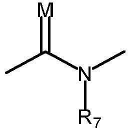
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
R_{B3} is selected from the group consisting of hydrogen, and C₁-C₄ alkyl, wherein each C₁-C₄ alkyl in R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B1} is selected from the group consisting of C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is hydrogen;
R₈ is hydrogen;
R₉ is hydrogen;
R₁₁ is hydrogen;
Q is 6- or 10 membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), - N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
wherein, when J is 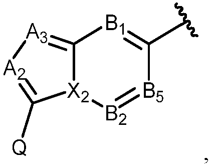 A₂ is N, and A₃, B₁, B₂, B₅, X₂, and Q are as defined above;
or a salt thereof.

5. The compound according to any of claims 1 to 4, wherein
n is 1,
J is selected from the group consisting of 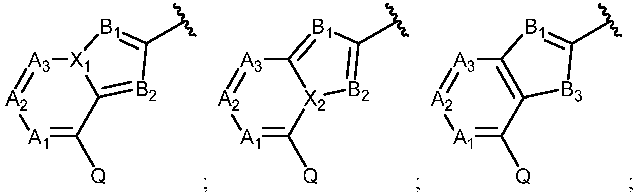 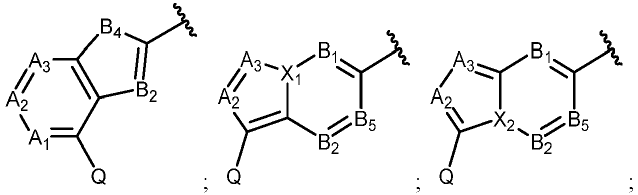 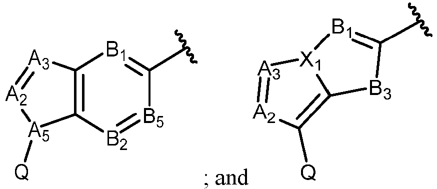
A₁ is selected from the group consisting of N and CR_{A1}
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is N;
X₂ is N;
G is 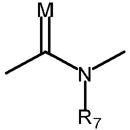
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, and C₁-C₄ alkyl, wherein each C₁-C₄ alkyl in R_{A1}, R_{A2}, R_{A3}, R_{B2}, and R_{B5} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R_{B3} is selected from the group consisting of hydrogen, and C₁-C₄alkyl, wherein each C₁-C₄ alkyl in R_{B3} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), - C(O)N(C₁-C₄ alkyl)₂, and C₁-C₄ alkoxy;
R_{B1} is selected from the group consisting of
C₁-C₄ alkyl, -N(C₁-C₄ alkyl)₂, wherein each C₁-C₄ alkyl in R_{B1} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents independently selected from the group consisting of halogen, cyano, nitro, hydroxy, oxo, C₁-C₄ alkyl, C₃-C₆ cycloalkyl, C₁-C₄ halogenoalkyl, C₁-C₄ alkoxy, - NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, - S(O)C₁-C₄-halogenoalkyl and -SO₂C₁-C₄ halogenoalkyl;
R_{B4} is C₁-C₄ alkyl, wherein each C₁-C₄ alkyl in R_{B4} may be optionally substituted with 1, 2 or 3 substituents independently selected from the group consisting of halogen, hydroxy, -NH₂, -NH(C₁-C₄ alkyl), -N(C₁-C₄ alkyl)₂, cyano, carboxy, carbamoyl, C₁-C₄ alkoxycarbonyl, -C(O)NH(C₁-C₄ alkyl), -C(O)N(C₁-C₄ alkyl)₂, and C₁-C₄ alkoxy;
R₇ is hydrogen;
R₈ is hydrogen;
R₉ is hydrogen;
R₁₁ is hydrogen;
Q is phenyl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), -SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
wherein, when J is 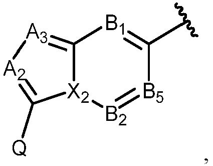 A₂ is N, and A₃, B₁, B₂, B₅, X₂, and Q are as defined above;
or a salt thereof.

6. The compound according to any one of claims 1 to 5, wherein
n is 1,
J is selected from the group consisting of 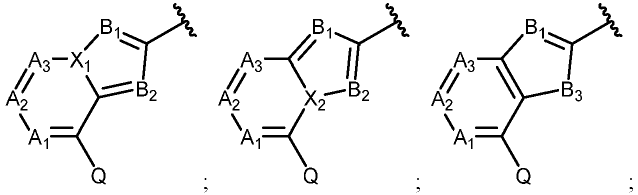 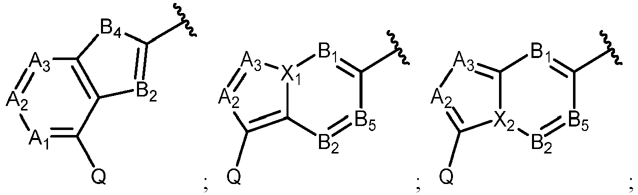 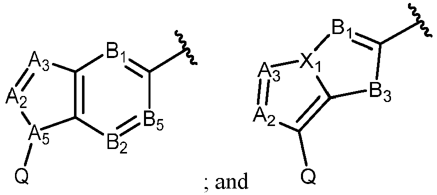
A₁ is selected from the group consisting of N and CR_{A1}
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is N;
X₂ is N;
G is 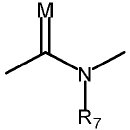
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, halogen, C₁-C₄ alkyl;
R_{B3} is selected from the group consisting of hydrogen, and C₁-C₄ alkyl;
R_{B1} is selected from the group consisting of C₁-C₄ alkyl, -N(C₁-C₄ alkyl)₂, wherein each C₁-C₄-alkyl in R_{B1} may be optionally substituted with 1, 2 or 3 substituents of hydroxy; and
a 4- to 7-membered heterocycloalkyl containing 1, 2, or 3 heteroatoms independently selected from N or O, wherein each heterocycloalkyl in R_{B1} is optionally substituted with 1, 2 or 3 substituents of halogen;
R_{B4} is C₁-C₄alkyl;
R₇ is hydrogen;
R₈ is hydrogen;
R₉ is hydrogen;
R₁₁ is hydrogen;
Q is phenyl having 1 to 5 halogen atoms;
wherein, when J is 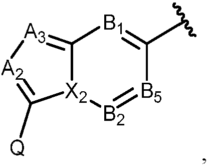 A₂ is N, and A₃, B₁, B₂, B₅, X₂, and Q are as defined above;
or a salt thereof.

7. The compound according to any one of claims 1 to 6, wherein
n is 1,
J is selected from the group consisting of 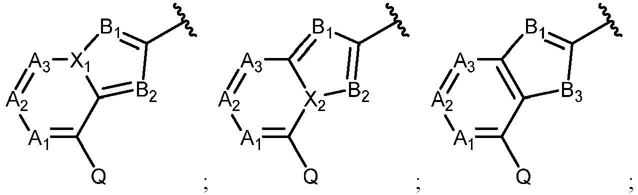 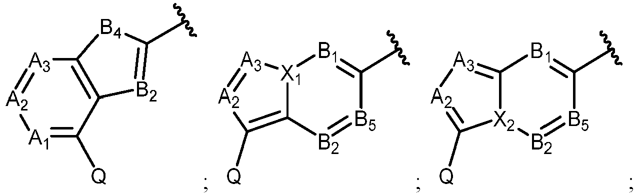 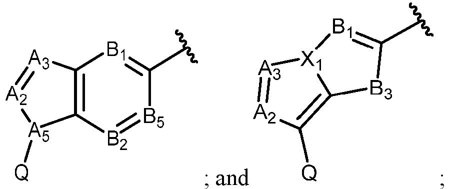
A₁ is selected from the group consisting of N and CR_{A1}
A₂ is selected from the group consisting of N and CR_{A2};
A₃ is selected from the group consisting of N and CR_{A3};
A₅ is N;
B₁ is CR_{B1};
B₂ is selected from the group consisting of N and CR_{B2};
B₃ is selected from the group consisting of O, S, and NR_{B3};
B₄ is NR_{B4};
B₅ is selected from the group consisting of N and CR_{B5};
X₁ is selected from the group consisting of N;
X₂ is selected from the group consisting of N;
G is selected from the group consisting of 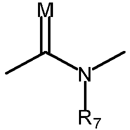
M is O;
Y₁ is CR₈R₉;
Y₂ is O;
Z₁ is CR₁₁;
Z₂ is CR₁₁;
Z₃ is CR₁₁;
Z₄ is CR₁₁;
R_{A1}, R_{A2}, R_{A3}, R_{B2}, and R_{B5} are each independently selected from the group consisting of hydrogen, fluoro, and methyl;
R_{B3} are independently selected from the group consisting of hydrogen, and methyl;
R_{B1} is independently selected from the group consisting of 4-morpholino, isopropyl, 2-hydroxyisopropyl, 3-fluoroazetidinyl, and NMe₂;
R_{B4} is isopropyl;
R₇ is hydrogen;
R₈ is hydrogen;
R₉ is hydrogen;
R₁₁ is hydrogen;
Q is selected from the group consisting of 2,3,5-trifluorophenyl and 2,6-difluorophenyl;
wherein, when J is 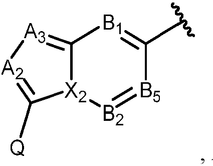 A₂ is N, and A₃, B₁, B₂, B₅, X₂, and Q are as defined above;
or a salt thereof.

8. The compound according to any one of claims 1 to 7, wherein
J is selected from the group consisting of 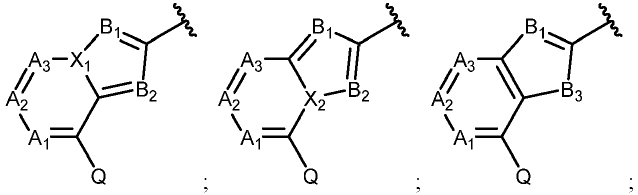 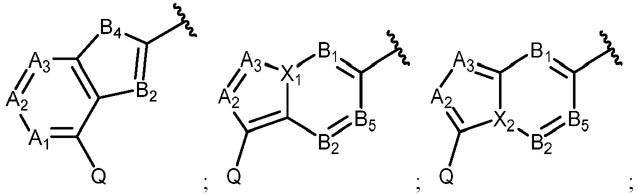 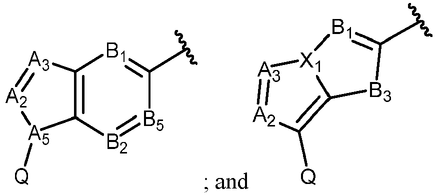
wherein, when J is 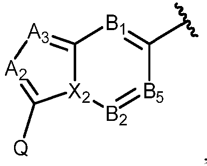 A₂ is N, and A₃, B₁, B₂, B₅, X₂, and Q are as defined in any one of claims 1 to 7;
or a salt thereof.

9. The compound according to any one of claims 1 to 8, wherein
G is 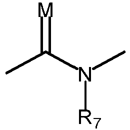 ; and
M is O;
or a salt thereof, more preferably
M is O; and
R₇ is hydrogen.

10. The compound according to any one of claims 1 to 9, wherein
Q is a 6- or 10 membered aryl optionally substituted with 1, 2 or 3 substituents independently selected from the group consisting of halogen, cyano, nitro, hydroxy, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl;
or a salt thereof.

11. The compound according to any one of claims 1 to 10, wherein
Q is a 6-membered aryl optionally substituted with 1, 2, 3, 4, or 5 substituents independently selected from the group consisting of halogen, cyano, nitro, hydroxyl, C₁-C₄ alkyl, C₁-C₄ halogenoalkyl, C₁-C₄ alkoxy, C₃-C₆ cycloalkyl, -C(O)R₁₇, -NH₂, -NH(C₁-C₄ alkyl), -N(C₁-C₄ alkyl)₂, -NH(C₃-C₆ cycloalkyl), -N(C₁-C₄ alkyl)(C₃-C₆-cycloalkyl), -NHSO₂(C₁-C₄ alkyl), - SC₁-C₄ alkyl, -S(O)C₁-C₄ alkyl, -SO₂C₁-C₄ alkyl, -S(O)C₁-C₄-halogenoalkyl and -SO₂C₁-C₄ halogenoalkyl, wherein the 6- or 10 membered aryl is optionally fused with a 4- to 7-membered heterocycloalkyl having 1 or 2 heteroatoms selected from the group O, S, and N and wherein the carbons of the heterocycloalkyl are optionally substituted with 1, 2 or 3 substituents independently selected from the group halogen, cyano, nitro, hydroxyl, oxo, C₁-C₄ alkyl, C₃-C₆ cycloalkyl, C₁-C₄ halogenoalkyl, C₁-C₄ alkoxy, -NH₂, -NH(C₁-C₄ alkyl), and -N(C₁-C₄ alkyl)₂ and any N in the heterocycloalkyl is, valency permitting, substituted with a substituent selected from the group consisting of hydrogen, C₁-C₄ alkyl, and C₃-C₆ cycloalkyl.

12. The compound according to any one of claims 1 to 11, or a salt thereof, wherein Q is selected from: 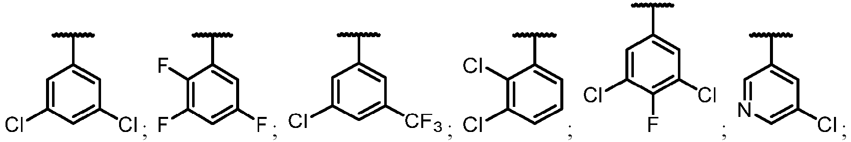 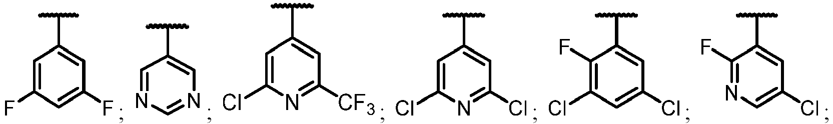 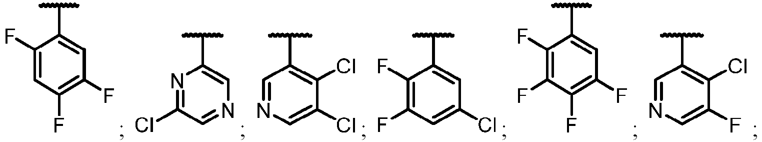 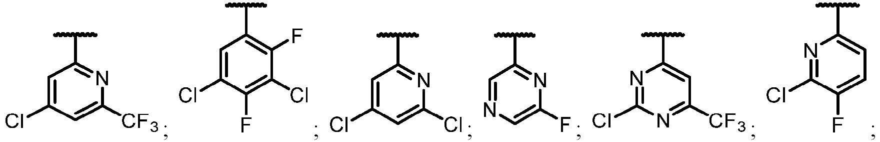 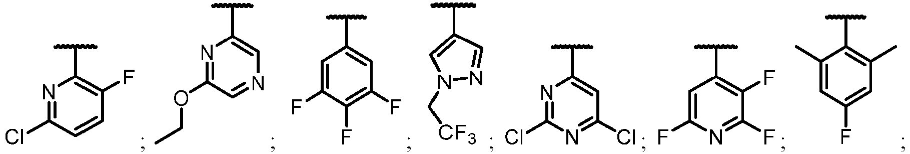 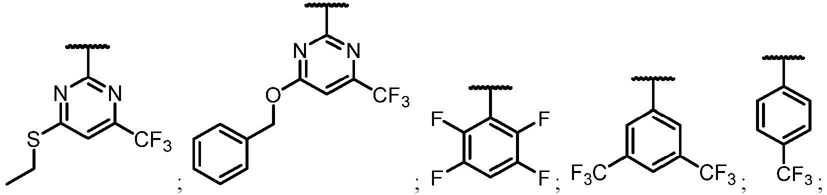 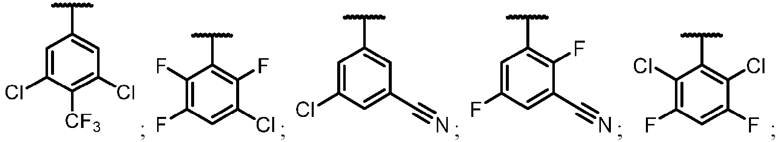 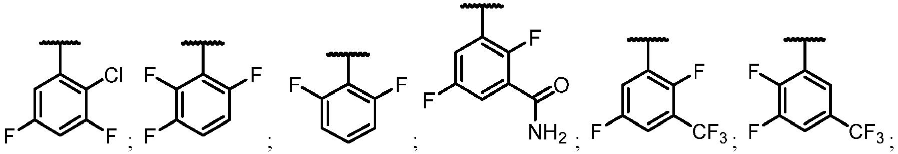 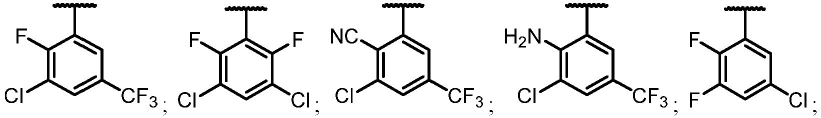 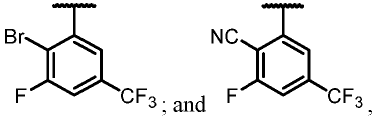 more preferably Q is

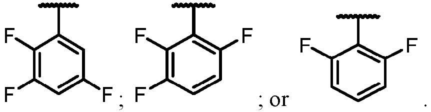

13. The compound according to any one of claims 1 to 12, wherein
n is 1, Y₁ is CR₈R₉, Y₂ is O, Z₁ is CR₁₁, Z₂ is CR₁₁, Z₃ is CR₁₁, Z₄ is CR₁₁,
or a salt thereof.

14. The compound according to any one of claims 1 to 13, wherein R_{B1} or R_{B4}, when present, is selected from the group consisting of C₁-C₄ alkyl, hydroxy-substituted C₁-C₄ alkyl, C₃-C₆ cycloalkyl, -N(C₁-C₄ alkyl)₂, and 4- to 7-membered heterocycloalkyl; or a salt thereof.

15. The compound according to any one of claims 1 to 14, or a salt thereof, wherein R_{B1} or R_{B4}, when present, is selected from:

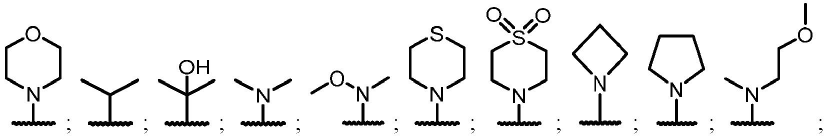

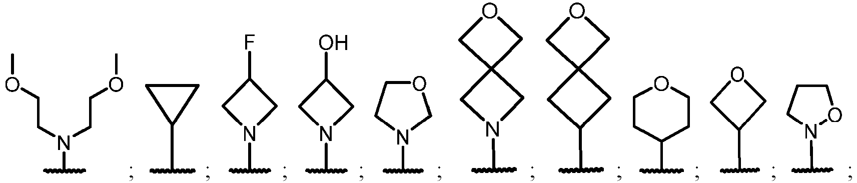

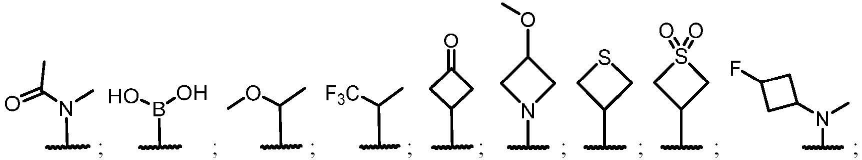

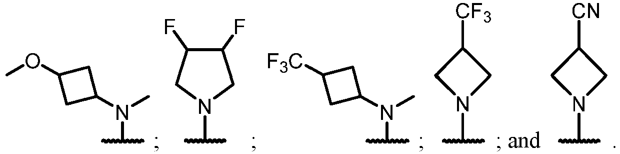

16. The compound of any one of claims 1 to 15, wherein the compound is selected from the group consisting of: 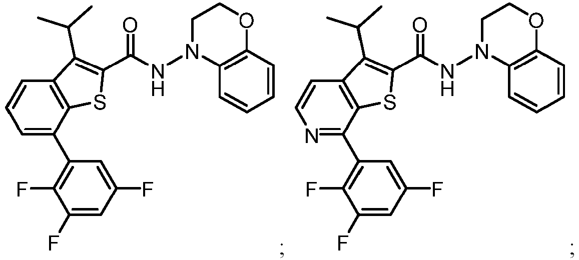 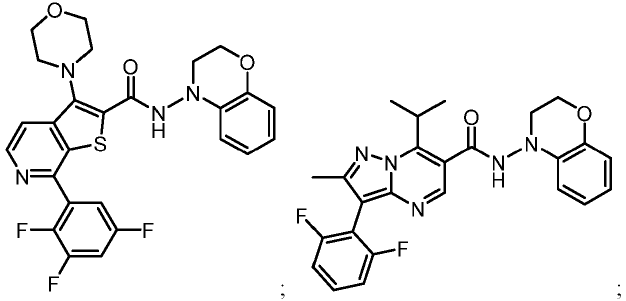 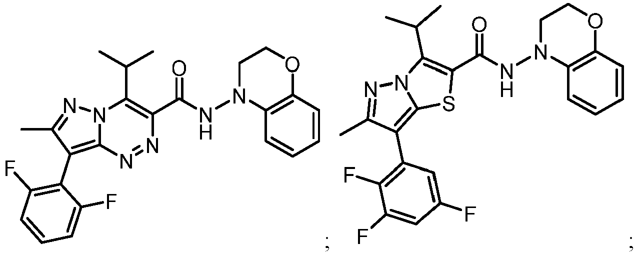 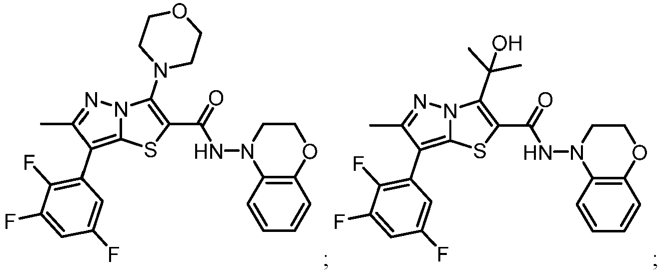 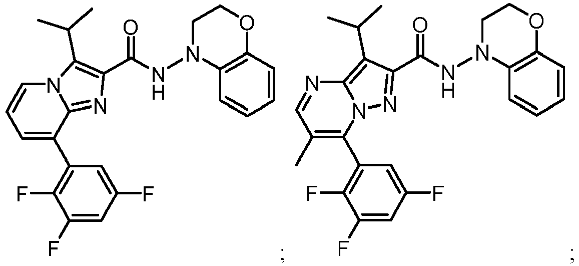 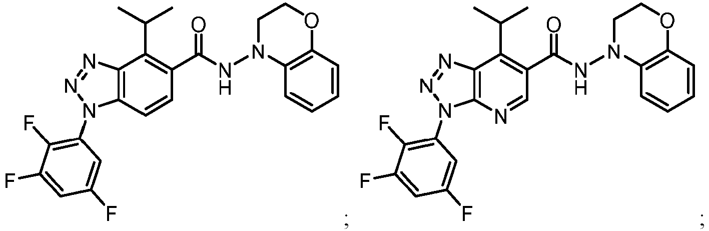 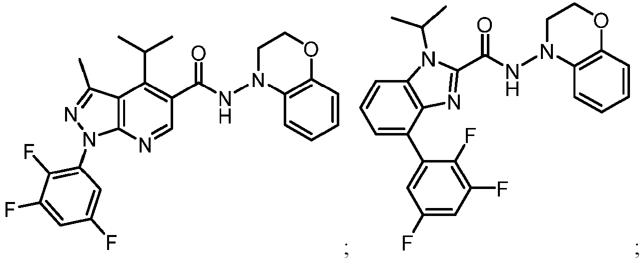 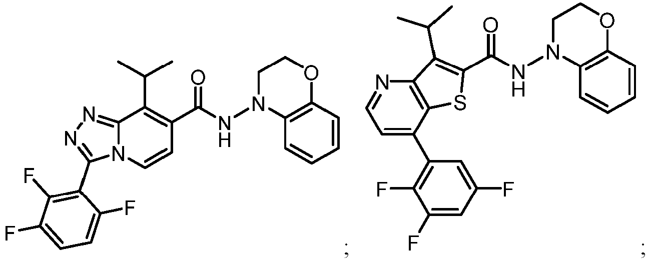 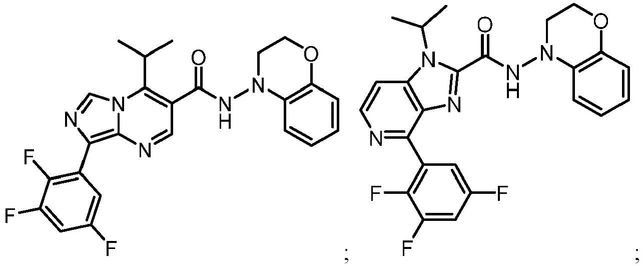 and 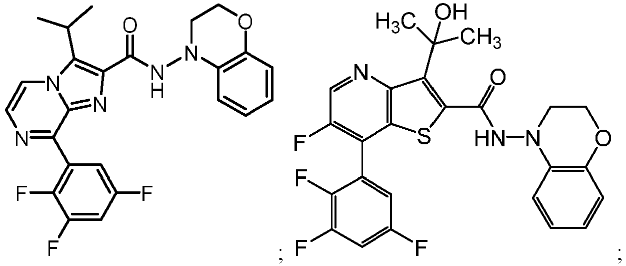 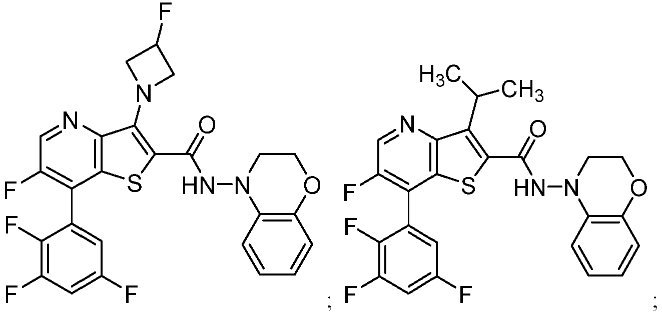 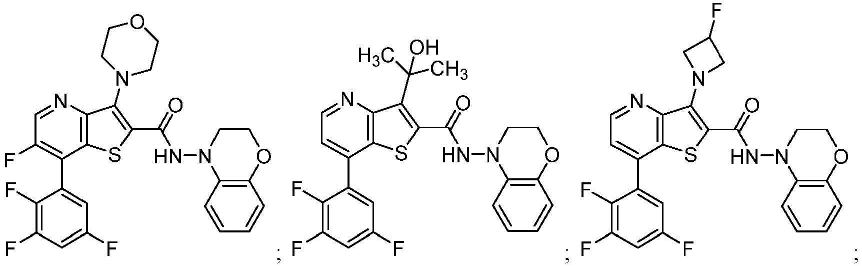 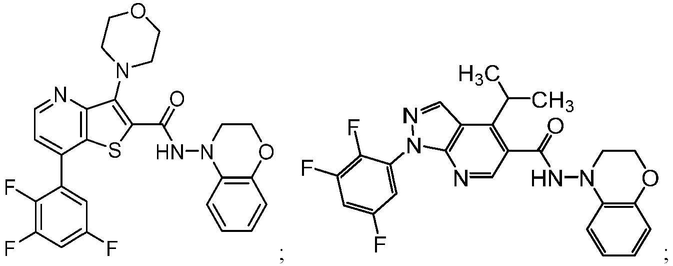 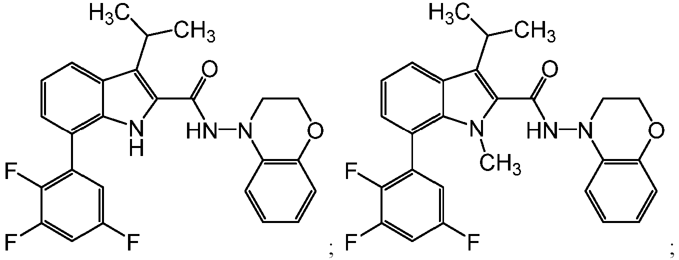 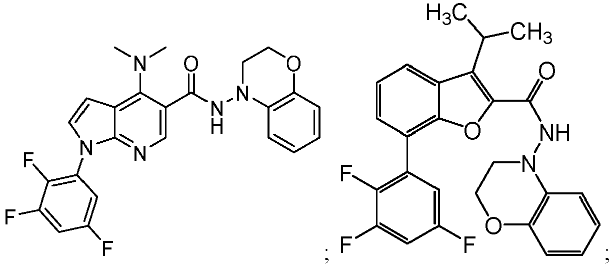 or a salt of any of the foregoing compounds.

17. A pharmaceutical composition comprising a compound of formula (I) according to any one of claims 1 to 16, or a salt thereof, and at least one acceptable carrier.

18. A compound of formula (I) according to any one of claims 1 to 16 or a pharmaceutical composition according to claim 17 for use in the control, treatment and/or prevention of a disease, wherein the disease is preferably an infection caused by endoparasites, more preferably a helminthic infection, even more preferably a heartworm infection.

## Patentansprüche

1. Verbindung der Formel (I) 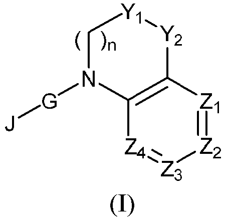 ausgewählt ist;
wobei
n 0 oder 1 ist;
J aus der Gruppe bestehend aus 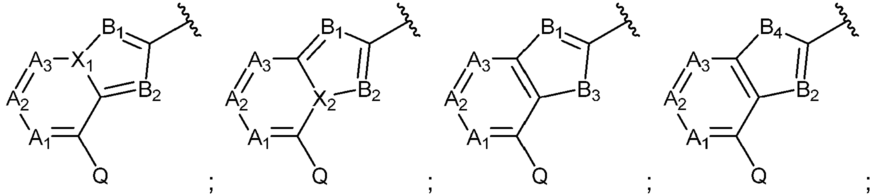 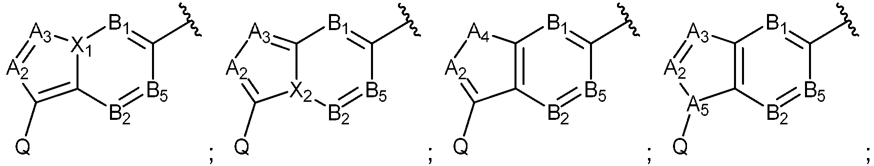 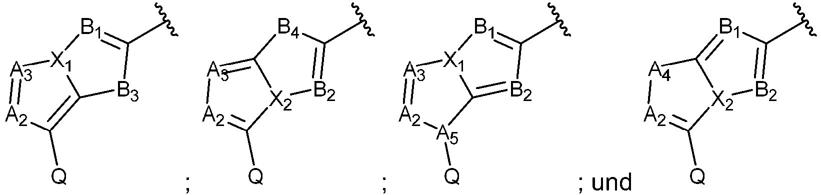 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₄ aus der Gruppe bestehend aus O, S und NR_{A4} ausgewählt ist;
A₅ aus der Gruppe bestehend aus N ausgewählt ist;
B₁ aus der Gruppe bestehend aus N und CR_{B1} ausgewählt ist;
B₂ aus der Gruppe bestehend aus N und CR_{B2} ausgewählt ist;
B₃ aus der Gruppe bestehend aus O, S und NR_{B3} ausgewählt ist;
B₄ aus der Gruppe bestehend aus O, S und NR_{B4} ausgewählt ist;
B₅ aus der Gruppe bestehend aus N und CR_{B5} ausgewählt ist;
X₁ aus der Gruppe bestehend aus N ausgewählt ist;
X₂ aus der Gruppe bestehend aus N ausgewählt ist;
G aus der Gruppe bestehend aus 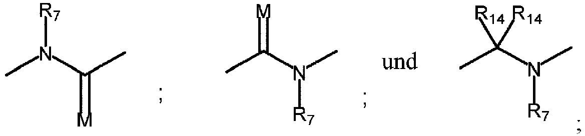 ausgewählt ist;
M aus der Gruppe bestehend aus N-R₁₃, O und S ausgewählt ist;
Y₁ aus der Gruppe bestehend aus CR₈R₉, O, S und NR₁₀ ausgewählt ist;
Y₂ aus der Gruppe bestehend aus CR₈R₉, O, S und NR₁₀ ausgewählt ist;
wobei mindestens eine der Gruppen Y₁ oder Y₂ CR₈R₉ ist;
Z₁ aus der Gruppe bestehend aus N, O, S und CR₁₁ ausgewählt ist;
Z₂ aus der Gruppe bestehend aus einer Bindung, N und CR₁₁ ausgewählt ist;
Z₃ aus der Gruppe bestehend aus einer Bindung, N und CR₁₁ ausgewählt ist;
Z₄ aus der Gruppe bestehend aus N, O, S und CR₁₁ ausgewählt ist;
wobei nicht mehr als 2 von Z₁, Z₂, Z₃ und Z₄ N sind und wobei nur eines von Z₁ und Z₄ O oder S ist, Z₂ nur dann eine Bindung ist, wenn Z₁ O oder S ist, und Z₃ nur dann eine Bindung ist, wenn Z₄ O oder S ist;
R_{A1}, R_{A2}, R_{A3}, R_{B2}, und R_{B5} jeweils unabhängig voneinander aus der Gruppe bestehend aus Wasserstoff, Halogen, Hydroxyl, -SH, -SC₁-C₄-Alkyl, - S(O)(C₁-C₄-Alkyl), -S(O)₂(C₁-C₄-Alkyl), Cyano, C₁-C₄-Alkyl, C₁-C₄-Halogenalkyl, C₁-C₄-Alkoxy, -B(OR₁₅)(OR₁₆) ausgewählt sind, wobei R₁₅, jedes Mal, wenn es genommen wird, aus der Gruppe bestehend aus Wasserstoff, C₁-C₄-Alkyl, und C₃-C₆-Cycloalkyl ausgewählt ist, R₁₆, jedes Mal, wenn es genommen wird, aus der Gruppe bestehend aus Wasserstoff, C₁-C₄-Alkyl, und C₃-C₆-Cycloalkyl ausgewählt ist, oder R₁₅ und R₁₆ gemeinsam mit den Sauerstoffatomen, an die sie gebunden sind, einen 5- bis 7-gliedrigen Ring bilden, der gegebenenfalls mit 1 bis 4 C₁-C₄-Alkyl substituiert ist; -NH₂, -NH(C₁-C₄-Alkyl) und -N(C₁-C₄-Alkyl)₂;
R_{A4} und R_{B3} sind unabhängig voneinander aus der Gruppe bestehend aus Wasserstoff, C₁-C₄-Alkyl und C₁-C₄-Halogenoalkyl ausgewählt;
R_{B1} aus der Gruppe bestehend aus Halogen, Cyano, -CHO, Hydroxyl, C₁-C₄-Alkyl, C₂-C₄-Alkenyl, C₂-C₄-Alkinyl, C₃-C₆-Cycloalkyl, C₁-C₄-Halogenalkyl, C₁-C₄-alkoxysubstituiertem C₁-C₄-Alkyl, gegebenenfalls mit 1 bis 5 Halogenatomen substituiertem Benzyl, C₁-C₄-Alkoxy, -NH₂, -NH(C₁-C₄-Alkyl), -N(C₁-C₄-Alkyl)₂, - NH(C₃-C₆-Cycloalkyl), -N(C₁-C₄-Alkyl)(C₃-C₆-Cycloalkyl), -N(C₁-C₄-Alkyl)(4- bis 7-gliedrigem Heterocycloalkyl), -NH(4- bis 7-gliedrigem Heterocycloalkyl), -N(C₁-C₄-Alkyl)(C₁-C₄-Alkoxy), -C(O)NH(C₁-C₄-Alkyl), -C(O)N(C₁-C₄-Alkyl)₂, -C(O)N(C₁-C₄-Alkyl)(4- bis 7-gliedrigem Heterocycloalkyl), -NHSO₂(C₁-C₄-Alkyl), -SC₁-C₄-Alkyl, - S(O)C₁-C₄-Alkyl, -SO₂C₁-C₄-Alkyl, -B(OR₁₅)(OR₁₆) ausgewählt ist, wobei R₁₅, jedes Mal, wenn es genommen wird, aus der Gruppe bestehend aus Wasserstoff, C₁-C₄-Alkyl, und C₃-C₆-Cycloalkyl ausgewählt ist, R₁₆, jedes Mal, wenn es genommen wird, aus der Gruppe bestehend aus Wasserstoff, C₁-C₄-Alkyl, und C₃-C₆-Cycloalkyl ausgewählt ist, oder R₁₅ und R₁₆ gemeinsam mit den Sauerstoffatomen, an die sie gebunden sind, einen 5- bis 7-gliedrigen Ring bilden, der gegebenenfalls mit 1 bis 4 C₁-C₄-Alkyl substituiert ist; 6- oder 10-gliedriges Aryl; ein monocyclischer Heterocyclus, ausgewählt aus der Gruppe von 4- bis 7-gliedrigem Heterocycloalkyl, 5-gliedrigem Heteroaryl mit mindestens einem Stickstoffatom, über das der 5-gliedrige Heteroarylring mit dem Rest des Moleküls verbunden ist, und 6-gliedrigem Heteroaryl mit mindestens einem Stickstoffatom; jeder der Aryl-, Heterocycloalkyl- und Heteroarylringe in R_{B1} gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind; wobei das C₃-C₆-Cycloalkyl und die Heterocycloalkylringe in R_{B1} gegebenenfalls mit einer Spirogruppe substituiert sind, wobei die Spirogruppe ein 3- bis 6-gliedriges Cycloalkyl oder 4- bis 6-gliedriges Heterocycloalkyl ist, das 1, 2 oder 3 Heteroatome enthält, die unabhängig voneinander aus N, S oder O ausgewählt sind, wobei die Spirogruppe gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), - NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind; und wobei jedes C₁-C₄-Alkyl, C₃-C₆-Cycloalkyl und C₁-C₄-Alkoxy in R_{B1} gegebenenfalls mit 1, 2 oder 3 Substituenten substituiert sein kann, die unabhängig voneinander aus der Gruppe bestehend aus Halogen, Hydroxy, Oxo, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R_{B4} aus der Gruppe bestehend aus -CHO, Hydroxyl, C₁-C₄-Alkyl, C₂-C₄-Alkenyl, C₂-C₄-Alkinyl, C₃-C₆-Cycloalkyl, C₁-C₄-Halogenalkyl, C₁-C₄-alkoxysubstituiertem C₁-C₄-Alkyl, gegebenenfalls mit 1 bis 5 Halogenatomen substituiertem Benzyl, C₁-C₄-Alkoxy, -NH₂, -NH(C₁-C₄-Alkyl), -N(C₁-C₄-Alkyl)₂, - NH(C₃-C₆-Cycloalkyl), -N(C₁-C₄-Alkyl)(C₃-C₆-Cycloalkyl), -N(C₁-C₄-Alkyl)(4- bis 7-gliedrigem Heterocycloalkyl), -NH(4- bis 7-gliedrigem Heterocycloalkyl), -N(C₁-C₄-Alkyl)(C₁-C₄-Alkoxy), -C(O)NH(C₁-C₄-Alkyl), -C(O)N(C₁-C₄-Alkyl)₂, -C(O)N(C₁-C₄-Alkyl)(4- bis 7-gliedrigem Heterocycloalkyl), -NHSO₂(C₁-C₄-Alkyl), -SC₁-C₄-Alkyl, - S(O)C₁-C₄-Alkyl, -SO₂C₁-C₄-Alkyl, -B(OR₁₅)(OR₁₆) ausgewählt ist, wobei R₁₅, jedes Mal, wenn es genommen wird, aus der Gruppe bestehend aus Wasserstoff, C₁-C₄-Alkyl, und C₃-C₆-Cycloalkyl ausgewählt ist, R₁₆, jedes Mal, wenn es genommen wird, aus der Gruppe bestehend aus Wasserstoff, C₁-C₄-Alkyl, und C₃-C₆-Cycloalkyl ausgewählt ist, oder R₁₅ und R₁₆ gemeinsam mit den Sauerstoffatomen, an die sie gebunden sind, einen 5- bis 7-gliedrigen Ring bilden, der gegebenenfalls mit 1 bis 4 C₁-C₄-Alkyl substituiert ist; 6- oder 10-gliedriges Aryl; ein monocyclischer Heterocyclus, ausgewählt aus der Gruppe von 4- bis 7-gliedrigem Heterocycloalkyl, 5-gliedrigem Heteroaryl und 6-gliedrigem Heteroaryl; jeder der Aryl-, Heterocycloalkyl- und Heteroarylringe in Rₑ₄ gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind; wobei das C₃-C₆-Cycloalkyl und die Heterocycloalkylringe in Rₑ₄ gegebenenfalls mit einer Spirogruppe substituiert sind, wobei die Spirogruppe ein 3- bis 6-gliedriges Cycloalkyl oder 4- bis 6-gliedriges Heterocycloalkyl ist, das 1, 2 oder 3 Heteroatome enthält, die unabhängig voneinander aus N, S oder O ausgewählt sind, wobei die Spirogruppe gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), - NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind; und wobei jedes C₁-C₄-Alkyl, C₃-C₆-Cycloalkyl und C₁-C₄-Alkoxy in R_{B4} gegebenenfalls mit 1, 2 oder 3 Substituenten substituiert sein kann, die unabhängig voneinander aus der Gruppe bestehend aus Halogen, Hydroxy, Oxo, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R₇ aus der Gruppe bestehend aus Wasserstoff, C₁-C₄-Alkyl und C₃-C₆-Cycloalkyl ausgewählt ist, gegebenenfalls substituiert mit 1 bis 5 Halogenatomen, -C(H)O, C₂-C₄-Alkenyl, C₂-C₄-Alkinyl, C₁-C₄-Halogenalkyl und C₁-C₄-Alkoxy;
R₃ jedes Mal, wenn es ausgewählt wird, unabhängig voneinander aus der Gruppe bestehend aus Wasserstoff, Fluor und (C₁-C₄)-Alkyl ausgewählt ist;
R₉ jedes Mal, wenn es ausgewählt wird, unabhängig voneinander aus der Gruppe bestehend aus Wasserstoff, Fluor und (C₁-C₄)-Alkyl ausgewählt ist;
R₁₀ aus der Gruppe bestehend aus Wasserstoff und C₁-C₄-Alkyl ausgewählt ist;
R₁₁ jedes Mal, wenn es ausgewählt wird, unabhängig voneinander aus der Gruppe bestehend aus Wasserstoff, Halogen, Hydroxyl, Cyano, C₁-C₄-Alkyl, C₁-C₄-Halogenalkyl, C₁-C₄-Alkoxy, C₃-C₆-Cycloalkyl, -NH₂, -NH(C₁-C₄-Alkyl) und - N(C₁-C₄-Alkyl)₂ ausgewählt ist; und
Q aus der Gruppe ausgewählt ist, bestehend aus
(i) 6- oder 10-gliedrigem Aryl, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -C(O)R₁₇, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind, wobei das 6- oder 10-gliedrige Aryl gegebenenfalls mit einem 4- bis 7-gliedrigen Heterocycloalkyl mit 1 oder 2 Heteroatomen, ausgewählt aus der Gruppe O, S und N, kondensiert ist, und wobei die Kohlenstoffe des Heterocycloalkyls gegebenenfalls mit 1, 2 oder 3 Substituenten substituiert sind, die unabhängig voneinander aus der Gruppe Halogen, Cyano, Nitro, Hydroxyl, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), und -N(C₁-C₄-Alkyl)₂ ausgewählt sind, und jedes N im Heterocycloalkyl, sofern es die Valenz erlaubt, mit einem Substituenten substituiert ist, der aus der Gruppe bestehend aus Wasserstoff, C₁-C₄-Alkyl, und C₃-C₆-Cycloalkyl ausgewählt ist;
(ii) 5- bis 10-gliedrigem Heteroaryl mit 1, 2 oder 3 Heteroatomen, die unabhängig voneinander aus der Gruppe O, S und N ausgewählt sind, und wobei die Kohlenstoffe des 5- bis 10-gliedrigen Heteroaryls gegebenenfalls mit 1, 2, 3, 4 oder 5 Substituenten substituiert sind, die unabhängig voneinander aus der Gruppe bestehend aus Halogen, Cyano, Nitro, Hydroxyl, C₁-C₄-Alkyl, C₃-C₆-Cycloalkyl, C₁-C₄-Halogenalkyl, C₁-C₄-Alkoxy, Benzyloxy, -C(O)R₁₇, -NH₂, -NH(C₁-C₄-Alkyl), -N(C₁-C₄-Alkyl)₂, -SC₁-C₄-Alkyl, -S(O)C₁-C₄-Alkyl, -SO₂C₁-C₄-Alkyl, - S(O)C₁-C₄-Halogenalkyl und -SO₂C₁-C₄-Halogenalkyl ausgewählt sind, und jedes N im Heteroaryl, sofern es die Valenz erlaubt, gegebenenfalls mit einem Substituenten substituiert ist, der aus der Gruppe bestehend aus Wasserstoff, C₁-C₄-Alkyl, und C₃-C₆-Cycloalkyl ausgewählt ist;
(iii) 4- bis 7-gliedrigem Heterocycloalkyl mit 1, 2 oder 3 Heteroatomen, die unabhängig voneinander aus der Gruppe O, S, N ausgewählt sind, wobei das Heterocycloalkyl gegebenenfalls benzokondensiert ist, wobei die Kohlenstoffe des 4- bis 7-gliedrigen Heterocycloalkyls oder gegebenenfalls des benzokondensierten 4- bis 7-gliedrigen Heterocycloalkyls gegebenenfalls mit 1, 2, 3 oder 4 Substituenten substituiert sind, die unabhängig voneinander aus der Gruppe bestehend aus Halogen, Cyano, Nitro, Hydroxyl, Oxo, C₁-C₄-Alkyl, C₃-C₆-Cycloalkyl, C₁-C₄-Halogenalkyl, C₁-C₄-Alkoxy, -C(O)R₁₇, -NH₂, -NH(C₁-C₄-Alkyl), und -N(C₁-C₄-Alkyl)₂ ausgewählt sind, und jedes N im Heterocycloalkyl gegebenenfalls mit einem Substituenten substituiert ist, der aus der Gruppe bestehend aus Wasserstoff, C₁-C₄-Alkyl, und C₃-C₆-Cycloalkyl ausgewählt ist;
(iv) 6- oder 10-gliedrigem Aryloxy, das gegebenenfalls mit 1, 2 oder 3 Substituenten substituiert ist, die unabhängig voneinander aus der Gruppe bestehend aus Halogen, Cyano, Nitro, Hydroxyl, C₁-C₄-Alkyl, C₃-C₆-Cycloalkyl, C₁-C₄-Halogenalkyl, C₁-C₄-Alkoxy, -C(O)R₁₇, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), - SC₁-C₄-Alkyl, -S(O)C₁-C₄-Alkyl, -SO₂C₁-C₄-Alkyl, -S(O)C₁-C₄-Halogenalkyl und - SO₂C₁-C₄-Halogenalkyl ausgewählt sind;
(v) 6- oder 10-gliedrigem Arylthio-oxy, das gegebenenfalls mit 1, 2 oder 3 Substituenten substituiert ist, die unabhängig voneinander aus der Gruppe, bestehend aus Halogen, Cyano, Nitro, Hydroxyl, C₁-C₄-Alkyl, C₃-C₆-Cycloalkyl, C₁-C₄-Halogenalkyl, C₁-C₄-Alkoxy, -C(O)R₁₇, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), - SC₁-C₄-Alkyl, -S(O)C₁-C₄-Alkyl, -SO₂C₁-C₄-Alkyl, -S(O)C₁-C₄-Halogenalkyl und - SO₂C₁-C₄-Halogenalkyl ausgewählt sind; und
(vi) 5- bis 10-gliedrigem Heteroaryloxy, das gegebenenfalls mit 1, 2 oder 3 Substituenten substituiert ist, die unabhängig voneinander aus der Gruppe bestehend aus Halogen, Cyano, Nitro, Hydroxyl, Oxo, C₁-C₄-Alkyl, C₃-C₆-Cycloalkyl, C₁-C₄-Halogenalkyl, C₁-C₄-Alkoxy, -C(O)R₁₇, -NH₂, -NH(C₁-C₄-Alkyl), - N(C₁-C₄-Alkyl)₂, -NH(C₃-C₆-Cycloalkyl), -N(C₁-C₄-Alkyl)(C₃-C₆-Cycloalkyl), - NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
R₁₃ aus der Gruppe bestehend aus Hydroxy, C₁-C₄-Alkoxy und -NH₂ ausgewählt ist;
R₁₄ jedes Mal, wenn es ausgewählt wird, unabhängig voneinander aus der Gruppe bestehend aus Wasserstoff, Halogen, Cyano, Nitro, Hydroxyl, C₁-C₄-Alkyl, C₃-C₆-Cycloalkyl, C₁-C₄-Halogenalkyl, C₁-C₄-Alkoxy, C₁-C₄-Halogenalkoxy, -NH₂, - NH(C₁-C₄-Alkyl) und -N(C₁-C₄-Alkyl)₂ ausgewählt ist; und
R₁₇ jedes Mal, wenn es ausgewählt wird, unabhängig voneinander aus der Gruppe bestehend aus C₁-C₄-Alkyl, C₃-C₆-Cycloalkyl, C₁-C₄-Halogenalkyl, C₁-C₄-Alkoxy, C₁-C₄-Halogenalkoxy, -OH, -NH₂, -NH(C₁-C₄-Alkyl), -N(C₁-C₄-Alkyl)₂, - NH(C₃-C₆-Cycloalkyl) und -N(C₁-C₄-Alkyl)(C₃-C₆-Cycloalkyl) ausgewählt ist;
wobei, wenn J 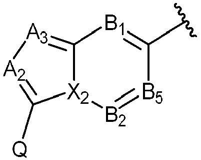 ist, A₂ N ist und A₃, B₁, B₂, B₅, X₂ und Q sind wie oben definiert;
oder ein Salz davon.

2. Verbindung nach Anspruch 1, wobei
n 0 oder 1 ist,
J aus der Gruppe bestehend aus 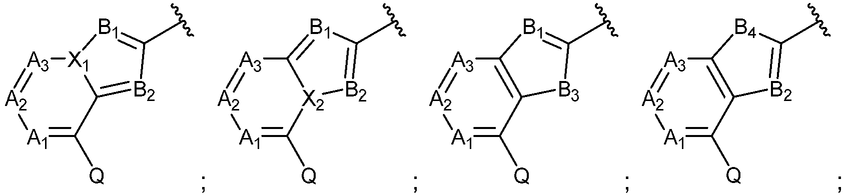 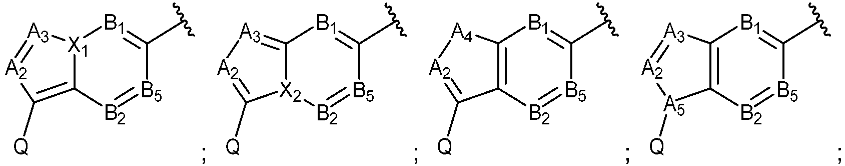 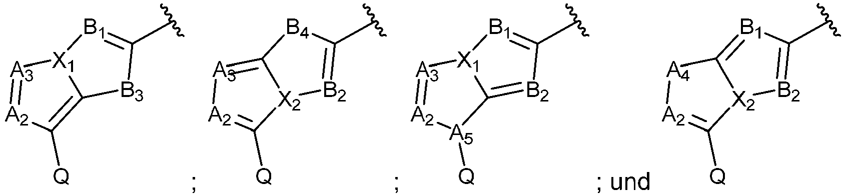 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₄ aus der Gruppe bestehend aus O, S und NR_{A4} ausgewählt ist;
A₅ aus der Gruppe bestehend aus N ausgewählt ist;
B₁ aus der Gruppe bestehend aus N und CR_{B1} ausgewählt ist;
B₂ aus der Gruppe bestehend aus N und CR_{B2} ausgewählt ist;
B₃ aus der Gruppe bestehend aus O, S und NR_{B3} ausgewählt ist;
B₄ aus der Gruppe bestehend aus O, S und NR_{B4} ausgewählt ist;
B₅ aus der Gruppe bestehend aus N und CR_{B5} ausgewählt ist;
X₁ aus der Gruppe bestehend aus N ausgewählt ist;
X₂ aus der Gruppe bestehend aus N ausgewählt ist;
G aus der Gruppe bestehend aus
ausgewählt ist; 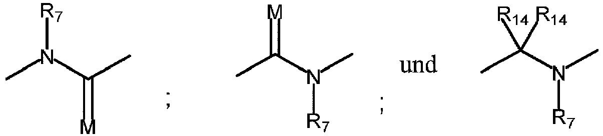
M aus der Gruppe bestehend aus N-R₁₃, O und S ausgewählt ist;
Y₁ aus der Gruppe bestehend aus CR₈R₉, O, S und NR₁₀ ausgewählt ist;
Y₂ aus der Gruppe bestehend aus CR₈R₉, O, S und NR₁₀ ausgewählt ist;
wobei mindestens eine der Gruppen Y₁ oder Y₂ CR₈R₉ ist;
Z₁ aus der Gruppe bestehend aus N, O, S und CR₁₁ ausgewählt ist;
Z₂ aus der Gruppe bestehend aus einer Bindung, N und CR₁₁ ausgewählt ist;
Z₃ aus der Gruppe bestehend aus einer Bindung, N und CR₁₁ ausgewählt ist;
Z₄ aus der Gruppe bestehend aus N, O, S und CR₁₁ ausgewählt ist;
wobei nicht mehr als 2 von Z₁, Z₂, Z₃ und Z₄ N sind und wobei nur eines von Z₁ und Z₄ O oder S ist, Z₂ nur dann eine Bindung ist, wenn Z₁ O oder S ist, und Z₃ nur dann eine Bindung ist, wenn Z₄ O oder S ist;
R_{A1}, R_{A2}, R_{A3}, R_{B2}, und R_{B5} jeweils unabhängig voneinander aus der Gruppe bestehend aus Wasserstoff, Halogen, Hydroxyl, -SH, NH₂, C₁-C₄-Alkyl, C₁-C₄-Halogenalkyl, C₁-C₄-Alkoxy und -SC₁-C₄Alkyl ausgewählt sind, wobei jedes C₁-C₄-Alkyl in R_{A1}, R_{A2}, R_{A3}, R_{B2}, und R_{B5} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), - C(O)N(C₁-C₄-Alkyl)₂, und C₁-C₄-Alkoxy ausgewählt sind;
R_{A4} und R_{B3} unabhängig voneinander aus der Gruppe bestehend aus Wasserstoff, Halogen, Hydroxyl, -SH, NH₂, C₁-C₄Alkyl, C₁-C₄Halogenalkyl, C₁-C₄-Alkoxy und -SC₁-C₄-Alkyl ausgewählt sind, wobei jedes C₁-C₄-Alkyl in R_{A4} und R_{B3} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R_{B1} aus der Gruppe ausgewählt ist, bestehend aus
Halogen, Hydroxyl, -SH, NH₂, C₁-C₄-Alkyl, C₃-C₆-Cycloalkyl, C₁-C₄-Halogenalkyl, C₁-C₄-Alkoxy, -SC₁-C₄-Alkyl und -NH(C₁-C₄-Alkyl), -N(C₁-C₄-Alkyl)₂, wobei jedes C₁-C₄-Alkyl in R_{B1} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind; und
einem 4- bis 7-gliedrigen Heterocycloalkyl, das 1, 2 oder 3 Heteroatome enthält, die unabhängig voneinander aus N oder O ausgewählt sind, wobei jedes Heterocycloalkyl in R_{B1} gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, - SO₂C₁-C₄-Alkyl, -S(O)C₁-C₄-Halogenalkyl und -SO₂C₁-C₄-Halogenalkyl ausgewählt sind;
R_{B4} aus der Gruppe bestehend aus Halogen, Hydroxyl, -SH, NH₂, C₁-C₄ - Alkyl, C₃-C₆-Cycloalkyl, C₁-C₄-Halogenalkyl, C₁-C₄-Alkoxy und -SC₁-C₄-Alkyl ausgewählt ist, wobei jedes C₁-C₄-Alkyl in R_{B4} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), - C(O)N(C₁-C₄-Alkyl)₂ und C₁-C₄-Alkoxy ausgewählt sind;
R₇ aus der Gruppe bestehend aus Wasserstoff, C₁-C₄-Alkyl und C₃-C₆-Cycloalkyl ausgewählt ist, das gegebenenfalls mit 1 bis 5 Halogenatomen substituiert ist;
R₃ jedes Mal, wenn es ausgewählt wird, unabhängig voneinander aus der Gruppe bestehend aus Wasserstoff, Fluor und (C₁-C₄)-Alkyl ausgewählt ist;
R₉ jedes Mal, wenn es ausgewählt wird, unabhängig voneinander aus der Gruppe bestehend aus Wasserstoff, Fluor und (C₁-C₄)-Alkyl ausgewählt ist;
R₁₀ aus der Gruppe bestehend aus Wasserstoff und C₁-C₄-Alkyl ausgewählt ist;
R₁₁ aus der Gruppe bestehend aus Wasserstoff, Halogen, C₁-C₄-Alkyl, C₁-C₄ -Halogenalkyl, C₃-C₆-Cycloalkyl ausgewählt ist;
Q 6- oder 10-gliedriges Aryl ist, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
R₁₃ aus der Gruppe bestehend aus Hydroxy, C₁-C₄-Alkoxy und -NH₂ ausgewählt ist;
R₁₄ jedes Mal, wenn es ausgewählt wird, unabhängig voneinander aus der Gruppe bestehend aus Wasserstoff und Halogen ausgewählt ist; und
R₁₇ jedes Mal, wenn es ausgewählt wird, unabhängig voneinander aus der Gruppe bestehend aus C₁-C₄-Alkyl, C₃-C₆-Cycloalkyl, C₁-C₄-Halogenalkyl, C₁-C₄-Alkoxy, C₁-C₄-Halogenalkoxy, -OH, -NH₂, -NH(C₁-C₄-Alkyl), -N(C₁-C₄-Alkyl)₂, - NH(C₃-C₆-Cycloalkyl) und -N(C₁-C₄-Alkyl)(C₃-C₆-Cycloalkyl) ausgewählt ist;
wobei, wenn J 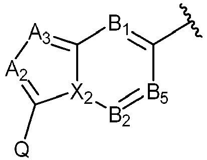 ist, A₂ N ist und A₃, B₁, B₂, B₅, X₂ und Q sind wie oben definiert;
oder ein Salz davon.

3. Verbindung nach Anspruch 1 oder 2, wobei
n 0 oder 1 ist,
J aus der Gruppe bestehend aus 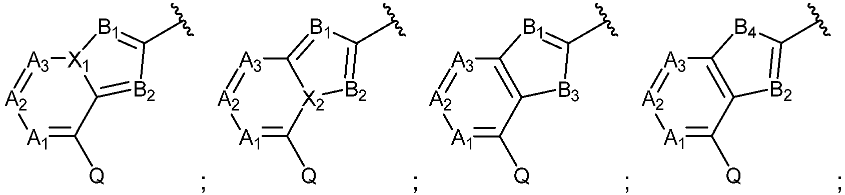 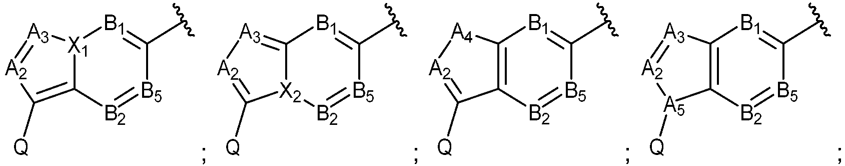 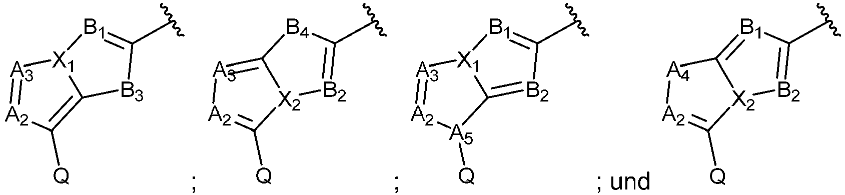 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₄ aus der Gruppe bestehend aus O, S und NR_{A4} ausgewählt ist;
A₅ aus der Gruppe bestehend aus N ausgewählt ist;
B₁ aus der Gruppe bestehend aus N und CR_{B1} ausgewählt ist;
B₂ aus der Gruppe bestehend aus N und CR_{B2} ausgewählt ist;
B₃ aus der Gruppe bestehend aus O, S und NR_{B3} ausgewählt ist;
B₄ aus der Gruppe bestehend aus O, S und NR_{B4} ausgewählt ist;
B₅ aus der Gruppe bestehend aus N und CR_{B5} ausgewählt ist;
X₁ aus der Gruppe bestehend aus N ausgewählt ist;
X₂ aus der Gruppe bestehend aus N ausgewählt ist;
G aus der Gruppe bestehend aus
ausgewählt ist; 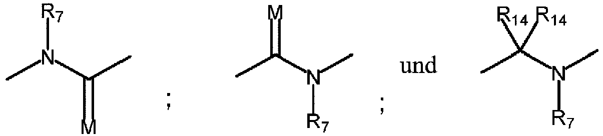
M aus der Gruppe bestehend aus N-R₁₃, O und S ausgewählt ist;
Y₁ aus der Gruppe bestehend aus CR₈R₉, O, S und NR₁₀ ausgewählt ist;
Y₂ aus der Gruppe bestehend aus CR₈R₉, O, S und NR₁₀ ausgewählt ist;
wobei mindestens eine der Gruppen Y₁ oder Y₂ CR₈R₉ ist;
Z₁ aus der Gruppe bestehend aus N, O, S und CR₁₁ ausgewählt ist;
Z₂ aus der Gruppe bestehend aus einer Bindung, N und CR₁₁ ausgewählt ist;
Z₃ aus der Gruppe bestehend aus einer Bindung, N und CR₁₁ ausgewählt ist;
Z₄ aus der Gruppe bestehend aus N, O, S und CR₁₁ ausgewählt ist;
wobei nicht mehr als 2 von Z₁, Z₂, Z₃ und Z₄ N sind und wobei nur eines von Z₁ und Z₄ O oder S ist, Z₂ nur dann eine Bindung ist, wenn Z₁ O oder S ist, und Z₃ nur dann eine Bindung ist, wenn Z₄ O oder S ist;
R_{A1}, R_{A2}, R_{A3}, R_{B2}, und R_{B5} jeweils unabhängig voneinander aus der Gruppe bestehend aus Wasserstoff, Halogen und C₁-C₄ -Alkyl ausgewählt sind, wobei jedes C₁-C₄-Alkyl in R_{A1}, R_{A2}, R_{A3}, R_{B2}, und R_{B5} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂, und C₁-C₄-Alkoxy ausgewählt sind;
R_{A4} und R_{B3} unabhängig voneinander aus der Gruppe bestehend aus Wasserstoff, C₁-C₄-Alkyl ausgewählt sind, wobei jedes C₁-C₄-Alkyl in R_{A4} und R_{B3} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R_{B1} aus der Gruppe ausgewählt ist, bestehend aus
C₁-C₄-Alkyl, -N(C₁-C₄-Alkyl)₂, wobei jedes C₁-C₄-Alkyl in R_{B1} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind; und
einem 4- bis 7-gliedrigen Heterocycloalkyl, das 1, 2 oder 3 Heteroatome enthält, die unabhängig voneinander aus N oder O ausgewählt sind, wobei jedes Heterocycloalkyl in R_{B1} gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, - SO₂C₁-C₄-Alkyl, -S(O)C₁-C₄-Halogenalkyl und -SO₂C₁-C₄-Halogenalkyl ausgewählt sind;
R_{B4} C₁-C₄-Alkyl ist, wobei jedes C₁-C₄-Alkyl in R_{B4} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R₇ Wasserstoff ist;
R₈ Wasserstoff ist;
R₉ Wasserstoff ist;
R₁₀ aus der Gruppe bestehend aus Wasserstoff und C₁-C₄-Alkyl ausgewählt ist;
R₁₁ Wasserstoff ist;
Q 6- oder 10-gliedriges Aryl ist, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
R₁₃ aus der Gruppe bestehend aus Hydroxy, C₁-C₄-Alkoxy und -NH₂ ausgewählt ist;
wobei, wenn J 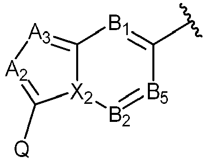 ist, A₂ N ist und A₃, B₁, B₂, B₅, X₂ und Q sind wie oben definiert;
oder ein Salz davon.

4. Verbindung nach einem der Ansprüche 1 bis 3, wobei
n 1 ist,
J aus der Gruppe bestehend aus 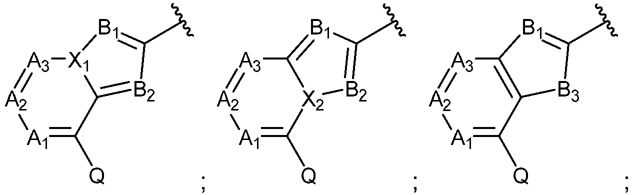 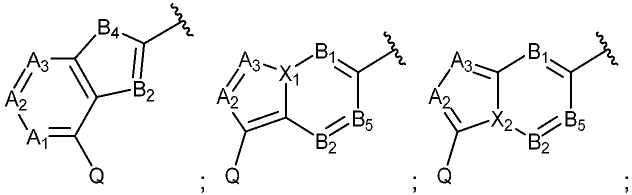 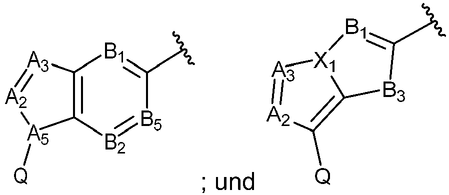 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₅ aus der Gruppe bestehend aus N ausgewählt ist;
B₁ CR_{B1} ist;
B₂ aus der Gruppe bestehend aus N und CR_{B2} ausgewählt ist;
B₃ aus der Gruppe bestehend aus O, S und NR_{B3} ausgewählt ist;
B₄ NR_{B4} ist;
B₅ aus der Gruppe bestehend aus N und CR_{B5} ausgewählt ist;
X₁ aus der Gruppe bestehend aus N ausgewählt ist;
X₂ aus der Gruppe bestehend aus N ausgewählt ist;
G aus der Gruppe ausgewählt ist, bestehend aus 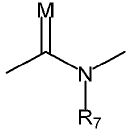
M ist O;
Y₁ CR₈R₉ ist;
Y₂ O ist;
Z₁ CR₁₁ ist;
Z₂ CR₁₁ ist;
Z₃ CR₁₁ ist;
Z₄ CR₁₁ ist;
R_{A1}, R_{A2}, R_{A3}, R_{B2}, und R_{B5} jeweils unabhängig voneinander aus der Gruppe bestehend aus Wasserstoff, Halogen, C₁-C₄-Alkyl ausgewählt sind, wobei jedes C₁-C₄-Alkyl in R_{A1}, R_{A2}, R_{A3}, R_{B2}, und R_{B5} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), - C(O)N(C₁-C₄-Alkyl)₂, und C₁-C₄-Alkoxy ausgewählt sind;
R_{B3} aus der Gruppe bestehend aus Wasserstoff und C₁-C₄-Alkyl ausgewählt ist, wobei jedes C₁-C₄-Alkyl in R_{B3} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), - C(O)N(C₁-C₄-Alkyl)₂ und C₁-C₄-Alkoxy ausgewählt sind;
R_{B1} aus der Gruppe ausgewählt ist, bestehend aus
C₁-C₄-Alkyl, -N(C₁-C₄-Alkyl)₂, wobei jedes C₁-C₄-Alkyl in R_{B1} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind; und
einem 4- bis 7-gliedrigen Heterocycloalkyl, das 1, 2 oder 3 Heteroatome enthält, die unabhängig voneinander aus N oder O ausgewählt sind, wobei jedes Heterocycloalkyl in R_{B1} gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, - SO₂C₁-C₄-Alkyl, -S(O)C₁-C₄-Halogenalkyl und -SO₂C₁-C₄-Halogenalkyl ausgewählt sind;
R_{B4} C₁-C₄-Alkyl ist, wobei jedes C₁-C₄-Alkyl in R_{B4} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R₇ Wasserstoff ist;
R₈ Wasserstoff ist;
R₉ Wasserstoff ist;
R₁₁ Wasserstoff ist;
Q 6- oder 10-gliedriges Aryl ist, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
wobei, wenn J 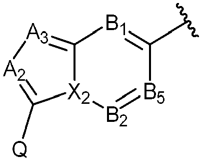 ist, A₂ N ist und A₃, B₁, B₂, B₅, X₂ und Q sind wie oben definiert;
oder ein Salz davon.

5. Verbindung nach einem der Ansprüche 1 bis 4, wobei
n 1 ist,
J aus der Gruppe bestehend aus 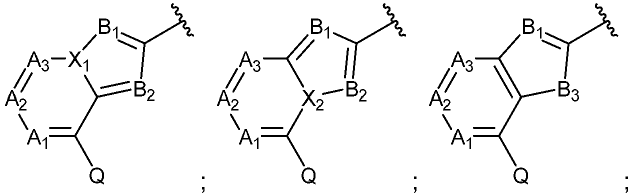 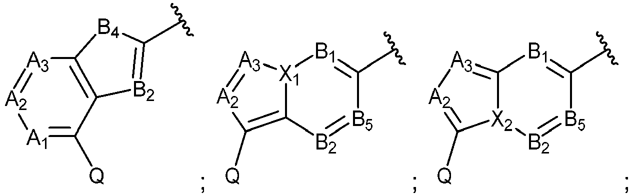 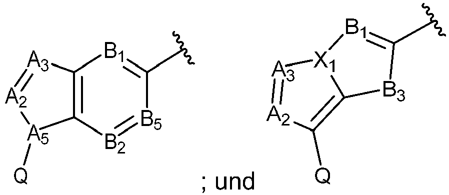 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₅ N ist;
B1 CR_{B1} ist;
B₂ aus der Gruppe bestehend aus N und CR_{B2} ausgewählt ist;
B₃ aus der Gruppe bestehend aus O, S und NR_{B3} ausgewählt ist;
B₄ NR_{B4} ist;
B₅ aus der Gruppe bestehend aus N und CR_{B5} ausgewählt ist;
X₁ N ist;
X₂ N ist;
G ist 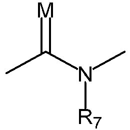
M O ist;
Y₁ CR₈R₉ ist;
Y₂ O ist;
Z₁ CR₁₁ ist;
Z₂ CR₁₁ ist;
Z₃ CR₁₁ ist;
Z₄ CR₁₁ ist;
R_{A1}, R_{A2}, R_{A3}, R_{B2}, und R_{B5} jeweils unabhängig voneinander aus der Gruppe bestehend aus Wasserstoff, Halogen und C₁-C₄-Alkyl ausgewählt sind, wobei jedes C₁-C₄-Alkyl in R_{A1}, R_{A2}, R_{A3}, R_{B2}, und R_{B5} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂, und C₁-C₄-Alkoxy ausgewählt sind;
R_{B3} aus der Gruppe bestehend aus Wasserstoff und C₁-C₄-Alkyl ausgewählt ist, wobei jedes C₁-C₄-Alkyl in R_{B3} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), - C(O)N(C₁-C₄-Alkyl)₂ und C₁-C₄-Alkoxy ausgewählt sind;
R_{B1} aus der Gruppe ausgewählt ist, bestehend aus
C₁-C₄-Alkyl, -N(C₁-C₄-Alkyl)₂, wobei jedes C₁-C₄-Alkyl in R_{B1} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind; und
einem 4- bis 7-gliedrigen Heterocycloalkyl, das 1, 2 oder 3 Heteroatome enthält, die unabhängig voneinander aus N oder O ausgewählt sind, wobei jedes Heterocycloalkyl in R_{B1} gegebenenfalls mit 1, 2 oder 3 Substituenten substituiert ist, die unabhängig voneinander aus der Gruppe bestehend aus Halogen, Cyano, Nitro, Hydroxy, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, - SO₂C₁-C₄-Alkyl, -S(O)C₁-C₄-Halogenalkyl und -SO₂C₁-C₄-Halogenalkyl ausgewählt sind;
R_{B4} C₁-C₄-Alkyl ist, wobei jedes C₁-C₄-Alkyl in R_{B4} gegebenenfalls mit 1, 2 oder 3 Substituenten substituiert sein kann, die unabhängig voneinander aus der Gruppe bestehend aus Halogen, Hydroxy, -NH₂, -NH(C₁-C₄-Alkyl), -N(C₁-C₄-Alkyl)₂, Cyano, Carboxy, Carbamoyl, C₁-C₄-Alkoxycarbonyl, -C(O)NH(C₁-C₄-Alkyl), -C(O)N(C₁-C₄-Alkyl)₂ und C₁-C₄-Alkoxy ausgewählt sind;
R₇ Wasserstoff ist;
R₈ Wasserstoff ist;
R₉ Wasserstoff ist;
R₁₁ Wasserstoff ist;
Q Phenyl ist, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
wobei, wenn J 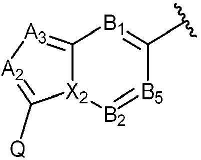 ist, A₂ N ist und A₃, B₁, B₂, B₅, X₂ und Q sind wie oben definiert;
oder ein Salz davon.

6. Verbindung nach einem der Ansprüche 1 bis 5, wobei
n 1 ist,
J aus der Gruppe bestehend aus 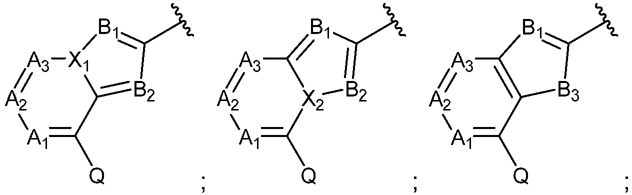 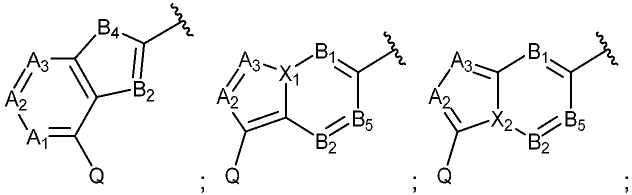 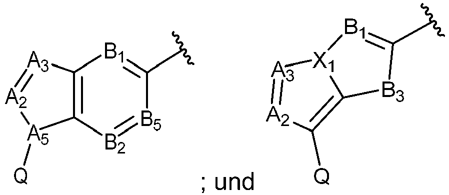 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₅ N ist;
B₁ CR_{B1} ist;
B₂ aus der Gruppe bestehend aus N und CR_{B2} ausgewählt ist;
B₃ aus der Gruppe bestehend aus O, S und NR_{B3} ausgewählt ist;
B₄ NR_{B4} ist;
B₅ aus der Gruppe bestehend aus N und CR_{B5} ausgewählt ist;
X₁ N ist;
X₂ N ist;
G ist 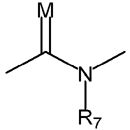
M O ist;
Y₁ CR₈R₉ ist;
Y₂ O ist;
Z₁ CR₁₁ ist;
Z₂ CR₁₁ ist;
Z₃ CR₁₁ ist;
Z₄ CR₁₁ ist;
R_{A1}, R_{A2}, R_{A3}, R_{B2} und R_{B5} jeweils unabhängig voneinander aus der Gruppe bestehend aus Wasserstoff, Halogen, C₁-C₄-Alkyl ausgewählt sind;
R_{B3} aus der Gruppe bestehend aus Wasserstoff und C₁-C₄-Alkyl ausgewählt ist;
R_{B1} aus der Gruppe ausgewählt ist, bestehend aus
C₁-C₄-Alkyl, -N(C₁-C₄-Alkyl)₂, wobei jedes C₁-C₄-Alkyl in R_{B1} gegebenenfalls mit 1, 2 oder 3 Hydroxy-Substituenten substituiert sein kann; und
einem 4- bis 7-gliedrigen Heterocycloalkyl, das 1, 2 oder 3 Heteroatome enthält, die unabhängig voneinander aus N oder O ausgewählt sind, wobei jedes Heterocycloalkyl in R_{B1} gegebenenfalls mit 1, 2 oder 3 Halogensubstituenten substituiert ist;
R_{B4} C₁-C₄-Alkyl ist;
R₇ Wasserstoff ist;
R₈ Wasserstoff ist;
R₉ Wasserstoff ist;
R₁₁ Wasserstoff ist;
Q Phenyl mit 1 bis 5 Halogenatomen ist;
wobei, wenn J 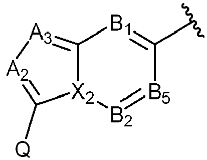 ist, A₂ N ist und A₃, B₁, B₂, B₅, X₂ und Q sind wie oben definiert;
oder ein Salz davon.

7. Verbindung nach einem der Ansprüche 1 bis 6, wobei
n 1 ist,
J aus der Gruppe bestehend aus 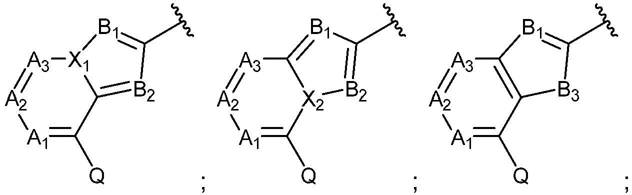 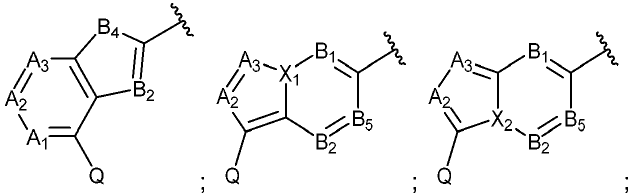 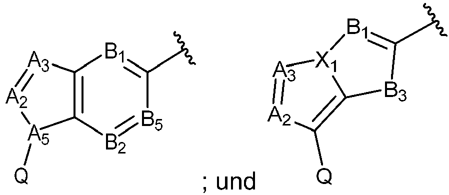 ausgewählt ist;
A₁ aus der Gruppe bestehend aus N und CR_{A1} ausgewählt ist;
A₂ aus der Gruppe bestehend aus N und CR_{A2} ausgewählt ist;
A₃ aus der Gruppe bestehend aus N und CR_{A3} ausgewählt ist;
A₅ N ist;
B1 CR_{B1} ist;
B₂ aus der Gruppe bestehend aus N und CR_{B2} ausgewählt ist;
B₃ aus der Gruppe bestehend aus O, S und NR_{B3} ausgewählt ist;
B₄ NR_{B4} ist;
B₅ aus der Gruppe bestehend aus N und CR_{B5} ausgewählt ist;
X₁ aus der Gruppe bestehend aus N ausgewählt ist;
X₂ aus der Gruppe bestehend aus N ausgewählt ist;
G aus der Gruppe ausgewählt ist, bestehend aus 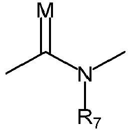
M ist O;
Y₁ CR₈R₉ ist;
Y₂ O ist;
Z₁ CR₁₁ ist;
Z₂ CR₁₁ ist;
Z₃ CR₁₁ ist;
Z₄ CR₁₁ ist;
R_{A1}, R_{A2}, R_{A3}, R_{B2} und R_{B5} jeweils unabhängig voneinander aus der Gruppe bestehend aus Wasserstoff, Fluor und Methyl ausgewählt sind;
R_{B3} unabhängig aus der Gruppe bestehend aus Wasserstoff und Methyl ausgewählt ist;
R_{B1} unabhängig aus der Gruppe bestehend aus 4-Morpholino, Isopropyl, 2-Hydroxyisopropyl, 3-Fluorazetidinyl und NMe₂ ausgewählt ist;
R_{B4} Isopropyl ist;
R₇ Wasserstoff ist;
R₈ Wasserstoff ist;
R₉ Wasserstoff ist;
R₁₁ Wasserstoff ist;
Q aus der Gruppe bestehend aus 2,3,5-Trifluorphenyl und 2,6-Difluorphenyl ausgewählt ist;
wobei, wenn J 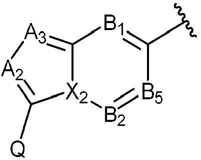 ist, A₂ N ist und A₃, B₁, B₂, B₅, X₂ und Q sind wie oben definiert;
oder ein Salz davon.

8. Verbindung nach einem der Ansprüche 1 bis 7, wobei
J aus der Gruppe bestehend aus 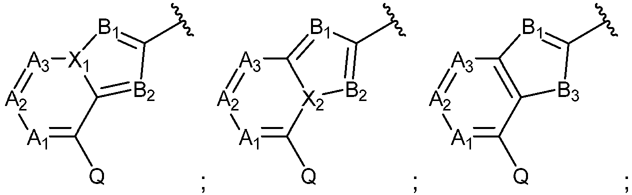 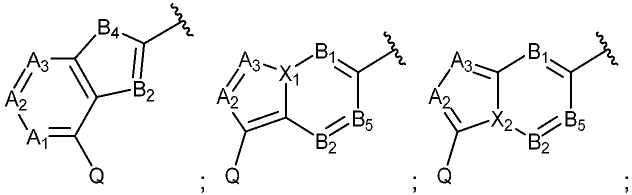 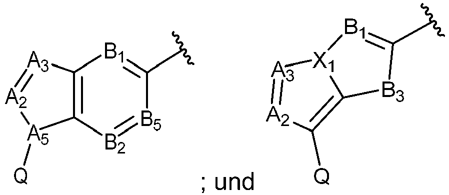 ausgewählt ist;
wobei, wenn J 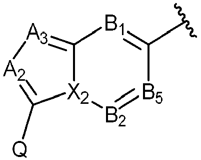 ist, A₂ N ist und A₃, B₁, B₂, B₅, X₂ und Q wie in einem der Ansprüche 1 bis 7 definiert sind;
oder ein Salz davon.

9. Verbindung nach einem der Ansprüche 1 bis 8, wobei
G 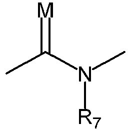 ist; und
M O ist;
oder ein Salz davon, mehr bevorzugt
M O ist; und
R₇ Wasserstoff ist.

10. Verbindung nach einem der Ansprüche 1 bis 9, wobei
Q ein 6- oder 10-gliedriges Aryl ist, das gegebenenfalls mit 1, 2 oder 3 Substituenten substituiert ist, die unabhängig voneinander aus der Gruppe bestehend aus Halogen, Cyano, Nitro, Hydroxy, C₁-C₄-Alkyl, C₁-C₄-Halogenalkyl, C₁-C₄-Alkoxy, C₃-C₆-Cycloalkyl, -C(O)R₁₇, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind;
oder ein Salz davon.

11. Verbindung nach einem der Ansprüche 1 bis 10, wobei
Q ein 6-gliedriges Aryl ist, das gegebenenfalls mit 1, 2, 3, 4 oder 5 Substituenten substituiert ist, die unabhängig voneinander aus der Gruppe bestehend aus Halogen, Cyano, Nitro, Hydroxyl, C₁-C₄-Alkyl, C₁-C₄-Halogenalkyl, C₁-C₄-Alkoxy, C₃-C₆-Cycloalkyl, -C(O)R₁₇, -NH₂, -NH(C₁-C₄-Alkyl), -N(C₁-C₄-Alkyl)₂, -NH(C₃-C₆-Cycloalkyl), -N(C₁-C₄-Alkyl)(C₃-C₆-Cycloalkyl), -NHSO₂(C₁-C₄-Alkyl), -SC₁-C₄-Alkyl, -S(O)C₁-C₄-Alkyl, -SO₂C₁-C₄-Alkyl, -S(O)C₁-C₄-Halogenalkyl und -SO₂C₁-C₄-Halogenalkyl ausgewählt sind, wobei das 6- oder 10-gliedrige Aryl gegebenenfalls mit einem 4- bis 7-gliedrigen Heterocycloalkyl mit 1 oder 2 Heteroatomen, ausgewählt aus der Gruppe O, S und N, kondensiert ist, und wobei die Kohlenstoffe des Heterocycloalkyls gegebenenfalls mit 1, 2 oder 3 Substituenten substituiert sind, die unabhängig voneinander aus der Gruppe Halogen, Cyano, Nitro, Hydroxyl, Oxo, C₁-C₄-Alkyl, C₃-C₆-Cycloalkyl, C₁-C₄-Halogenalkyl, C₁-C₄-Alkoxy, -NH₂, -NH(C₁-C₄-Alkyl), und -N(C₁-C₄-Alkyl)₂ ausgewählt sind, und jedes N im Heterocycloalkyl, sofern es die Valenz erlaubt, mit einem Substituenten substituiert ist, der aus der Gruppe bestehend aus Wasserstoff, C₁-C₄-Alkyl, und C₃-C₆-Cycloalkyl ausgewählt ist.

12. Verbindung nach einem der Ansprüche 1 bis 11 oder ein Salz davon, wobei Q aus: 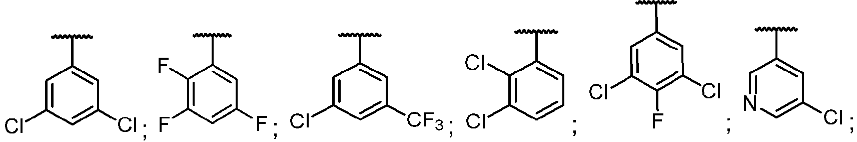 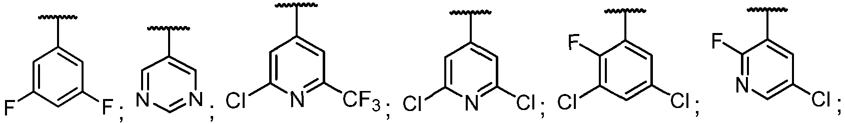 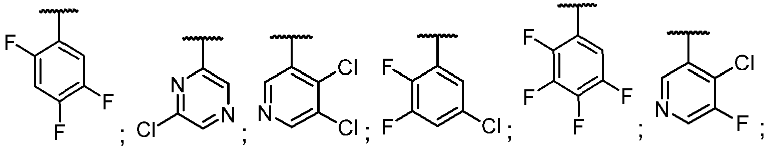 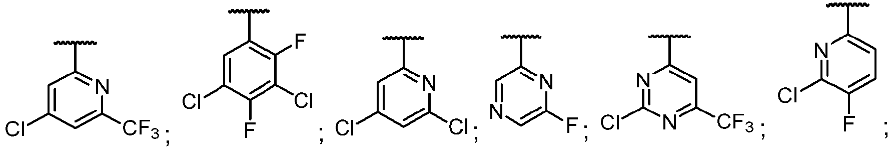 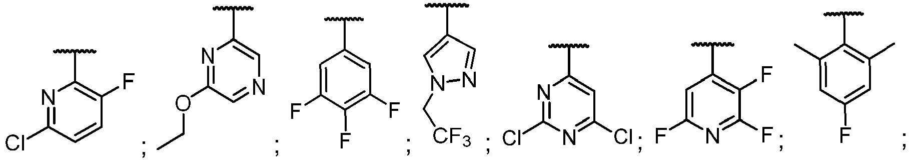 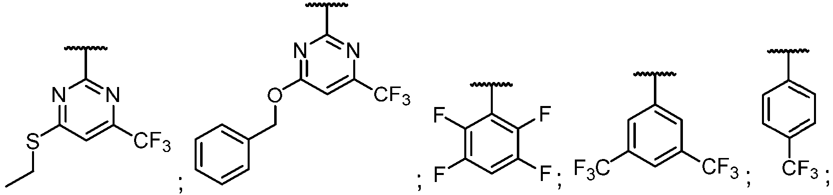 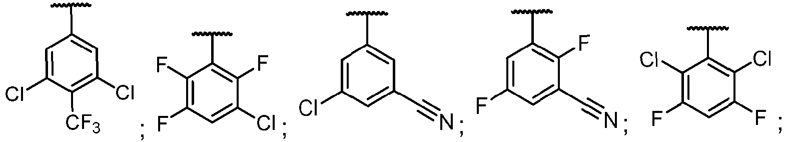 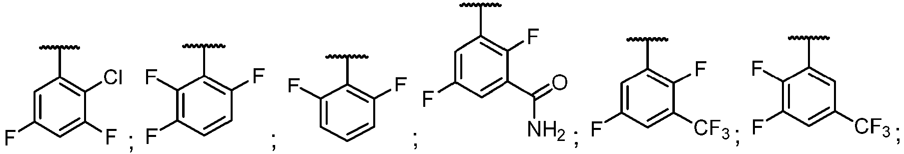 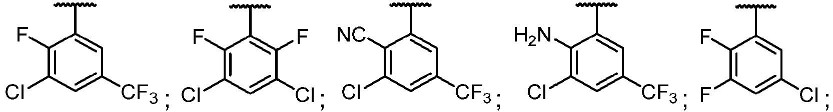 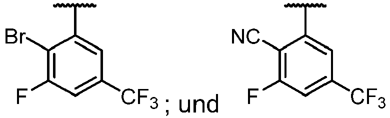 ausgewählt ist, wobei Q mehr bevorzugt 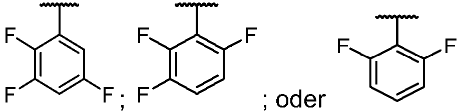 ist.

13. Verbindung nach einem der Ansprüche 1 bis 12, wobei
n 1 ist, Y₁ CR₈R₉ ist, Y₂ O ist, Z₁ CR₁₁ ist, Z₂ CR₁₁ ist, Z₃ CR₁₁ ist, Z₄ CR₁₁ ist,
oder ein Salz davon.

14. Verbindung nach einem der Ansprüche 1 bis 13, wobei R_{B1} oder R_{B4}, wenn vorhanden, aus der Gruppe bestehend aus C₁-C₄-Alkyl, hydroxysubstituiertem C₁-C₄-Alkyl, C₃-C₆-Cycloalkyl, -N(C₁-C₄-Alkyl)₂ und 4- bis 7-gliedrigem Heterocycloalkyl ausgewählt ist; oder ein Salz davon.

15. Verbindung nach einem der Ansprüche 1 bis 14 oder ein Salz davon, wobei R_{B1} oder R_{B4}, wenn vorhanden, aus: 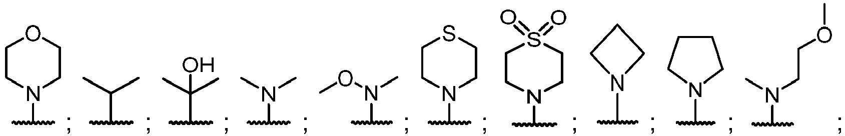 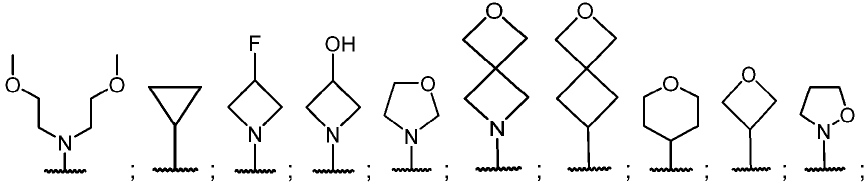 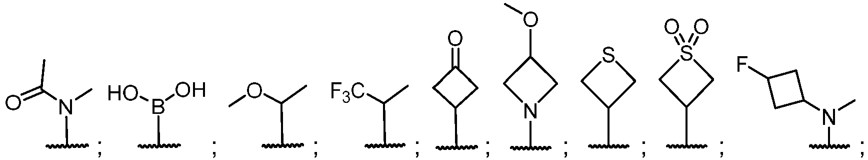 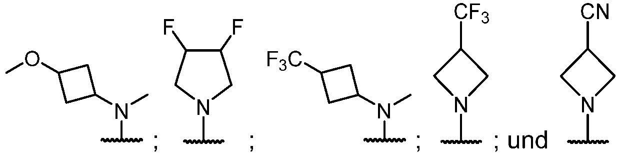 ausgewählt ist.

16. Verbindung nach einem der Ansprüche 1 bis 15, wobei die Verbindung aus der Gruppe bestehend aus: 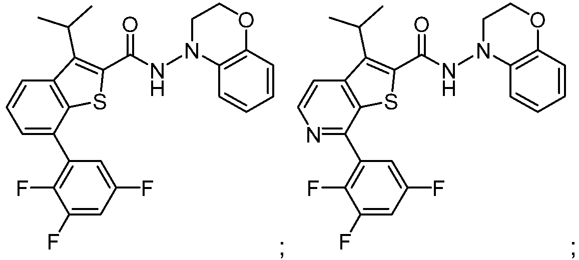 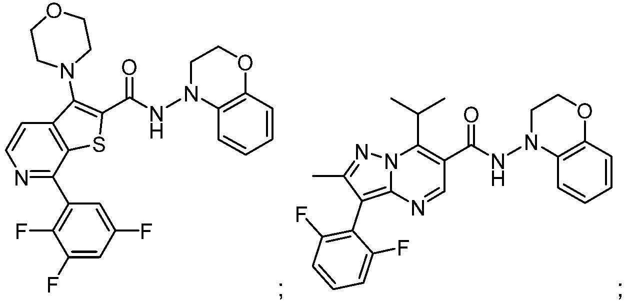 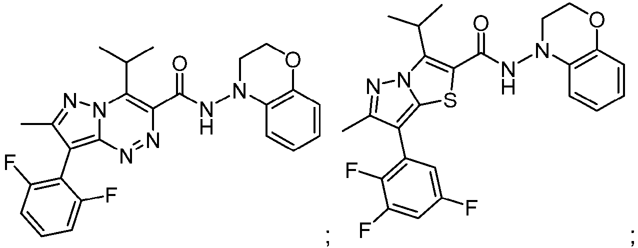 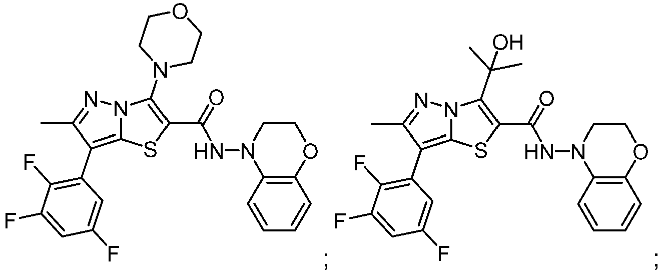 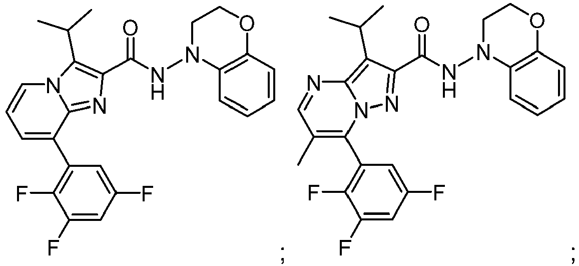 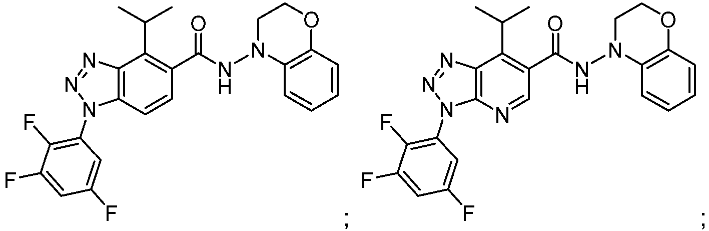 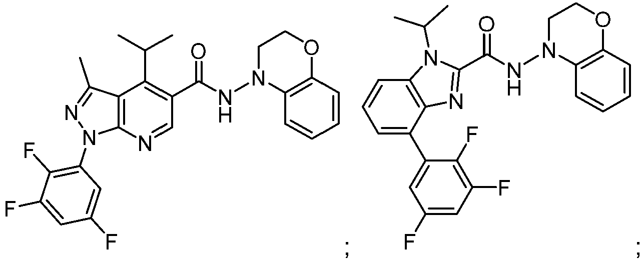 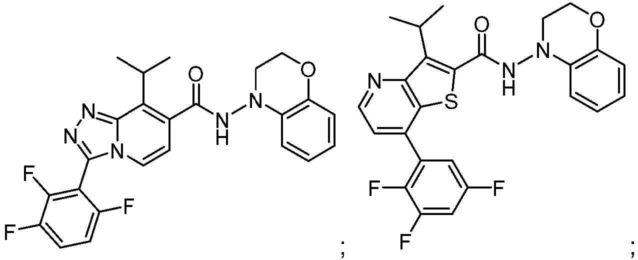 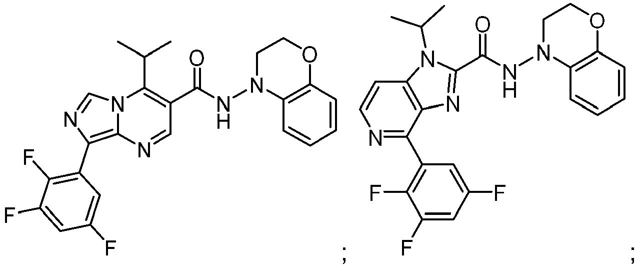 und 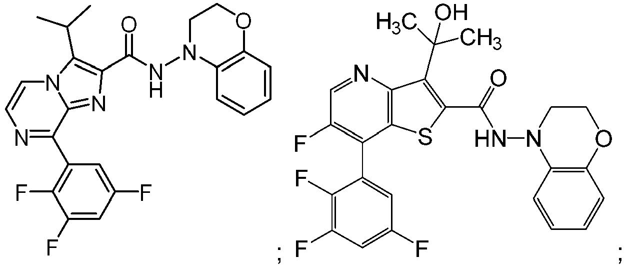 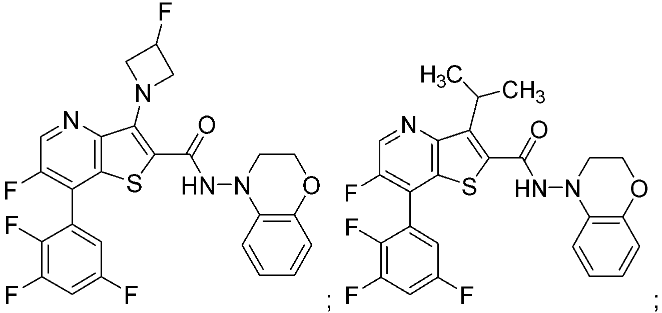 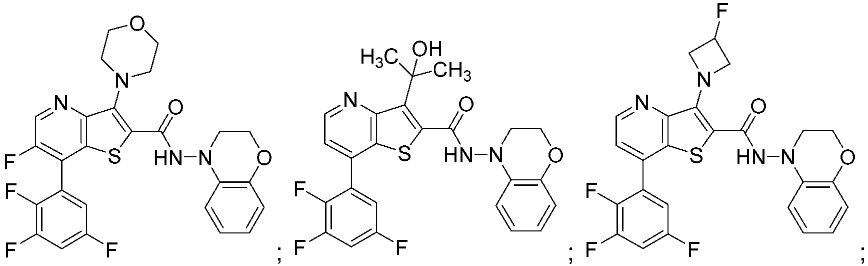 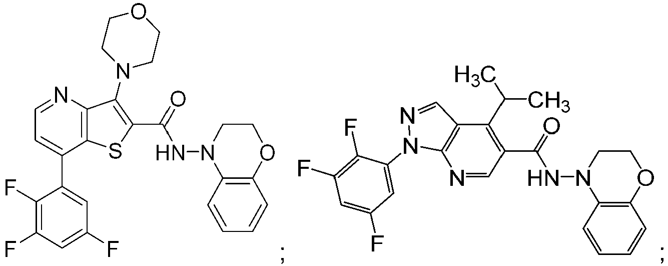 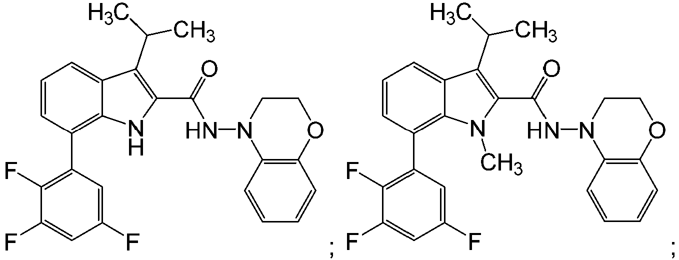 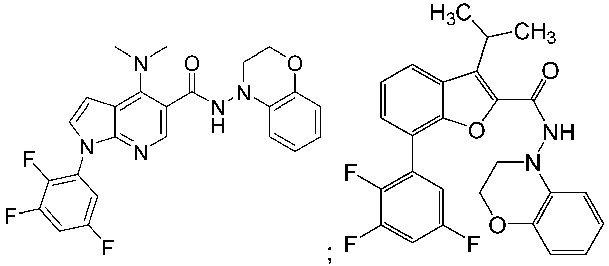 ausgewählt ist;
oder ein Salz einer der vorstehenden Verbindungen.

17. Pharmazeutische Zusammensetzung, umfassend eine Verbindung der Formel (I) nach einem der Ansprüche 1 bis 16 oder ein Salz davon und mindestens einen geeigneten Träger.

18. Verbindung der Formel (I) nach einem der Ansprüche 1 bis 16 oder eine pharmazeutische Zusammensetzung nach Anspruch 17 zur Verwendung bei der Bekämpfung, Behandlung und/oder Vorbeugung einer Krankheit, wobei die Krankheit vorzugsweise eine durch Endoparasiten verursachte Infektion ist, mehr bevorzugt eine Helmintheninfektion, noch mehr bevorzugt eine Herzwurminfektion.

## Revendications

1. Composé de formule (I) 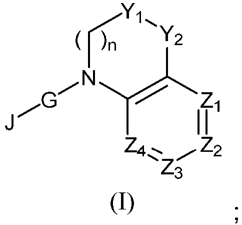 dans lequel
n vaut 0 ou 1 ;
J est choisi dans le groupe constitué par 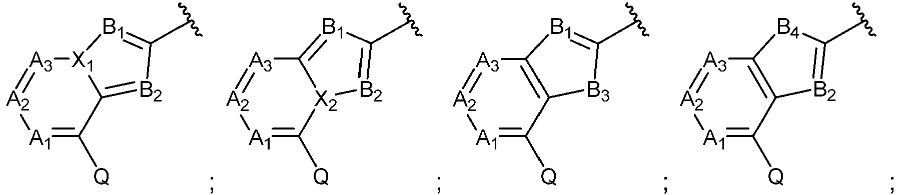 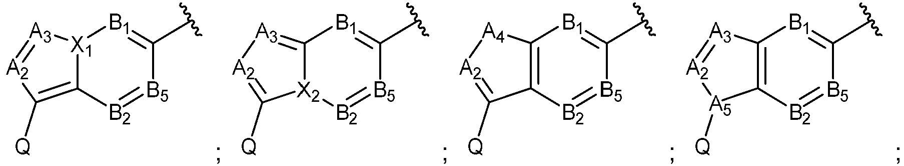 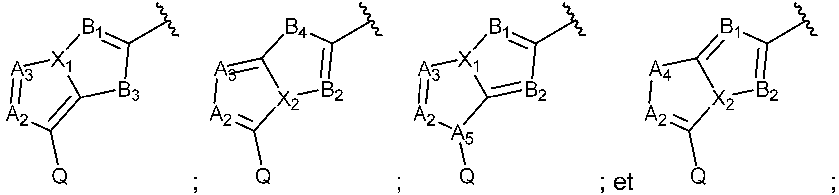
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₄ est choisi dans le groupe constitué par O, S et NR_{A4} ;
A₅ est choisi dans le groupe constitué par N ;
B₁ est choisi dans le groupe constitué par N et CR_{B1} ;
B₂ est choisi dans le groupe constitué par N et CR_{B2} ;
B₃ est choisi dans le groupe constitué par O, S et NR_{B3} ;
B₄ est choisi dans le groupe constitué par O, S et NR_{B4} ;
B₅ est choisi dans le groupe constitué par N et CR_{B5} ;
X₁ est choisi dans le groupe constitué par N ;
X₂ est choisi dans le groupe constitué par N ;
G est choisi dans le groupe constitué par 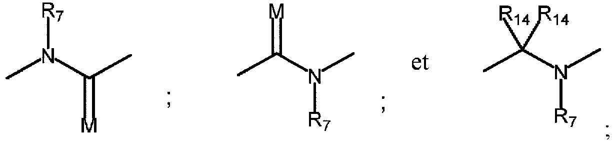
M est choisi dans le groupe constitué par N-R₁₃, O et S ;
Y₁ est choisi dans le groupe constitué par CR₈R₉, O, S et NR₁₀ ;
Y₂ est choisi dans le groupe constitué par CR₈R₉, O, S et NR₁₀ ;
dans lequel au moins l'un des groupes Y₁ ou Y₂ est CR₈R₉ ;
Z₁ est choisi dans le groupe constitué par N, O, S et CR₁₁ ;
Z₂ est choisi dans le groupe constitué par nul, N et CR₁₁ ;
Z₃ est choisi dans le groupe constitué par nul, N et CR₁₁ ;
Z₄ est choisi dans le groupe constitué par N, O, S et CR₁₁ ;
dans lequel pas plus de 2 parmi Z₁, Z₂, Z₃ et Z₄ sont N et dans lequel un seul parmi Z₁ et Z₄ est O ou S, Z₂ est nul uniquement lorsque Z₁ est O ou S, et Z₃ est nul uniquement lorsque Z₄ est O ou S ;
R_{A1}, R_{A2}, R_{A3}, R_{B2} et R_{B5} sont chacun indépendamment choisis dans le groupe constitué par hydrogène, halogène, hydroxyle, -SH, -S-alkyle en C₁ à C₄, - S(O)(alkyle en C₁ à C₄), -S(O)₂(alkyle en C₁ à C₄), cyano, alkyle en C₁ à C₄, halogénoalkyle en C₁ à C₄, alcoxy en C₁ à C₄, -B(OR₁₅)(OR₁₆) dans lequel R₁₅ est, à chaque reprise, choisi dans le groupe constitué par hydrogène, alkyle en C₁ à C₄ et cycloalkyle en C₃ à C₆, R₁₆ est, à chaque reprise, choisi dans le groupe constitué par hydrogène, alkyle en C₁ à C₄ et cycloalkyle en C₃ à C₆, ou R₁₅ et R₁₆ conjointement avec les atomes d'oxygène auxquels ils sont fixés forment un cycle de 5 à 7 chaînons qui est facultativement substitué par 1 à 4 alkyles en C₁ à C₄ ; - NH₂, -NH(alkyle en C₁ à C₄), et -N(alkyle en C₁ à C₄)₂ ;
R_{A4} et R_{B3} sont indépendamment choisis dans le groupe constitué par hydrogène, alkyle en C₁ à C₄, et halogénoalkyle en C₁ à C₄ ;
R_{B1} est choisi dans le groupe constitué par halogène, cyano, -CHO, hydroxyle, alkyle en C₁ à C₄, alcényle en C₂ à C₄, alcynyle en C₂ à C₄, cycloalkyle en C₃ à C₆, halogénoalkyle en C₁ à C₄, alkyle en C₁ à C₄ à substitution alcoxy en C₁ à C₄, benzyle facultativement substitué par 1 à 5 atomes d'halogène, alcoxy en C₁ à C₄, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -N(alkyle en C₁ à C₄)(hétérocycloalkyle de 4 à 7 chaînons), -NH(hétérocycloalkyle de 4 à 7 chaînons), -N(alkyle en C₁ à C₄)(alcoxy en C₁ à C₄), -C(O)NH(alkyle en C₁ à C₄), -C(O)N(alkyle en C₁ à C₄)₂, -C(O)N(alkyle en C₁ à C₄)(hétérocycloalkyle de 4 à 7 chaînons), -NHSO₂(alkyle en C₁ à C₄), -S-alkyle en C₁ à C₄, -S(O)alkyle en C₁ à C₄, -SO₂-alkyle en C₁ à C₄, -B(OR₁₅)(OR₁₆) dans lequel R₁₅ est, à chaque reprise, choisi dans le groupe constitué par hydrogène, alkyle en C₁ à C₄ et cycloalkyle en C₃ à C₆, R₁₆ est, à chaque reprise, choisi dans le groupe constitué par hydrogène, alkyle en C₁ à C₄,et cycloalkyle en C₃ à C₆, ou R₁₅ et R₁₆ conjointement avec les atomes d'oxygène auxquels ils sont fixés forment un cycle de 5 à 7 chaînons qui est facultativement substitué par 1 à 4 alkyles en C₁ à C₄ ; aryle de 6 ou 10 chaînons ; un hétérocycle monocyclique choisi dans le groupe d'hétérocycloalkyle de 4 à 7 chaînons, hétéroaryle de 5 chaînons ayant au moins un atome d'azote par l'intermédiaire duquel le cycle hétéroaryle de 5 chaînons est relié au reste de la molécule, et hétéroaryle de 6 chaînons ayant au moins un atome d'azote ; chacun des cycles aryle, hétérocycloalkyle et hétéroaryle in R_{B1} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ; dans lequel les cycles cycloalkyle en C₃ à C₆ et hétérocycloalkyle dans R_{B1} sont facultativement substitués par un groupe spiro, dans lequel ledit groupe spiro est un cycloalkyle en 3 à 6 chaînons ou un hétérocycloalkyle de 4 à 6 chaînons contenant 1, 2 ou 3 hétéroatomes indépendamment choisis parmi N, S ou O, dans lequel ledit groupe spiro est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, -NH₂, - NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ; et dans lequel chaque alkyle en C₁ à C₄, cycloalkyle en C₃ à C₆ et alcoxy en C₁ à C₄ dans R_{B1} peut être facultativement substitué par 1, 2 ou 3 substituants indépendamment choisis dans le groupe constitué par halogène, hydroxy, oxo, -NH₂, -NH(alkyle en C₁ à C₄), - N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂, et alcoxy en C₁ à C₄ ;
R_{B4} est choisi dans le groupe constitué par -CHO, hydroxyle, alkyle en C₁ à C₄, alcényle en C₂ à C₄, alcynyle en C₂ à C₄, cycloalkyle en C₃ à C₆, halogénoalkyle en C₁ à C₄, alkyle en C₁ à C₄ à substitution alcoxy en C₁ à C₄, benzyle facultativement substitué par 1 à 5 atomes d'halogène, alcoxy en C₁ à C₄, -NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -N(alkyle en C₁ à C₄)(hétérocycloalkyle de 4 à 7 chaînons), -NH(hétérocycloalkyle de 4 à 7 chaînons), -N(alkyle en C₁ à C₄)(alcoxy en C₁ à C₄), -C(O)NH(alkyle en C₁ à C₄), -C(O)N(alkyle en C₁ à C₄)₂, -C(O)N(alkyle en C₁ à C₄)(hétérocycloalkyle de 4 à 7 chaînons), -NHSO₂(alkyle en C₁ à C₄), -S-alkyle en C₁ à C₄, -S(O)alkyle en C₁ à C₄, -SO-alkyle en ₂C₁ à C₄, -B(OR₁₅)(OR₁₆) dans lequel R₁₅ est, à chaque reprise, choisi dans le groupe constitué par hydrogène, alkyle en C₁ à C₄ et cycloalkyle en C₃ à C₆, R₁₆ est, à chaque reprise, choisi dans le groupe constitué par hydrogène, alkyle en C₁ à C₄, et cycloalkyle en C₃ à C₆, ou R₁₅ et R₁₆ conjointement avec les atomes d'oxygène auxquels ils sont fixés forment un cycle de 5 à 7 chaînons qui est facultativement substitué par 1 à 4 alkyles en C₁ à C₄ ; aryle de 6 ou 10 chaînons ; un hétérocycle monocyclique choisi dans le groupe d'hétérocycloalkyle de 4 à 7 chaînons, hétéroaryle de 5 chaînons, et hétéroaryle de 6 chaînons ; chacun des cycles aryle, hétérocycloalkyle et hétéroaryle in R_{B4} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ; dans lequel les cycles cycloalkyle en C₃ à C₆ et hétérocycloalkyle dans R_{B4} sont facultativement substitués par un groupe spiro, dans lequel ledit groupe spiro est un cycloalkyle en 3 à 6 chaînons ou un hétérocycloalkyle de 4 à 6 chaînons contenant 1, 2 ou 3 hétéroatomes indépendamment choisis parmi N, S ou O, dans lequel ledit groupe spiro est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, -NH₂, - NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ; et dans lequel chaque alkyle en C₁ à C₄, cycloalkyle en C₃ à C₆ et alcoxy en C₁ à C₄ dans R_{B4} peut être facultativement substitué par 1, 2 ou 3 substituants indépendamment choisis dans le groupe constitué par halogène, hydroxy, oxo, -NH₂, -NH(alkyle en C₁ à C₄), - N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂, et alcoxy en C₁ à C₄ ;
R₇ est choisi dans le groupe constitué par hydrogène, alkyle en C₁ à C₄, et cycloalkyle en C₃ à C₆ facultativement substitué par 1 à 5 atomes d'halogène, - C(H)O, alcényle en C₂ à C₄, alcynyle en C₂ à C₄, halogénoalkyle en C₁ à C₄, et alcoxy en C₁ à C₄ ;
R₈ est, chaque fois qu'il est choisi, indépendamment choisi dans le groupe constitué par hydrogène, fluoro, et alkyle en C₁ à C₄ ;
R₉ est, chaque fois qu'il est choisi, indépendamment choisi dans le groupe constitué par hydrogène, fluoro, et alkyle en C₁ à C₄ ;
R₁₀ est choisi dans le groupe constitué par hydrogène et alkyle en C₁ à C₄ ;
R₁₁ est, chaque fois qu'il est choisi, indépendamment choisi dans le groupe constitué par hydrogène, halogène, hydroxyle, cyano, alkyle en C₁ à C₄, halogénoalkyle en C₁ à C₄, alcoxy en C₁ à C₄, cycloalkyle en C₃ à C₆, -NH₂, - NH(alkyle en C₁ à C₄), et -N(alkyle en C₁ à C₄)₂ ; et
Q est choisi dans le groupe constitué par
(i) aryle de 6 ou 10 chaînons facultativement substitué par 1, 2, 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄, dans lequel l'aryle de 6 ou 10 chaînons est facultativement condensé avec un hétérocycloalkyle de 4 à 7 chaînons ayant 1 ou 2 hétéroatomes choisis dans le groupe O, S et N et dans lequel les carbones de l'hétérocycloalkyle sont facultativement substitués par 1, 2 ou 3 substituants indépendamment choisis dans le groupe halogène, cyano, nitro, hydroxyle, oxo, alkyle en C₁ à C₄, cycloalkyle en C₃ à C₆, halogénoalkyle en C₁ à C₄, alcoxy en C₁ à C₄, -NH₂, - NH(alkyle en C₁ à C₄), et -N(alkyle en C₁ à C₄)₂ et l'un quelconque N dans l'hétérocycloalkyle est, si la valence le permet, substitué par un substituant choisi dans le groupe constitué par hydrogène, alkyle en C₁ à C₄ et cycloalkyle en C₃ à C₆ ;
(ii) hétéroaryle de 5 à 10 chaînons ayant 1, 2 ou 3 hétéroatomes indépendamment choisis dans le groupe O, S et N et dans lequel les carbones de l'hétéroaryle de 5 à 10 chaînons sont facultativement substitués par 1, 2, 3, 4 ou 5 substituants indépendamment choisis dans le groupe constitué par halogène, cyano, nitro, hydroxyle, alkyle en C₁ à C₄, cycloalkyle en C₃ à C₆, halogénoalkyle en C₁ à C₄, alcoxy en C₁ à C₄, benzyloxy, -C(O)R₁₇, -NH₂, -NH(alkyle en C₁ à C₄), - N(alkyle en C₁ à C₄)₂, -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄, et l'un quelconque N dans l'hétéroaryle, si la valence le permet, est facultativement substitué par un substituant choisi dans le groupe constitué par hydrogène, alkyle en C₁ à C₄ et cycloalkyle en C₃ à C₆ ;
(iii) hétérocycloalkyle de 4 à 7 chaînons ayant 1, 2 ou 3 hétéroatomes indépendamment choisis dans le groupe O, S, N, dans lequel l'hétérocycloalkyle est facultativement benzo-condensé, dans lequel les carbones de l'hétérocycloalkyle de 4 à 7 chaînons ou facultativement de l'hétérocycloalkyle de 4 à 7 chaînons benzo-condensé sont facultativement substitués par 1, 2, 3 ou 4 substituants indépendamment choisis dans le groupe constitué par halogène, cyano, nitro, hydroxyle, oxo, alkyle en C₁ à C₄, cycloalkyle en C₃ à C₆, halogénoalkyle en C₁ à C₄, alcoxy en C₁ à C₄, -C(O)R₁₇, -NH₂, -NH(alkyle en C₁ à C₄), et -N(alkyle en C₁ à C₄)₂ et l'un quelconque N dans l'hétérocycloalkyle est facultativement substitué par un substituant choisi dans le groupe constitué par hydrogène, alkyle en C₁ à C₄, et cycloalkyle en C₃ à C₆ ;
(iv) aryloxy de 6 ou 10 chaînons facultativement substitué par 1, 2 ou 3 substituants indépendamment choisis dans le groupe constitué par halogène, cyano, nitro, hydroxyle, alkyle en C₁ à C₄, cycloalkyle en C₃ à C₆, halogénoalkyle en C₁ à C₄, alcoxy en C₁ à C₄, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ;
(v) arylthio-oxy de 6 ou 10 chaînons facultativement substitué par 1, 2 ou 3 substituants indépendamment choisis dans le groupe constitué par halogène, cyano, nitro, hydroxyle, alkyle en C₁ à C₄, cycloalkyle en C₃ à C₆, halogénoalkyle en C₁ à C₄, alcoxy en C₁ à C₄, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ; et
(vi) hétéroaryloxy de 5 ou 10 chaînons facultativement substitué par 1, 2 ou 3 substituants indépendamment choisis dans le groupe constitué par halogène, cyano, nitro, hydroxyle, oxo, alkyle en C₁ à C₄, cycloalkyle en C₃ à C₆, halogénoalkyle en C₁ à C₄, alcoxy en C₁ à C₄, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et - SO₂halogénoalkyle en C₁ à C₄ ;
R₁₃ est choisi dans le groupe constitué par hydroxy, alcoxy en C₁ à C₄ et - NH₂ ;
R₁₄ est, chaque fois qu'il est choisi, indépendamment choisi dans le groupe constitué par hydrogène, halogène, cyano, nitro, hydroxyle, alkyle en C₁ à C₄, cycloalkyle en C₃ à C₆, halogénoalkyle en C₁ à C₄, alcoxy en C₁ à C₄, halogénoalcoxy en C₁ à C₄, -NH₂, -NH(alkyle en C₁ à C₄) et -N(alkyle en C₁ à C₄)₂ ; et
R₁₇ est, chaque fois qu'il est choisi, indépendamment choisi dans le groupe constitué par alkyle en C₁ à C₄, cycloalkyle en C₃ à C₆, halogénoalkyle en C₁ à C₄, alcoxy en C₁ à C₄, halogénoalcoxy en C₁ à C₄, -OH, -NH₂, -NH(alkyle en C₁ à C₄), -N(alkyle en C₁ à C₄)₂, -NH(cycloalkyle en C₃ à C₆) et -N(alkyle en C₁ à C₄)(cycloalkyle en C₃ à C₆) ;
dans lequel, lorsque J est 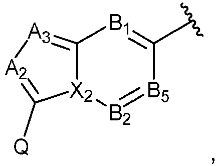 A₂ est **N,** et A₃, B₁, B₂, B₅, X₂ et Q sont tels que définis ci-dessus ;
ou un sel de celui-ci.

2. Composé selon la revendication 1, dans lequel
n est 0 ou 1,
J est choisi dans le groupe constitué par 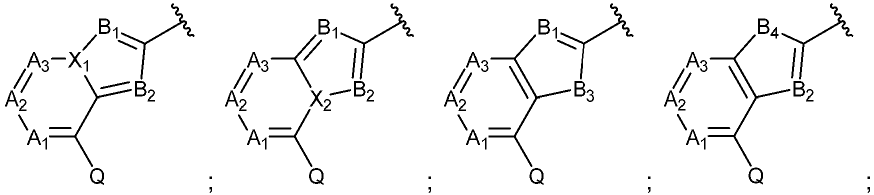 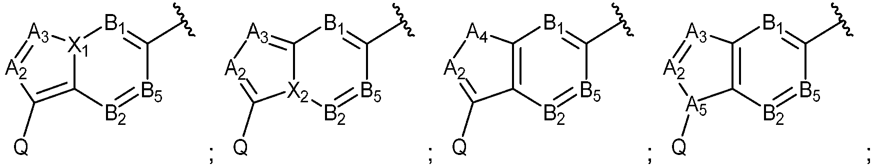 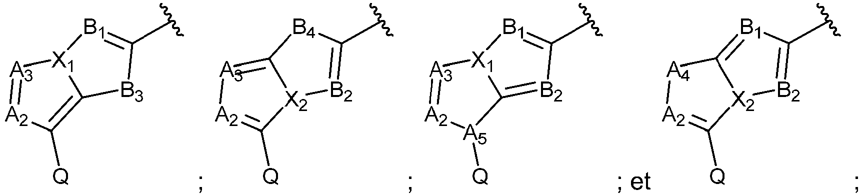
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₄ est choisi dans le groupe constitué par O, S et NR_{A4} ;
A₅ est choisi dans le groupe constitué par N ;
B₁ est choisi dans le groupe constitué par N et CR_{B1} ;
B₂ est choisi dans le groupe constitué par N et CR_{B2} ;
B₃ est choisi dans le groupe constitué par O, S et NR_{B3} ;
B₄ est choisi dans le groupe constitué par O, S et NR_{B4} ;
B₅ est choisi dans le groupe constitué par N et CR_{B5} ;
X₁ est choisi dans le groupe constitué par N ;
X₂ est choisi dans le groupe constitué par N ;
G est choisi dans le groupe constitué par 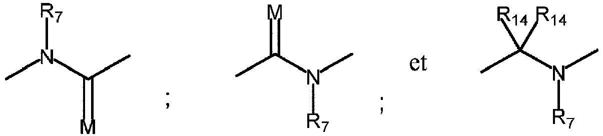
M est choisi dans le groupe constitué par N-R₁₃, O et S ;
Y₁ est choisi dans le groupe constitué par CR₈R₉, O, S et NR₁₀ ;
Y₂ est choisi dans le groupe constitué par CR₈R₉, O, S et NR₁₀ ;
dans lequel au moins l'un des groupes Y₁ ou Y₂ est CR₈R₉ ;
Z₁ est choisi dans le groupe constitué par N, O, S et CR₁₁ ;
Z₂ est choisi dans le groupe constitué par nul, N et CR₁₁ ;
Z₃ est choisi dans le groupe constitué par nul, N et CR₁₁ ;
Z₄ est choisi dans le groupe constitué par N, O, S et CR₁₁ ;
dans lequel pas plus de 2 parmi Z₁, Z₂, Z₃ et Z₄ sont N et dans lequel un seul parmi Z₁ et Z₄ est O ou S, Z₂ est nul uniquement lorsque Z₁ est O ou S, et Z₃ est nul uniquement lorsque Z₄ est O ou S ;
R_{A1}, R_{A2}, R_{A3}, R_{B2} et R_{B5} sont chacun indépendamment choisis dans le groupe constitué par hydrogène, halogène, hydroxyle, -SH, NH₂, alkyle en C₁ à C₄, halogénoalkyle en C₁ à C₄, alcoxy en C₁ à C₄ et -S-alkyle en C₁ à C₄, dans lequel chaque alkyle en C₁ à C₄ dans R_{A1}, R_{A2}, R_{A3}, R_{B2} et R_{B5} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{A4} et R_{B3} sont indépendamment choisis dans le groupe constitué par hydrogène, halogène, hydroxyle, -SH, NH₂, alkyle en C₁ à C₄, halogénoalkyle en C₁ à C₄, alcoxy en C₁ à C₄, et -S-alkyle en C₁ à C₄, dans lequel chaque alkyle en C₁ à C₄ dans R_{A4} et R_{B3} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), - C(O)N(alkyle en C₁ à C₄)₂ et alcoxy en C₁ à C₄ ;
R_{B1} est choisi dans le groupe constitué par
halogène, hydroxyle, -SH, NH₂, alkyle en C₁ à C₄, cycloalkyle en C₃ à C₆, halogénoalkyle en C₁ à C₄, alcoxy en C₁ à C₄, -S-alkyle en C₁ à C₄, et -NH(alkyle en C₁ à C₄), -N(alkyle en C₁ à C₄)₂, dans lequel chaque alkyle en C₁ à C₄ dans R_{B1} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ; et
un hétérocycloalkyle de 4 à 7 chaînons contenant 1, 2 ou 3 hétéroatomes indépendamment choisis parmi N ou O, dans lequel chaque hétérocycloalkyle dans R_{B1} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ;
R_{B4} est choisi dans le groupe constitué par halogène, hydroxyle, -SH, NH₂, alkyle en C₁ à C₄, cycloalkyle en C₃ à C₆, halogénoalkyle en C₁ à C₄, alcoxy en C₁ à C₄ et -S-alkyle en C₁ à C₄, dans lequel chaque alkyle en C₁ à C₄ dans R_{B4} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ;
R₇ est choisi dans le groupe constitué par hydrogène, alkyle en C₁ à C₄ et cycloalkyle en C₃ à C₆ facultativement substitué par 1 à 5 atomes d'halogène ;
R₈ est, chaque fois qu'il est choisi, indépendamment choisi dans le groupe constitué par hydrogène, fluoro, et alkyle en C₁ à C₄ ;
R₉ est, chaque fois qu'il est choisi, indépendamment choisi dans le groupe constitué par hydrogène, fluoro, et alkyle en C₁ à C₄ ;
R₁₀ est choisi dans le groupe constitué par hydrogène et alkyle en C₁ à C₄ ;
R₁₁ est choisi dans le groupe constitué par hydrogène, halogène, alkyle en C₁ à C₄, halogénoalkyle en C₁ à C₄, cycloalkyle en C₃ à C₆ ;
Q est un aryle de 6 ou 10 chaînons facultativement substitué par 1, 2 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄ ;
R₁₃ est choisi dans le groupe constitué par hydroxy, alcoxy en C₁ à C₄ et - NH₂ ;
R₁₄ est, chaque fois qu'il est choisi, indépendamment choisi dans le groupe constitué par hydrogène et halogène ; et
R₁₇ est, chaque fois qu'il est choisi, indépendamment choisi dans le groupe constitué par alkyle en C₁ à C₄, cycloalkyle en C₃ à C₆, halogénoalkyle en C₁ à C₄, alcoxy en C₁ à C₄, halogénoalcoxy en C₁ à C₄, -OH, -NH₂, -NH(alkyle en C₁ à C₄), -N(alkyle en C₁ à C₄)₂, -NH(cycloalkyle en C₃ à C₆) et -N(alkyle en C₁ à C₄)(cycloalkyle en C₃ à C₆) ;
dans lequel, lorsque J est 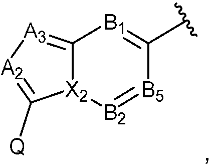 A₂ est N, et A₃, B₁, B₂, B₅, X₂ et Q sont tels que définis ci-dessus ;
ou un sel de celui-ci.

3. Composé selon la revendication 1 ou 2, dans lequel
n est 0 ou 1,
J est choisi dans le groupe constitué par 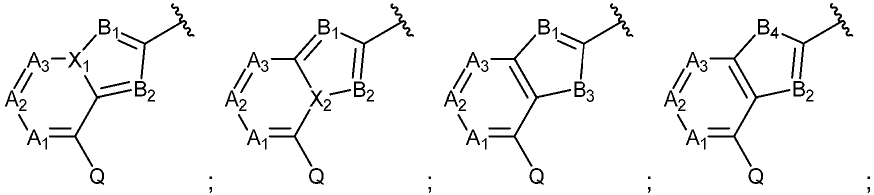 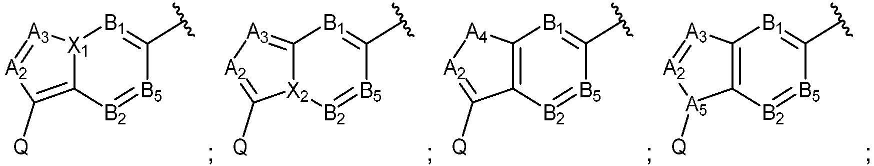 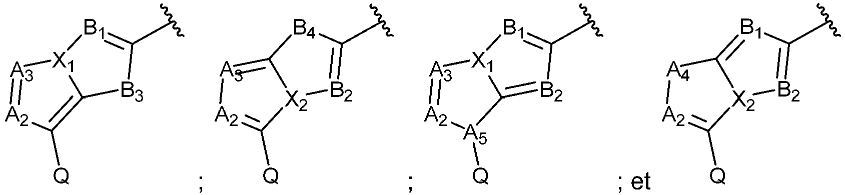
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₄ est choisi dans le groupe constitué par O, S et NR_{A4} ;
A₅ est choisi dans le groupe constitué par N ;
B₁ est choisi dans le groupe constitué par N et CR_{B1} ;
B₂ est choisi dans le groupe constitué par N et CR_{B2} ;
B₃ est choisi dans le groupe constitué par O, S et NR_{B3} ;
B₄ est choisi dans le groupe constitué par O, S et NR_{B4} ;
B₅ est choisi dans le groupe constitué par N et CR_{B5} ;
X₁ est choisi dans le groupe constitué par N ;
X₂ est choisi dans le groupe constitué par N ;
G est choisi dans le groupe constitué par
; 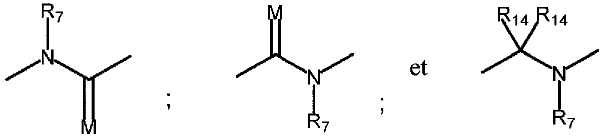
M est choisi dans le groupe constitué par N-R₁₃, O et S ;
Y₁ est choisi dans le groupe constitué par CR₈R₉, O, S et NR₁₀ ;
Y₂ est choisi dans le groupe constitué par CR₈R₉, O, S et NR₁₀ ;
dans lequel au moins l'un des groupes Y₁ ou Y₂ est CR₈R₉ ;
Z₁ est choisi dans le groupe constitué par N, O, S et CR₁₁ ;
Z₂ est choisi dans le groupe constitué par nul, N et CR₁₁ ;
Z₃ est choisi dans le groupe constitué par nul, N et CR₁₁ ;
Z₄ est choisi dans le groupe constitué par N, O, S et CR₁₁ ;
dans lequel pas plus de 2 parmi Z₁, Z₂, Z₃ et Z₄ sont N et dans lequel un seul parmi Z₁ et Z₄ est O ou S, Z₂ est nul uniquement lorsque Z₁ est O ou S, et Z₃ est nul uniquement lorsque Z₄ est O ou S ;
R_{A1}, R_{A2}, R_{A3}, R_{B2} et R_{B5} sont chacun indépendamment choisis dans le groupe constitué par hydrogène, halogène, et alkyle en C₁ à C₄, dans lequel chaque alkyle en C₁ à C₄ dans in R_{A1}, R_{A2}, R_{A3}, R_{B2} et R_{B5} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{A4} et R_{B3} sont indépendamment choisis dans le groupe constitué par hydrogène, alkyle en C₁ à C₄, dans lequel chaque alkyle en C₁ à C₄ dans R_{A4} et R_{B3} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ;
R_{B1} est choisi dans le groupe constitué par
alkyle en C₁ à C₄, -N(alkyle en C₁ à C₄)₂, dans lequel chaque alkyle en C₁ à C₄ dans R_{B1} peut être facultativement substitué par 1, 2 ou 3 substituants indépendamment choisis dans le groupe constitué par halogène, hydroxy, -NH₂, - NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ; et
un hétérocycloalkyle de 4 à 7 chaînons contenant 1, 2 ou 3 hétéroatomes indépendamment choisis parmi N ou O, dans lequel chaque hétérocycloalkyle dans R_{B1} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ;
R_{B4} est alkyle en C₁ à C₄, dans lequel chaque alkyle en C₁ à C₄ dans R_{B4} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ;
R₇ est hydrogène ;
R₈ est hydrogène ;
R₉ est hydrogène ;
R₁₀ est choisi dans le groupe constitué par hydrogène et alkyle en C₁ à C₄;
R₁₁ est hydrogène ;
Q est un aryle de 6 ou 10 chaînons facultativement substitué par 1, 2 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄ ;
R₁₃ est choisi dans le groupe constitué par hydroxy, alcoxy en C₁ à C₄ et - NH₂ ;
dans lequel, lorsque J est 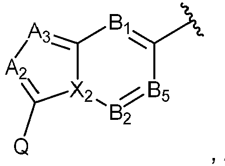 A₂ est N, et A₃, B₁, B₂, B₅, X₂ et Q sont tels que définis ci-dessus ;
ou un sel de celui-ci.

4. Composé selon l'une quelconque des revendications 1 à 3, dans lequel
n vaut 1,
J est choisi dans le groupe constitué par 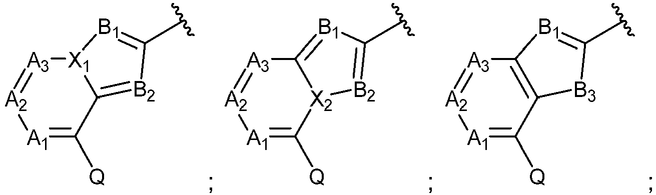 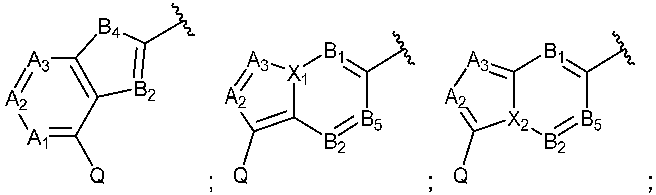 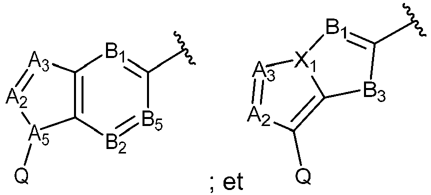
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₅ est choisi dans le groupe constitué par N ;
B₁ est CR_{B1} ;
B₂ est choisi dans le groupe constitué par N et CR_{B2} ;
B₃ est choisi dans le groupe constitué par O, S et NR_{B3} ;
B₄ est NR_{B4} ;
B₅ est choisi dans le groupe constitué par N et CR_{B5} ;
X₁ est choisi dans le groupe constitué par N ;
X₂ est choisi dans le groupe constitué par N ;
G est choisi dans le groupe constitué par 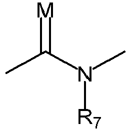
M est O ;
Y₁ est CR₈R₉ ;
Y₂ est O ;
Z₁ est CR₁₁ ;
Z₂ est CR₁₁ ;
Z₃ est CR₁₁ ;
Z₄ est CR₁₁ ;
R_{A1}, R_{A2}, R_{A3}, R_{B2} et R_{B5} sont chacun indépendamment choisis dans le groupe constitué par hydrogène, halogène, et alkyle en C₁ à C₄, dans lequel chaque alkyle en C₁ à C₄ dans R_{A1}, R_{A2}, R_{A3}, R_{B2} et R_{B5} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{B3} est choisi dans le groupe constitué par hydrogène et alkyle en C₁ à C₄, dans lequel chaque alkyle en C₁ à C₄ dans R_{B3} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{B1} est choisi dans le groupe constitué par
alkyle en C₁ à C₄, -N(alkyle en C₁ à C₄)₂, dans lequel chaque alkyle en C₁ à C₄ dans R_{B1} peut être facultativement substitué par 1, 2 ou 3 substituants indépendamment choisis dans le groupe constitué par halogène, hydroxy, -NH₂, - NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ; et
un hétérocycloalkyle de 4 à 7 chaînons contenant 1, 2 ou 3 hétéroatomes indépendamment choisis parmi N ou O, dans lequel chaque hétérocycloalkyle dans R_{B1} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ;
R_{B4} est alkyle en C₁ à C₄, dans lequel chaque alkyle en C₁ à C₄ dans R_{B4} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ;
R₇ est hydrogène ;
R₈ est hydrogène ;
R₉ est hydrogène ;
R₁₁ est hydrogène ;
Q est un aryle de 6 ou 10 chaînons facultativement substitué par 1, 2, 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₁ à C₄)₂, -NH(cycloalkyle en C₃ à C₆), -N(cycloalkyle en C₁ à C₄)(cycloalkyle en C₃ à C₆), -NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄ ;
dans lequel, lorsque J est 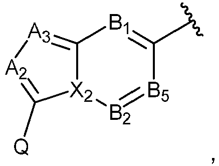 A₂ **est N, et** A₃, B₁, B₂, B₅, X₂ et Q sont tels que définis ci-dessus ;
ou un sel de celui-ci.

5. Composé selon l'une quelconque des revendications 1 à 4, dans lequel
n vaut 1,
J est choisi dans le groupe constitué par 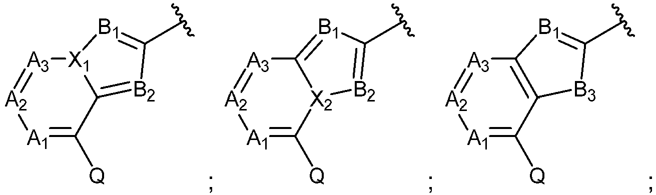 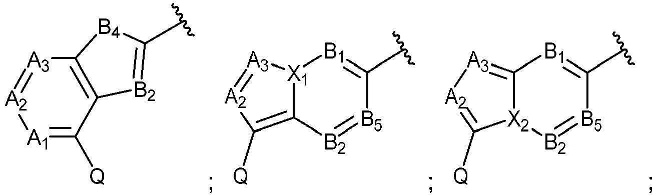 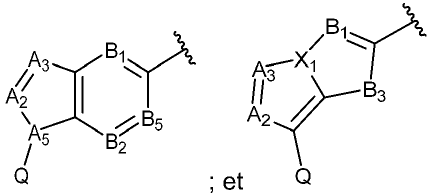
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₅ est N ;
B₁ est CR_{B1} ;
B₂ est choisi dans le groupe constitué par N et CR_{B2} ;
B₃ est choisi dans le groupe constitué par O, S et NR_{B3} ;
B₄ est NR_{B4} ;
B₅ est choisi dans le groupe constitué par N et CR_{B5} ;
X₁ est N ;
X₂ est N ;
G est 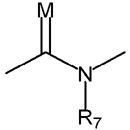
M est O ;
Y₁ est CR₈R₉ ;
Y₂ est O ;
Z₁ est CR₁₁ ;
Z₂ est CR₁₁ ;
Z₃ est CR₁₁ ;
Z₄ est CR₁₁ ;
R_{A1}, R_{A2}, R_{A3}, R_{B2} et R_{B5} sont chacun indépendamment choisis dans le groupe constitué par hydrogène, halogène, et alkyle en C₁ à C₄, dans lequel chaque alkyle en C₁ à C₄ dans in R_{A1}, R_{A2}, R_{A3}, R_{B2} et R_{B5} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{B3} est choisi dans le groupe constitué par hydrogène et alkyle en C₁ à C₄, dans lequel chaque alkyle en C₁ à C₄ dans R_{B3} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, - C(O)NH(alkyle en C₁ à C₄), -C(O)N(alkyle en C₁ à C₄)₂ et alcoxy en C₁ à C₄ ;
R_{B1} est choisi dans le groupe constitué par
alkyle en C₁ à C₄, -N(alkyle en C₁ à C₄)₂, dans lequel chaque alkyle en C₁ à C₄ dans R_{B1} peut être facultativement substitué par 1, 2 ou 3 substituants indépendamment choisis dans le groupe constitué par halogène, hydroxy, -NH₂, - NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄ ; et
un hétérocycloalkyle de 4 à 7 chaînons contenant 1, 2 ou 3 hétéroatomes indépendamment choisis parmi N ou O, dans lequel chaque hétérocycloalkyle dans R_{B1} est facultativement substitué par 1, 2 ou 3 substituants indépendamment choisis dans le groupe constitué par halogène, cyano, nitro, hydroxy, oxo, alkyle en C₁ à C₄, cycloalkyle en C₃ à C₆, halogénoalkyle en C₁ à C₄, alcoxy en C₁ à C₄, - NH₂, -NH(alkyle en C₁ à C₄), -N(alkyle en C₁ à C₄)₂, -NH(cycloalkyle en C₃ à C₆), - N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄ ;
R_{B4} est alkyle en C₁ à C₄, dans lequel chaque alkyle en C₁ à C₄ dans R_{B4} peut être facultativement substitué par 1, 2 ou 3 substituants indépendamment choisis dans le groupe constitué par halogène, hydroxy, -NH₂, -NH(alkyle en C₁ à C₄), -N(alkyle en C₁ à C₄)₂, cyano, carboxy, carbamoyle, alcoxycarbonyle en C₁ à C₄, -C(O)NH(alkyle en C₁ à C₄), -C(O)N(alkyle en C₁ à C₄)₂ et alcoxy en C₁ à C₄;
R₇ est hydrogène ;
R₈ est hydrogène ;
R₉ est hydrogène ;
R₁₁ est hydrogène ;
Q est un phényle facultativement substitué par 1, 2, 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -NH₂, -NH(alkyle en C₁ à C₄), -N(alkyle en C₄ à C₄)₂, - NH(cycloalkyle en C₃ à C₆), -N(alkyle en C₁ à C₄)(cycloalkyle en C₃ à C₆), - NHSO₂(alkyle en C₁ à C₄), -S-alkyle en C₁ à C₄, -S(O)-alkyle en C₁ à C₄, -SO₂-alkyle en C₁ à C₄, -S(O)-halogénoalkyle en C₁ à C₄ et -SO₂-halogénoalkyle en C₁ à C₄ ;
dans lequel, lorsque J est 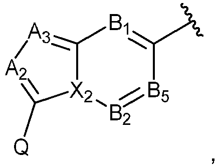 A₂ **est N, et** A₃, B₁, B₂, B₅, X₂ et Q sont tels que définis ci-dessus ;
ou un sel de celui-ci.

6. Composé selon l'une quelconque des revendications 1 à 5, dans lequel
n vaut 1,
J est choisi dans le groupe constitué par 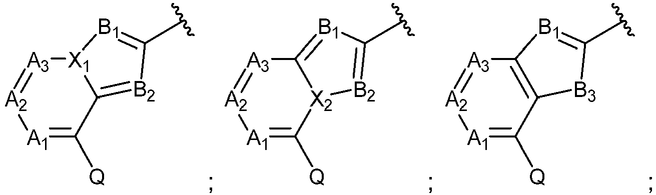 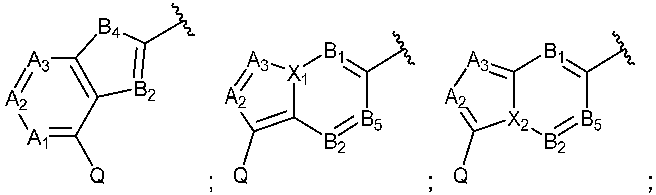 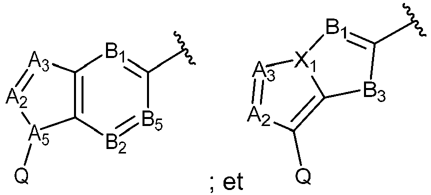
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₅ est N ;
B₁ est CR_{B1} ;
B₂ est choisi dans le groupe constitué par N et CR_{B2} ;
B₃ est choisi dans le groupe constitué par O, S et NR_{B3} ;
B₄ est NR_{B4} ;
B₅ est choisi dans le groupe constitué par N et CR_{B5} ;
X₁ est N ;
X₂ est N ;
G est 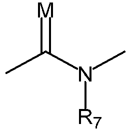
M est O ;
Y₁ est CR₈R₉ ;
Y₂ est O ;
Z₁ est CR₁₁ ;
Z₂ est CR₁₁ ;
Z₃ est CR₁₁ ;
Z₄ est CR₁₁ ;
R_{A1}, R_{A2}, R_{A3}, R_{B2} et R_{B5} sont chacun indépendamment choisis dans le groupe constitué par hydrogène, halogène, alkyle en C₁ à C₄ ;
R_{B3} est choisi dans le groupe constitué par hydrogène et alkyle en C₁ à C₄ ;
R_{B1} est choisi dans le groupe constitué par
alkyle en C₁ à C₄, -N(alkyle en C₁ à C₄)₂, dans lequel chaque alkyle en C₁ à C₄ dans R_{B1} peut être facultativement substitué par 1, 2 ou 3 substituants d'hydroxy ; et
un hétérocycloalkyle de 4 à 7 chaînons contenant 1, 2 ou 3 hétéroatomes indépendamment choisis parmi N ou O, dans lequel chaque hétérocycloalkyle dans R_{B1} est facultativement substitué par 1, 2 ou 3 substituants d'halogène ;
R_{B4} est alkyle en C₁ à C₄ ;
R₇ est hydrogène ;
R₈ est hydrogène ;
R₉ est hydrogène ;
R₁₁ est hydrogène ;
Q est phényle ayant 1 à 5 atomes d'halogène ;
dans lequel, lorsque J est 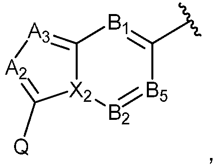 A₂ est N, et A₃, B₁, B₂, B₅, X₂ et Q sont tels que définis ci-dessus ;
ou un sel de celui-ci.

7. Composé selon l'une quelconque des revendications 1 à 6, dans lequel
n vaut 1,
J est choisi dans le groupe constitué par 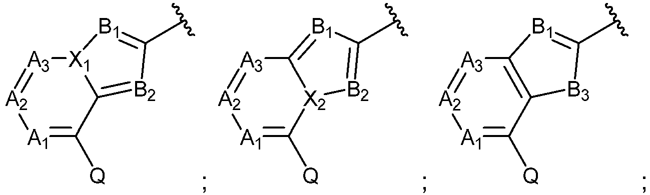 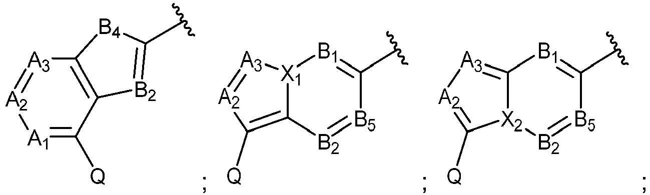 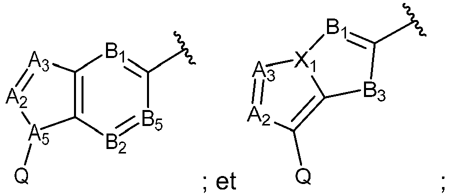
A₁ est choisi dans le groupe constitué par N et CR_{A1} ;
A₂ est choisi dans le groupe constitué par N et CR_{A2} ;
A₃ est choisi dans le groupe constitué par N et CR_{A3} ;
A₅ est N ;
B₁ est CR_{B1} ;
B₂ est choisi dans le groupe constitué par N et CR_{B2} ;
B₃ est choisi dans le groupe constitué par O, S et NR_{B3} ;
B₄ est NR_{B4} ;
B₅ est choisi dans le groupe constitué par N et CR_{B5} ;
X₁ est choisi dans le groupe constitué par N ;
X₂ est choisi dans le groupe constitué par N ;
G est choisi dans le groupe constitué par 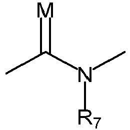
M est O ;
Y₁ est CR₈R₉ ;
Y₂ est O ;
Z₁ est CR₁₁ ;
Z₂ est CR₁₁ ;
Z₃ est CR₁₁ ;
Z₄ est CR₁₁ ;
R_{A1}, R_{A2}, R_{A3}, R_{B2} et R_{B5} sont chacun indépendamment choisis dans le groupe constitué par hydrogène, fluoro et méthyle ;
R_{B3} sont indépendamment choisis dans le groupe constitué par hydrogène et méthyle ;
R_{B1} est indépendamment choisi dans le groupe constitué de 4-morpholino, isopropyle, 2-hydroxyisopropyle, 3-fluoroazétidinyle et NMe₂ ;
R_{B4} est isopropyle ;
R₇ est hydrogène ;
R₈ est hydrogène ;
R₉ est hydrogène ;
R₁₁ est hydrogène ;
Q est choisi dans le groupe constitué par 2,3,5-trifluorophényle et 2,6-difluorophényle ;
dans lequel, lorsque J est 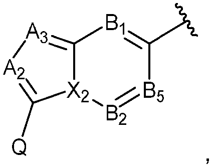 A₂ est N, et A₃, B₁, B₂, B₅, X₂ et Q sont tels que définis ci-dessus ;
ou un sel de celui-ci.

8. Composé selon l'une quelconque des revendications 1 à 7, dans lequel
J est choisi dans le groupe constitué par 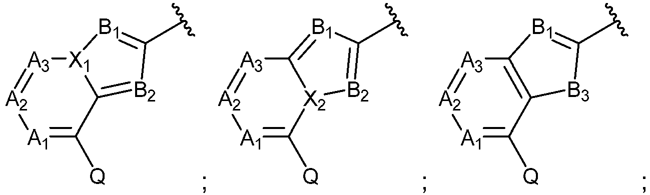 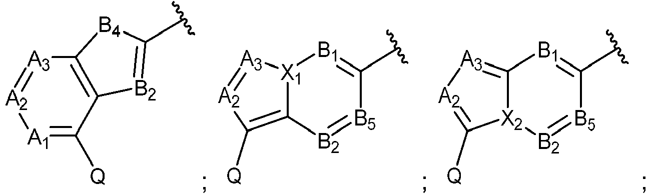 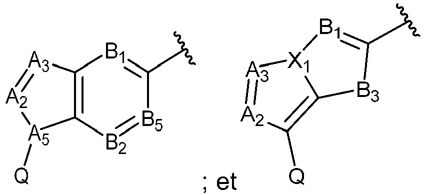
dans lequel, lorsque J est 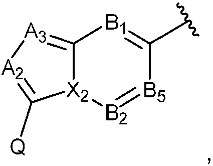 A₂ est N, et A₃, B₁, B₂, B₅, X₂ et Q sont tels que définis dans l'une quelconque des revendications 1 à 7 ;
ou un sel de celui-ci.

9. Composé selon l'une quelconque des revendications 1 à 8, dans lequel
G est 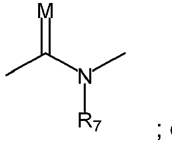 et
M est O ;
ou un sel de celui-ci, plus préférablement
M est O ; et
R₇ est hydrogène.

10. Composé selon l'une quelconque des revendications 1 à 9, dans lequel
Q est un aryle de 6 ou 10 chaînons facultativement substitué par 1, 2 ou 3 substituants indépendamment choisis dans le groupe constitué par halogène, cyano, nitro, hydroxy, alkyle en C₁ à C₄, halogénoalkyle en C₁ à C₄, alcoxy en C₁ à C₄, cycloalkyle en C₃ à C₆, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄;
ou un sel de celui-ci.

11. Composé selon l'une quelconque des revendications 1 à 10, dans lequel
Q est un aryle de 6 chaînons facultativement substitué par 1, 2, 3, 4 ou 5 substituants indépendamment choisis dans le groupe constitué par halogène, cyano, nitro, hydroxyle, alkyle en C₁ à C₄, halogénoalkyle en C₁ à C₄, alcoxy en C₁ à C₄, cycloalkyle en C₃ à C₆, -C(O)R₁₇, -NH₂, -NH(alkyle en C₁ à C₄), -N(alkyle en C₁ à C₄)₂, -NH(cycloalkyle en C₃ à C₆), -N(alkyle en C₁ à C₄)(cycloalkyle en C₃ à C₆), -NHSO₂(alkyle en C₁ à C₄), -S-alkyle en C₁ à C₄, -S(O)alkyle en C₁ à C₄, -SO₂alkyle en C₁ à C₄, -S(O)halogénoalkyle en C₁ à C₄ et -SO₂halogénoalkyle en C₁ à C₄, dans lequel l'aryle de 6 ou 10 chaînons est facultativement condensé avec un hétérocycloalkyle de 4 à 7 chaînons ayant 1 ou 2 hétéroatomes choisis dans le groupe O, S et N et dans lequel les carbones de l'hétérocycloalkyle sont facultativement substitués par 1, 2 ou 3 substituants indépendamment choisis dans le groupe halogène, cyano, nitro, hydroxyle, oxo, alkyle en C₁ à C₄, cycloalkyle en C₃ à C₆, halogénoalkyle en C₁ à C₄, alcoxy en C₁ à C₄, -NH₂, - NH(alkyle en C₁ à C₄), et -N(alkyle en C₁ à C₄)₂ et l'un quelconque N dans l'hétérocycloalkyle est, si la valence le permet, substitué par un substituant choisi dans le groupe constitué par hydrogène, alkyle en C₁ à C₄ et cycloalkyle en C₃ à C₆.

12. Composé selon l'une quelconque des revendications 1 à 11, ou sel de celui-ci, dans lequel Q est choisi parmi : 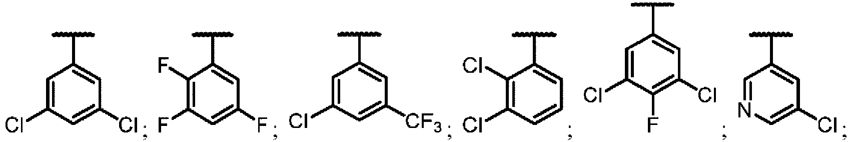 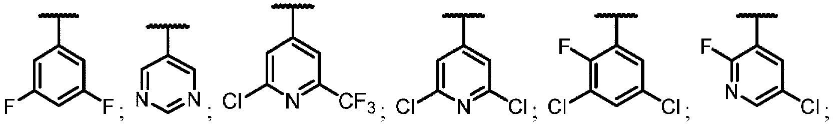 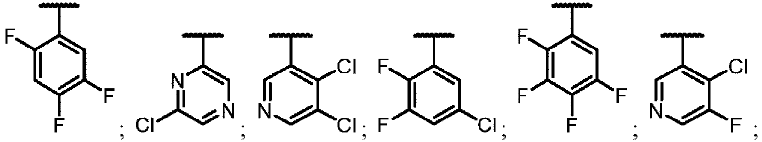 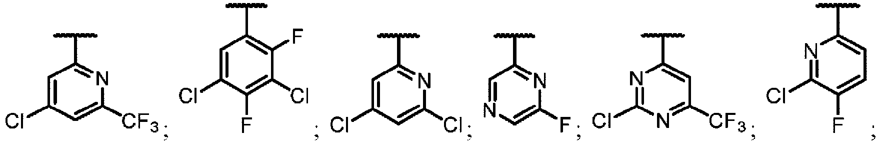 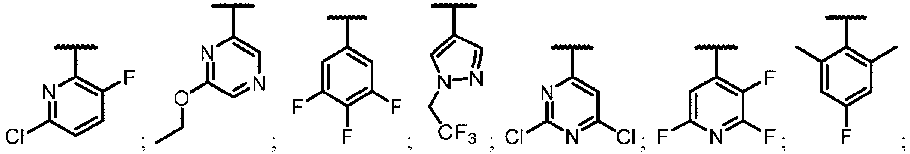 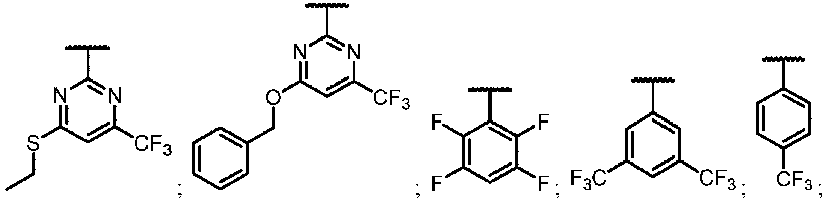 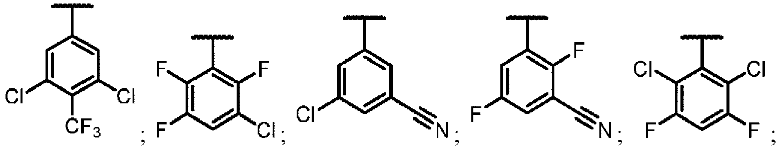 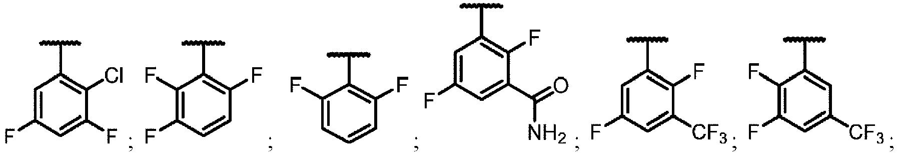 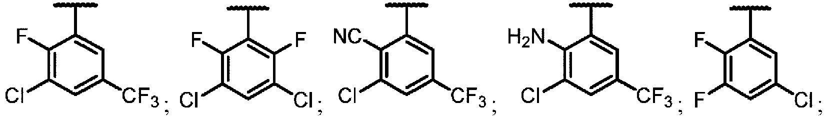 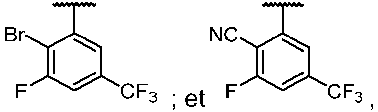 plus préférablement Q est

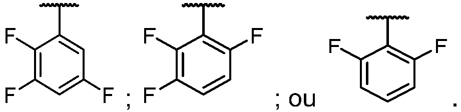

13. Composé selon l'une quelconque des revendications 1 à 12, dans lequel
n vaut 1, Y₁ est CR₈R₉, Y₂ est O, Z₁ est CR₁₁, Z₂ est CR₁₁, Z₃ est CR₁₁, Z₄ est CR₁₁,
ou un sel de celui-ci.

14. Composé selon l'une quelconque des revendications 1 à 13, dans lequel R_{B1} ou R_{B4}, lorsqu'il est présent, est choisi dans le groupe constitué par alkyle en C₁ à C₄, alkyle en C₁ à C₄ à substitution hydroxy, cycloalkyle en C₃ à C₆, -N(alkyle en C₁ à C₄)₂ et hétérocycloalkyle de 4 à 7 chaînons ; ou un sel de celui-ci.

15. Composé selon l'une quelconque des revendications 1 à 14, ou sel de celui-ci, dans lequel R_{B1} ou R_{B4}, lorsqu'il est présent, est choisi parmi :

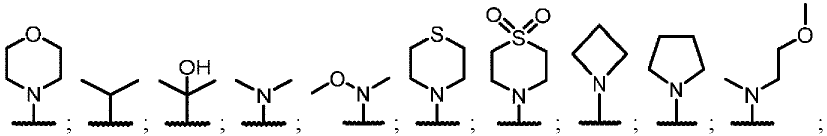

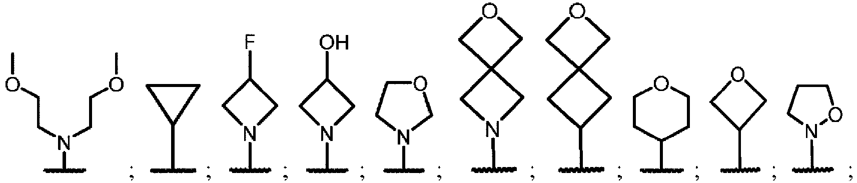

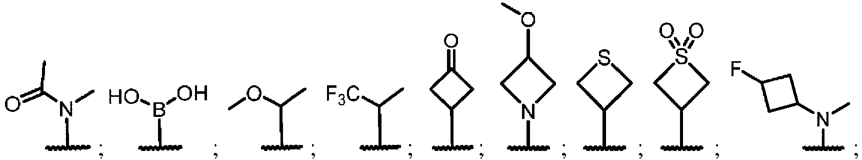

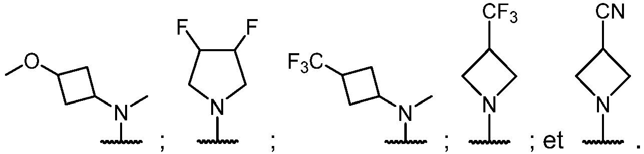

16. Composé selon l'une quelconque des revendications 1 à 15, dans lequel le composé est choisi dans le groupe constitué par : 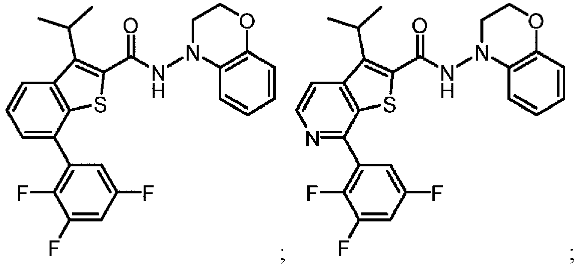 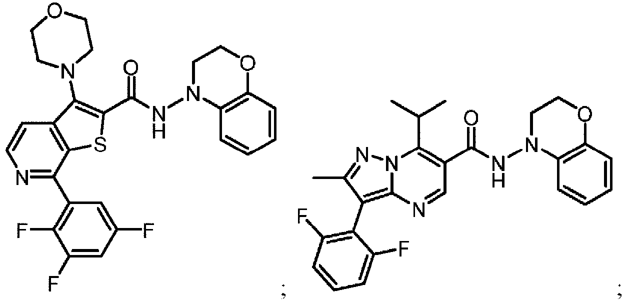 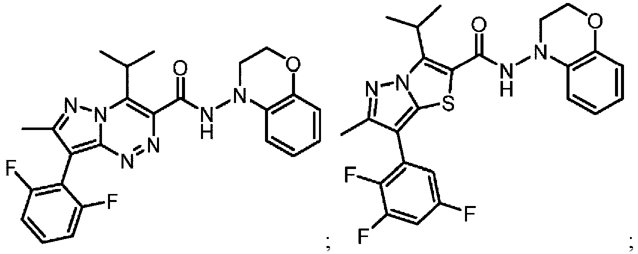 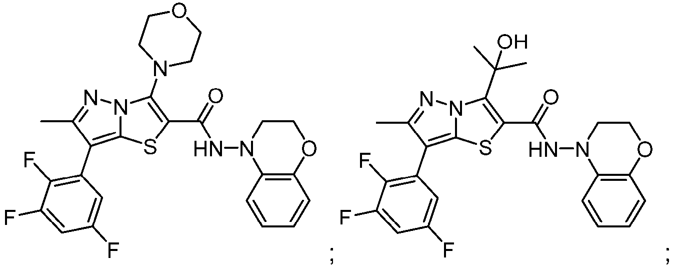 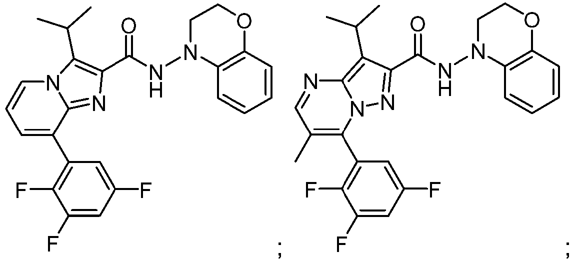 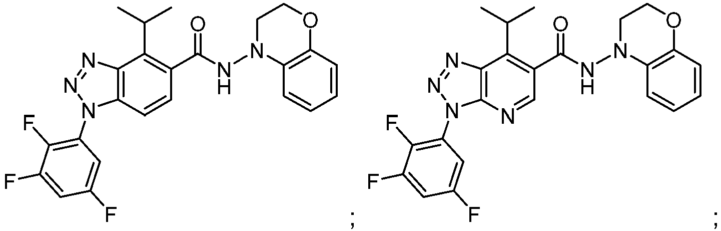 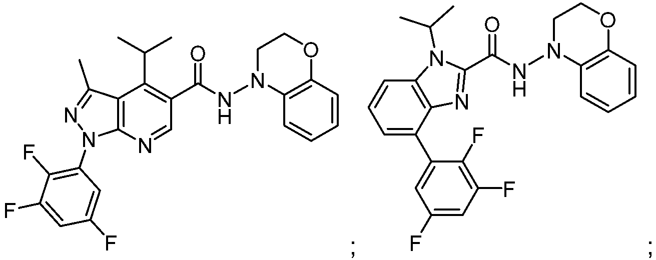 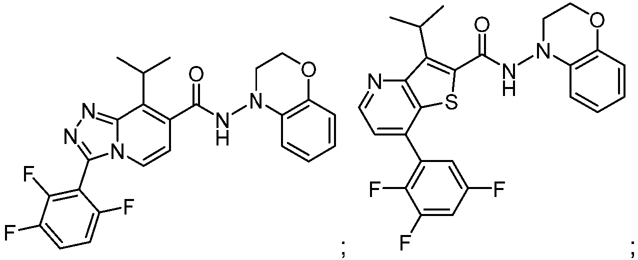 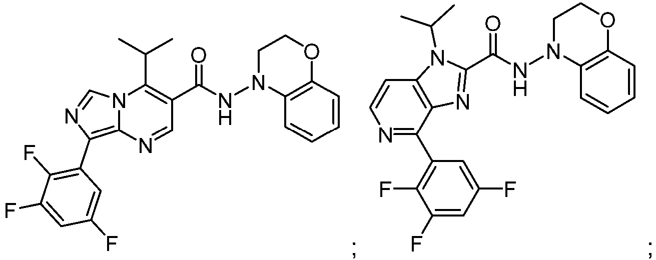 et 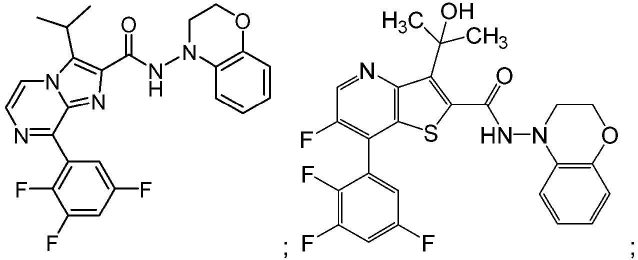 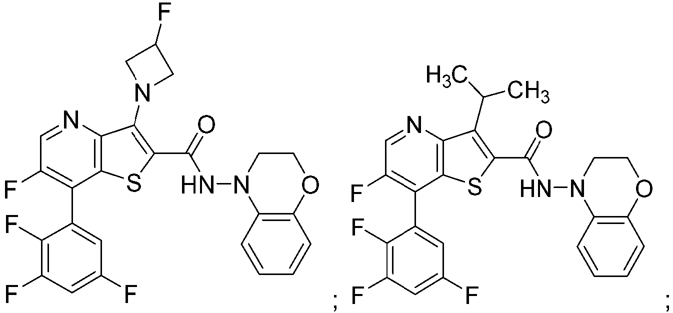 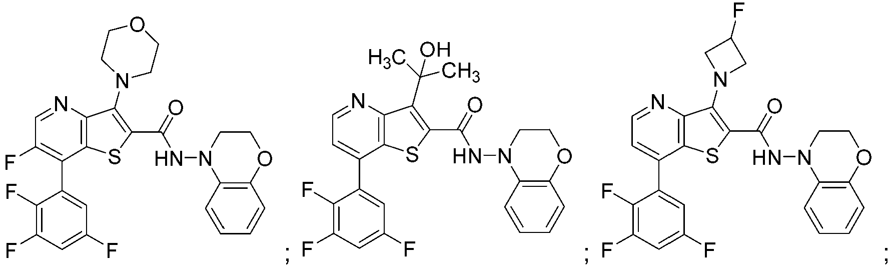 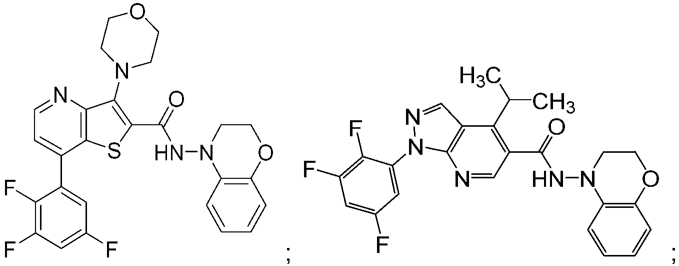 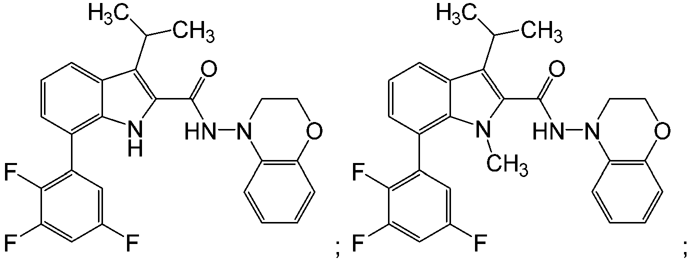 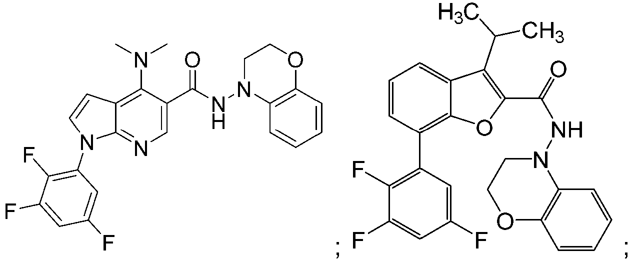 ou un sel de l'un quelconque des composés qui précèdent.

17. Composition pharmaceutique comprenant un composé de formule (I) selon l'une quelconque des revendications 1 à 16, ou un sel de celui-ci, et au moins un véhicule acceptable.

18. Composé de formule (I) selon l'une quelconque des revendications 1 à 16 ou composition pharmaceutique selon la revendication 17 pour utilisation dans le contrôle, le traitement et/ou la prévention d'une maladie, dans lequel la maladie est de préférence une infection causée par des endoparasites, plus préférablement une infection helminthique, même plus préférablement une dirofilariose cardiopulmonaire.
